(19) Europäisches Patentamt
European Patent Office
Office européen des brevets

(11) EP 4 435 104 A1

(12) EUROPEAN PATENT APPLICATION
published in accordance with Art. 153(4) EPC

(43) Date of publication:
**25.09.2024 Bulletin 2024/39**

(21) Application number: **22894956.6**

(22) Date of filing: **18.11.2022**

(51) International Patent Classification (IPC):
***C12N 15/113*** *(2010.01)* ***A61K 31/713*** *(2006.01)*
***A61P 9/12*** *(2006.01)* ***A61P 9/10*** *(2006.01)*
***A61P 13/12*** *(2006.01)*

(52) Cooperative Patent Classification (CPC):
**A61K 31/713; A61P 9/10; A61P 9/12; A61P 13/12; C12N 15/113**

(86) International application number:
**PCT/CN2022/132883**

(87) International publication number:
**WO 2023/088427 (25.05.2023 Gazette 2023/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.11.2021 CN 202111398206**
**16.12.2021 CN 202111542323**
**20.05.2022 CN 202210554195**

(71) Applicant: **Tuojie Biotech (Shanghai) Co., Ltd.**
**Shanghai 201203 (CN)**

(72) Inventors:
- **LIN, Xiaoyan**
  **Shanghai 201203 (CN)**
- **LI, Yunfei**
  **Shanghai 201203 (CN)**
- **ZHANG, Zhen**
  **Shanghai 201203 (CN)**
- **DONG, Yuqiong**
  **Shanghai 201203 (CN)**
- **HOU, Zhe**
  **Shanghai 201203 (CN)**
- **ZHANG, Jianyu**
  **Shanghai 201203 (CN)**
- **GENG, Jun**
  **Shanghai 201203 (CN)**
- **MAO, Song**
  **Shanghai 201203 (CN)**
- **HUANG, Longfei**
  **Shanghai 201203 (CN)**
- **ZHOU, Yaqin**
  **Shanghai 201203 (CN)**
- **LV, Zhenzhen**
  **Shanghai 201203 (CN)**
- **HUANG, Yanfen**
  **Shanghai 201203 (CN)**
- **HUANG, Jinyu**
  **Shanghai 201203 (CN)**

(74) Representative: **Regimbeau**
**20, rue de Chazelles**
**75847 Paris Cedex 17 (FR)**

# (54) SIRNA TARGETING ANGIOTENSINOGEN AND PHARMACEUTICAL USE OF SIRNA

(57) An siRNA targeting angiotensinogen (AGT) and a pharmaceutical use of the siRNA. Specifically, the present invention relates to the siRNA targeting an AGT gene, an siRNA conjugate or a dsRNA, a composition, and a pharmaceutical use of the siRNA. The present invention also relates to a pharmaceutical composition, a cell or a kit comprising the siRNA, and a method for the siRNA to treat and/or prevent AGT-related disorders (such as hypertension).

EP 4 435 104 A1

## Description

The present disclosure claims the priority to the Chinese Patent Application CN202111398206.0 filed on Nov. 19, 2021, the Chinese Patent Application CN202111542323.X filed on Dec. 16, 2021, and the Chinese Patent Application CN202210554195.9 filed on May 20, 2022, which are incorporated herein by reference in their entirety.

## TECHNICAL FIELD

The present disclosure relates to an siRNA targeting angiotensinogen (AGT), a double-stranded ribonucleic acid (dsRNA), a conjugate, and use thereof.

## BACKGROUND

AGT is also known as SERPINA8 or ANHU. AGT, encoded by an AGT gene and mainly produced by the liver, is a rate-limiting substrate of the renin-angiotensin system (RAS), and is also a precursor to an angiotensin peptide. Under the action of active renin in the plasma, AGT is cleaved into angiotensin I, which is subsequently converted to angiotensin II by the action of an angiotensin-converting enzyme (ACE) expressed cyclically and locally. Angiotensin II, the most important bioactive substance in the renin-angiotensin-aldosterone system (RAAS), causes arterial vasoconstriction and secretion of adrenocortical aldosterone by binding to the angiotensin II type 1 receptor (AT1R), and thus plays a key role in the regulation of blood pressure in the body. When angiotensin II is overproduced or AT1R is overstimulated, the RAAS system is dysfunctional, leading to hypertension. Numerous studies have indicated that the polymorphism on the AGT gene is closely related to the blood pressure regulation and control capability of the body, and association studies in the Chinese Han population have shown that the polymorphism of AGT M235T site is related to the occurrence of essential hypertension (ER).

Hypertension is a common cardiovascular disease worldwide, has a global prevalence of up to 10% to 20%, and can cause cerebrovascular, cardiac and renal complications, which is a major risk factor for various diseases, disorders and conditions (e.g., stroke, coronary heart disease, chronic kidney disease, myocardial infarction, heart failure, aneurysm, peripheral arterial disease, heart damage, and other cardiovascular-related diseases). At present, there are five main classes of drugs for treating hypertension, including diuretics, β-blockers, calcium channel blockers, angiotensin-converting enzyme inhibitors, and angiotensin II receptor blockers. Despite the wide variety of antihypertensive drugs available for the treatment of hypertension, more than two thirds of subjects cannot be controlled with one antihypertensive drug and need two or more different classes of antihypertensive drugs. Since compliance and side effects increase with increasing medication, this further reduces the number of subjects with controlled blood pressure. RNA interference (RNAi) is an effective way to silence gene expression, which can specifically degrade mRNA of a target gene through a post-transcriptional regulatory mechanism. Statistically, more than 80% of the disease-related proteins are non-druggable proteins as they cannot be targeted by conventional small-molecule drugs and biomacromolecule formulations. AGT is the most upstream precursor in the RAAS, and the cascade effect thereof in the regulation of blood pressure has been proved. The inhibition of AGT synthesis in the liver may lead to the persistent reduction of the AGT protein, and finally to the persistent reduction of angiotensin AngII. This inhibitory effect will have a good antihypertensive effect.

## SUMMARY

The present disclosure provides an siRNA targeting angiotensinogen (AGT).

In some embodiments, the present disclosure provides an siRNA, which comprises a sense strand and an antisense strand forming a double-stranded region;

the sense strand comprises a sequence differing by no more than 3 (e.g., 0, 1, 2 or 3) nucleotides from any one of the nucleotide sequences of SEQ ID NO: 1 to SEQ ID NO: 6 and comprises at least 15 (e.g., 16, 17, 18, 19, 20 or 21) contiguous nucleotides;

the antisense strand comprises a sequence differing by no more than 3 (e.g., 0, 1, 2 or 3) nucleotides from any one of the nucleotide sequences of SEQ ID NO: 7 to SEQ ID NO: 12 and comprises at least 15 (e.g., 16, 17, 18, 19, 20 or 21) contiguous nucleotides.

In some embodiments, the antisense strand is at least partially reverse complementary to a target sequence to mediate RNA interference. In some embodiments, there are no more than 5, no more than 4, no more than 3, no more than 2 or no more than 1 mismatch between the antisense strand and the target sequence. In some embodiments, the antisense strand is fully reverse complementary to the target sequence.

In some embodiments, the sense strand is at least partially reverse complementary to the antisense strand to

form a double-stranded region. In some embodiments, there are no more than 5, no more than 4, no more than 3, no more than 2 or no more than 1 mismatch between the sense strand and the antisense strand. In some embodiments, the sense strand is fully reverse complementary to the antisense strand.

In some embodiments, the siRNA of the present disclosure comprises one or two blunt ends.

In some specific embodiments, each strand of the siRNA independently comprises 1 to 2 unpaired nucleotides.

In some embodiments, the siRNA of the present disclosure comprises an overhang at the 3' end of the antisense strand of the siRNA.

In some embodiments, the sense strand and the antisense strand each independently have 16 to 35, 16 to 34, 17 to 34, 17 to 33, 18 to 33, 18 to 32, 18 to 31, 18 to 30, 18 to 29, 18 to 28, 18 to 27, 18 to 26, 18 to 25, 18 to 24, 18 to 23, 19 to 25, 19 to 24 or 19 to 23 nucleotides.

In some embodiments, the sense strand and the antisense strand are identical or different in length; the sense strand is 19-23 nucleotides in length, and the antisense strand is 19-26 nucleotides in length. A ratio of the length of the sense strand to the length of the antisense strand of the siRNA provided by the present disclosure can be 19/19, 19/20, 19/21, 19/22, 19/23, 19/24, 19/25, 19/26, 20/19, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25 or 23/26. In some embodiments, a ratio of the length of the sense strand to the length of the antisense strand of the siRNA is 19/21, 21/23 or 23/25. In some embodiments, a ratio of the length of the sense strand to the length of the antisense strand of the siRNA is 19/21.

In some embodiments, the sense strand comprises at least 15 contiguous nucleotides and differs by no more than 2 nucleotides from any one of the nucleotide sequences of SEQ ID NO: 1 to SEQ ID NO: 6. In some embodiments, the nucleotide sequence differs by no more than 1 nucleotide; in some embodiments, the difference is 1 nucleotide.

In some embodiments, the antisense strand comprises at least 15 contiguous nucleotides and differs by no more than 2 nucleotides from any one of the nucleotide sequences of SEQ ID NO: 7 to SEQ ID NO: 12. In some embodiments, the nucleotide sequence differs by no more than 1 nucleotide; in some embodiments, the difference is 1 nucleotide.

In some embodiments, the sense strand comprises at least 15 contiguous nucleotides of any one of the nucleotide sequences of SEQ ID NO: 1 to SEQ ID NO: 6. In some embodiments, the sense strand comprises at least 16 contiguous nucleotides of any one of the nucleotide sequences of SEQ ID NO: 1 to SEQ ID NO: 6. In some embodiments, the sense strand comprises at least 18 contiguous nucleotides of any one of the nucleotide sequences of SEQ ID NO: 1 to SEQ ID NO: 6. In some embodiments, the sense strand comprises at least 19 contiguous nucleotides of any one of the nucleotide sequences of SEQ ID NO: 1 to SEQ ID NO: 6.

In some embodiments, the antisense strand comprises at least 15 contiguous nucleotides of any one of the nucleotide sequences of SEQ ID NO: 7 to SEQ ID NO: 12. In some embodiments, the antisense strand comprises at least 18 contiguous nucleotides of any one of the nucleotide sequences of SEQ ID NO: 7 to SEQ ID NO: 12. In some embodiments, the antisense strand comprises at least 19 contiguous nucleotides of any one of the nucleotide sequences of SEQ ID NO: 7 to SEQ ID NO: 12. In some embodiments, the antisense strand comprises at least 20 contiguous nucleotides of any one of the nucleotide sequences of SEQ ID NO: 7 to SEQ ID NO: 12. In some embodiments, the antisense strand comprises at least 21 contiguous nucleotides of any one of the nucleotide sequences of SEQ ID NO: 7 to SEQ ID NO: 12.

In some embodiments, the sense strand comprises a nucleotide sequence selected from the group consisting of the following: SEQ ID NO: 1 to SEQ ID NO: 6.

In some embodiments, the antisense strand comprises a nucleotide sequence selected from the group consisting of the following: SEQ ID NO: 7 to SEQ ID NO: 12.

In some embodiments, at least one nucleotide comprised in each of the sense strand and the antisense strand of the present disclosure is a modified nucleotide. In some embodiments, all of the nucleotides of the sense strand and all of the nucleotides of the antisense strand of the present disclosure are modified nucleotides.

The present disclosure provides an siRNA, which comprises a sense strand and an antisense strand forming a double-stranded region;

> the sense strand comprises a sequence differing by no more than 3 nucleotides from any one of the nucleotide sequences of SEQ ID NO: 1 to SEQ ID NO: 6 and comprises at least 15 contiguous nucleotides; the antisense strand comprises a sequence differing by no more than 3 nucleotides from any one of the nucleotide sequences of SEQ ID NO: 7 to SEQ ID NO: 12 and comprises at least 15 contiguous nucleotides; at least one nucleotide at positions 2 to 8 (e.g., position 2, position 3, position 4, position 5, position 6, position 7 or position 8) of the 5' region of the antisense strand comprises a 2'-methoxy modification or a chemical modification of formula (I) or a tautomeric modification thereof;
>
> formula (I) is selected from:

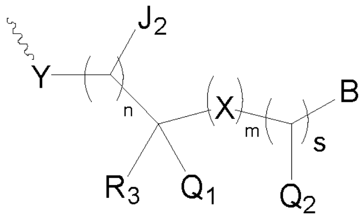

;

(I)

wherein Y is selected from the group consisting of O, NH and S;

each X is independently selected from the group consisting of $CR_4(R_4')$, S, $NR_5$ and NH-CO, wherein $R_4$, $R_4'$ and $R_5$ are each independently H or $C_1$-$C_6$ alkyl;

$J_2$ is H or $C_1$-$C_6$ alkyl;

n = 0, 1 or 2; m = 0, 1 or 2; s = 0 or 1;

$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;

$Q_1$ is

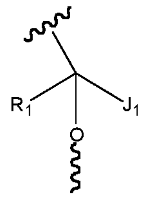

,

and $Q_2$ is $R_2$; or $Q_1$ is $R_2$, and $Q_2$ is

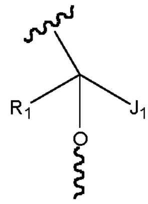

;

wherein:

$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;

$J_1$ is H or $C_1$-$C_6$ alkyl;

$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;

optionally, $R_1$ and $R_2$ are directly linked to form a ring;

B is a base;

wherein the chemical modification of formula (I) or the tautomeric modification thereof is not

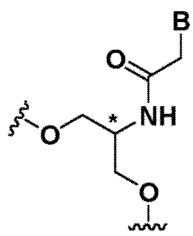

.

In some embodiments, when X is NH-CO, $R_1$ is not H.

In some embodiments, B is a base; for example, B is selected from the group consisting of purine bases, pyrimidine bases, indole, 5-nitroindole and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.

In some embodiments, B is a base at a position corresponding to the modified nucleotide of the antisense strand.

In some embodiments, formula (I) is selected from formula (I-1):

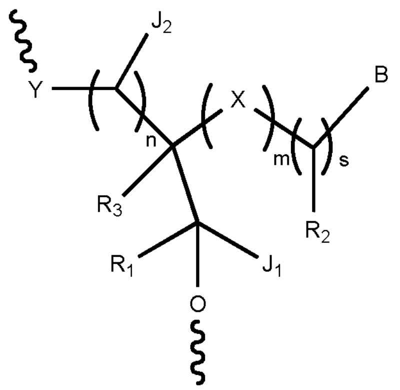

(Formula I-1)

wherein:

- Y is selected from the group consisting of O, NH and S;
- each X is independently selected from the group consisting of $CR_4(R_4')$, S, $NR_5$ and NH-CO, wherein $R_4$, $R_4'$ and $R_5$ are each independently H or $C_1$-$C_6$ alkyl;
- each $J_1$ and each $J_2$ is independently H or $C_1$-$C_6$ alkyl;
- n = 0, 1 or 2; m = 0, 1 or 2; s = 0 or 1;
- $R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
- $R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
- $R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
- optionally, $R_1$ and $R_2$ are directly linked to form a ring;
- B is as defined in formula (I).

In some embodiments of formula (I-1), B is a base; for example, B is selected from the group consisting of purine bases, pyrimidine bases, indole, 5-nitroindole and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.

In some embodiments, B is a base at a position corresponding to the modified nucleotide of the antisense strand.

In some embodiments, formula (I) is selected from formula (I-2):

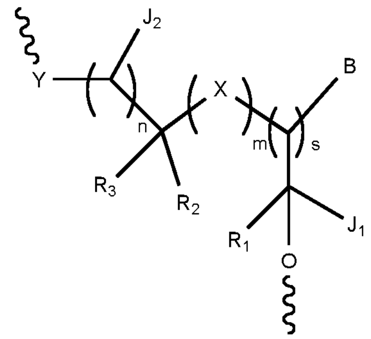

(I-2)

wherein,

Y is selected from the group consisting of O, NH and S;
each X is independently selected from the group consisting of $CR_4(R_4')$, S, $NR_5$ and NH-CO, wherein $R_4$, $R_4'$ and $R_5$ are each independently H or $C_1$-$C_6$ alkyl;
n = 0, 1 or 2; m = 0, 1 or 2; s = 0 or 1;
each $J_1$ and each $J_2$ is independently H or $C_1$-$C_6$ alkyl;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
$R_2$ is selected from the group consisting of H, $C_1$-$C_6$ alkyl, $C_1$-$C_6$ alkoxy, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
optionally, $R_1$ and $R_2$ are directly linked to form a ring;
B is as defined in formula (I).

In some embodiments of formula (I-2), B is a base; for example, B is selected from the group consisting of purine bases, pyrimidine bases, indole, 5-nitroindole and 3-nitropyrrole.
In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.
In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.
In some embodiments, B is a base at a position corresponding to the modified nucleotide of the antisense strand.
In some embodiments, the chemical modification or the tautomeric modification thereof described above is not

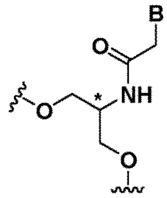

.

In some embodiments, each X is independently selected from the group consisting of $CR_4(R_4')$, S, $NR_5$ and NH-CO, wherein $R_4$, $R_4'$ and $R_5$ are each independently H or $C_1$-$C_3$ alkyl;

n = 0, 1 or 2; m = 0, 1 or 2; s = 0 or 1;
each $J_1$ and each $J_2$ is independently H or $C_1$-$C_3$ alkyl;
$R_3$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$

alkynyl, $S\text{-}CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_3$ alkyl, $C_1$-$C_3$ alkoxy, $C_2$-$C_4$ alkenyl, $C_2$-$C_4$ alkynyl and $(CH_2)_qR_7$, wherein $R_7$ is selected from the group consisting of OH, halogen, methoxy, ethoxy, $N_3$, $C_2$-$C_4$ alkenyl and $C_2$-$C_4$ alkynyl, and q = 1, 2 or 3;
$R_2$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$ alkynyl, $S\text{-}CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_4$ alkenyl and $C_2$-$C_4$ alkynyl, and r = 1, 2 or 3;
optionally, $R_1$ and $R_2$ are directly linked to form a ring.
B is as defined in formula (I).

In some embodiments, B is a base; for example, B is selected from the group consisting of purine bases, pyrimidine bases, indole, 5-nitroindole and 3-nitropyrrole.
In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.
In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.
In some embodiments, B is a base at a position corresponding to the modified nucleotide of the antisense strand.
In some embodiments, each X is independently selected from the group consisting of $CR_4(R_4')$, S, $NR_5$ and NH-CO, wherein $R_4$, $R_4'$ and $R_5$ are each independently H, methyl, ethyl, n-propyl or isopropyl;

n = 0, 1 or 2; m = 0, 1 or 2; s = 0 or 1;
each $J_1$ and each $J_2$ is independently H or methyl;
$R_3$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, $S\text{-}CH_3$, $NCH_3(CH_3)$, $OCH_2CH_2OCH_3$, -O-methylamino, -O-ethylamino and $(CH_2)_pR_6$, wherein $R_6$ is selected from the group consisting of OH, F, Cl, methoxy, ethoxy, $N_3$, vinyl, allyl, ethynyl and propargyl, and p = 1 or 2;
$R_1$ is selected from the group consisting of H, methyl, ethyl, n-propyl, isopropyl, methoxy, ethoxy, n-propoxy, isopropoxy, vinyl, allyl, ethynyl, propargyl and $(CH_2)_qR_7$, wherein $R_7$ is selected from the group consisting of OH, F, Cl, methoxy, ethoxy, $N_3$, vinyl, allyl, ethynyl and propargyl, and q = 1 or 2;
$R_2$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, $S\text{-}CH_3$, $NCH_3(CH_3)$, $OCH_2CH_2OCH_3$, -O-methylamino, -O-ethylamino and $(CH_2)_rR_8$, wherein $R_8$ is selected from the group consisting of OH, F, Cl, methoxy, ethoxy, $N_3$, vinyl, allyl, ethynyl, and propargyl, and r = 1 or 2;
optionally, $R_1$ and $R_2$ are directly linked to form a ring.
B is as defined in formula (I).

In some embodiments, B is a base; for example, B is selected from the group consisting of purine bases, pyrimidine bases, indole, 5-nitroindole and 3-nitropyrrole.
In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.
In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.
In some embodiments, B is a base at a position corresponding to the modified nucleotide of the antisense strand.
B is as defined in formula (I).
In some embodiments, B is a base; for example, B is selected from the group consisting of those of purine bases, pyrimidine bases, indole, 5-nitroindole and 3-nitropyrrole.
In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.
In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.
In some embodiments, B is a base at a position corresponding to the modified nucleotide of the antisense strand.

In some embodiments, Y is O or NH; each X is independently selected from the group consisting of NH-CO, $CH_2$ and NH;

n = 0 or1; m = 0 or1; s = 0 or 1;
each $J_1$ and each $J_2$ is independently H;
$R_1$ is selected from the group consisting of H, methyl and $CH_2OH$;
$R_2$ is selected from the group consisting of H, OH, $NH_2$, methyl and $CH_2OH$;
$R_3$ is selected from the group consisting of H, OH, $NH_2$, methyl and $CH_2OH$;
optionally, $R_1$ and $R_2$ are directly linked to form a ring;
B is as defined in formula (I).

In some embodiments, B is a base; for example, B is selected from the group consisting of purine bases, pyrimidine bases, indole, 5-nitroindole and 3-nitropyrrole.
In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.
In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.
In some embodiments, B is a base at a position corresponding to the modified nucleotide of the antisense strand.
In some embodiments, Y is O or NH; each X is independently selected from the group consisting of NH-CO, $CH_2$ and NH;

n = 0 or1; m = 0 or1; s = 0 or 1;
each $J_1$ and each $J_2$ is independently H;
$R_1$ is selected from the group consisting of H, methyl and $CH_2OH$;
$R_2$ is selected from the group consisting of H, methyl and $CH_2OH$;
$R_3$ is selected from the group consisting of H, OH, $NH_2$, methyl and $CH_2OH$;
optionally, $R_1$ and $R_2$ are directly linked to form a ring.

In some embodiments, B is a base; for example, B is selected from the group consisting of purine bases, pyrimidine bases, indole, 5-nitroindole and 3-nitropyrrole.
In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.
In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.
In some embodiments, B is a base at a position corresponding to the modified nucleotide of the antisense strand.
In some embodiments, in some embodiments, the chemical modification of formula (I) or the tautomeric modification thereof is selected from the group consisting of:

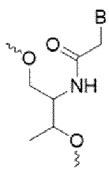 , 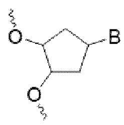 , 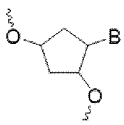 , 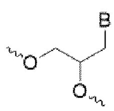 , 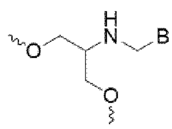 , 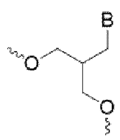 , 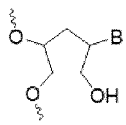 ,

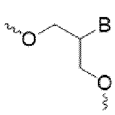 , 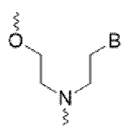 , 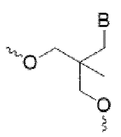 , 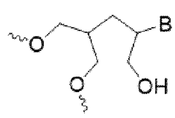 , 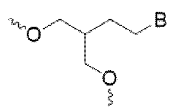 ,

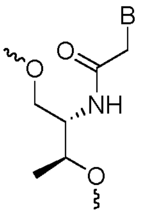, 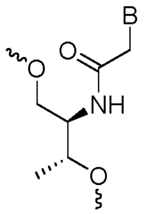, 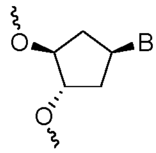, 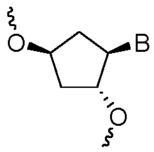, 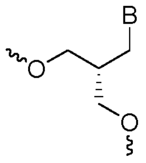,

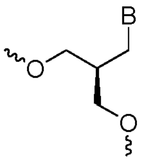, 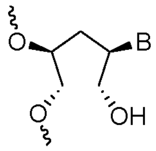, 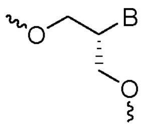, 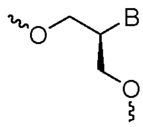, 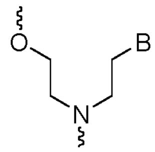,

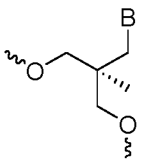, 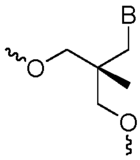, 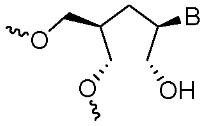, 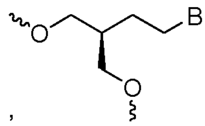, 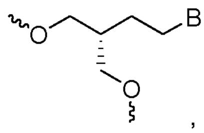,

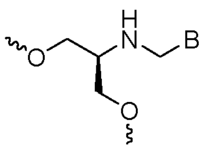, 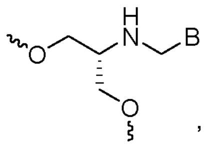, 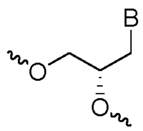, 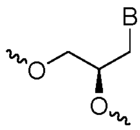,

wherein B is a base; for example, B is selected from the group consisting of purine bases, pyrimidine bases, indole, 5-nitroindole and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.

In some embodiments, B is a base at a position corresponding to the modified nucleotide of the antisense strand.

In some embodiments, the chemical modification of formula (I) or the tautomeric modification thereof is selected from the group consisting of:

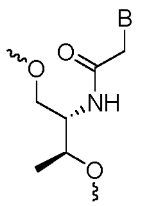, 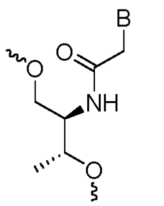, 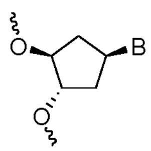, 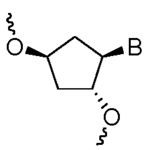, 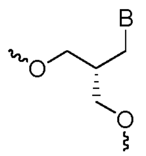,

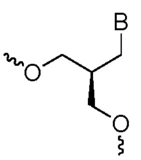, ,

wherein B is a base; for example, B is selected from the group consisting of purine bases, pyrimidine bases, indole, 5-nitroindole and 3-nitropyrrole.

In some embodiments, B is selected from the group consisting of adenine, guanine, isoguanine, hypoxanthine,

xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.

In some embodiments, B is a base at a position corresponding to the modified nucleotide of the antisense strand.

In some embodiments, the chemical modification of formula (I) or the tautomeric modification thereof is selected from the group consisting of:

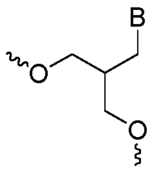 , 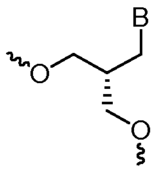 or 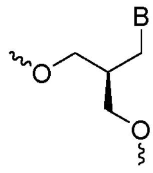 ;

wherein B is selected from the group consisting of adenine, guanine, 2,6-diaminopurine, 6-dimethylaminopurine, 2-aminopurine, cytosine, uracil, thymine, indole, 5-nitroindole and 3-nitropyrrole.

In some embodiments, B is a base at a corresponding position among positions 2 to 8 of the 5' region of the antisense strand.

In some embodiments, when the chemical modification of formula (I) or the tautomeric modification thereof is at position 5 of the 5' region, B is selected from the group consisting of adenine, guanine, 2,6-diaminopurine, 6-dimethylaminopurine, 2-aminopurine, cytosine, uracil, thymine, indole, 5-nitroindole and 3-nitropyrrole. In some embodiments, B is a base corresponding to position 5 of the 5' region of the antisense strand.

In some embodiments, when the chemical modification of formula (I) or the tautomeric modification thereof is at position 6 of the 5' region, B is selected from the group consisting of adenine, guanine, 2,6-diaminopurine, 6-dimethylaminopurine, 2-aminopurine, cytosine, uracil, thymine, indole, 5-nitroindole and 3-nitropyrrole. In some embodiments, B is a base corresponding to position 6 of the 5' region of the antisense strand.

In some embodiments, when the chemical modification of formula (I) or the tautomeric modification thereof is at position 7 of the 5' region, B is selected from the group consisting of adenine, guanine, 2,6-diaminopurine, 6-dimethylaminopurine, 2-aminopurine, cytosine, uracil, thymine, indole, 5-nitroindole and 3-nitropyrrole. In some embodiments, B is a base corresponding to position 7 of the 5' region of the antisense strand.

In some embodiments, a nucleotide comprising a chemical modification of formula (I) or a tautomeric modification thereof is selected from a nucleotide comprising a chemical modification of formula (I') or a tautomeric modification thereof,

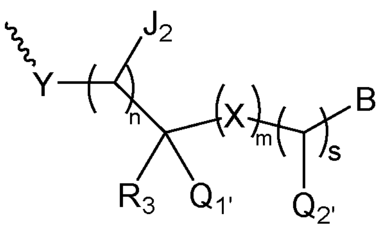

(I')

wherein Y is selected from the group consisting of O, NH and S;

each X is independently selected from the group consisting of $CR_4(R_4')$, S, $NR_5$ and NH-CO, wherein $R_4$, $R_4'$ and $R_5$ are each independently H or $C_1$-$C_6$ alkyl;

$J_2$ is H or $C_1$-$C_6$ alkyl;

n = 0, 1 or 2; m = 0, 1 or 2; s = 0 or 1;

$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;

$Q_{1'}$ is

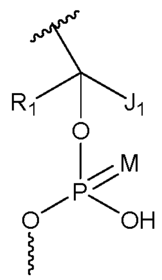

,

and $Q_{2'}$ is $R_2$; or $Q_{1'}$ is $R_2$, and $Q_{2'}$ is

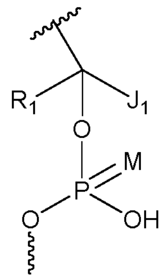

;

wherein:

$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;

$J_1$ is H or $C_1$-$C_6$ alkyl;

$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;

optionally, $R_1$ and $R_2$ are directly linked to form a ring;

B is a base;

M is O or S;

wherein the chemical modification of formula (I') or the tautomeric modification thereof is not

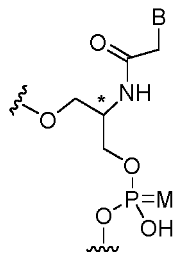

.

In some embodiments, when X is NH-CO, $R_1$ is not H.

In some embodiments of formula (I'), B is a base; for example, B is selected from the group consisting of purine bases, pyrimidine bases, indole, 5-nitroindole and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.

In some embodiments, B is a base at a position corresponding to the modified nucleotide of the antisense strand.

In some embodiments, formula (I') is selected from formula (I'-1):

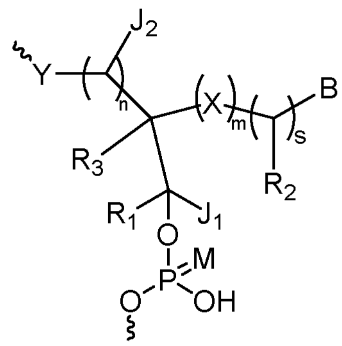

(I'-1)

wherein Y is selected from the group consisting of O, NH and S;
each X is independently selected from the group consisting of $CR_4(R_4')$, S, $NR_5$ and NH-CO, wherein $R_4$, $R_4'$ and $R_5$ are each independently H or $C_1$-$C_6$ alkyl;
each $J_1$ and each $J_2$ is independently H or $C_1$-$C_6$ alkyl;
n = 0, 1 or 2; m = 0, 1 or 2; s = 0 or 1;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
M is O or S;
optionally, $R_1$ and $R_2$ are directly linked to form a ring;
B is as defined in formula (I').

In some embodiments of formula (I'-1), B is a base; for example, B is selected from the group consisting of purine bases, pyrimidine bases, indole, 5-nitroindole and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.

In some embodiments, B is a base at a position corresponding to the modified nucleotide of the antisense strand.

In some embodiments, formula (I') is selected from formula (I'-2):

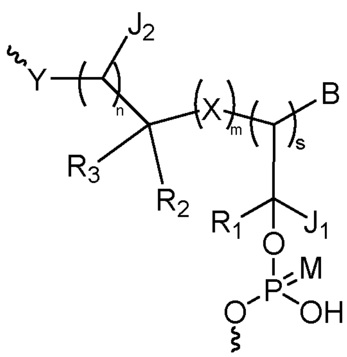

(I'-2)

wherein Y is selected from the group consisting of O, NH and S;
each X is independently selected from the group consisting of $CR_4(R_4')$, S, $NR_5$ and NH-CO, wherein $R_4$, $R_4'$ and $R_5$ are each independently H or $C_1$-$C_6$ alkyl;
n = 0, 1 or 2; m = 0, 1 or 2; s = 0 or 1;
each $J_1$ and each $J_2$ is independently H or $C_1$-$C_6$ alkyl;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
$R_2$ is selected from the group consisting of H, $C_1$-$C_6$ alkyl, $C_1$-$C_6$ alkoxy, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
optionally, $R_1$ and $R_2$ are directly linked to form a ring;
M is O or S;
B is as defined in formula (I').

In some embodiments of formula (I'-2), B is a base; for example, B is selected from the group consisting of purine bases, pyrimidine bases, indole, 5-nitroindole and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.

In some embodiments, B is a base at a position corresponding to the modified nucleotide of the antisense strand.

In some embodiments, the chemical modification or the tautomeric modification thereof described above is not

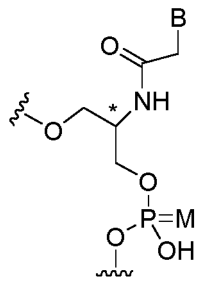

.

In some embodiments, when X is NH-CO, $R_1$ is not H.

In some embodiments, each X is independently selected from the group consisting of $CR_4(R_4')$, S, $NR_5$ and NH-CO, wherein $R_4$, $R_4'$ and $R_5$ are each independently H or $C_1$-$C_3$ alkyl;

n = 0, 1 or 2; m = 0, 1 or 2; s = 0 or 1;
each $J_1$ and each $J_2$ is independently H or $C_1$-$C_3$ alkyl;
$R_3$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_3$ alkyl, $C_1$-$C_3$ alkoxy, $C_2$-$C_4$ alkenyl, $C_2$-$C_4$ alkynyl and $(CH_2)_qR_7$, wherein $R_7$ is selected from the group consisting of OH, halogen, methoxy, ethoxy, $N_3$, $C_2$-$C_4$ alkenyl and $C_2$-$C_4$ alkynyl, and q = 1, 2 or 3;
$R_2$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_4$ alkenyl and $C_2$-$C_4$ alkynyl, and r = 1, 2 or 3;
optionally, $R_1$ and $R_2$ are directly linked to form a ring.

In some embodiments, each X is independently selected from the group consisting of $CR_4(R_4')$, S, $NR_5$ and NH-CO, wherein $R_4$, $R_4'$ and $R_5$ are each independently H, methyl, ethyl, n-propyl or isopropyl;

n = 0, 1 or 2; m = 0, 1 or 2; s = 0 or 1;
each $J_1$ and each $J_2$ is independently H or methyl;
$R_3$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, S-$CH_3$, $NCH_3(CH_3)$, $OCH_2CH_2OCH_3$, -O-methylamino, -O-ethylamino and $(CH_2)_pR_6$, wherein $R_6$ is selected from the group consisting of OH, F, Cl, methoxy, ethoxy, $N_3$, vinyl, allyl, ethynyl and propargyl, and p = 1 or 2;
$R_1$ is selected from the group consisting of H, methyl, ethyl, n-propyl, isopropyl, methoxy, ethoxy, n-propoxy, isopropoxy, vinyl, allyl, ethynyl, propargyl and $(CH_2)_qR_7$, wherein $R_7$ is selected from the group consisting of OH, F, Cl, methoxy, ethoxy, $N_3$, vinyl, allyl, ethynyl and propargyl, and q = 1 or 2;
$R_2$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, S-$CH_3$, $NCH_3(CH_3)$, $OCH_2CH_2OCH_3$, -O-methylamino, -O-ethylamino and $(CH_2)_rR_8$, wherein $R_8$ is selected from the group consisting of OH, F, Cl, methoxy, ethoxy, $N_3$, vinyl, allyl, ethynyl, and propargyl, and r = 1 or 2;
optionally, $R_1$ and $R_2$ are directly linked to form a ring.

In some embodiments, B is a base; for example, B is selected from the group consisting of purine bases, pyrimidine bases, indole, 5-nitroindole and 3-nitropyrrole.
In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.
In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.
In some embodiments, B is a base at a position corresponding to the modified nucleotide of the antisense strand.
In some embodiments, Y is O or NH; each X is independently selected from the group consisting of NH-CO, $CH_2$ and NH;

n = 0 or1; m = 0 or1; s = 0 or 1;
each $J_1$ and each $J_2$ is independently H;
$R_1$ is selected from the group consisting of H, methyl and $CH_2OH$;
$R_2$ is selected from the group consisting of H, OH, $NH_2$, methyl and $CH_2OH$;
$R_3$ is selected from the group consisting of H, OH, $NH_2$, methyl and $CH_2OH$;
optionally, $R_1$ and $R_2$ are directly linked to form a ring.

In some embodiments, B is a base; for example, B is selected from the group consisting of purine bases, pyrimidine bases, indole, 5-nitroindole and 3-nitropyrrole.
In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.
In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.
In some embodiments, B is a base at a position corresponding to the modified nucleotide of the antisense strand.
In some embodiments, Y is O or NH; each X is independently selected from the group consisting of NH-CO, $CH_2$ and NH;

n = 0 or1; m = 0 or1; s = 0 or 1;
each $J_1$ and each $J_2$ is independently H;
$R_1$ is selected from the group consisting of H, methyl and $CH_2OH$;
$R_2$ is selected from the group consisting of H, methyl and $CH_2OH$;
$R_3$ is selected from the group consisting of H, OH, $NH_2$, methyl and $CH_2OH$;
optionally, $R_1$ and $R_2$ are directly linked to form a ring.

In some embodiments, B is a base; for example, B is selected from the group consisting of purine bases, pyrimidine bases, indole, 5-nitroindole and 3-nitropyrrole.
In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.

In some embodiments, B is a base at a position corresponding to the modified nucleotide of the antisense strand.

In some embodiments, the chemical modification of formula (I') or the tautomeric modification thereof is selected from the group consisting of:

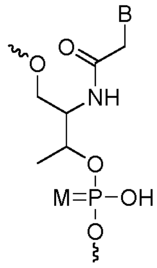
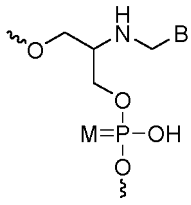
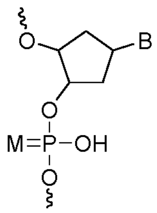
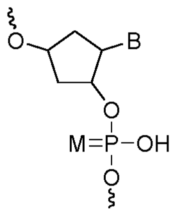
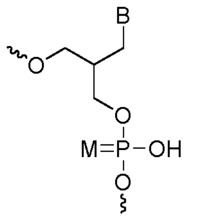

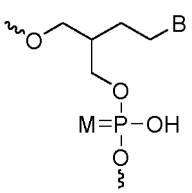
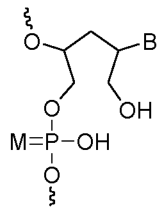
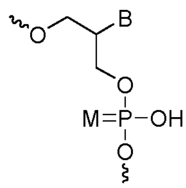
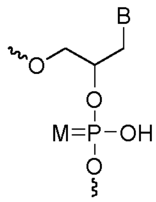
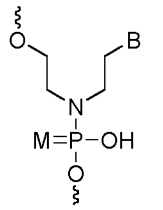

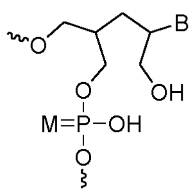
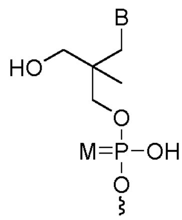

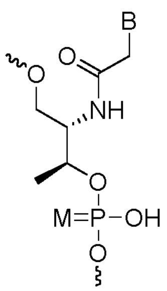
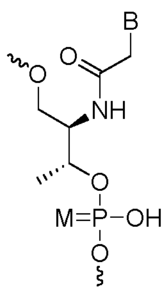
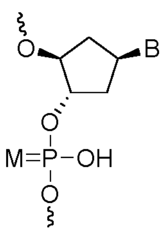
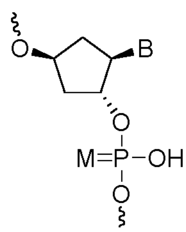
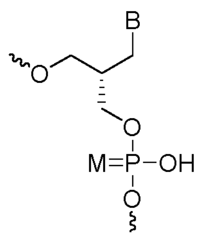

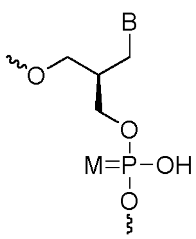
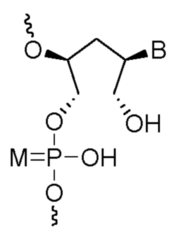
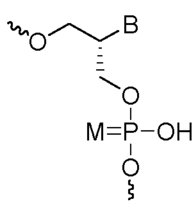
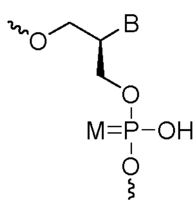
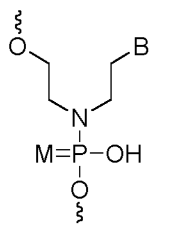

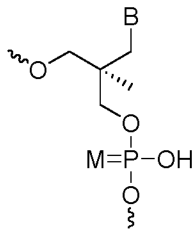
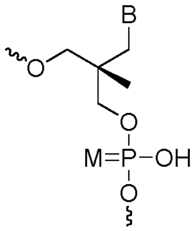
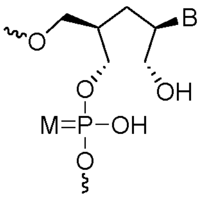
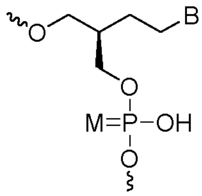
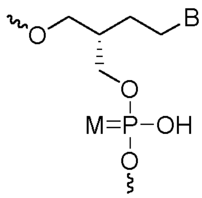

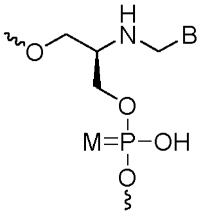
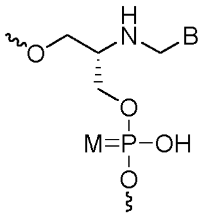
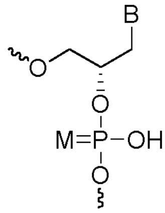
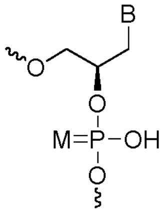

wherein M is O or S;
B is as defined in formula (I').

In some embodiments, B is a base; for example, B is selected from the group consisting of purine bases, pyrimidine bases, indole, 5-nitroindole and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.

In some embodiments, B is a base at a position corresponding to the modified nucleotide of the antisense strand.

In some embodiments, the chemical modification of formula (I') or the tautomeric modification thereof is selected from the group consisting of:

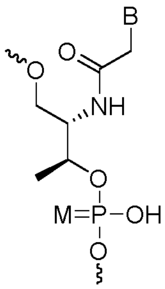
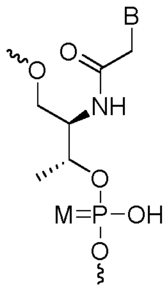
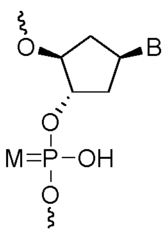
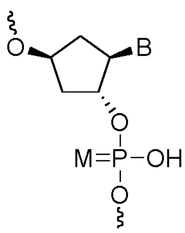
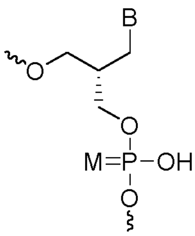

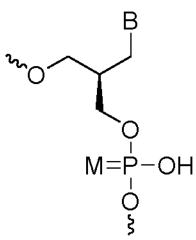
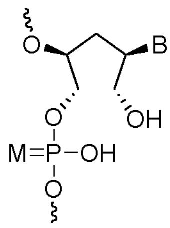
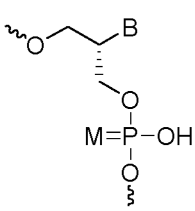
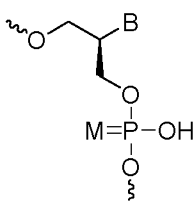
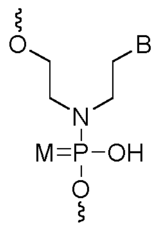

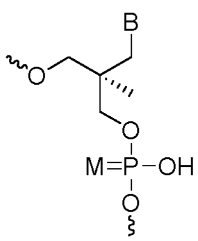 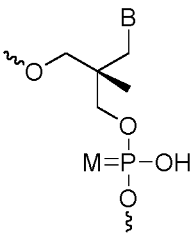 ;

wherein M is O or S;

B is as defined in formula (I').

In some embodiments, B is a base; for example, B is selected from the group consisting of purine bases, pyrimidine bases, indole, 5-nitroindole and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.

In some embodiments, B is a base at a position corresponding to the modified nucleotide of the antisense strand.

In some embodiments, the chemical modification of formula (I') or the tautomeric modification thereof is selected from the group consisting of:

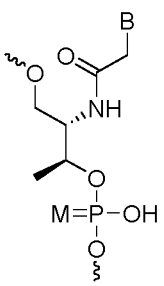 , 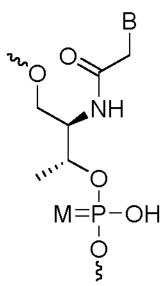 , 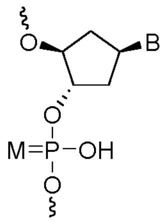 , 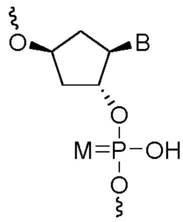 , 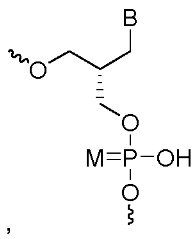 ,

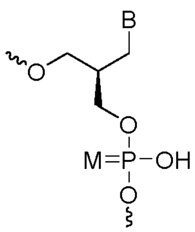

wherein M is O or S;

B is as defined in formula (I').

In some embodiments, B is a base; for example, B is selected from the group consisting of purine bases, pyrimidine bases, indole, 5-nitroindole and 3-nitropyrrole.

In some embodiments, B is selected from the group consisting of adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some embodiments, B is selected from the group consisting of adenine, guanine, 2,6-diaminopurine, 6-dimethylaminopurine, 2-aminopurine, cytosine, uracil, thymine, indole, 5-nitroindole and 3-nitropyrrole.

In some embodiments, B is a base at a position corresponding to the modified nucleotide of the antisense strand.

In some embodiments, the chemical modification of formula (I') or the tautomeric modification thereof includes, but is not limited to:

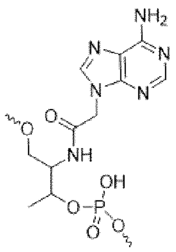, 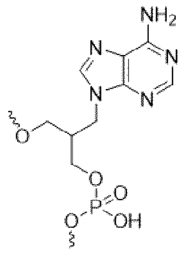, 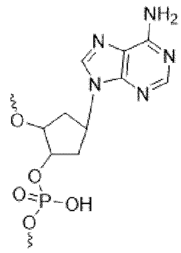, 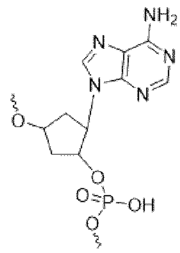, 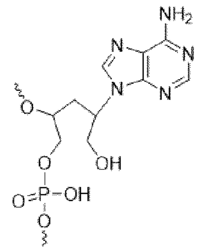

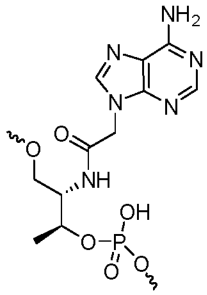, 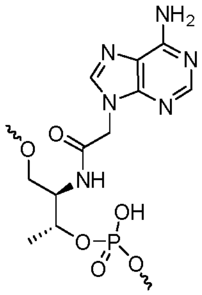, 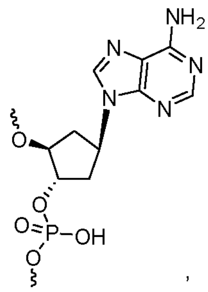, 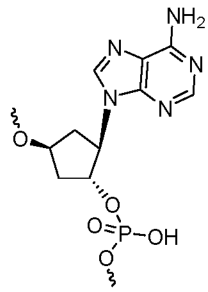,

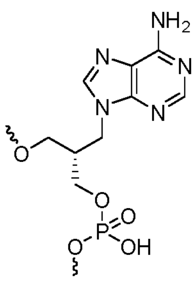, 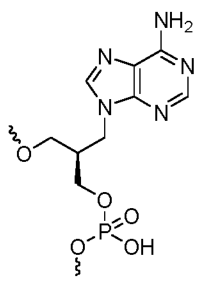, 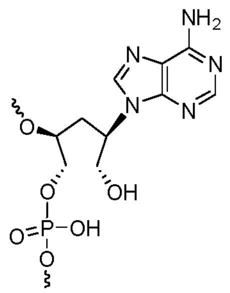,

and those where adenine in the structure is replaced with guanine, cytosine, uracil or thymine.

In some embodiments, at least one nucleotide at positions 2 to 8 (e.g., position 2, position 3, position 4, position 5, position 6, position 7 or position 8) of the 5' region of the antisense strand comprises a 2'-methoxy modification.

In some embodiments, any one of the nucleotides at positions 5, 6 and 7 of the 5' region of the antisense strand comprises a 2'-methoxy modification.

In some embodiments, the modified nucleotide is located at any one of positions 2 to 8 of the 5' region of the antisense strand.

In some embodiments, the modified nucleotide is located at any one of position 5, 6 or 7 of the 5' region of the antisense strand.

In some embodiments, when the chemical modification of formula (I') or the tautomeric modification thereof is at position 5 of the 5' region, B is selected from the group consisting of adenine, guanine, 2,6-diaminopurine, 6-dimethylaminopurine, 2-aminopurine, cytosine, uracil, thymine, indole, 5-nitroindole and 3-nitropyrrole; in some embodiments, B is a base corresponding to position 5 of the 5' region of the antisense strand.

In some embodiments, when the chemical modification of formula (I') or the tautomeric modification thereof is at position 6 of the 5' region, B is selected from the group consisting of adenine, guanine, 2,6-diaminopurine, 6-dimethylaminopurine, 2-aminopurine, cytosine, uracil, thymine, indole, 5-nitroindole and 3-nitropyrrole; in some embodiments, B is a base corresponding to position 6 of the 5' region of the antisense strand.

In some embodiments, when the chemical modification of formula (I') or the tautomeric modification thereof is at position 7 of the 5' region, B is selected from the group consisting of adenine, guanine, 2,6-diaminopurine, 6-dimethylaminopurine, 2-aminopurine, cytosine, uracil, thymine, indole, 5-nitroindole and 3-nitropyrrole; in some embodiments, B is a base corresponding to position 7 of the 5' region of the antisense strand.

In some embodiments, at least one additional nucleotide in the sense strand and/or the antisense strand of the siRNA is a modified nucleotide selected from the group consisting of: a 2'-methoxy-modified nucleotide, a 2'-substituted alkoxy-modified nucleotide, a 2'-alkyl-modified nucleotide, a 2'-substituted alkyl-modified nucleotide, a 2'-amino-modified nucleotide, a 2'-substituted amino-modified nucleotide, a 2'-fluoro-modified nucleotide, a 2'-deoxynucleotide, a 2'-deoxy-2'-fluoro-modified nucleotide, a 3'-deoxy-thymine nucleotide, an isonucleotide, LNA, ENA, cET, UNA and GNA. In some

embodiments, the modified nucleotides are each independently selected from the group consisting of: a 2’-methoxy-modified nucleotide, a 2’-fluoro-modified nucleotide and a 2’-deoxy-modified nucleotide.

In some embodiments, three contiguous nucleotides in the sense strand of the siRNA are 2’-fluoro-modified nucleotides.

In some embodiments, three contiguous nucleotides at positions 7-9 of the 5’ end in the sense strand of the siRNA are 2’-fluoro-modified nucleotides.

In some embodiments, three contiguous nucleotides at positions 7-9 of the 5’ end in the sense strand of the siRNA are 2’-fluoro-modified nucleotides, and the remaining positions of the sense strand are all non-2’-fluoro-modified nucleotides.

In some embodiments, in a 5’-end to 3’-end direction, nucleotides at positions 2, 4, 6, 9, 12, 14, 16 and 18 of the antisense strand are each independently a 2’-fluoro-modified nucleotide.

In some other embodiments, in a 5’-end to 3’-end direction, nucleotides at positions 2, 4, 6, 10, 12, 14, 16 and 18 of the antisense strand are each independently a 2’-fluoro-modified nucleotide.

In some embodiments, the sense strand comprises the nucleotide sequence of the formula shown below (5’-3’):

$N_aN_aN_aN_aXN_aN_bN_bN_bN_aN_aN_aN_aN_aN_aN_aN_aN_aN_a$
wherein each X is independently $N_a$ or $N_b$;
$N_a$ is a 2’-methoxy-modified nucleotide, and $N_b$ is a 2’-fluoro-modified nucleotide.

In some embodiments, the sense strand comprises a nucleotide sequence of the formula shown below:

5’-$N_aN_aN_aN_aN_aN_aN_bN_bN_bN_aN_aN_aN_aN_aN_aN_aN_aN_aN_a$-3’; or,
5’-$N_aN_aN_aN_aN_bN_aN_bN_bN_bN_aN_aN_aN_aN_aN_aN_aN_aN_aN_a$-3’;
wherein $N_a$ is a 2’-methoxy-modified nucleotide, and $N_b$ is a 2’-fluoro-modified nucleotide.

In some embodiments, the sense strand is selected from a nucleotide sequence of the formula shown below:

5’-$N_aN_aN_aN_aN_aN_aN_bN_bN_bN_aN_aN_aN_aN_aN_aN_aN_aN_aN_a$-3’; or,
5’-$N_aN_aN_aN_aN_bN_aN_bN_bN_bN_aN_aN_aN_aN_aN_aN_aN_aN_aN_a$-3’;
wherein $N_a$ is a 2’-methoxy-modified nucleotide, and $N_b$ is a 2’-fluoro-modified nucleotide.

In some embodiments, the antisense strand comprises a nucleotide sequence of the formula shown below:

5’-$N_a'N_b'N_a'N_b'N_a'N_b'W'N_a'N_a'N_b'N_a'N_b'N_a'N_b'N_a'N_b'N_a'N_b'N_a'N_a'N_a'$-3’; or,
5’-$N_a'N_b'N_a'N_b'N_a'N_b'W'N_a'N_b'N_a'N_a'N_b'N_a'N_b'N_a'N_b'N_a'N_b'N_a'N_a'N_a'$-3’;
wherein $N_a'$ is a 2’-methoxy-modified nucleotide, and $N_b'$ is a 2’-fluoro-modified nucleotide; W’ represents a 2’-methoxy-modified nucleotide or a nucleotide comprising a chemical modification of formula (I) or a tautomeric modification thereof.

In some specific embodiments, W’ represents a nucleotide comprising a chemical modification of formula (I) or a tautomeric modification thereof.

In some specific embodiments, formula (I) is selected from the group consisting of:

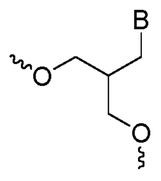,
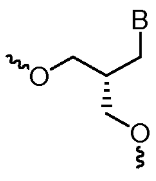 or
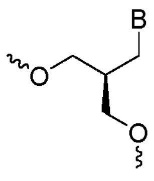;

wherein B is selected from the group consisting of guanine, adenine, cytosine or uracil. In some specific embodiments, B is selected from a base corresponding to position 7 of the 5’ region of the antisense strand. In some specific embodiments, formula (I) is selected from the group consisting of:

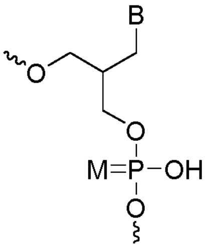, 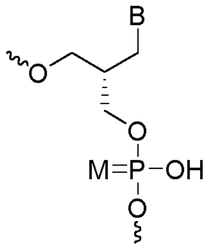 and 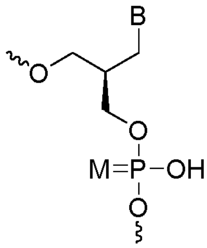;

wherein M is O or S; wherein B is selected from the group consisting of guanine, adenine, cytosine or uracil. In some specific embodiments, B is selected from a base corresponding to position 7 of the 5' region of the antisense strand.

In some specific embodiments, M is S. In some specific embodiments, M is O.

In some specific embodiments, W' represents a 2'-methoxy-modified nucleotide.

In some embodiments, at least one phosphoester group in the sense strand and/or the antisense strand is a phosphoester group with a modification group. The modification group makes the siRNA have increased stability in a biological sample or environment. In some embodiments, the phosphoester group with a modification group is a phosphorothioate group.

In some embodiments, the phosphorothioate group is present in at least one of the positions selected from the group consisting of:

a position between the 1st and 2nd nucleotides of the 5' end of the sense strand;
a position between the 2nd and 3rd nucleotides of the 5' end of the sense strand;
an end of the 1st nucleotide of the 3' end of the sense strand;
a position between the 1st and 2nd nucleotides of the 3' end of the sense strand;
a position between the 2nd and 3rd nucleotides of the 3' end of the sense strand;
a position between the 1st and 2nd nucleotides of the 5' end of the antisense strand;
a position between the 2nd and 3rd nucleotides of the 5' end of the antisense strand;
an end of the 1st nucleotide of the 3' end of the antisense strand;
a position between the 1st and 2nd nucleotides of the 3' end of the antisense strand; and
a position between the 2nd and 3rd nucleotides of the 3' end of the antisense strand.

In some embodiments, the sense strand and/or the antisense strand comprise a plurality of phosphorothioate groups that are present:

between the 1st and 2nd nucleotides of the 5' end of the sense strand; and
between the 2nd and 3rd nucleotides of the 5' end of the sense strand; and
between the 1st and 2nd nucleotides of the 5' end of the antisense strand; and
between the 2nd and 3rd nucleotides of the 5' end of the antisense strand; and
between the 1st and 2nd nucleotides of the 3' end of the antisense strand; and
between the 2nd and 3rd nucleotides of the 3' end of the antisense strand.

In some embodiments, the sense strand comprises a nucleotide sequence of the formula shown below:

5'-NmsNmsNmNmNfNmNfNfNfNmNmNmNmNmNmNmNmNmNm-3', or
5'-NmsNmsNmNmNmNmNfNfNfNmNmNmNmNmNmNmNmNmNm-3', or
5'-NmsNmsNmNmNfNmNfNfNfNmNmNmNmNmNmNmNmNmNms-3', or
5'-NmsNmsNmNmNmNmNfNfNfNmNmNmNmNmNmNmNmNmNms-3', or
5'-NmsNmsNmNmNfNmNfNfNfNmNmNmNmNmNmNmNmNmsNm-3', or
5'-NmsNmsNmNmNmNmNfNfNfNmNmNmNmNmNmNmNmNmsNm-3', or
5'-NmsNmsNmNmNfNmNfNfNfNmNmNmNmNmNmNmNmNmsNms-3', or
5'-NmsNmsNmNmNmNmNfNfNfNmNmNmNmNmNmNmNmNmsNms-3',
wherein Nm represents any 2'-methoxy-modified nucleotide, such as 2'-methoxy-modified C, G, U or A; Nf represents any 2'-fluoro-modified nucleotide, such as 2'-fluoro-modified C, G, U or A;
the lowercase letter s indicates that the two nucleotides adjacent to the letter s are linked by a phosphorothioate group; the lowercase letter s, when being the first at the 3' end, indicates that the upstream phosphoester group adjacent to the letter s is a phosphorothioate group.

In some embodiments, the antisense strand comprises a nucleotide sequence of the formula shown below:

5’-Nm’sNf sNm’NfNm’NfW’Nm’Nm’NfNm’NfNm’NfNm’NfNm’NfNm’sNm’sNm’-3’, or
5’-Nm’sNf sNm’NfNm’NfW’Nm’NfNm’Nm’NfNm’NfNm’NfNm’NfNm’sNm’sNm’-3’

wherein Nm’ represents any 2’-methoxy-modified nucleotide, such as 2’-methoxy-modified C, G, U or A; Nf represents any 2’-fluoro-modified nucleotide, such as 2’-fluoro-modified C, G, U or A;

the lowercase letter s indicates that the two nucleotides adjacent to the letter s are linked by a phosphorothioate group; the lowercase letter s, when being the first at the 3’ end, indicates that the upstream phosphoester group adjacent to the letter s is a phosphorothioate group;

W’ represents a 2’-methoxy-modified nucleotide or a nucleotide comprising a chemical modification of formula (I) or a tautomeric modification thereof.

In some embodiments, formula (I) is selected from the group consisting of:

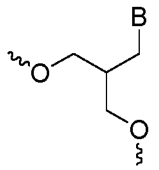 , 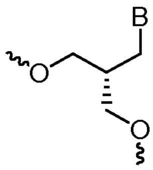 or 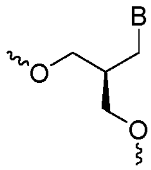 ;

wherein B is selected from the group consisting of guanine, adenine, cytosine or uracil. In some embodiments, B is a base corresponding to position 7 of the 5’ region of the antisense strand.

In some embodiments, formula (I) is selected from the group consisting of:

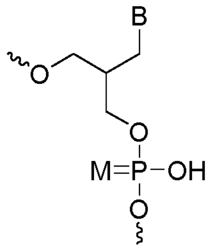 , 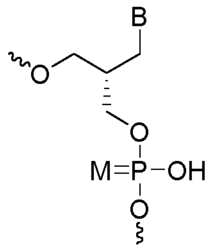 and 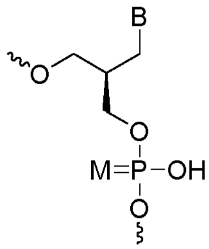 ;

wherein M is O or S; wherein B is selected from the group consisting of guanine, adenine, cytosine or uracil. In some specific embodiments, B is a base corresponding to position 7 of the 5’ region of the antisense strand.

In some specific embodiments, M is S. In some specific embodiments, M is O.

In some embodiments, the sense strand comprises a sequence differing by no more than 3 nucleotides from any one of the nucleotide sequences of SEQ ID NO: 1 to SEQ ID NO: 6 and comprises at least 15 contiguous nucleotides.

In some embodiments, the sense strand comprises a sequence differing by no more than 3 nucleotides from any one of the nucleotide sequences of SEQ ID NO: 1 to SEQ ID NO: 6 and comprises at least 19 contiguous nucleotides; in some embodiments, the nucleotide sequence differs by no more than 1 nucleotide.

In some embodiments, the antisense strand comprises a sequence differing by no more than 3 nucleotides from any one of the nucleotide sequences of SEQ ID NO: 13 to SEQ ID NO: 18 and comprises at least 21 contiguous nucleotides; in some embodiments, the nucleotide sequence differs by no more than 1 nucleotide; wherein W’ represents a 2’-methoxy-modified nucleotide, or a nucleotide comprising a chemical modification of formula (I) or a tautomeric modification thereof.

In some embodiments, the nucleotide of the sense strand is selected from any one of SEQ ID NO: 1 to SEQ ID NO: 6.

In some embodiments, the nucleotide of the antisense strand is selected from any one of SEQ ID NO: 13 to SEQ ID NO: 18, wherein W’ represents a 2’-methoxy-modified nucleotide, or a nucleotide comprising a chemical modification of formula (I) or a tautomeric modification thereof.

In some embodiments, formula (I) is selected from the group consisting of:

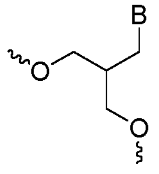, 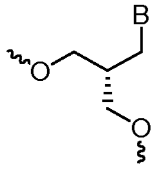 or 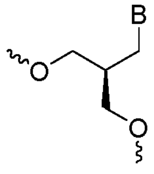;

wherein B is selected from the group consisting of guanine, adenine, cytosine or uracil. In some embodiments, B is a base corresponding to position 7 of the 5' region of the antisense strand.

In some embodiments, formula (I) is selected from the group consisting of:

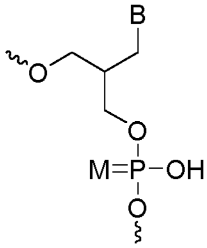, 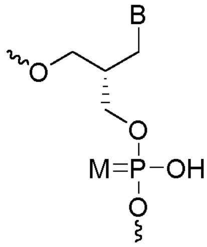 and 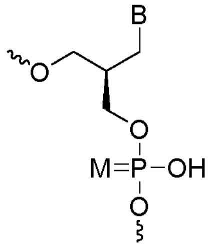;

wherein M is O or S; wherein B is selected from the group consisting of guanine, adenine, cytosine and uracil; in some specific embodiments, B is a base corresponding to position 7 of the 5' region of the antisense strand.

In some specific embodiments, M is S. In some specific embodiments, M is O.

In some specific embodiments, the sense strand comprises any one of SEQ ID NO: 135 to SEQ ID NO: 162.

In some specific embodiments, the antisense strand comprises any one of SEQ ID NO: 189 to SEQ ID NO: 216, SEQ ID NO: 243 to SEQ ID NO: 259 and SEQ ID NO: 273 to SEQ ID NO: 289.

In some specific embodiments, the sense strand is selected from any one of SEQ ID NO: 135 to SEQ ID NO: 162; in some specific embodiments, the antisense strand is selected from any one of SEQ ID NO: 189 to SEQ ID NO: 216, SEQ ID NO: 243 to SEQ ID NO: 259 and SEQ ID NO: 273 to SEQ ID NO: 289.

In some specific embodiments, the siRNA comprises a sense strand and an antisense strand selected from any one of those shown in Table 26.

In some specific embodiments, the siRNA of the present disclosure is selected from any one of the following:

the siRNA of the present disclosure, wherein the sense strand is selected from SEQ ID NO: 140, and the antisense strand is selected from SEQ ID NO: 194; or

the siRNA of the present disclosure, wherein the sense strand is selected from SEQ ID NO: 143, and the antisense strand is selected from SEQ ID NO: 197; or

the siRNA of the present disclosure, wherein the sense strand is selected from SEQ ID NO: 144, and the antisense strand is selected from SEQ ID NO: 199; or

the siRNA of the present disclosure, wherein the sense strand is selected from SEQ ID NO: 146, and the antisense strand is selected from SEQ ID NO: 200; or

the siRNA of the present disclosure, wherein the sense strand is selected from SEQ ID NO: 147, and the antisense strand is selected from SEQ ID NO: 201; or

the siRNA of the present disclosure, wherein the sense strand is selected from SEQ ID NO: 148, and the antisense strand is selected from SEQ ID NO: 202; or

the siRNA of the present disclosure, wherein the sense strand is selected from SEQ ID NO: 149, and the antisense strand is selected from SEQ ID NO: 203; or

the siRNA of the present disclosure, wherein the sense strand is selected from SEQ ID NO: 150, and the antisense strand is selected from SEQ ID NO: 204; or

the siRNA of the present disclosure, wherein the sense strand is selected from SEQ ID NO: 151, and the antisense strand is selected from SEQ ID NO: 205; or

the siRNA of the present disclosure, wherein the sense strand is selected from SEQ ID NO: 152, and the antisense strand is selected from SEQ ID NO: 206; or

the siRNA of the present disclosure, wherein the sense strand is selected from SEQ ID NO: 153, and the antisense strand is selected from SEQ ID NO: 207; or

the siRNA of the present disclosure, wherein the sense strand is selected from SEQ ID NO: 154, and the antisense strand is selected from SEQ ID NO: 208; or

the siRNA of the present disclosure, wherein the sense strand is selected from SEQ ID NO: 155, and the antisense strand is selected from SEQ ID NO: 209; or
the siRNA of the present disclosure, wherein the sense strand is selected from SEQ ID NO: 156, and the antisense strand is selected from SEQ ID NO: 210.

The present disclosure also provides an siRNA conjugate or a dsRNA, which comprises any siRNA described above and a targeting ligand linked to the siRNA.
In some embodiments, the targeting ligand comprises a targeting moiety.
In some embodiments, the siRNA and the targeting ligand are linked covalently or non-covalently.
In some embodiments, the targeting ligand targets the liver. In some embodiments, the targeting ligand binds to an asialoglycoprotein receptor (ASGPR). In some embodiments, the targeting ligand includes a galactose cluster or a galactose derivative cluster. The galactose derivative is selected from the group consisting of N-acetyl-galactosamine, N-trifluoroacetyl-galactosamine, N-propionyl-galactosamine, N-n-butyrylgalactosamine and N-isobutyrylgalactosamine.
In some embodiments, the targeting ligand is linked to the 3' end of the sense strand of the siRNA.
In some embodiments, to promote entry of the siRNA into a cell, a lipophilic group such as cholesterol can be introduced into an end of the sense strand of the siRNA. The lipophilic group is covalently bonded to the siRNA, for example, cholesterol, lipoprotein, vitamin E, etc., are introduced to the end to facilitate going through the cell membrane consisting of a lipid bilayer and interacting with the mRNA in the cell. Meanwhile, the siRNA can also be modified by non-covalent bonding, for example, bonding to a phospholipid molecule, a polypeptide, a cationic polymer, etc, by a hydrophobic bond or an ionic bond to increase stability and biological activity.
In some embodiments, the targeting ligand is linked to an end of the siRNA by a phosphoester group, a phosphorothioate group or a phosphonic acid group. In some embodiments, the targeting ligand is indirectly linked to an end of the siRNA by a phosphoester group, a phosphorothioate group or a phosphonic acid group. In some embodiments, the targeting ligand is directly linked to an end of the siRNA by a phosphoester group, a phosphorothioate group or a phosphonic acid group. In some embodiments, the targeting ligand is directly linked to an end of the siRNA by a phosphoester group or a phosphorothioate group. In some embodiments, the targeting ligand is directly linked to the 3' end of the sense strand of the siRNA by a phosphoester group or a phosphorothioate group.
In some embodiments, the targeting ligand assists in directing the delivery of the therapeutic agent linked thereto to the desired target position. In some cases, the targeting moiety can bind to a cell or cellular receptor and initiate endocytosis to promote entry of the therapeutic agent into the cell. The targeting moiety can comprise a compound with affinity for a cellular receptor or a cell surface molecule. Various targeting ligands comprising targeting moieties can be linked to therapeutic agents and other compounds to target the agents at cells and specific cellular receptors.
In some embodiments, the types of the targeting moieties include carbohydrates, cholesterol and cholesterol groups, or steroids. Targeting moieties that can bind to cellular receptors include saccharides such as galactose, galactose derivatives (e.g., N-acetyl-galactosamine, N-trifluoroacetylgalactosamine, N-propionylgalactosamine, N-n-butyrylgalactosamine and N-isobutyrylgalactosamine), mannose, and mannose derivatives.
It is known that targeting moieties that bind to asialoglycoprotein receptors (ASGPR) can be particularly used for directing the delivery of oligomeric compounds (e.g., an siRNA or a conjugate thereof or a dsRNA) to the liver. Asialoglycoprotein receptors are extensively expressed on liver cells. The targeting moieties of cellular receptors targeting ASCPR include galactose and galactose derivatives. Specifically, clusters of galactose derivatives, including clusters consisting of 2, 3, 4 or more than 4 N-acetyl-galactosamines (GalNAc or NAG), can promote the uptake of certain compounds by hepatocytes. The GalNAc cluster coupled to the oligomeric compound is used for directing the therapeutic agent to the liver, and the N-acetyl-galactosamine saccharide can bind to the asialoglycoprotein receptors on the liver cell surface. It is believed that the binding to the asialoglycoprotein receptors will initiate receptor-mediated endocytosis, thereby promoting entry of the therapeutic agent into the interior of the cell.
In some embodiments, the targeting ligand can comprise 2, 3, 4 or more than 4 targeting moieties. In some embodiments, the targeting ligand disclosed herein can comprise 1, 2, 3, 4 or more than 4 targeting moieties linked to the branching group by $L_2$.
In some embodiments, the targeting ligand is in the form of a galactose cluster.
In some embodiments, each targeting moiety is independently a galactosamine derivative, which is N-acetyl-galactosamine. Other sugars that can be used as targeting moieties and that have affinity for asialoglycoprotein receptors can be selected from the group consisting of galactose, galactosamine, N-formyl-galactosamine, N-propionyl-galactosamine, N-n-butyryl-galactosamine, N-isobutyryl-galactosamine, etc.
In some embodiments, the targeting ligand in the present disclosure comprises N-acetylgalactosamine as a targeting moiety,

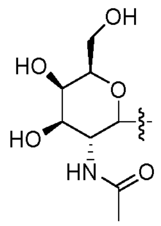

(H-2)

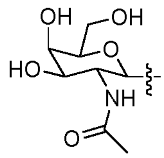

(H-2)

In some embodiments, the targeting ligand comprises three terminal galactosamines or galactosamine derivatives (such as N-acetyl-galactosamine), each of which has affinity for asialoglycoprotein receptors. In some embodiments, the targeting ligand comprises three terminal N-acetyl-galactosamine (GalNAc or NAG) as targeting moieties.

In some embodiments, the targeting ligand comprises four terminal galactosamines or galactosamine derivatives (such as N-acetyl-galactosamine), each of which has affinity for asialoglycoprotein receptors. In some embodiments, the targeting ligand comprises four terminal N-acetyl-galactosamine (GalNAc or NAG) as targeting moieties.

The terms commonly used in the art when referring to three terminal N-acetyl-galactosamines include tri-antennary, tri-valent and trimer.

The terms commonly used in the art when referring to four terminal N-acetyl-galactosamines include tetra-antennary, tetra-valent and tetramer.

In some embodiments, the targeting ligand is selected from the group consisting of a compound of formula (II) or a pharmaceutically acceptable salt thereof,

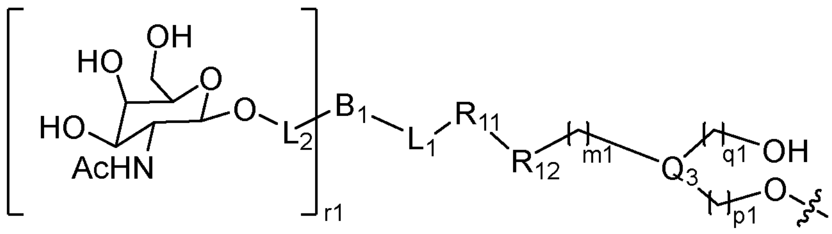

(II)

wherein $L_1$ is a $C_1$-$C_{30}$ alkyl chain, or comprises a $C_1$-$C_{30}$ alkyl chain interrupted by one or more oxygen, sulfur or nitrogen atoms or C=O;

$R_{11}$ and $R_{12}$ are independently chemical bonds, $NR_{16}$, C=O or -OC(=O)-;

$Q_3$ is

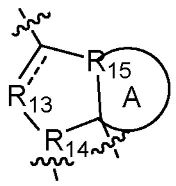

or

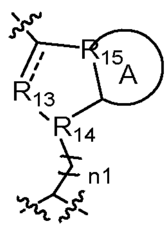

;

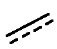

is a single bond or a double bond, and when

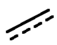

is a single bond, $R_{13}$ is independently $CR_{17}R_{18}$, $NR_{16}$, O or S;

when

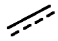

is a double bond, $R_{13}$ is independently $CR_{19}$ or N;

$R_{14}$ is independently $CR_{19}$ or N;

ring A is cycloalkyl, heterocycloalkyl, aryl or heteroaryl which is present or absent, and when ring A is present, $R_{15}$ is independently $CR_{19}$ or N; when ring A is absent, $R_{15}$ is independently $CR_{17}R_{18}$, $NR_{16}$ or O;

$R_{16}$ and $R_{19}$ are independently hydrogen, deuterium, alkyl, alkoxy, cycloalkyl, heterocycloalkyl, aryl, heteroaryl, SR', S(=O)R', $S(=O)_2R'$, $S(=O)_2NR'(R'')$, NR'(R"), C(=O)R', C(=O)OR' or C(=O)NR'(R"), wherein the alkyl, alkoxy, cycloalkyl, heterocycloalkyl, aryl or heteroaryl is optionally substituted with one or more groups selected from the group consisting of halogen, hydroxy, oxo, nitro, cyano, $C_{1-6}$ alkyl, $C_{1-6}$ alkoxy, $C_{3-7}$ cycloalkyl, 3- to 12-membered heterocycloalkyl, 6- to 12-membered aryl, 5- to 12-membered heteroaryl, SR', S(=O)R', $S(=O)_2R'$, $S(=O)_2NR'(R'')$, NR'(R"), C(=O)R', C(=O)OR' and C(=O)NR'(R");

$R_{17}$ and $R_{18}$ are independently hydrogen, deuterium, alkyl, alkoxy, cycloalkyl, heterocycloalkyl, aryl, heteroaryl, SR', S(=O)R', $S(=O)_2R'$, $S(=O)_2NR'(R'')$, NR'(R"), C(=O)R', C(=O)OR' or C(=O)NR'(R"), wherein the alkyl, alkoxy, cycloalkyl, heterocycloalkyl, aryl or heteroaryl is optionally substituted with one or more groups selected from the group consisting of halogen, hydroxy, oxo, nitro, cyano, $C_{1-6}$ alkyl, $C_{1-6}$ alkoxy, $C_{3-7}$ cycloalkyl, 3- to 12-membered heterocycloalkyl, 6- to 12-membered aryl, 5- to 12-membered heteroaryl, SR', S(=O)R', $S(=O)_2R'$, $S(=O)_2NR'(R'')$, NR'(R"), C(=O)R', C(=O)OR' and C(=O)NR'(R");

R' and R" are independently hydrogen, deuterium, hydroxy, alkyl, alkoxy, cycloalkyl, heterocycloalkyl, aryl or heteroaryl, wherein the alkyl, alkoxy, cycloalkyl, heterocycloalkyl, aryl or heteroaryl is optionally substituted with one or more substituents selected from the group consisting of halogen, hydroxy, oxo, nitro and cyano;

m1, n1, p1 and q1 are independently 0, 1, 2, 3 or 4;

B1 is

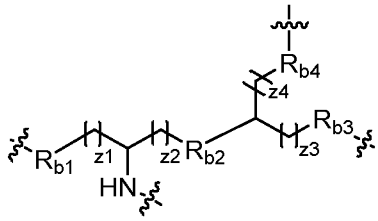

or

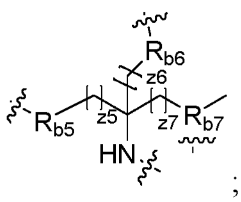

;

$R_{b1}$, $R_{b2}$, $R_{b3}$, $R_{b4}$, $R_{b5}$, $R_{b6}$ and $R_{b7}$ are independently -C(=O)-, -NHC(=O)-, -C(=O)O-, - $C(=O)-(CH_2)_{z8}-O-$ or $-NHC(=O)-(CH_2)_{z9}-O-$;

z1, z2, z3, z4, z5, z6, z7, z8 and z9 are independently integers of 0-10;

$L_2$ is a $C_1$-$C_{30}$ alkyl chain, or comprises a $C_1$-$C_{30}$ alkyl chain interrupted by one or more oxygen, sulfur or nitrogen atoms or C=O;

r1 is an integer of 1-10.

In some embodiments, $L_1$ is a $C_1$-$C_{30}$ alkyl chain, or comprises a $C_1$-$C_{30}$ alkyl chain interrupted by one or more oxygen, sulfur or nitrogen atoms or C=O;

$R_{11}$ and $R_{12}$ are independently chemical bonds, $NR_{16}$ or C=O;

$Q_3$ is

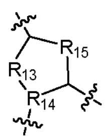

;

$R_{13}$ is $CR_{17}R_{18}$, $NR_{16}$, O or S;

$R_{14}$ is $CR_{19}$;

$R_{15}$ is independently $CR_{17}R_{18}$, $NR_{16}$ or O;

$R_{16}$ to $R_{19}$ are independently hydrogen, deuterium or alkyl;

m1, p1 and q1 are independently 0, 1, 2, 3 or 4;

B1 is

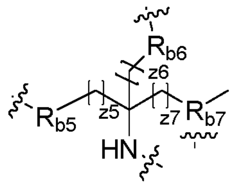

;

$R_{b5}$, $R_{b6}$ and $R_{b7}$ are independently -C(=O)-, -NHC(=O)-, -C(=O)O-, $-C(=O)-(CH_2)_{z8}-O-$ or $-NHC(=O)-(CH_2)_{z9}-O-$;
z5, z6, z7, z8 and z9 are independently integers of 0-10;
$L_2$ is a $C_1$-$C_{30}$ alkyl chain, or comprises a $C_1$-$C_{30}$ alkyl chain interrupted by one or more oxygen, sulfur or nitrogen atoms or C=O;
r1 is an integer of 1-10.

In some embodiments, $L_1$ is $-(CH_2)_{j11}-C(=O)-(CH_2)_{j12}-$;

$R_{11}$ and $R_{12}$ are independently chemical bonds, $NR_{16}$ or C=O;
$R_{16}$ is hydrogen or $C_{1-6}$ alkyl;
$Q_3$ is

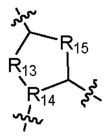

;

$R_{13}$ is $CR_{17}R_{18}$ or O;
$R_{14}$ is $CR_{19}$;
$R_{15}$ is independently $CR_{17}R_{18}$ or O;
$R_{17}$ to $R_{19}$ are independently hydrogen or alkyl;
m1, p1 and q1 are independently 0 or 1;
B1 is

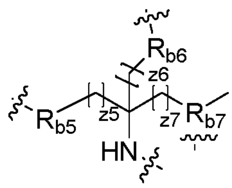

;

$R_{b5}$, $R_{b6}$ and $R_{b7}$ are independently $-C(=O)-(CH_2)_{z8}-O-$ or $-NHC(=O)-(CH_2)_{z9}-O-$; z8 and z9 are independently integers of 0-10;
$L_2$ is $-(CH_2)_{j15}-(OCH_2CH_2)_{1-4}-(CH_2)_{j16}-$ or

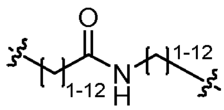

;

j 15 and j 16 are independently integers of 0-4;
r1 is 3, 4, 5 or 6.

In some embodiments, $L_1$ can be $L_3$ or $L_3$-$R_{110}$-$R_{111}$-$L_3$, wherein $L_3$ is independently a $C_1$-$C_{12}$ alkyl chain, $-(CH_2)_{j11}-C(=O)-(CH_2)_{j12}-$ or $-(CH_2)_{j13}-(CH_2CH_2O)_{1-4}-(CH_2)_{j14}-$; $R_{110}$ and $R_{111}$ are independently chemical bonds, $-NR_{112}-$, -C(=O)- or -OC(=O)-; $R_{112}$ is hydrogen or $C_1$-$C_{12}$ alkyl; j 11, j 12, j 13 and j 14 are independently integers of

0-10. In some embodiments, j 11, j 12, j 13 and j 14 are independently integers of 0-2 or 4-10. In some embodiments, j 11, j 12, j 13 and j 14 are independently 0, 1, 2, 6, 7, 8, 9 or 10.

In some embodiments, $L_1$ can be $-(CH_2)_{j11}-C(=O)-(CH_2)_{j12}-$, wherein j 11 and j 12 are as defined in any of the preceding embodiments.

In some embodiments, $L_1$ can be

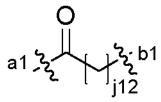
,

and j12 is as defined in any of the preceding embodiments, wherein the end a1 is attached to B1, and the end b1 is attached to $R_{11}$.

In some embodiments, $L_1$ can be

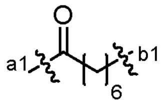
,
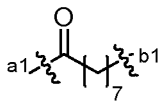
,
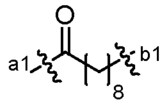
,
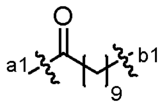
or
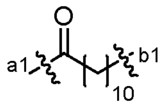
,

wherein the end a1 is attached to $B_1$, and the end b1 is attached to $R_{11}$.

In some embodiments, $R_{11}$ can be a chemical bond and $R_{12}$ can be C=O.

In some embodiments, $R_{11}$ can be a chemical bond and $R_{12}$ can be $NR_{16}$, wherein $R_{16}$ is as defined in any of the preceding embodiments.

In some embodiments, $R_{11}$ can be a chemical bond and $R_{12}$ can be -OC(=O)-.

In some embodiments, $R_{11}$ can be $NR_{16}$ and $R_{12}$ can be C=O, wherein $R_{16}$ is as defined in any of the preceding embodiments.

In some embodiments, $R_1$ can be $NR_{16}$ and $R_{12}$ can be -OC(=O)-, wherein $R_{16}$ is as defined in any of the preceding embodiments.

In some embodiments, $R_{12}$ can be $NR_{16}$ and $R_{11}$ can be C=O, wherein $R_{16}$ is as defined in any of the preceding embodiments.

In some embodiments, $R_{12}$ can be $NR_{16}$ and $R_{11}$ can be -OC(=O)-, wherein $R_{16}$ is as defined in any of the preceding embodiments.

In some embodiments, $R_{11}$ can be NH and $R_{12}$ can be C=O.

In some embodiments, $R_{12}$ can be NH and $R_{11}$ can be C=O.

In some embodiments, $R_{16}$ can be hydrogen or $C_{1-6}$ alkyl.

In some embodiments, $R_{16}$ can be hydrogen, methyl, ethyl, propyl or isopropyl.

In some embodiments, $R_{16}$ can be hydrogen.

In some embodiments, $R_{17}$ and $R_{18}$ can be hydrogen.

In some embodiments, $R_{19}$ can be hydrogen.

In some embodiments, when ring A is present, ring A can be $C_{6-12}$ aryl.

In some embodiments, ring A can be phenyl.

In some embodiments, m1 can be 0 or 1.

In some embodiments, m1 can be 3.

In some embodiments, n1 can be 0 or 1.

In some embodiments, p1 and q1 are independently 0 or 1.

In some embodiments, p1 = 1 and q1 = 1.

In some embodiments, p1 = 1 and q1 = 0.

In some embodiments, p1 = 0 and q1 = 1.

In some embodiments, p1 = 0 and q1 = 0.

In some embodiments, z1, z2, z3, z4, z5, z6, z7, z8 and z9 can independently be integers of 0-4. In some embodiments, z1, z2, z3, z4, z5, z6, z7, z8 and z9 can independently be integers of 0, 1 or 2.

In some embodiments, $B_1$ can be

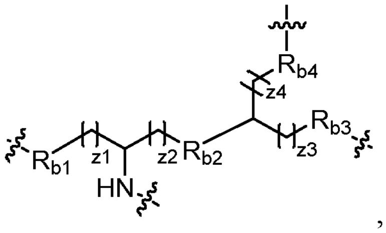

wherein $R_{b1}$, $R_{b2}$, $R_{b3}$ and $R_{b4}$ are independently -C(=O)- or -NHC(=O)-; the N atom is attached to $L_1$; z1, z2, z3 and z4 are as defined in any of the preceding embodiments.

In some embodiments, $B_1$ can be

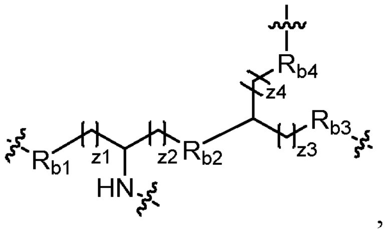

wherein $R_{b1}$, $R_{b2}$, $R_{b3}$ and $R_{b4}$ are independently -C(=O)- or -NHC(=O)-; the N atom is attached to $L_1$; $R_{b1}$, $R_{b3}$ and $R_{b4}$ are identical; z1, z2, z3 and z4 are as defined in any of the preceding embodiments.

In some embodiments, $B_1$ can be

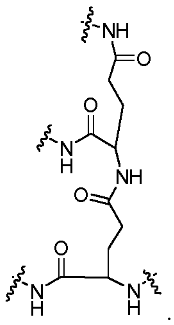

In some embodiments, $B_1$ can be

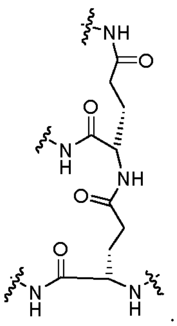

In some embodiments, $B_1$ can be

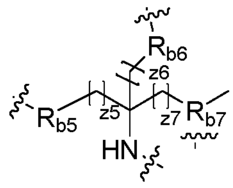

,

wherein $R_{b5}$, $R_{b6}$ and $R_{b7}$ are independently $-C(=O)-(CH_2)_{z8}-O-$ or $-NHC(=O)-(CH_2)_{z9}-O-$; the N atom is attached to $L_1$; z5, z6, z7, z8 and z9 are as defined in any of the preceding embodiments.

In some embodiments, $B_1$ can be

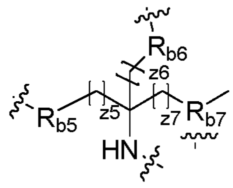

,

wherein $R_{b5}$, $R_{b6}$ and $R_{b7}$ are independently $-C(=O)-(CH_2)_{z8}-O-$ or $-NHC(=O)-(CH_2)_{z9}-O-$; the N atom is attached to $L_1$; $R_{b5}$, $R_{b6}$ and $R_{b7}$ are identical; z5, z6, z7, z8 and z9 are as defined in any of the preceding embodiments.

In some embodiments, $B_1$ can be

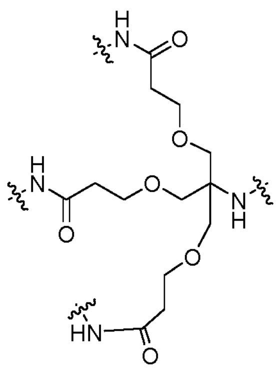

.

In some embodiments, $L_2$ can be $L_4$ or $L_4-R_{13}-R_{14}-L_4$, wherein $L_4$ is independently a $C_1$-$C_{12}$ alkyl chain or $-(CH_2)_{j15}-(OCH_2CH_2)_{1-4}-(CH_2)_{j16}-$; $R_{13}$ and $R_{14}$ are independently chemical bonds, $-NR_{115}-$, $-C(=O)-$ or $-OC(=O)-$; $R_{115}$ is independently hydrogen or $C_1$-$C_{12}$ alkyl; j 15 and j 16 are independently integers of 0-10. In some embodiments, j 15 and j 16 are independently integers of 0-6. In some embodiments, j 15 and j 16 are independently 0, 1, 2, 3 or 4.

In some embodiments, $L_2$ can be $-(CH_2)_{j15}-(OCH_2CH_2)_{1-4}-(CH_2)_{j16}-$, wherein j 15 and j 16 are as defined in any of the preceding embodiments.

In some embodiments, $L_2$ can be

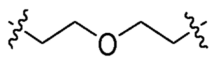

or

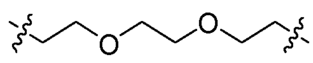

.

In some embodiments, $L_2$ can be

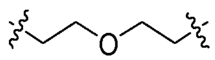

,

wherein one side is attached to the O atom, and the other side is attached to $B_1$.

In some embodiments, $L_2$ can be a $C_1$-$C_{12}$ alkyl chain.

In some embodiments, $L_2$ can be

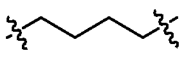, 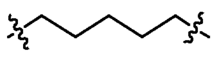, 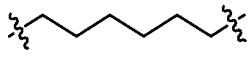,

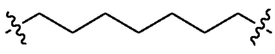 or 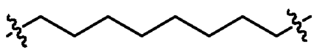.

In some embodiments, $L_2$ can be

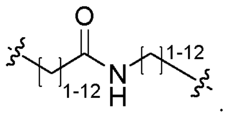.

In some embodiments, $L_2$ can be

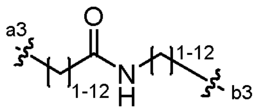.

In some embodiments, $L_2$ can be

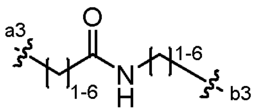.

In some embodiments, $L_2$ can be

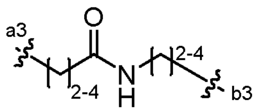,

wherein the end a3 is attached to the O atom, and the end b3 is attached to $B_1$.

In some embodiments, $L_2$ can be

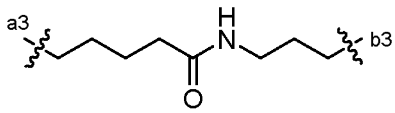,

wherein the end a3 is attached to the O atom, and the end b3 is attached to $B_1$.

In some embodiments, r1 can be 3, 4, 5 or 6. In some embodiments, r1 can be 3.

In some embodiments, $Q_3$ can be

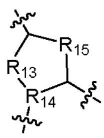 or 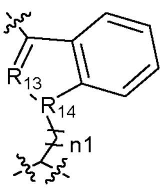.

In some embodiments, $Q_3$ can be

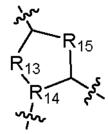
,

wherein $R_{13}$, $R_{14}$, $R_{15}$ and n1 are as defined in any of the preceding embodiments.

In some embodiments,

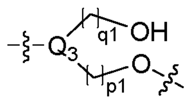

can be

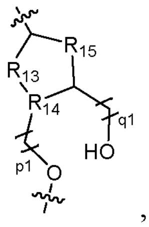
,

wherein $R_{13}$, $R_{14}$, $R_{15}$, p1 and q1 are as defined in any of the preceding embodiments.

In some embodiments,

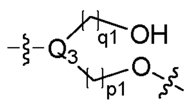

can be

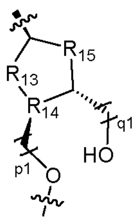
,
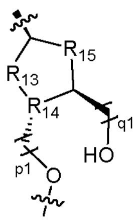
,
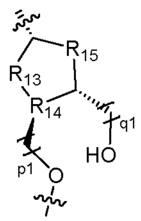

or

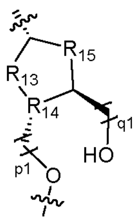
,

wherein $R_{13}$, $R_{14}$, $R_{15}$, p1 and q1 are as defined in any of the preceding embodiments.

In some embodiments,

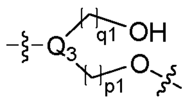

can be

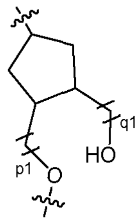 , 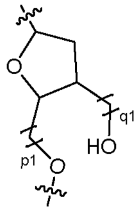 or 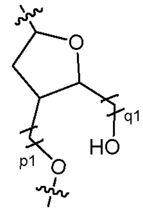 .

In some embodiments,

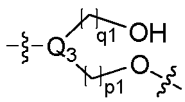

can be

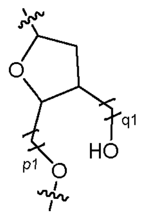 or 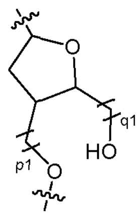 .

In some embodiments,

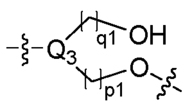

can be

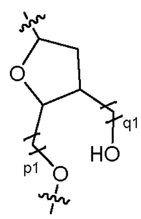 .

p1 and q1 are as defined in any of the preceding embodiments.

In some embodiments,

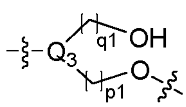

can be

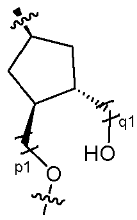, 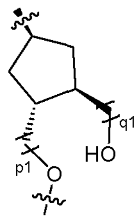, 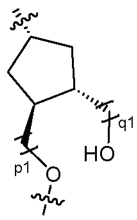,

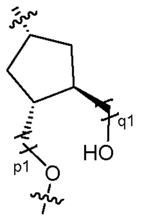, 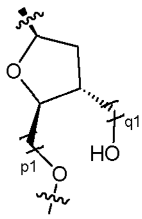, 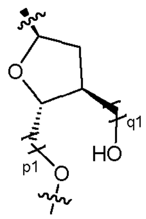, 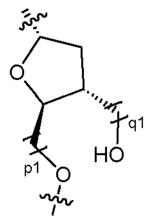, 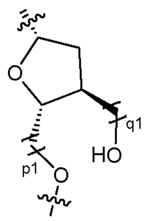, 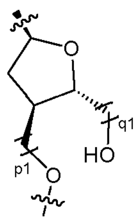,

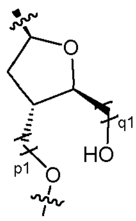, 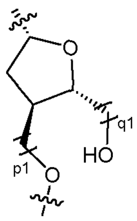 or 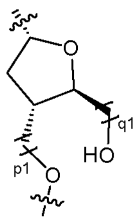.

In some embodiments,

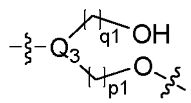

can be

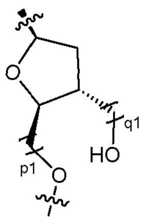, 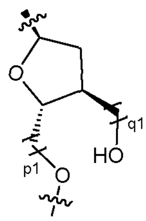, 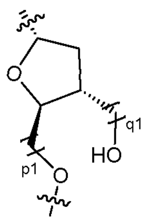,

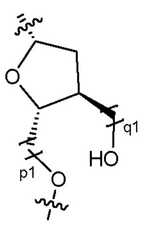, 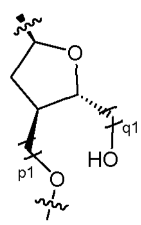, 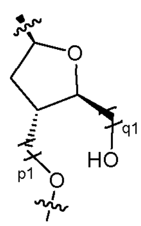, 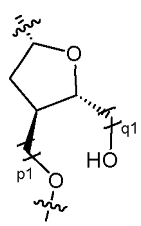 or 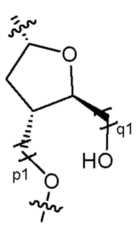.

In some embodiments,

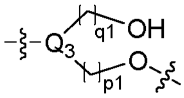

can be

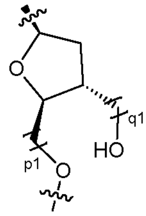
,
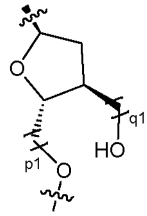
,
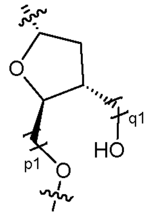

or

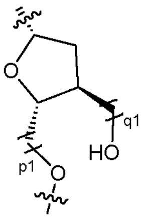
.

p1 and q1 are as defined in any of the preceding embodiments.

In some embodiments,

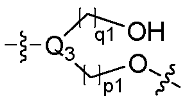

can be

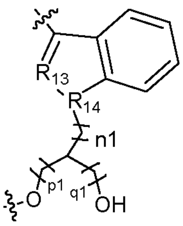
,

wherein $R_{13}$, $R_{14}$, n1, p1 and q1 are as defined in any of the preceding embodiments.

In some embodiments,

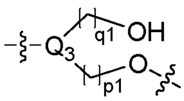

can be

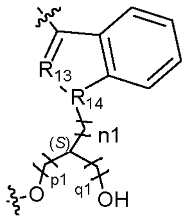 or 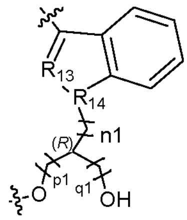 ,

wherein $R_{13}$, $R_{14}$, n1, p1 and q1 are as defined in any of the preceding embodiments.

In some embodiments,

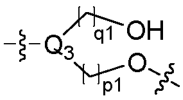

can be

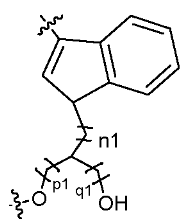 or 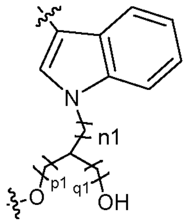 .

In some embodiments,

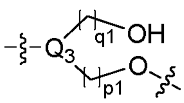

can be

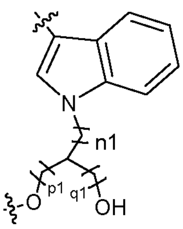 .

n1, p1 and q1 are as defined in any of the preceding embodiments.

In some embodiments,

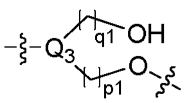

can be

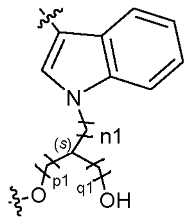

or

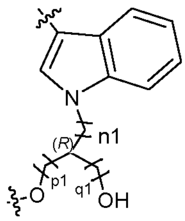

,

wherein n1, p1 and q1 are as defined in any of the preceding embodiments.

In some embodiments, the targeting ligand can be any of the following structures or pharmaceutically acceptable salts thereof:

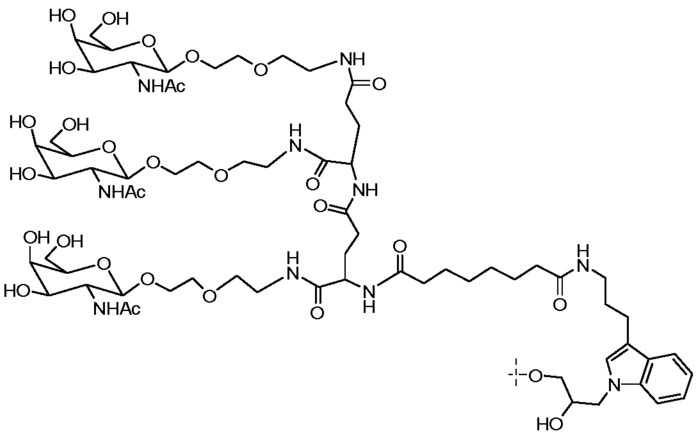

,

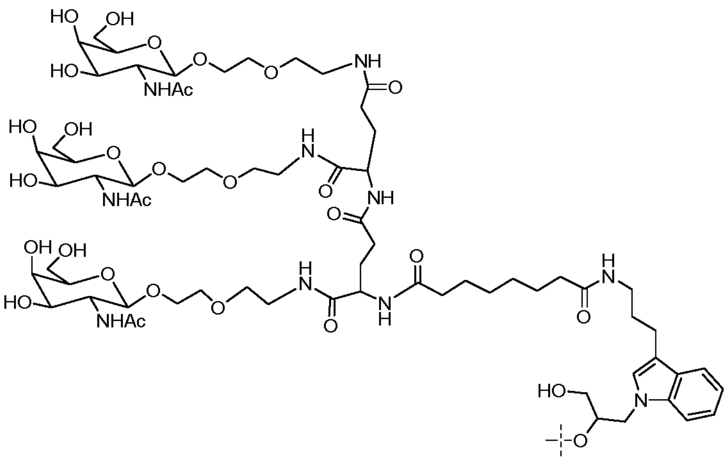

,

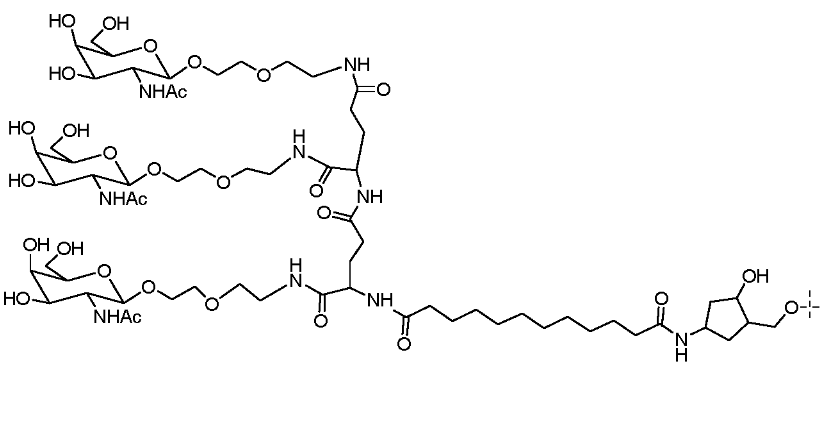

,

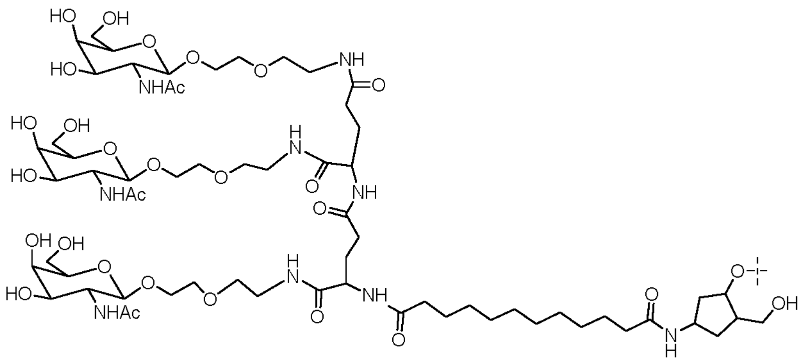

,

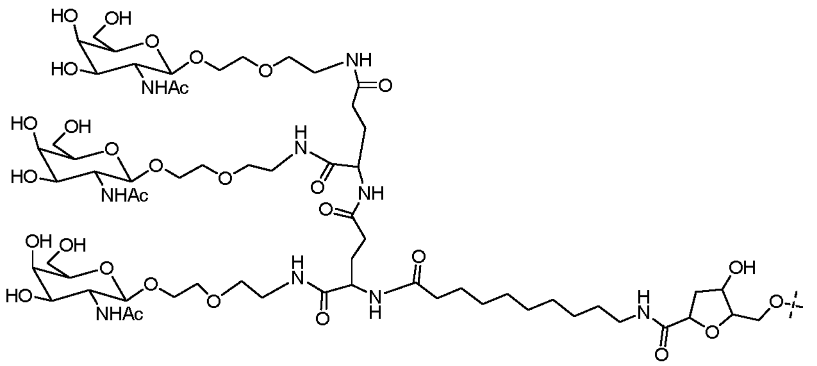

,

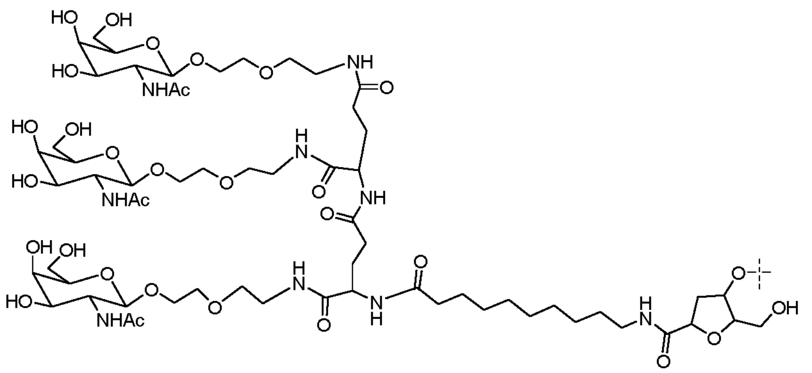

,

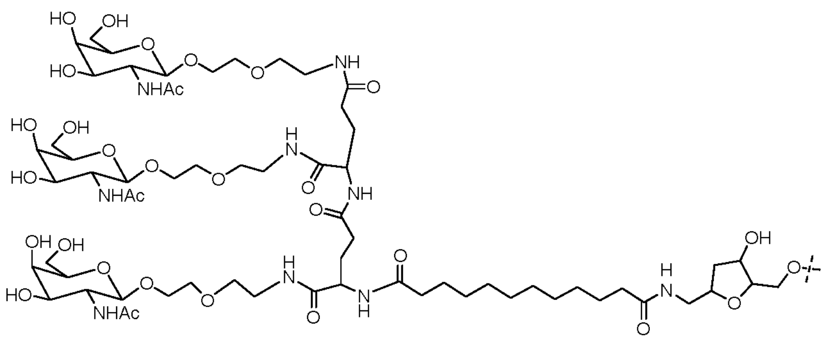

,

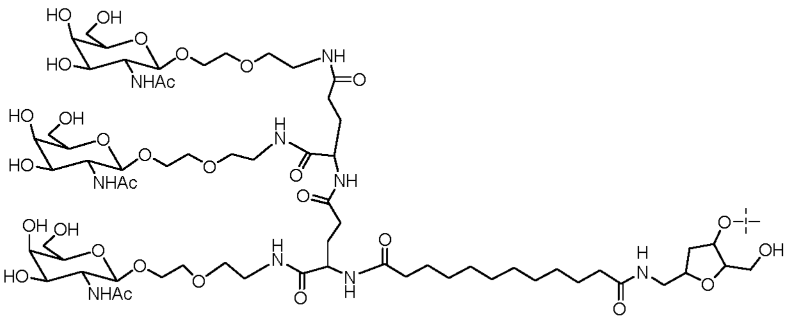

,

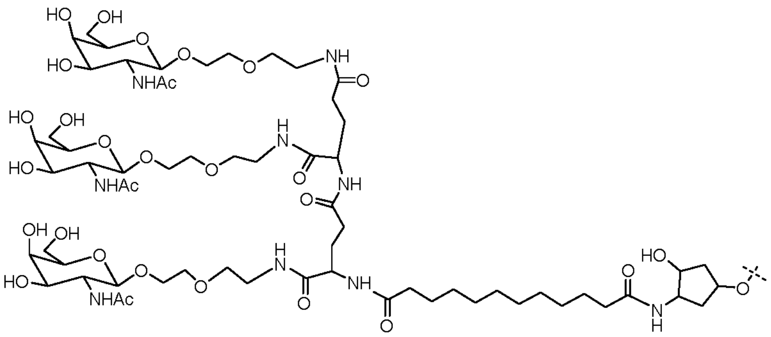

,

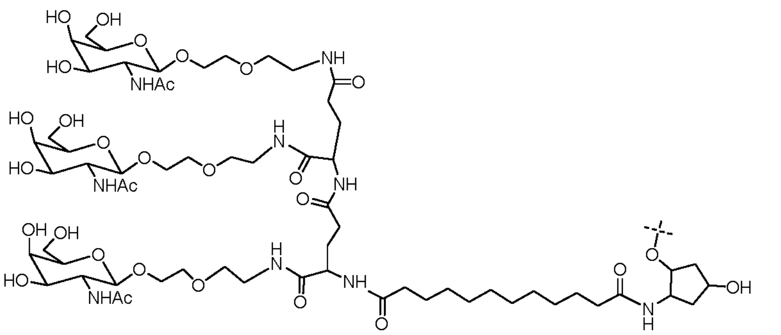

,

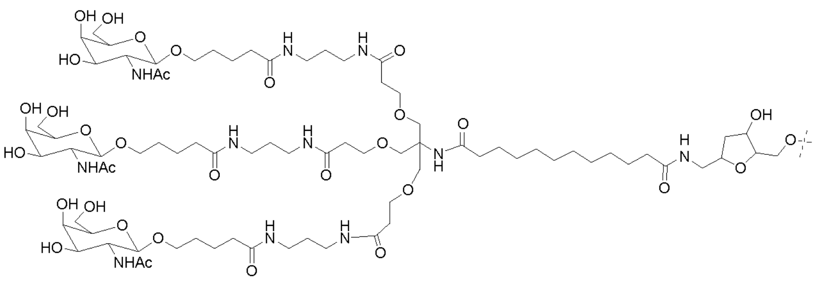

,

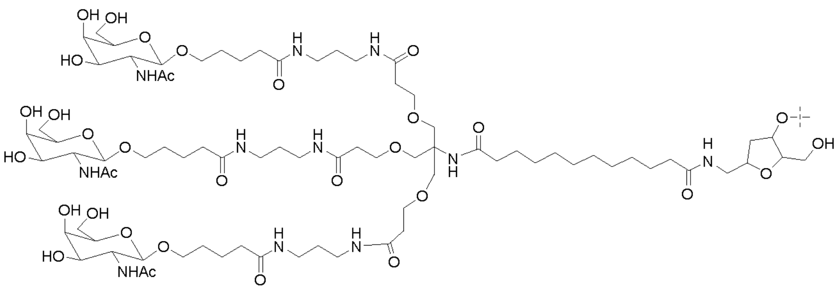

,

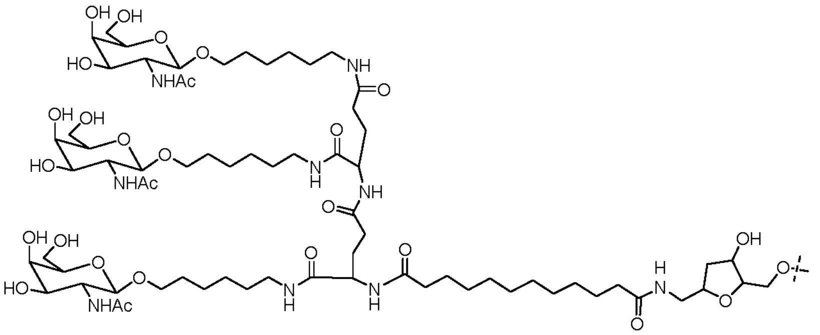

,

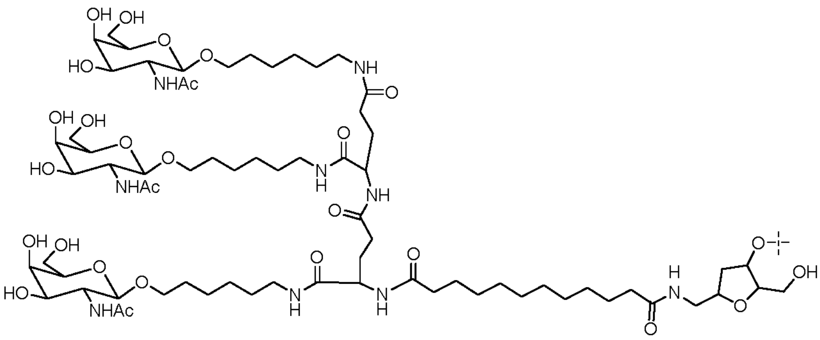

,

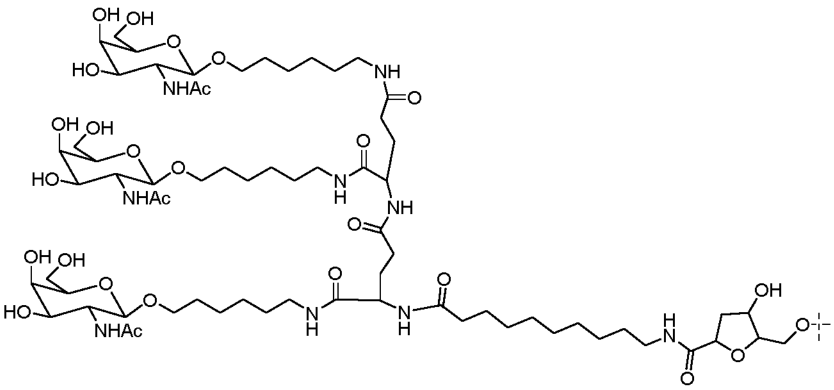

,

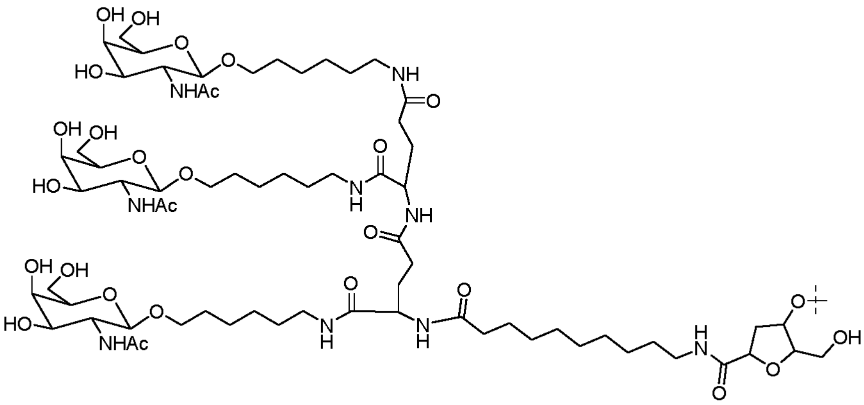

,

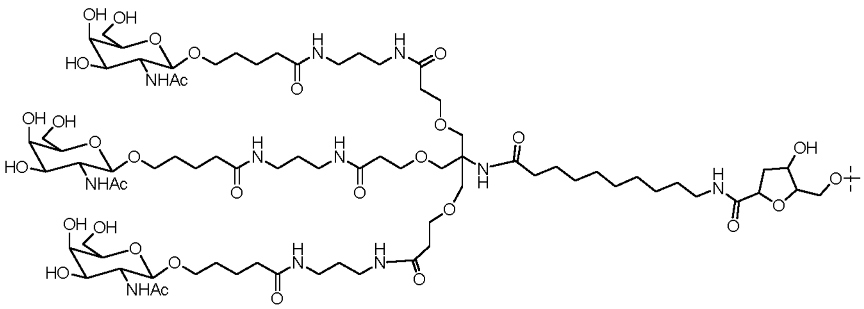

or

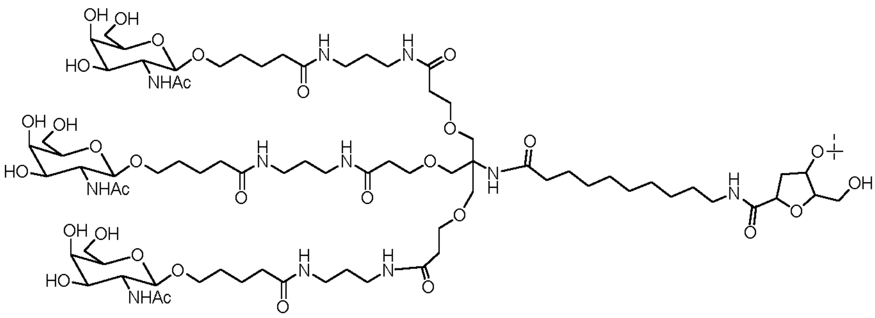

.

In some embodiments, the targeting ligand can be any of the following structures or pharmaceutically acceptable salts thereof:

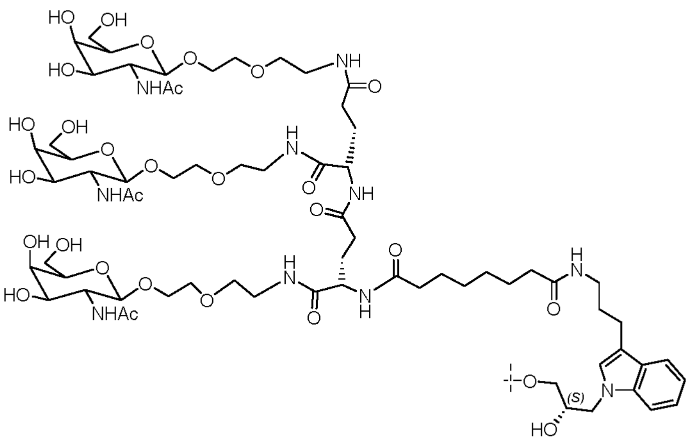

,

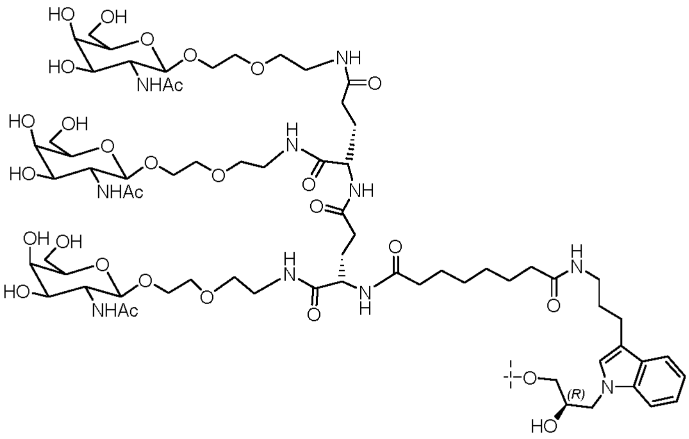

,

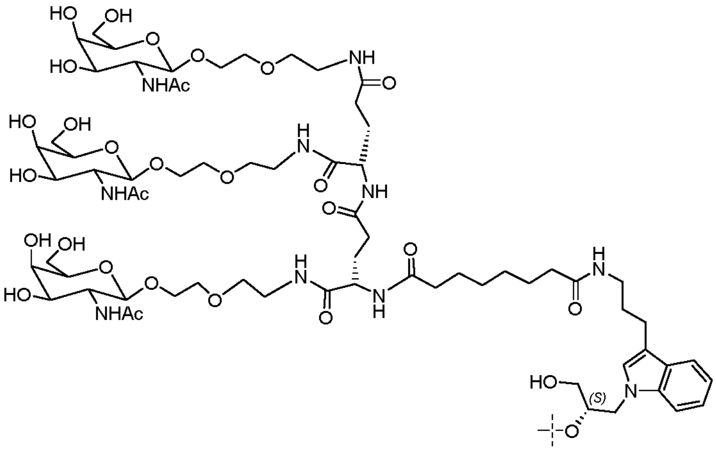

,

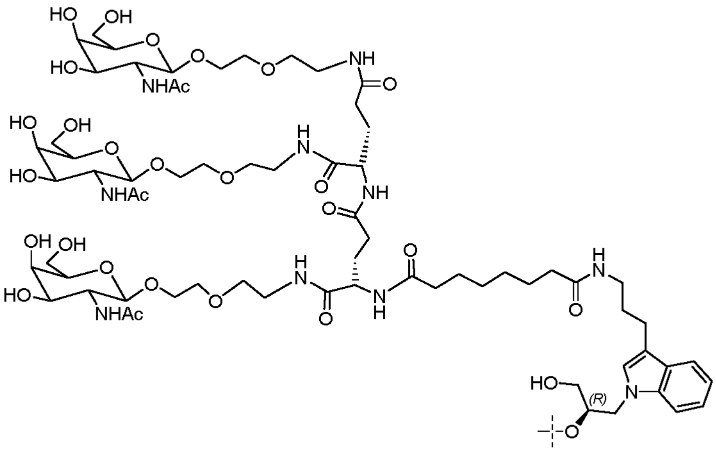

,

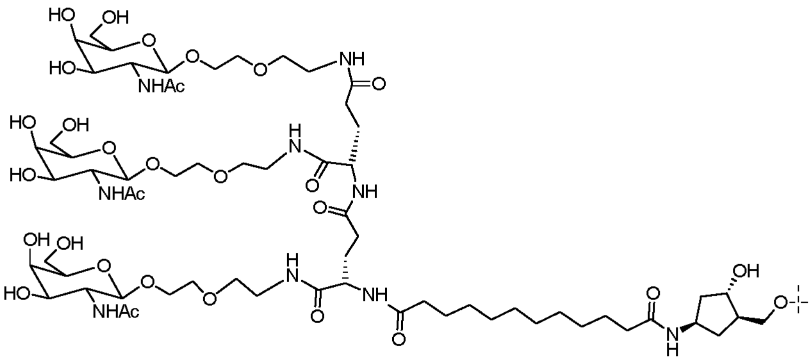

,

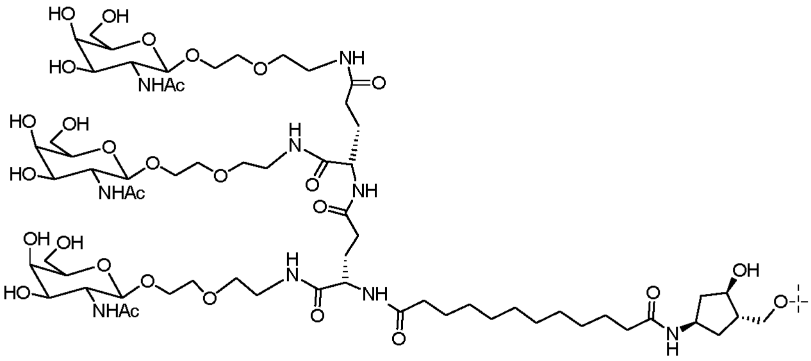

,

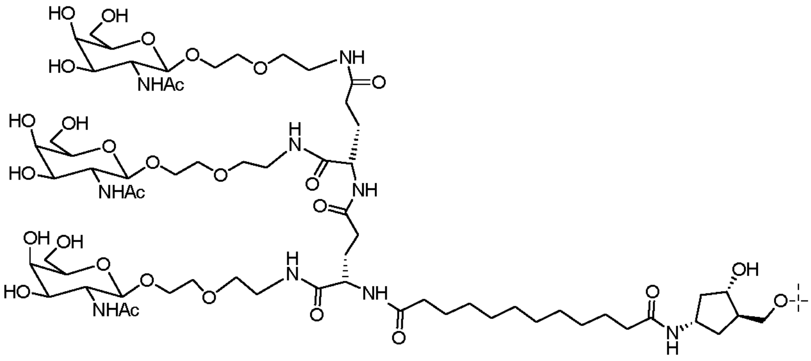

,

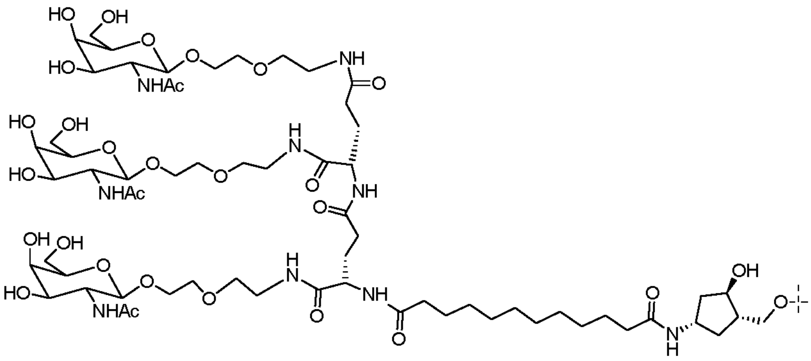

,

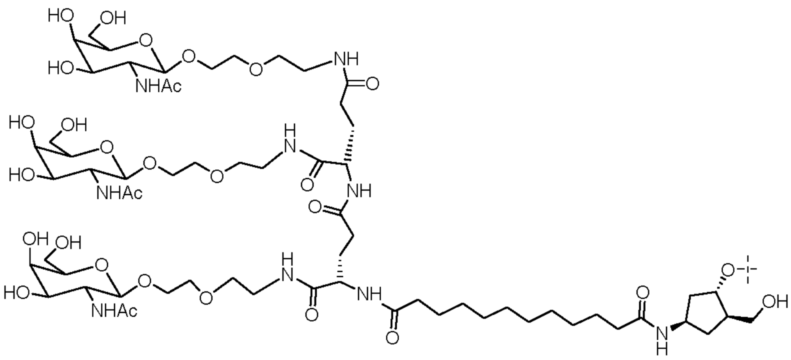

,

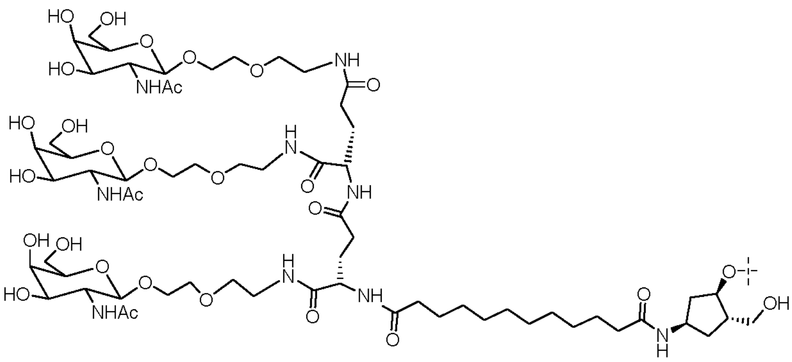

,

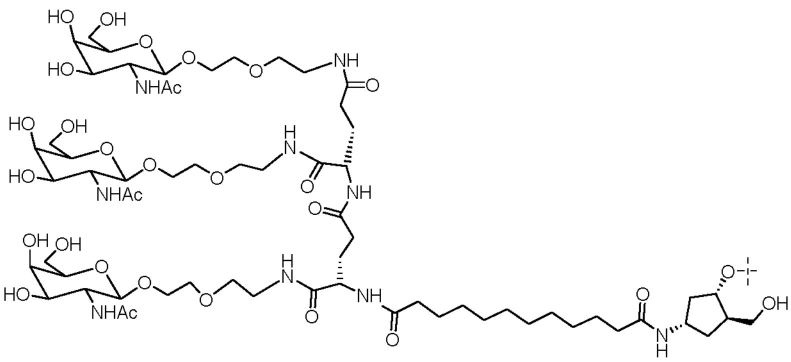

,

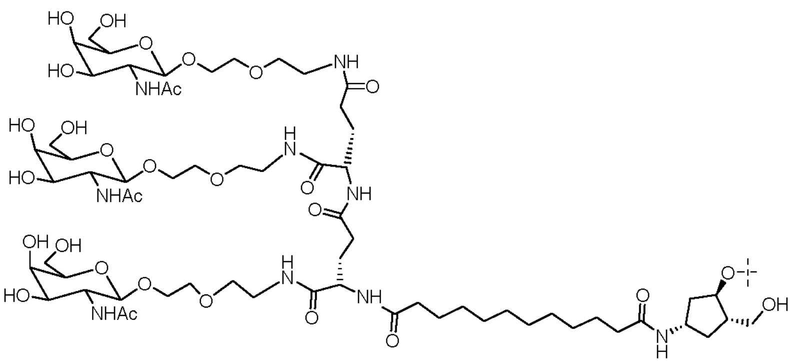

,

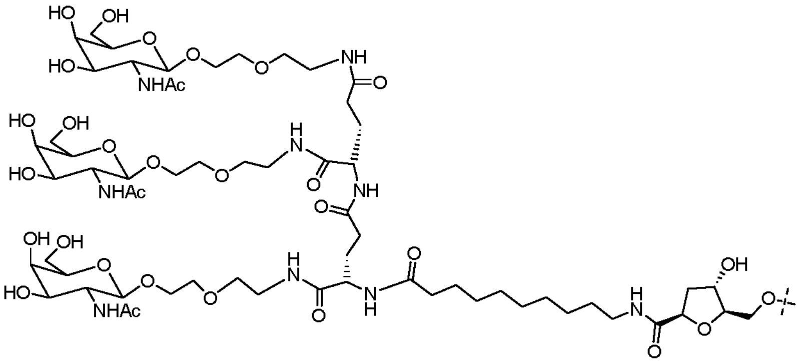

,

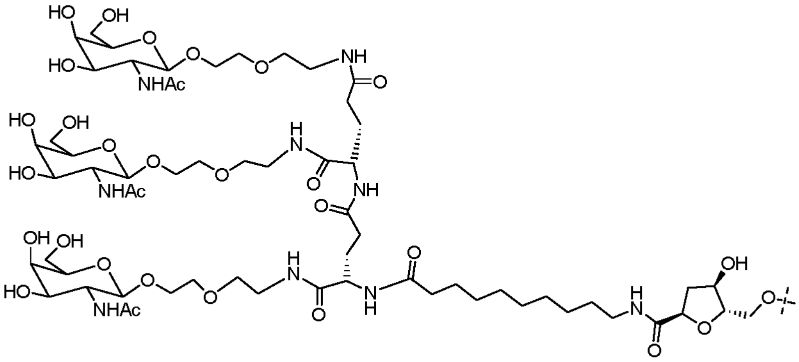

,

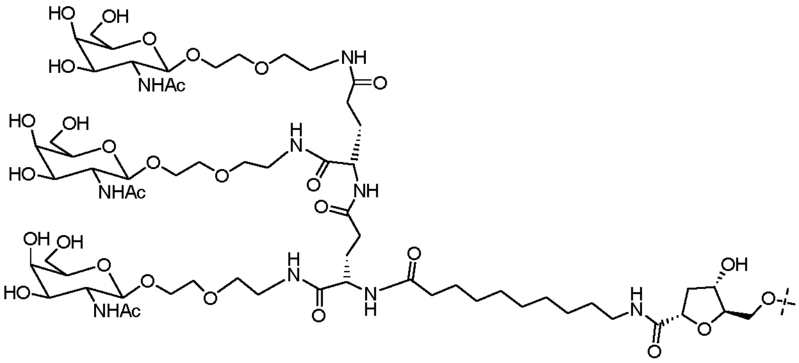

,

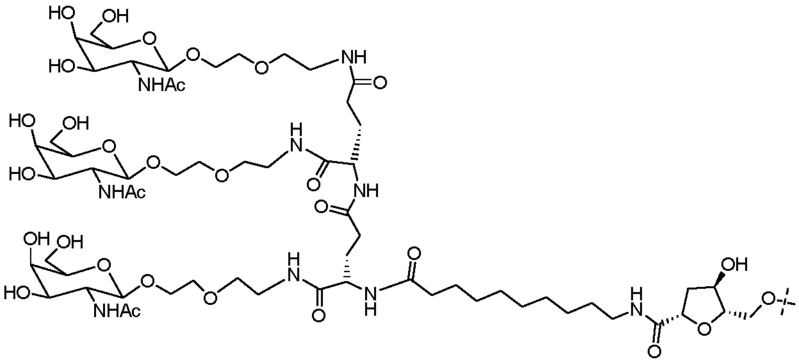

,

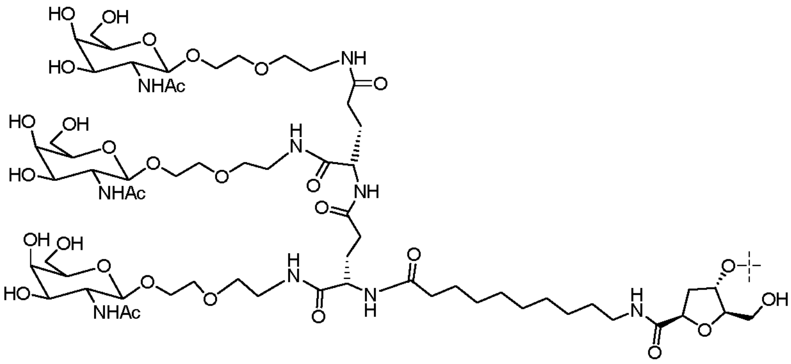

,

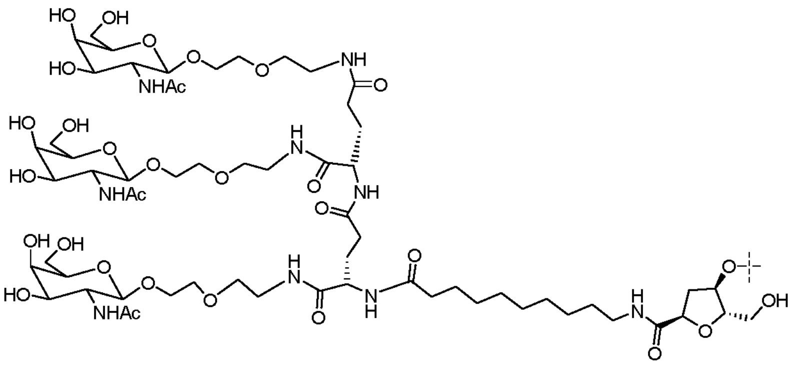

,

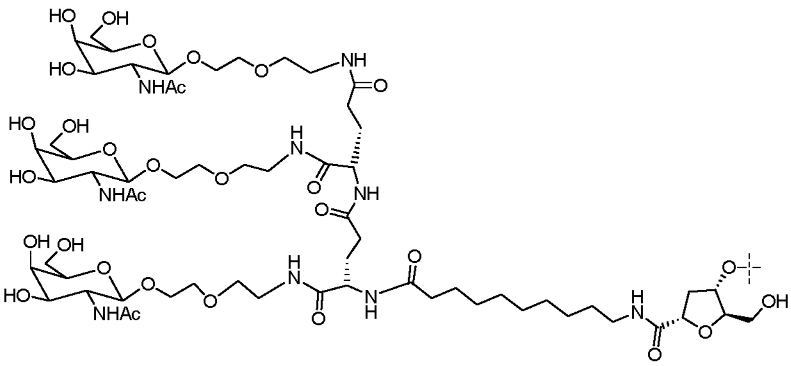

,

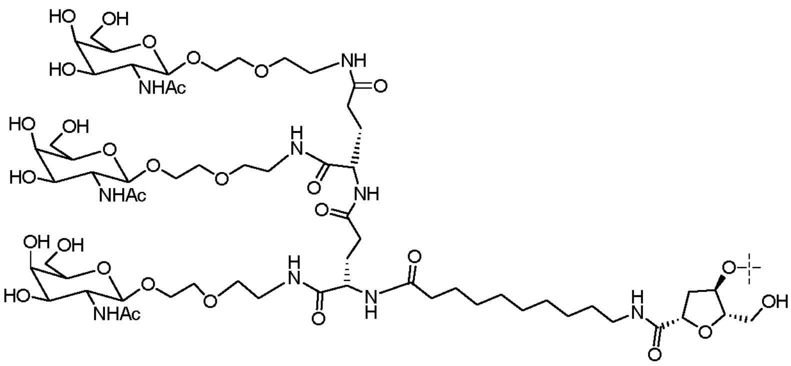

,

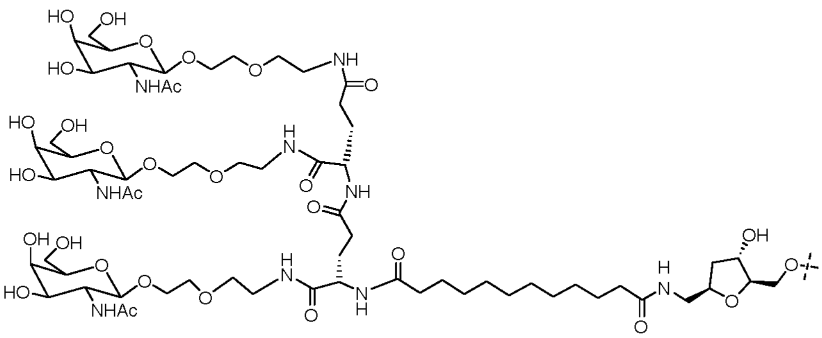

,

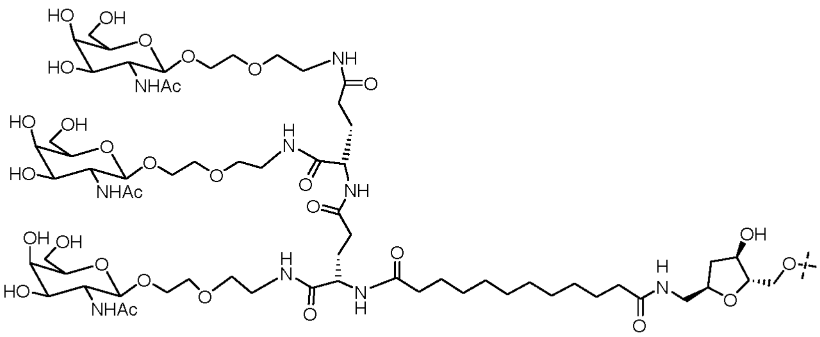

,

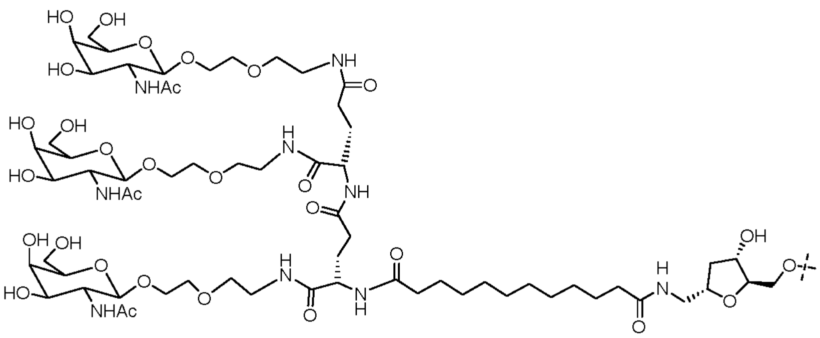

,

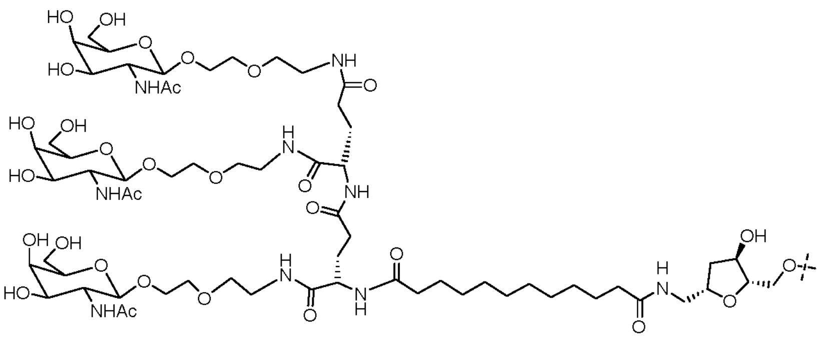

,

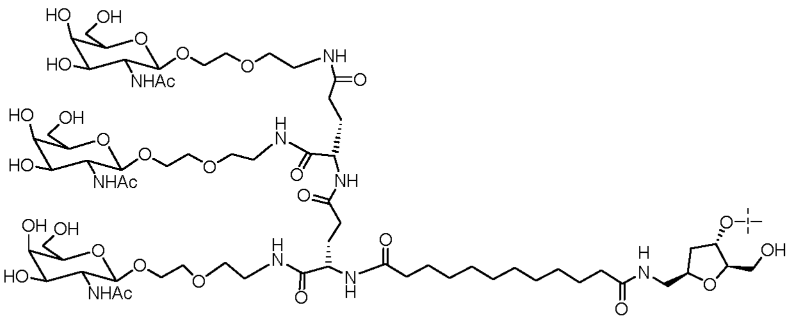

,

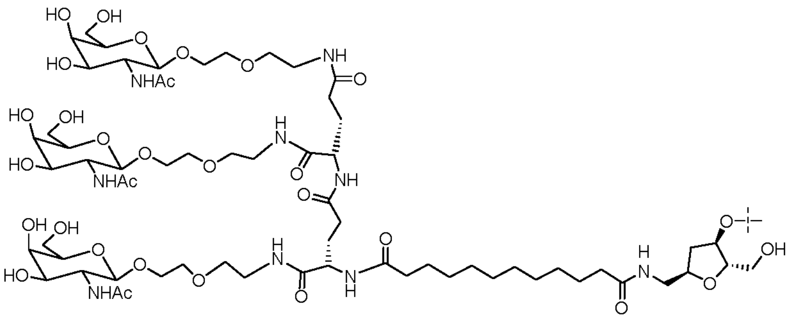

,

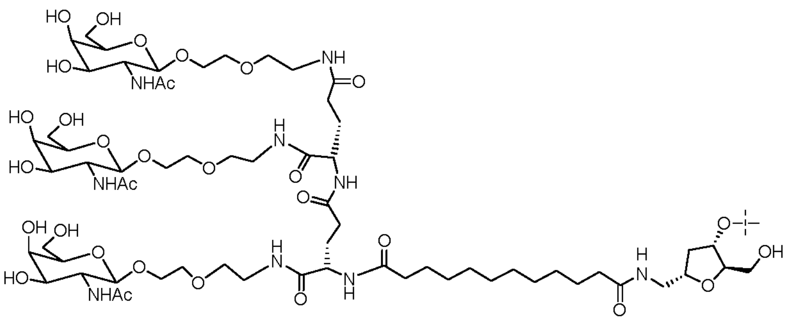

,

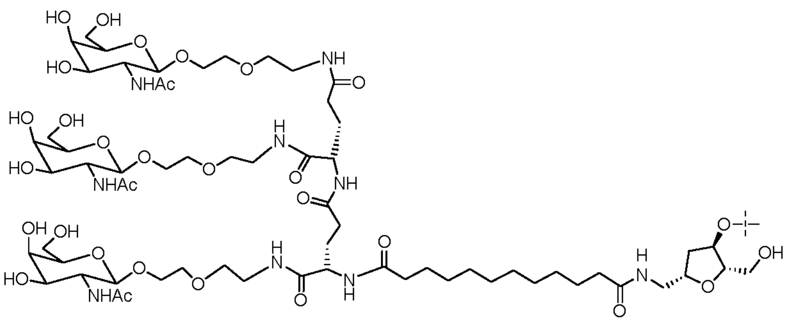

,

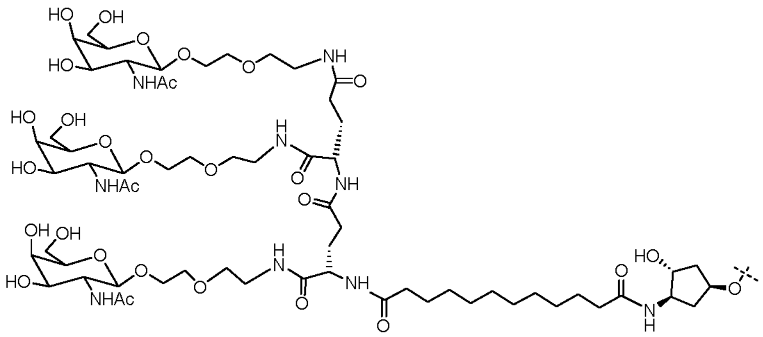

,

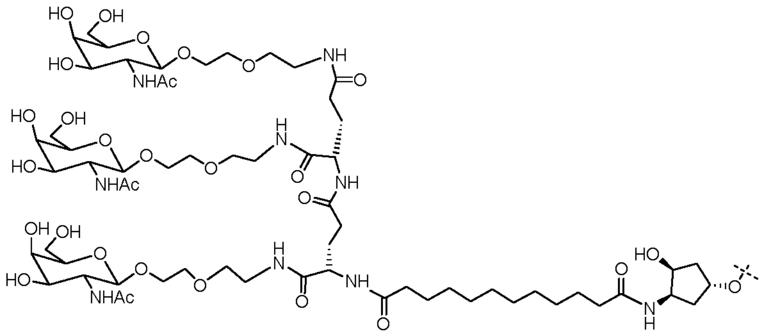

,

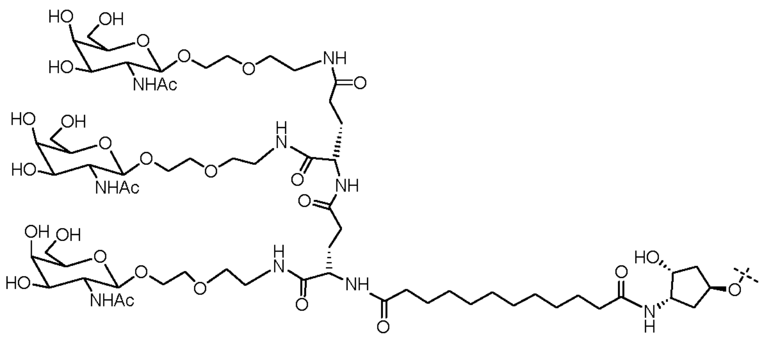

,

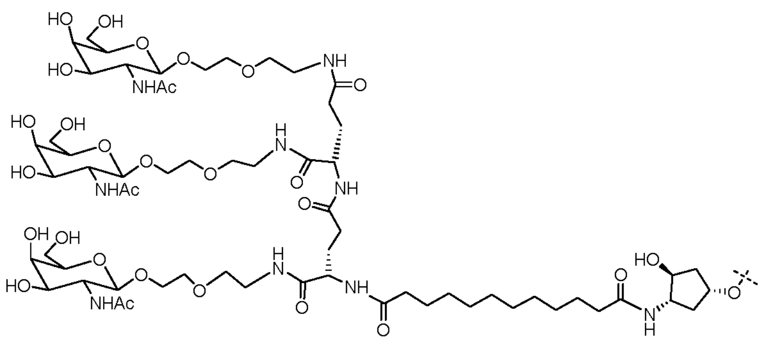

,

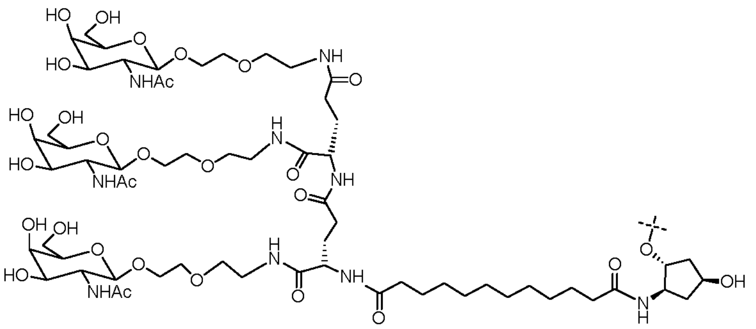

,

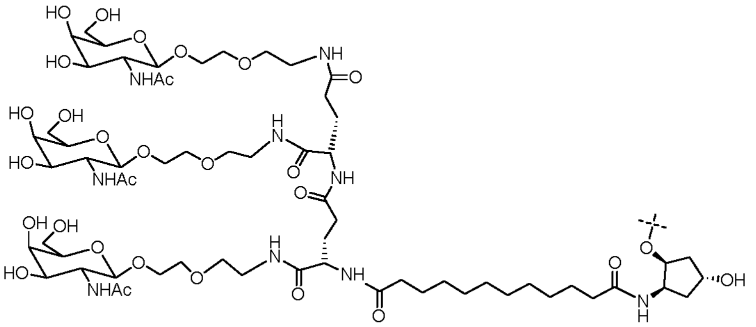

,

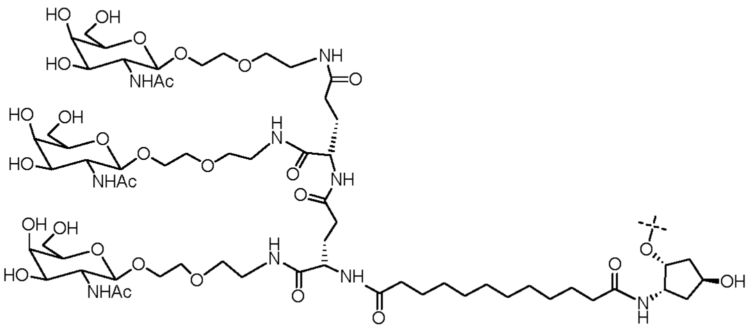

,

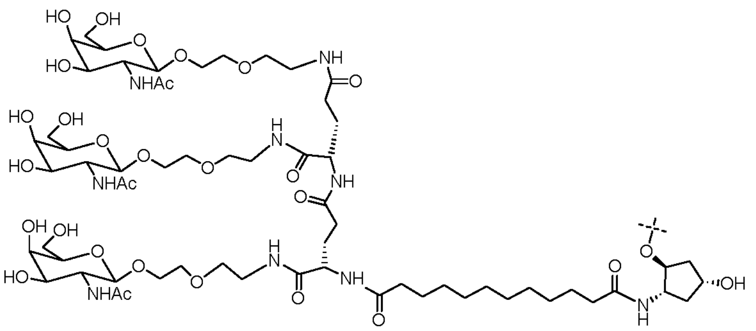

,

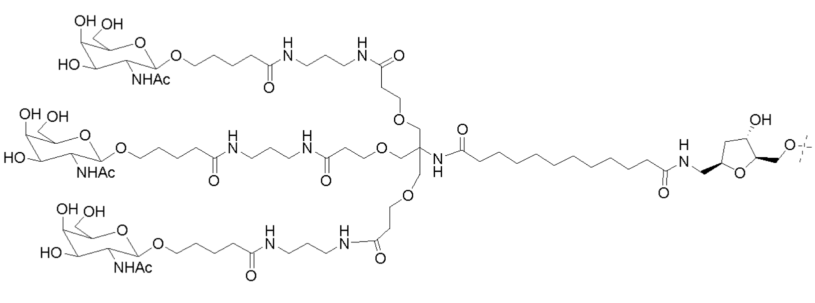

,

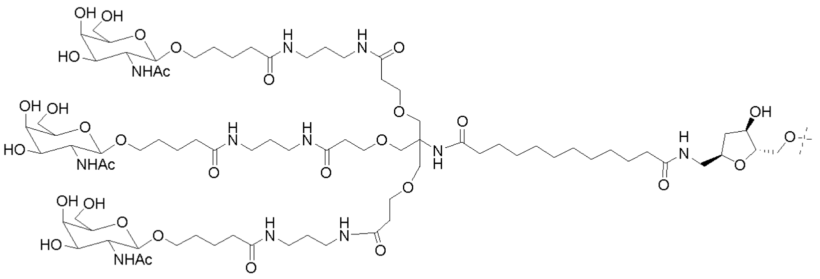

,

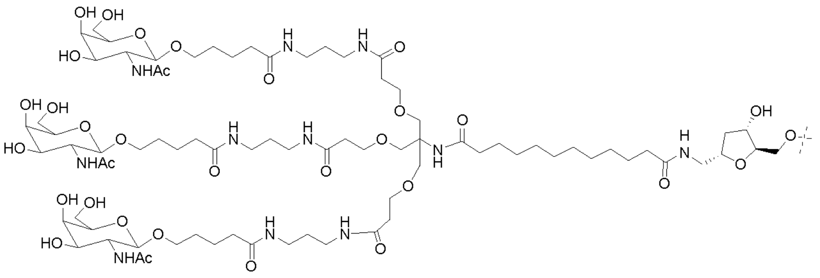

,

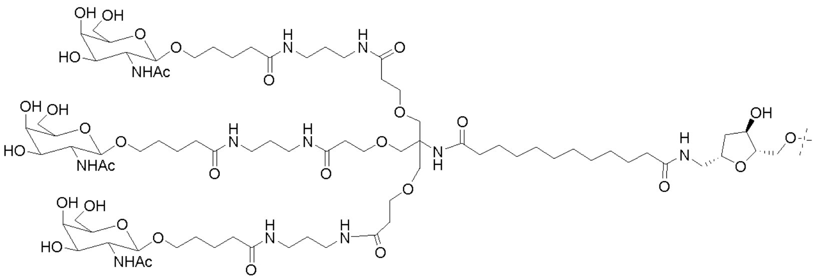

,

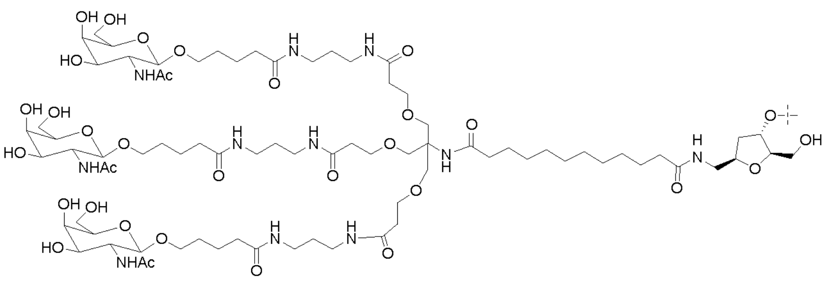

,

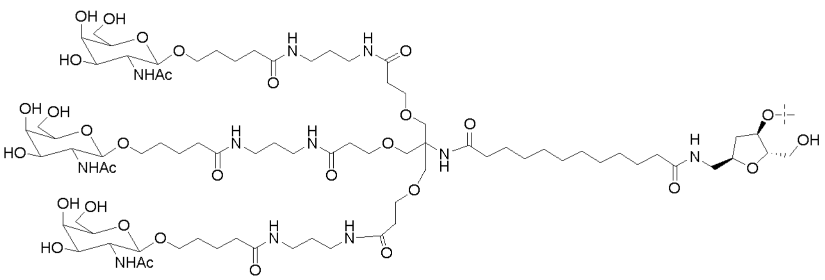

,

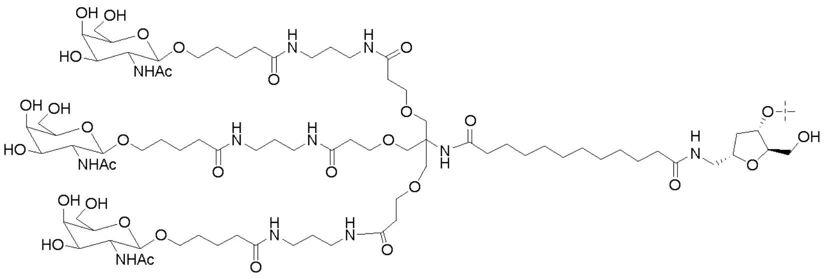

,

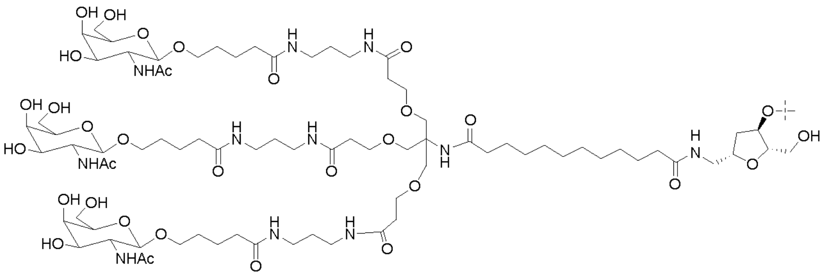

,

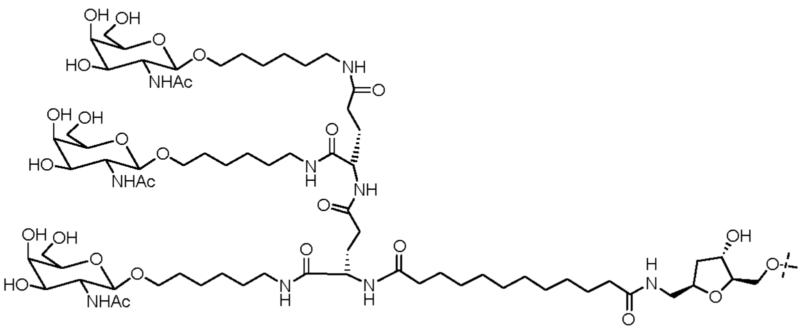

,

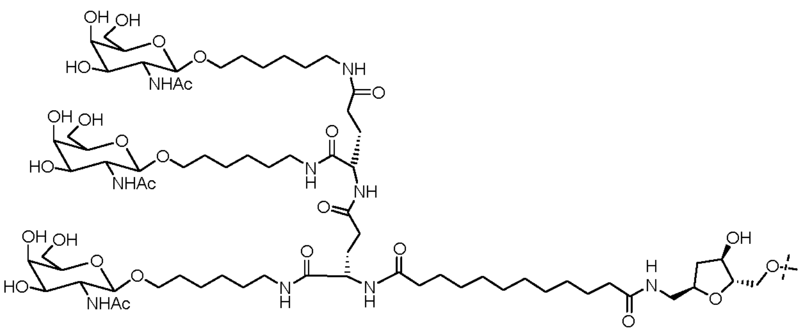

,

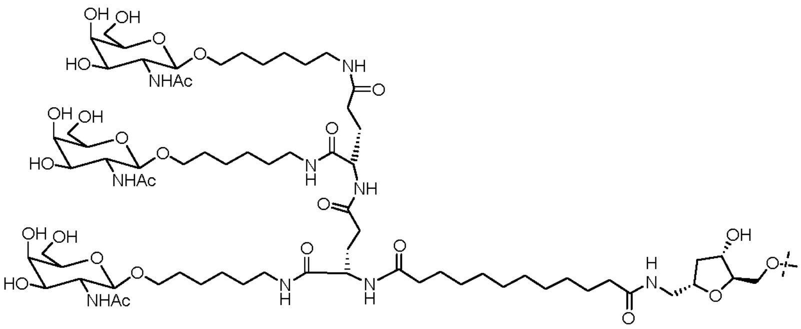

,

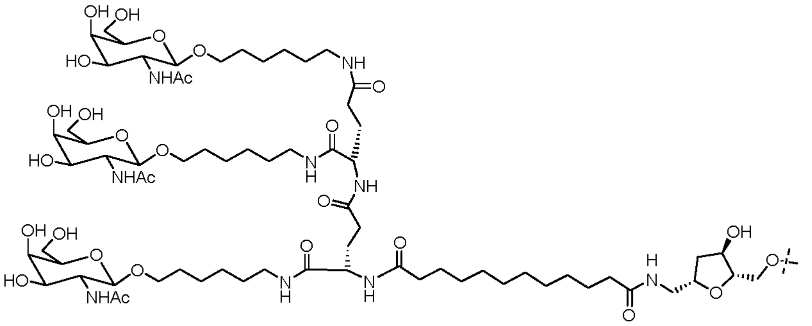

,

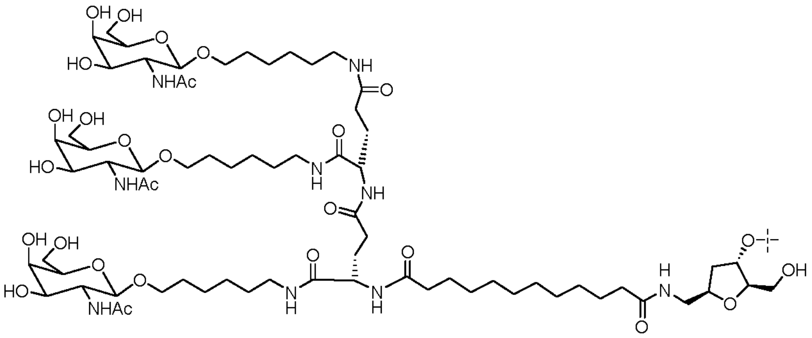

,

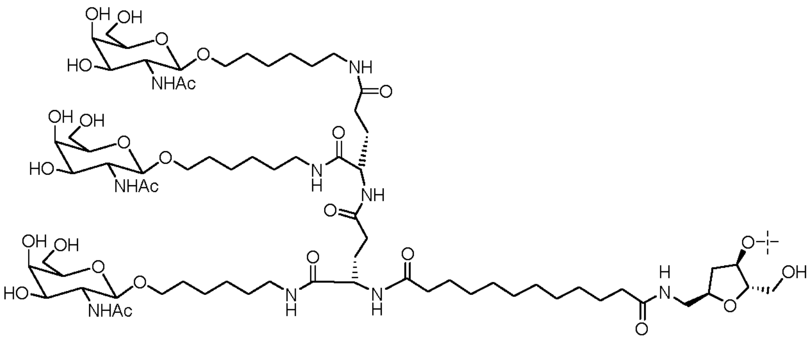

,

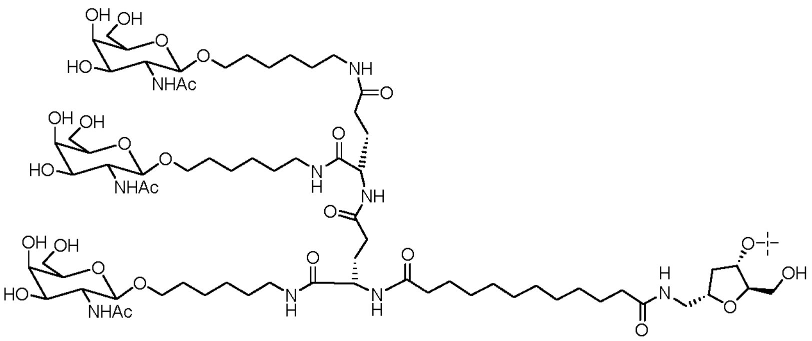

,

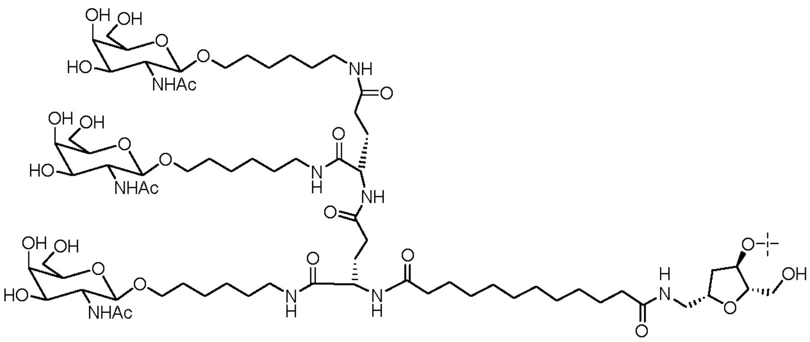

,

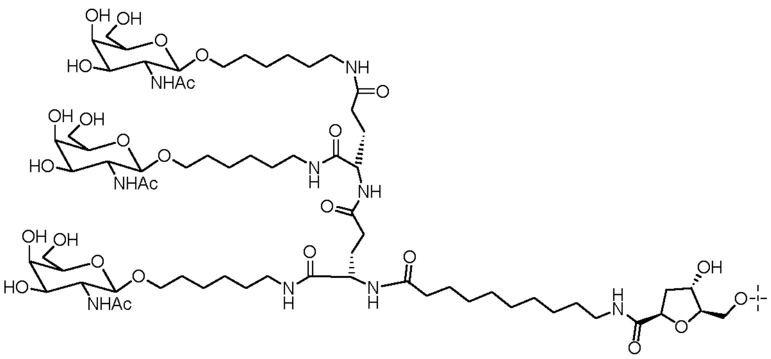

,

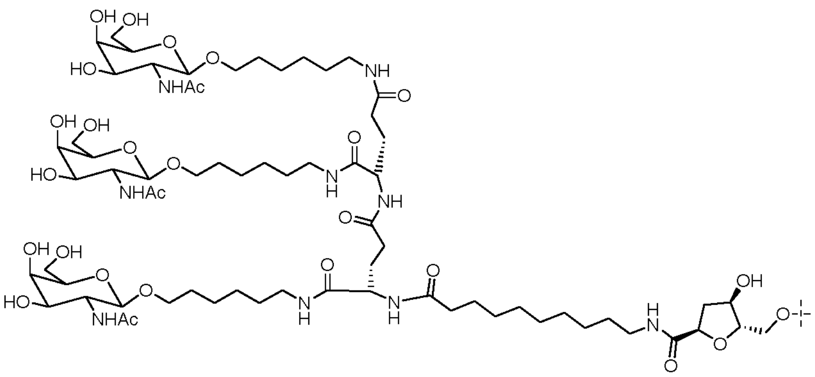

,

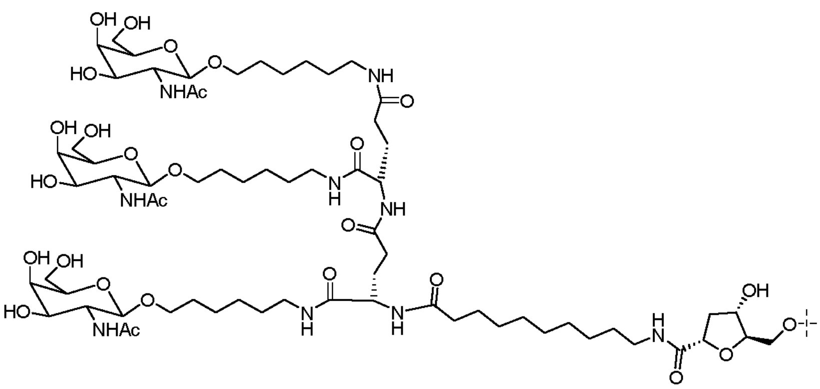

,

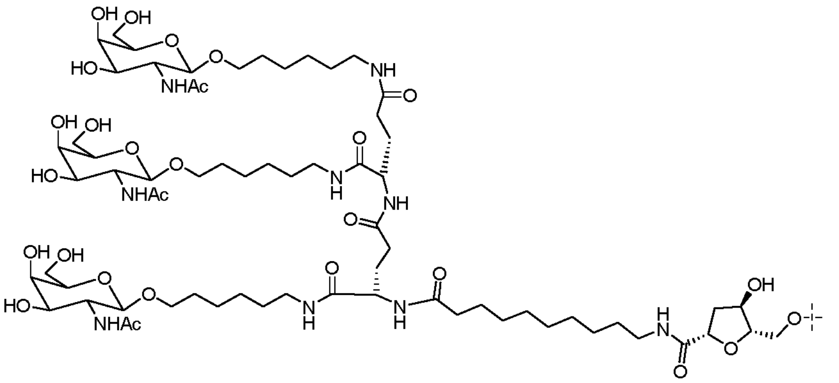

,

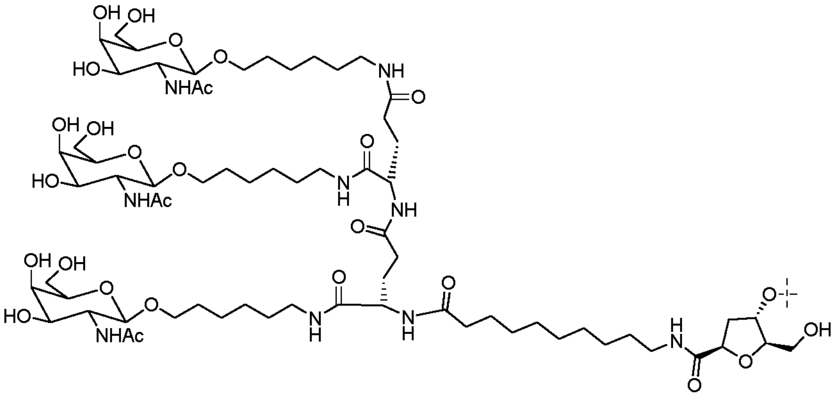

,

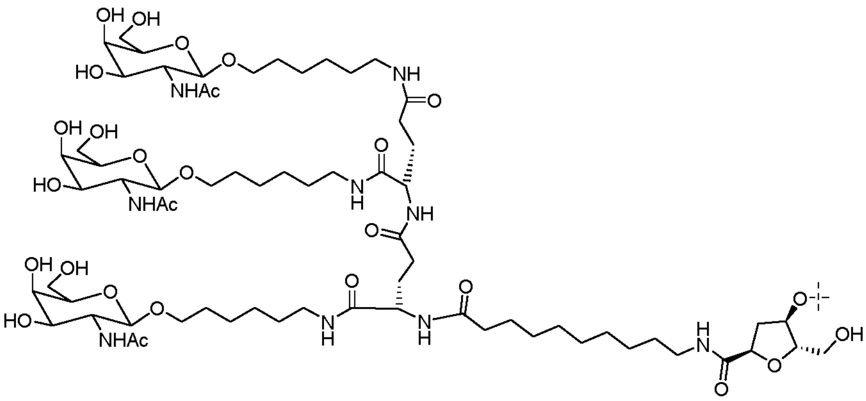

,

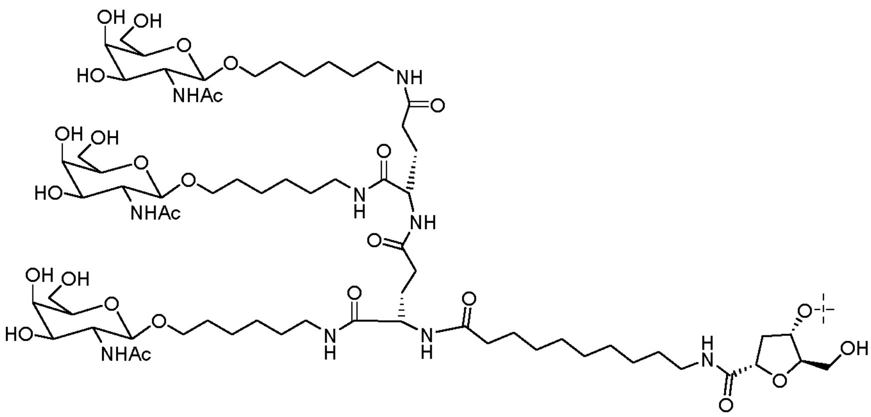

,

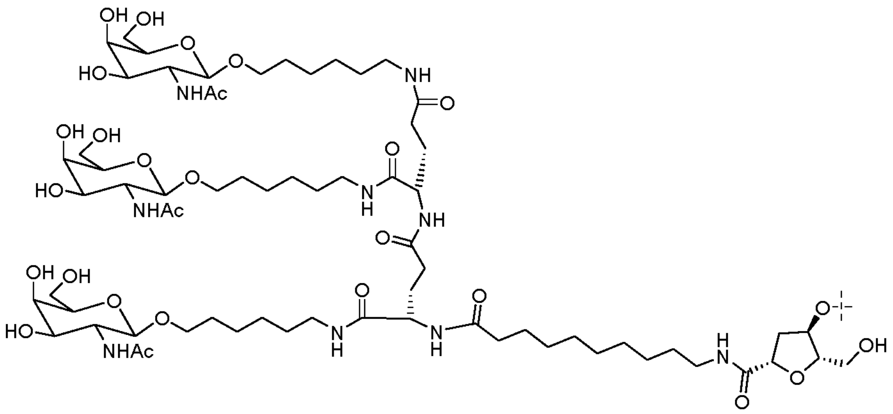

,

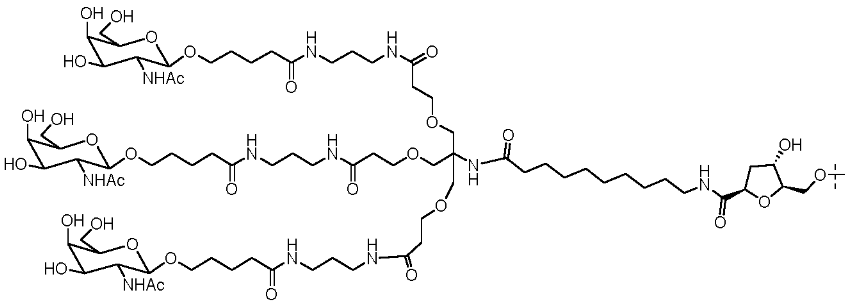

,

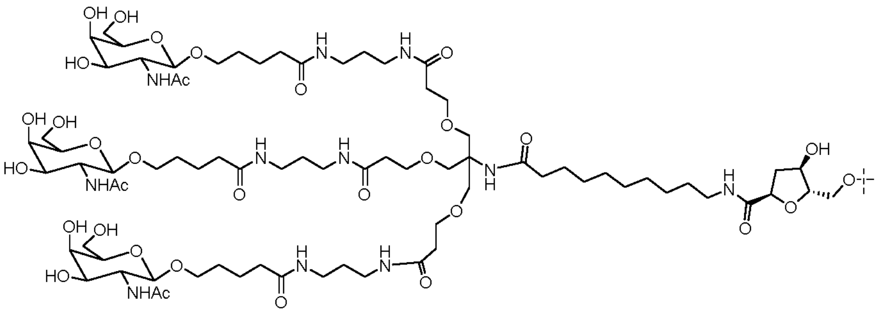

,

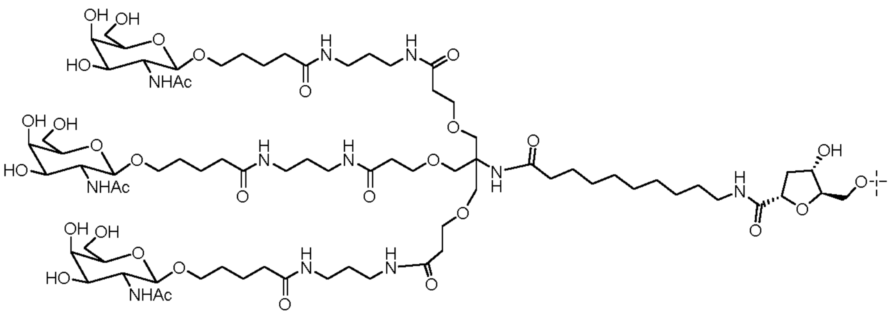

,

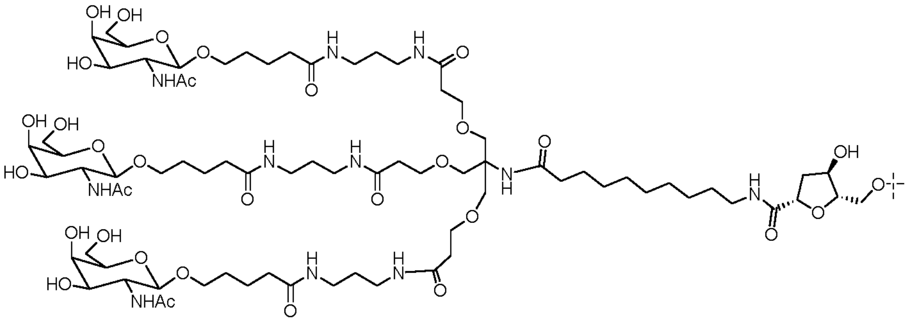

,

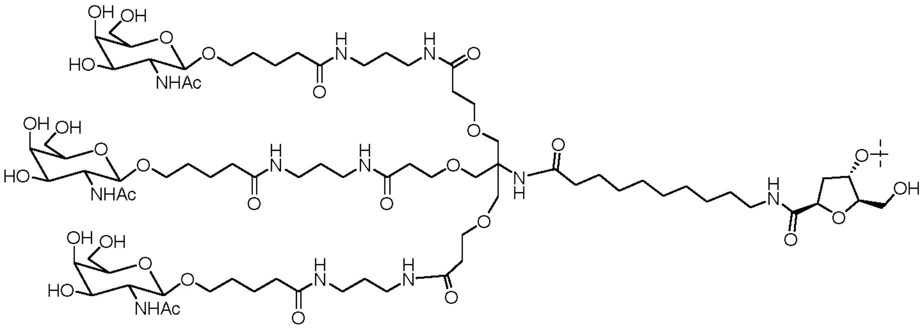

,

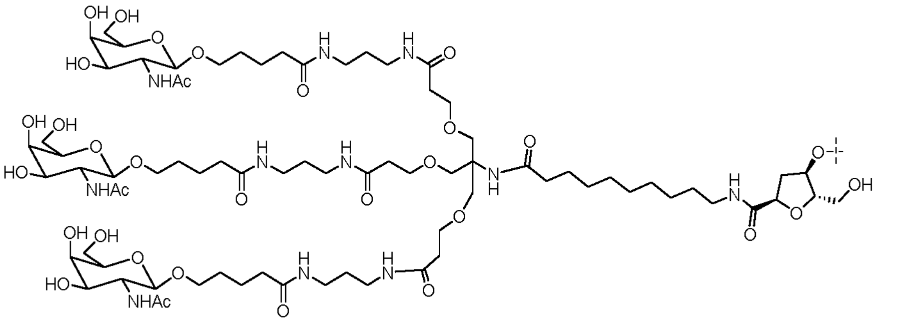

,

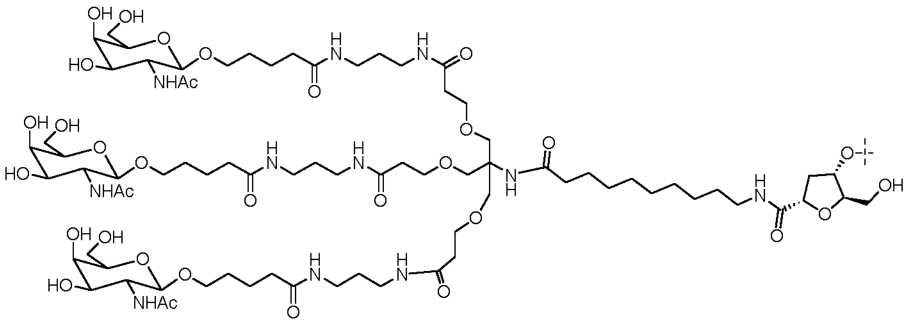

or

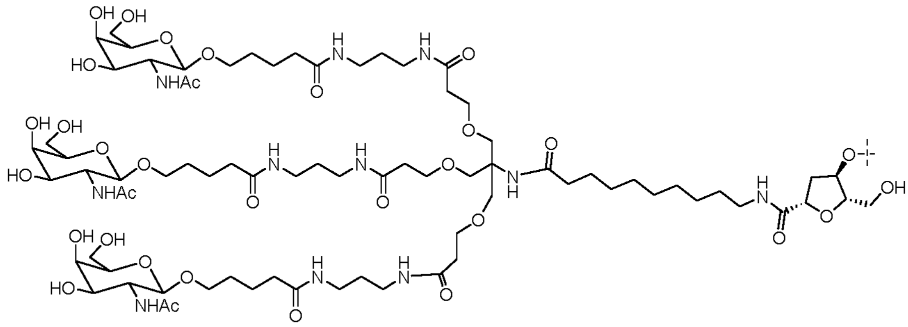

.

In some embodiments, the targeting ligand is selected from the group consisting of the following structure or a pharmaceutically acceptable salt thereof,

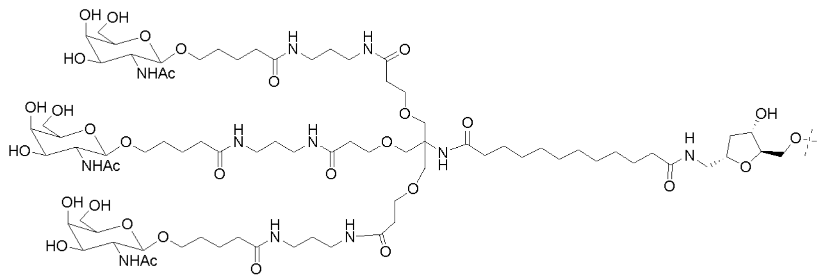

.

In some embodiments, the N-acetyl-galactosamine moiety in the above targeting ligand can be replaced with N-trifluoroacetylgalactosamine, N-propionylgalactosamine, N-n-butyrylgalactosamine or N-isobutyrylgalactosamine.

In some embodiments, the siRNA and the targeting ligand are linked covalently or non-covalently.

In some embodiments, the 3' end and/or the 5' end of the sense strand is conjugated to the targeting ligand.

In some embodiments, the 3' end of the sense strand is conjugated to the targeting ligand. In some embodiments, the targeting ligand is linked to an end of the siRNA by a phosphoester group (phosphodiester group) or a phosphorothioate group (phosphodiester group).

In some embodiments, the targeting ligand is linked to the 3' end of the sense strand of the siRNA by a phosphoester group (phosphodiester group) or a phosphorothioate group (phosphodiester group).

In some specific embodiments, the sense strand comprises any one of SEQ ID NO: 163 to SEQ ID NO: 188; in some specific embodiments, the antisense strand comprises any one of SEQ ID NO: 217 to SEQ ID NO: 242, SEQ ID NO: 260 to SEQ ID NO: 272 and SEQ ID NO: 290 to SEQ ID NO: 302.

In some specific embodiments, the sense strand is selected from any one of SEQ ID NO: 163 to SEQ ID NO: 188; in some specific embodiments, the antisense strand is selected from any one of SEQ ID NO: 217 to SEQ ID NO: 242, SEQ ID NO: 260 to SEQ ID NO: 272 and SEQ ID NO: 290 to SEQ ID NO: 302.

In some specific embodiments, the dsRNA or the siRNA conjugate comprises a sense strand and an antisense strand selected from any one of those defined in Table 26.

In some specific embodiments, the dsRNA or the siRNA conjugate is selected from any one of the following:

the dsRNA or the siRNA conjugate or the compound of the present disclosure, wherein the sense strand is selected from SEQ ID NO: 163, and the antisense strand is selected from SEQ ID NO: 217; or

the dsRNA or the siRNA conjugate or the compound of the present disclosure, wherein the sense strand is selected from SEQ ID NO: 172, and the antisense strand is selected from SEQ ID NO: 226; or

the dsRNA or the siRNA conjugate or the compound of the present disclosure, wherein the sense strand is selected from SEQ ID NO: 173, and the antisense strand is selected from SEQ ID NO: 227; or

the dsRNA or the siRNA conjugate or the compound of the present disclosure, wherein the sense strand is selected from SEQ ID NO: 174, and the antisense strand is selected from SEQ ID NO: 228; or

the dsRNA or the siRNA conjugate or the compound of the present disclosure, wherein the sense strand is selected from SEQ ID NO: 175, and the antisense strand is selected from SEQ ID NO: 229; or

the dsRNA or the siRNA conjugate or the compound of the present disclosure, wherein the sense strand is selected from SEQ ID NO: 176, and the antisense strand is selected from SEQ ID NO: 230.

In some embodiments, the dsRNA or the siRNA conjugate is of the following structures or pharmaceutically acceptable salts thereof:

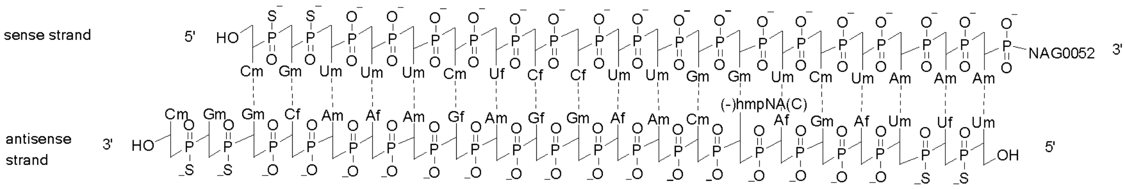

,

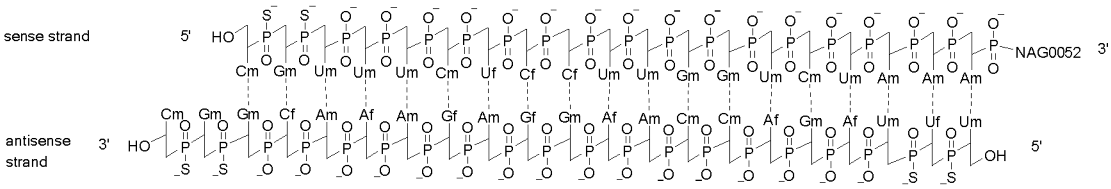

,

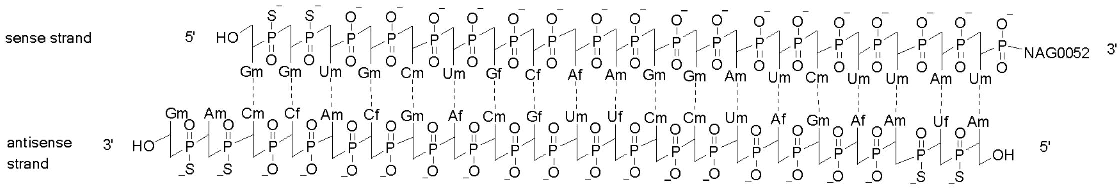

wherein,

Af = adenine 2'-F ribonucleoside;
Cf = cytosine 2'-F ribonucleoside;
Uf = uracil 2'-F ribonucleoside;
Gf = guanine 2'-F ribonucleoside;
Am = adenine 2'-OMe ribonucleoside;
Cm = cytosine 2'-OMe ribonucleoside;
Gm = guanine 2'-OMe ribonucleoside;
Um = uracil 2'-OMe ribonucleoside.

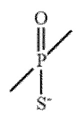

represents a phosphorothioate diester group,

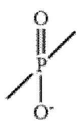

represents a phosphodiester group,
NAG0052 represents

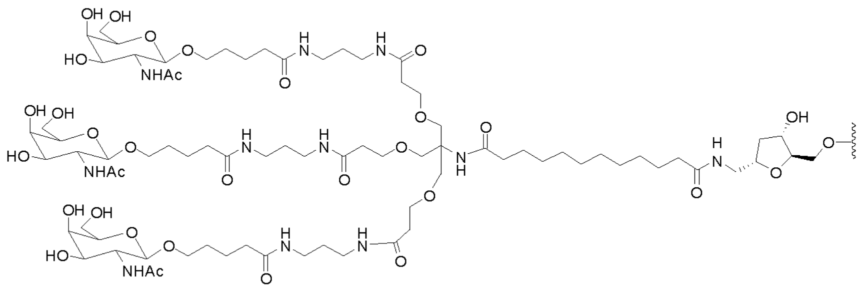

,

and
(-)hmpNA(C) represents

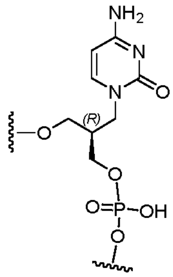

.

In some embodiments, the pharmaceutically acceptable salt can be a conventional salt in the art, including, but not limited to: sodium salts, potassium salts, ammonium salts, amine salts, etc. In some embodiments, the dsRNA or the siRNA conjugate is selected from the group consisting of TRD008096, TJR100053 and TJR100296.

In some embodiments, the dsRNA or the siRNA conjugate is TRD008096, which is of the following structure:

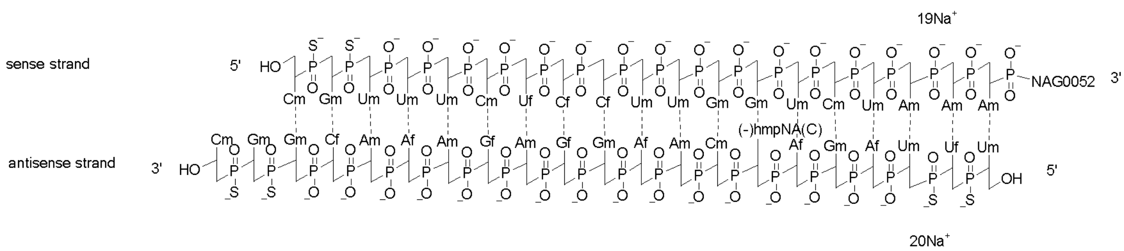

In some embodiments, the dsRNA or the siRNA conjugate is TJR100053, which is of the following structure:

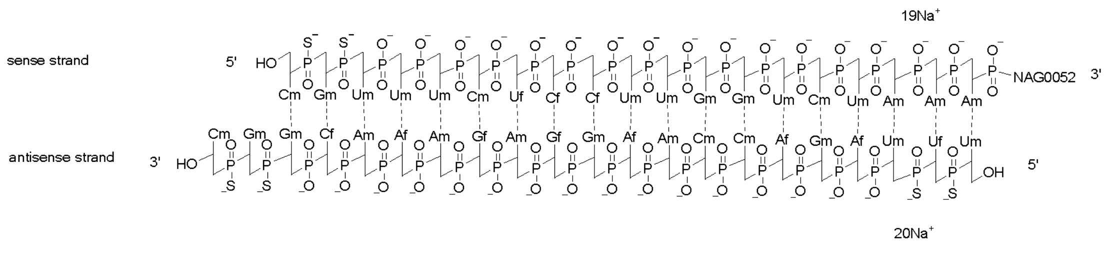

In some embodiments, the dsRNA is TJR100296, which is of the following structure:

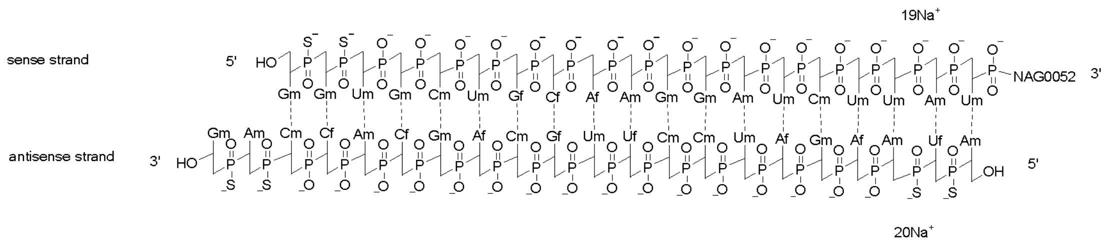

wherein,

Af = adenine 2'-F ribonucleoside;
Cf = cytosine 2'-F ribonucleoside;
Uf = uracil 2'-F ribonucleoside;
Gf = guanine 2'-F ribonucleoside;
Am = adenine 2'-OMe ribonucleoside;
Cm = cytosine 2'-OMe ribonucleoside;
Gm = guanine 2'-OMe ribonucleoside;
Um = uracil 2'-OMe ribonucleoside.

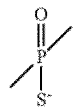

represents a phosphorothioate diester group,

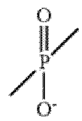

represents a phosphodiester group,
NAG0052 represents

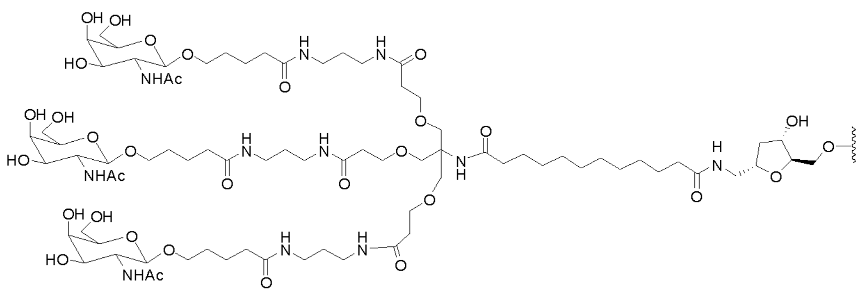

,

and
(-)hmpNA(C) represents

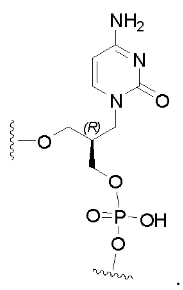

.

Another aspect of the present disclosure provides a compound selected from any one of groups 1) to 6):

group 1), a sense strand set forth in SEQ ID NO: 1 and an antisense strand set forth in SEQ ID NO: 7;
group 2), a sense strand set forth in SEQ ID NO: 2 and an antisense strand set forth in SEQ ID NO: 8;
group 3), a sense strand set forth in SEQ ID NO: 3 and an antisense strand set forth in SEQ ID NO: 9;
group 4), a sense strand set forth in SEQ ID NO: 4 and an antisense strand set forth in SEQ ID NO: 10;
group 5), a sense strand set forth in SEQ ID NO: 5 and an antisense strand set forth in SEQ ID NO: 11;
group 6), a sense strand set forth in SEQ ID NO: 6 and an antisense strand set forth in SEQ ID NO: 12.

The present disclosure provides a compound, comprising a sense strand and an antisense strand, wherein the sense strand is selected from any one of SEQ ID NO: 135 to SEQ ID NO: 162; the antisense strand is selected from any one of SEQ ID NO: 189 to SEQ ID NO: 216, SEQ ID NO: 243 to SEQ ID NO: 259 and SEQ ID NO: 273 to SEQ ID NO: 289.

The present disclosure provides a compound, comprising a sense strand and an antisense strand, wherein the sense strand is selected from any one of SEQ ID NO: 163 to SEQ ID NO: 188; the antisense strand is selected from any one of SEQ ID NO: 217 to SEQ ID NO: 242, SEQ ID NO: 260 to SEQ ID NO: 272 and SEQ ID NO: 290 to SEQ ID NO: 302.

The present disclosure provides a compound, which comprises a sense strand and an antisense strand selected from any one of those defined in Table 26.

Another aspect of the present disclosure provides a composition, which comprises the siRNA, the siRNA conjugate, the dsRNA or the compound described herein, and one or more pharmaceutically acceptable excipients, such as vehicles, carriers, diluents and/or delivery polymers.

Various delivery systems are known and can be used for the siRNA or the siRNA conjugate or the dsRNA of the present disclosure, e.g., encapsulation in liposomes, microparticles, microcapsules, recombinant cells capable of expressing the siRNA or siRNA conjugate or dsRNA, receptor-mediated endocytosis, and construction of a nucleic acid as part of a retroviral or other vectors.

Another aspect of the present disclosure provides use of the siRNA, the siRNA conjugate, the dsRNA or the composition described above in manufacturing a medicament for treating a disease in a subject; in some embodiments, the disease is selected from a hepatic disease.

Another aspect of the present disclosure provides a method for treating a disease in a subject, which comprises administering to the subject the siRNA, the siRNA conjugate, the dsRNA or the composition described above.

Another aspect of the present disclosure provides a method for inhibiting mRNA expression in a subject, which comprises administering to the subject the siRNA, the siRNA conjugate, the dsRNA or the composition described above.

Another aspect of the present disclosure provides a method for delivering an expression-inhibiting oligomeric compound to the liver *in vivo,* which comprises administering to a subject the siRNA, the siRNA conjugate, the dsRNA or the composition described above. The siRNA, the siRNA conjugate, the dsRNA or the composition and methods disclosed herein can reduce the level of a target mRNA in a cell, a cell population, a cell population, a tissue or a subject, which comprises: administering to the subject a therapeutically effective amount of the siRNA, the siRNA conjugate, the dsRNA or the composition described herein. The siRNA is linked to a targeting ligand, thereby inhibiting the expression of the target mRNA in the subject.

In some embodiments, the subject has been previously identified as having pathological upregulation of the target gene in the targeted cell or tissue.

The subject described in the present disclosure refers to a subject having (or suspected to have or susceptible to) a disease or condition that would benefit from reduction or inhibition of target mRNA expression.

Delivery can be accomplished by topical administration (e.g., direct injection, implantation or topical application), systemic administration, or through subcutaneous, intravenous, intraperitoneal, or parenteral routes, including intracranial (e.g., intraventricular, intraparenchymal and intrathecal), intramuscular, transdermal, airway (aerosol), nasal, oral, rectal, or topical (including buccal and sublingual) administration.

In optional embodiments, the pharmaceutical composition provided by the present disclosure can be administered by injection, for example, intravenous, intramuscular, intradermal, subcutaneous, intraduodenal, or intraperitoneal injection.

In optional embodiments, after the targeting ligand and siRNA are linked to form a conjugate, the conjugate can be packaged in a kit.

The present disclosure also provides a pharmaceutical composition, which comprises the siRNA or the siRNA conjugate or the dsRNA of the present disclosure.

In some embodiments, the pharmaceutical composition can further comprise a pharmaceutically acceptable auxiliary material and/or adjuvant; the auxiliary material can be one or more of various formulations or compounds conventionally used in the art. For example, the pharmaceutically acceptable auxiliary material can include at least one

of a pH buffer, a protective agent, and an osmotic pressure regulator.

In some embodiments, when the siRNA, the siRNA conjugate, the dsRNA or the pharmaceutical composition described above is in contact with a target gene-expressing cell, the siRNA, the siRNA conjugate, the dsRNA or the pharmaceutical composition described above inhibits the target gene expression by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, as measured by, for example, psiCHECK activity screening, luciferase reporter gene assay, PCR or branched DNA (bDNA)-based methods, or protein-based methods such as immunofluorescence assay, western blot or flow cytometry.

In some embodiments, when the siRNA, the siRNA conjugate, the dsRNA or the pharmaceutical composition described above is in contact with a target gene-expressing cell, the siRNA, the siRNA conjugate, the dsRNA or the pharmaceutical composition described above results in a percent remaining expression of the target gene's mRNA of no more than 99%, no more than 95%, no more than 90%, no more than 85%, no more than 80%, no more than 75%, no more than 70%, no more than 65%, no more than 60%, no more than 55%, no more than 50%, no more than 45%, no more than 40%, no more than 35%, no more than 30%, no more than 25%, no more than 20%, no more than 15%, or no more than 10%, as measured by, for example, psiCHECK activity screening and luciferase reporter gene assay, PCR or branched DNA (bDNA)-based methods, or protein-based methods such as immunofluorescence assay, western blot or flow cytometry.

In some embodiments, when the siRNA, the siRNA conjugate, the dsRNA or the pharmaceutical composition is in contact with a target gene-expressing cell, the siRNA, the siRNA conjugate, the dsRNA or the pharmaceutical composition reduces off-target activity by at least 20%, at least 25%, at least 30%, at least 35%, at least 40%, at least 45%, at least 50%, at least 55%, at least 60%, at least 65%, at least 70%, or at least 75%, while maintaining on-target activity, as measured by, for example, psiCHECK activity screening and luciferase reporter gene assay, PCR or branched DNA (bDNA)-based methods, or protein-based methods such as immunofluorescence assay, western blot or flow cytometry.

In some embodiments, when the siRNA, the siRNA conjugate, the dsRNA or the pharmaceutical composition is in contact with a target gene-expressing cell, the siRNA, the siRNA conjugate, the dsRNA or the pharmaceutical composition reduces off-target activity by at least 20%, at least 25%, at least 30%, at least 35%, at least 40%, at least 45%, at least 50%, at least 55%, at least 60%, at least 65%, at least 70%, or at least 75%, while reducing on-target activity by at most 20%, at most 19%, at most 15%, at most 10%, at most 5%, or more than 1%, as measured by, for example, psiCHECK activity screening and luciferase reporter gene assay, PCR or branched DNA (bDNA)-based methods, or protein-based methods such as immunofluorescence assay, western blot or flow cytometry.

In some embodiments, when the siRNA, the siRNA conjugate, the dsRNA or the pharmaceutical composition is in contact with a target gene-expressing cell, the siRNA, the siRNA conjugate, the dsRNA or the pharmaceutical composition reduces off-target activity by at least 20%, at least 25%, at least 30%, at least 35%, at least 40%, at least 45%, at least 50%, at least 55%, at least 60%, at least 65%, at least 70%, or at least 75%, while increasing on-target activity by at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, or at least 80%, as measured by, for example, psiCHECK activity screening and luciferase reporter gene assay, PCR or branched DNA (bDNA)-based methods, or protein-based methods such as immunofluorescence assay, western blot or flow cytometry.

The present disclosure also provides a cell, which comprises the siRNA or the siRNA conjugate or the dsRNA of the present disclosure.

The present disclosure also provides a kit, which comprises the siRNA or the siRNA conjugate or the dsRNA of the present disclosure.

The present disclosure also provides a method for silencing a target gene or mRNA of the target gene in a cell, which comprises the step of introducing the siRNA, the siRNA conjugate, the dsRNA and/or the pharmaceutical composition according to the present disclosure into the cell.

The present disclosure also provides a method for silencing a target gene or mRNA of the target gene in a cell *in vivo* or *in vitro,* which comprises the step of introducing the siRNA, the siRNA conjugate, the dsRNA and/or the pharmaceutical composition according to the present disclosure into the cell.

The present disclosure also provides a method for inhibiting the expression of a target gene or mRNA of the target gene, which comprises administering to a subject in need thereof an effective amount or effective dose of the siRNA, the siRNA conjugate, the dsRNA and/or the pharmaceutical composition according to the present disclosure.

In some embodiments, administration is carried out through routes of administration including intramuscular, intrabronchial, intrapleural, intraperitoneal, intraarterial, lymphatic, intravenous, subcutaneous, cerebrospinal, or combinations thereof.

In some embodiments, the effective amount or effective dose of the siRNA, the siRNA conjugate, the dsRNA

and/or the pharmaceutical composition is from about 0.001 mg/kg body weight to about 200 mg/kg body weight, from about 0.01 mg/kg body weight to about 100 mg/kg body weight, or from about 0.5 mg/kg body weight to about 50 mg/kg body weight.

In some embodiments, the target gene is an AGT gene.

The present disclosure also provides a pharmaceutical composition, which comprises the siRNA or the siRNA conjugate or the dsRNA of the present disclosure.

In some embodiments, the pharmaceutical composition can further comprise a pharmaceutically acceptable auxiliary material and/or adjuvant; the auxiliary material can be one or more of various formulations or compounds conventionally used in the art. For example, the pharmaceutically acceptable auxiliary material can include at least one of a pH buffer, a protective agent, and an osmotic pressure regulator.

In some embodiments, when the siRNA conjugate or the dsRNA or the pharmaceutical composition described above is in contact with an AGT-expressing cell, the siRNA conjugate or the dsRNA or the pharmaceutical composition described above inhibits the expression of AGT by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, as measured by, for example, PCR or branched DNA (bDNA)-based methods, or protein-based methods such as immunofluorescence assay, western blot or flow cytometry.

In some embodiments, when the siRNA conjugate or the dsRNA or the pharmaceutical composition described above is in contact with an AGT-expressing cell, the siRNA conjugate or the dsRNA or the pharmaceutical composition described above results in a percent remaining expression of AGT mRNA of no more than 99%, no more than 95%, no more than 90%, no more than 85%, no more than 80%, no more than 75%, no more than 70%, no more than 65%, no more than 60%, no more than 55%, no more than 50%, no more than 45%, no more than 40%, no more than 35%, no more than 30%, no more than 25%, no more than 20%, no more than 15%, or no more than 10%, as measured by, for example, PCR or branched DNA (bDNA)-based methods, or protein-based methods such as immunofluorescence assay, western blot or flow cytometry.

The present disclosure provides a method for inhibiting the expression of an AGT gene, comprising administering to a subject an effective amount or effective dose of the siRNA, the siRNA conjugate, the dsRNA or the pharmaceutical composition according to the present disclosure.

The present disclosure provides a method for treating and/or preventing a disease related to the expression of an AGT gene in a subject, comprising administering to the subject an effective amount or effective dose of the siRNA, the siRNA conjugate, the dsRNA or the pharmaceutical composition described herein.

The present disclosure provides a method for treating and/or preventing a disease related to a RAAS pathway in a subject, comprising administering to the subject an effective amount or effective dose of the siRNA, the siRNA conjugate, the dsRNA or the pharmaceutical composition described herein.

The present disclosure provides a method for treating and/or preventing a disease, comprising administering to a subject an effective amount or effective dose of the siRNA or the siRNA conjugate or the dsRNA or the pharmaceutical composition described herein. In some embodiments, the disease is selected from the group consisting of hypertension or heart disease. In some embodiments, the disease is selected from the group consisting of borderline hypertension, essential hypertension, secondary hypertension, isolated systolic or diastolic hypertension, pregnancy-related hypertension, diabetic hypertension, resistant hypertension, refractory hypertension, paroxysmal hypertension, renovascular hypertension, Goldblatt hypertension, hypertension associated with low plasma renin activity or plasma renin concentration, ocular hypertension, glaucoma, pulmonary hypertension, portal hypertension, systemic venous hypertension, systolic hypertension, labile hypertension; hypertensive heart disease, hypertensive nephropathy, atherosclerosis, arteriosclerosis, vasculopathy, diabetic nephropathy, diabetic retinopathy, chronic heart failure, cardiomyopathy, diabetic cardiomyopathy, glomerulosclerosis, coarctation of the aorta, aortic aneurysm, ventricular fibrosis, heart failure, myocardial infarction, angina pectoris, valvular heart disease, stroke, kidney disease, renal failure, systemic sclerosis, intrauterine growth restriction (IUGR), fetal growth restriction, obesity, hepatic steatosis/fatty liver, nonalcoholic steatohepatitis (NASH), nonalcoholic fatty liver disease (NAFLD); glucose intolerance, type 2 diabetes (non-insulin dependent diabetes mellitus), and metabolic syndrome.

The present disclosure provides a method for delivering an siRNA that inhibits the expression and/or replication of an AGT gene to the liver *in vivo*, comprising administering to a subject the siRNA or the siRNA conjugate or the dsRNA or the pharmaceutical composition described herein.

The present disclosure also provides a method for preparing an siRNA or an siRNA conjugate or a dsRNA, comprising synthesizing the siRNA, the siRNA conjugate, the dsRNA or the pharmaceutical composition described herein.

The present disclosure also provides an siRNA or an siRNA conjugate or a dsRNA, wherein one or more bases U, e.g., 1, 2, 3, 3, 5, 6, 7, 8, 9 or 10 bases U, of any siRNA or siRNA conjugate or dsRNA of the present disclosure are replaced with bases T.

The pharmaceutically acceptable salts of the compounds described herein are selected from the group consisting of inorganic salts and organic salts. The compounds described herein can react with acidic or basic substances to form corresponding salts.

In another aspect, where the configuration is not specified, the compounds of the present disclosure can be present in specific geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the present disclosure. Additional asymmetric carbon atoms can be present in substituents such as an alkyl group. All such isomers and mixtures thereof are included within the scope of the present disclosure.

In addition, wherein the configuration is not specified, the compounds and intermediates of the present disclosure can also be present in different tautomeric forms, and all such forms are included within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol and imine-enamine, lactam-lactim isomerization. An example of a lactam-lactim equilibrium is present between A and B as shown below.

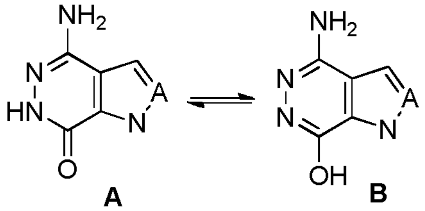

All compounds in the present disclosure can be drawn as form A or form B. All tautomeric forms are within the scope of the present disclosure. The names of the compounds do not exclude any tautomers.

The compounds of the present disclosure can be asymmetric; for example, the compounds have one or more stereoisomers. Unless otherwise specified, all stereoisomers include, for example, enantiomers and diastereomers. The compounds of the present disclosure containing asymmetric carbon atoms can be separated in optically active pure form or in racemic form. The optically active pure form can be isolated from a racemic mixture or synthesized using chiral starting materials or chiral reagents.

Optically active (R)- and (S)-enantiomers and D- and L-isomers can be prepared by chiral synthesis, chiral reagents or other conventional techniques. If one enantiomer of a certain compound of the present disclosure is desired, it can be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. Furthermore, separation of enantiomers and diastereomers is generally accomplished by chromatography using a chiral stationary phase, optionally in combination with chemical derivatization (e.g., carbamate formation from amines).

The present disclosure also includes isotopically labeled compounds that are identical to those recited herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure also comprises various deuterated forms of the compounds of formula I and formula II. Each available hydrogen atom connected to a carbon atom may be independently replaced by a deuterium atom. Those skilled in the art are able to synthesize the deuterated forms of the compounds of formula I and formula II with reference to the relevant literature. Commercially available deuterated starting materials can be used in preparing the deuterated forms of the compounds of formula I and formula II, or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, and the like.

Where the configurations are not specified, in the chemical structures of the compounds of the present disclosure, a bond

“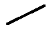”

indicates an unspecified configuration; that is, if chiral isomers exist in the chemical structures, the bond

“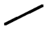”

may be

“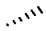”

or

“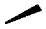”,

or includes both the configurations

“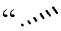”

and

“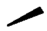”.

Although all of the above structural formulae are drawn as certain isomeric forms for the sake of simplicity, the present disclosure may include all isomers, such as tautomers, rotamers, geometric isomers, diastereomers, racemates, and enantiomers. In the chemical structure of the compound of the present disclosure, a bond

“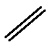”

does not specify a configuration; that is, the configuration for the bond

“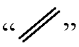”

can be an E configuration or a Z configuration, or includes both the E configuration and the Z configuration.

**Terms and Definitions**

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains. Unless otherwise specified, in the context of the present disclosure, the terms "angiotensinogen" and "AGT" are used interchangeably herein. AGT is also known as SERPINA8 and ANHU. AGTs include, but are not limited to, human AGT, cynomolgus monkey AGT, mouse AGT, and rat AGT, the amino acid and complete coding sequences and mRNA sequences of which are readily accessible using publicly available databases, e.g., GenBank, UniProt, OMIM, and the Macaca genome project website.

The term "AGT" also refers to naturally occurring DNA sequence variations of the AGT gene, such as a single nucleotide polymorphism (SNP) in the AGT gene. Exemplary SNPs may be found in the dbSNP database.

The term "target sequence" refers to a contiguous portion of the nucleotide sequence of an mRNA molecule

formed during the transcription of the AGT gene, including mRNA that is a product of RNA processing of a primary transcription product. The targeted portion in the target sequence should be long enough to serve as a substrate for iRNA-directed cleavage. In one embodiment, the target sequence is within the protein encoding region of AGT.

As used herein, in the context of RNA-mediated gene silencing, the sense strand (also referred to as SS or SS strand) of an siRNA refers to a strand that comprises a sequence that is identical or substantially identical to a target mRNA sequence; the antisense strand (also referred to as AS or AS strand) of an siRNA refers to a strand having a sequence complementary to a target mRNA sequence.

In the context describing the siRNA sense strand described herein, the term "a sequence differing by no more than 3 nucleotides from any one of the nucleotide sequences of SEQ ID NO: 1 to SEQ ID NO: 6 and comprises at least 15 contiguous nucleotides" is intended to mean that the siRNA sense strand described herein comprises at least 15 contiguous nucleotides of any one of the sense strands in SEQ ID NO: 1 to SEQ ID NO: 6 or a sequence differing by no more than 3 nucleotides (optionally, by no more than 2 nucleotides; optionally, by 1 nucleotide) from at least 15 contiguous nucleotides of any one of the sense strands in SEQ ID NO: 1 to SEQ ID NO: 6. Optionally, the siRNA sense strand described herein comprises at least 16 contiguous nucleotides of any one of the sense strands of SEQ ID NO: 1 to SEQ ID NO: 6 or a sequence differing by no more than 3 nucleotides (optionally, by no more than 2 nucleotides; optionally, by 1 nucleotide) from at least 16 contiguous nucleotides of any one of the sense strands of SEQ ID NO: 1 to SEQ ID NO: 6.

In the context describing the siRNA antisense strand described herein, the term "a sequence differing by no more than 3 nucleotides from any one of the antisense strands of SEQ ID NO: 7 to SEQ ID NO: 12 and comprises at least 15 contiguous nucleotides" is intended to mean that the siRNA antisense strand described herein comprises at least 15 contiguous nucleotides of any one of the antisense strands in SEQ ID NO: 7 to SEQ ID NO: 12 or a sequence differing by no more than 3 nucleotides (optionally, by no more than 2 nucleotides; optionally, by 1 nucleotide) from at least 15 contiguous nucleotides of any one of the antisense strands in SEQ ID NO: 7 to SEQ ID NO: 12.

In the present disclosure, the "5' region" of the sense or antisense strand, i.e., the "5' end" or "5' terminus", is used interchangeably. For example, the nucleotides at positions 2 to 8 of the 5' region of the antisense strand may be replaced with the nucleotides at positions 2 to 8 of the 5' end of the antisense strand. Likewise, the "3' region", "3' terminus" and "3' end" of the sense or antisense strand are also used interchangeably.

Unless otherwise specified, in the context of the present disclosure, the uppercase letters C, G, U, A and T represent base components of a nucleotide; the lowercase letter d indicates that the nucleotide downstream of and adjacent to the letter d is a deoxyribonucleotide; the lowercase letter m indicates that the nucleotide upstream of and adjacent to the letter m is a methoxy-modified nucleotide; the lowercase letter f indicates that the nucleotide upstream of and adjacent to the letter f is a fluoro-modified nucleotide; the lowercase letter s indicates that the two nucleotides adjacent to the letter s are linked by a phosphorothioate group.

As used in the present disclosure, the term "2'-fluoro-modified nucleotide" refers to a nucleotide in which the hydroxy group at the 2' position of the ribosyl group of the nucleotide is substituted with fluorine, and the term "non-2'-fluoro-modified nucleotide" refers to a nucleotide or a nucleotide analog in which the hydroxy group at the 2' position of the ribosyl group of the nucleotide is substituted with a non-fluorine group.

As used in the present disclosure, the term "2'-methoxy-modified nucleotide" refers to a nucleotide in which the 2'-hydroxy group of the ribosyl group is substituted with a methoxy group.

As used herein, the terms "complementary" and "reverse complementary" are used interchangeably and have the meaning well known to those skilled in the art; that is, in a double-stranded nucleic acid molecule, the bases of one strand are paired with the bases of the other strand in a complementary manner. In DNA, the purine base adenine (A) is always paired with the pyrimidine base thymine (t) (or uracil (U) in RNA), and the purine base guanine (C) is always paired with the pyrimidine base cytosine (G). Each base pair comprises a purine and a pyrimidine. When adenines of one strand are always paired with thymines (or uracils) of another strand and guanines are always paired with cytosines, the two strands are considered complementary to each other, and the sequences of the strands can be deduced from the sequences of their complementary strands. Accordingly, "mismatch" in the art means that in a double-stranded nucleic acid, the bases at the corresponding positions are not paired in a complementary manner.

As used herein, the term "inhibit" is used interchangeably with "decrease", "silence", "down-regulate", "repress" and other similar terms, and includes any level of inhibition. Inhibition can be assessed in terms of a decrease in the absolute or relative level of one or more of these variables relative to a control level. The control level can be any type of control level used in the art, such as a pre-dose baseline level or a level determined from an untreated or control (e.g., buffer-only control or inert agent control) treated subject, cell, or sample. For example, the remaining mRNA expression level can be used to characterize the degree of inhibition of target gene expression by the siRNA; for example, the remaining expression level of mRNA is not greater than 99%, not greater than 95%, not greater than 90%, not greater than 85%, not greater than 80%, not greater than 75%, not greater than 70%, not greater than 65%, not greater than 60%, not greater than 55%, not greater than 50%, not greater than 45%, not greater than 40%, not greater than 35%, not greater than 30%, not greater than 25%, not greater than 20%, not greater than 15%, or not greater than 10%. The

inhibition rate of target gene expression can be measured using Dual-Glo® Luciferase Assay System: the Firefly chemiluminescence value (Fir) and the Renilla chemiluminescence value (Ren) are each read, and the relative value Ratio = Ren/Fir is calculated; in the present disclosure, the ratio of the remaining mRNA expression level (or residual activity%) = Ratio (siRNA-treated group) / Ratio (siRNA-free control group), and the inhibition rate (%) = 100% - remaining mRNA expression level (%).

Unless otherwise specified, the "compound", "ligand", "nucleic acid-ligand conjugate", "nucleic acid", "conjugate", "chemical modification", "ligand", "dsRNA", and "RNAi" of the present disclosure can each independently exist in the form of a salt, mixed salts, or a non-salt (e.g., a free acid or free base). When existing in the form of a salt or mixed salts, it can be a pharmaceutically acceptable salt.

The term "pharmaceutically acceptable salt" includes pharmaceutically acceptable acid addition salts and pharmaceutically acceptable base addition salts.

"Pharmaceutically acceptable acid addition salt" refers to salts that are capable of retaining the biological effectiveness of free bases without having any undesirable effects and that are formed with inorganic or organic acids. Inorganic acid salts include, but are not limited to, hydrochlorides, hydrobromides, sulfates, nitrates, phosphates, etc.; organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, mesylates, benzenesulfonates, p-toluenesulfonates, alginates, ascorbates, salicylates, 4-aminosalicylates, napadisylates, etc. These salts can be prepared using methods known in the art.

"Pharmaceutically acceptable base addition salt" refers to salts that are capable of retaining the biological effectiveness of free acids without having any undesirable effects and that are formed with inorganic bases or organic bases. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts and magnesium salts; sodium salts are preferred. Salts derived from organic bases include, but are not limited to, salts of the following: primary, secondary and tertiary amines, substituted amines including naturally occurring substituted amines, cyclic amines and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, etc. Preferred organic bases include isopropylamine, diethylamine, ethanolamine, trimethylamine, dicyclohexylamine, choline, and caffeine. These salts can be prepared using methods known in the art.

"Effective amount" or "effective dosage" refers to the amount of a drug, a compound or a pharmaceutical composition necessary to obtain any one or more beneficial or desired therapeutic results. For preventive use, the beneficial or desired results include elimination or reduction of risk, reduction of severity or delay of the onset of a condition, including the biochemistry, histology and/or behavioral symptoms of the condition, complications thereof and intermediate pathological phenotypes that appear during the progression of the condition. For therapeutic applications, the beneficial or desired results include clinical results, such as reducing the incidence of various conditions related to the target gene, target mRNA or target protein of the present disclosure or alleviating one or more symptoms of the condition, reducing the dosage of other agents required to treat the condition, enhancing the therapeutic effect of another agent, and/or delaying the progression of conditions related to the target gene, target mRNA or target protein of the present disclosure in the patient.

As used herein, "patient", "subject" and "individual" are used interchangeably and include human or non-human animals, e.g., mammals, e.g., humans or monkeys.

The siRNA provided by the present disclosure can be obtained using a preparation method conventional in the art (e.g., solid-phase synthesis and liquid-phase synthesis). Solid phase synthesis has been commercially available as customization service. A modified nucleotide group can be introduced into the siRNA of the present disclosure using a nucleoside monomer with a corresponding modification. Methods of preparing a nucleoside monomer with a corresponding modification and introducing a modified nucleotide group into an siRNA are also well known to those skilled in the art.

The term "chemical modification" or "modification" includes all changes made to a nucleotide by chemical means, such as the addition or removal of a chemical moiety, or the substitution of one chemical moiety for another.

The term "base" encompasses any known DNA and RNA bases and base analogs such as purines or pyrimidines, and also include the natural compounds adenine, thymine, guanine, cytosine, uracil, inosine, and natural analogs.

The terms "blunt" and "blunt ended" are used interchangeably and mean that there are no unpaired nucleotides or nucleotide analogs at a given terminus of an siRNA, i.e., no nucleotide overhang. In most cases, an siRNA both ends of which are blunt ended will be double-stranded over its entire length.

The terms "about" and "approximately" mean that a numerical value is within an acceptable error range for the

specific value determined by those of ordinary skill in the art, and the numerical value depends in part on how the value is measured or determined (i.e., the limitations of the measurement system). For example, "about" may mean a standard deviation within 1 or more than 1. Alternatively, "about" or "comprising essentially" may mean a range of at most 20%, e.g., a change of between 1% and 15%, between 1% and 10%, between 1% and 5%, between 0.5% and 5%, or between 0.5% and 1%. In the present disclosure, every instance where a number or numerical range is preceded by the term "about" also includes an embodiment of the given number. Unless otherwise specified, when a specific value is provided in the present application and claims, the meaning of "about" or "substantially comprising" should be assumed to be within an acceptable error range for that specific value.

Unless otherwise specified, "optionally", "optional", "alternatively", or "alternative" means that the event or circumstance subsequently described may, but does not necessarily, occur, and this description includes instances where the event or circumstance occurs or does not occur. For example, "optionally, $R_1$ and $R_2$ are directly linked to form a ring" means that $R_1$ and $R_2$ being directly linked to form a ring may occur but does not necessarily exist, and this description includes an instance where $R_1$ and $R_2$ are directly linked to form a ring and an instance where $R_1$ and $R_2$ do not form a ring.

In the present disclosure, the term "comprise" can be replaced with "consist of'.

The term "alkyl" refers to a saturated aliphatic hydrocarbon group which is a straight-chain or branched-chain group containing 1 to 20 carbon atoms. In some embodiments, it is selected from the group consisting of alkyl groups containing 1 to 12 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched-chain isomers thereof, and the like. In some embodiments, it is selected from the group consisting of alkyl groups containing 1 to 6 carbon atoms; non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. Alkyl may be substituted or unsubstituted, and when it is substituted, the substituent may be substituted at any accessible point of attachment; the substituent, in some embodiments, is selected from the group consisting of one or more of the following groups, independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, or a carboxylate group.

The term "alkoxy" refers to -O-(alkyl) and -O-(unsubstituted cycloalkyl), wherein the alkyl group is as defined above. Non-limiting examples of alkoxy groups include: methoxy, ethoxy, propoxy, butoxy, cyclopropyloxy, cyclobutoxy, cyclopentyloxy, and cyclohexyloxy. Alkoxy may be optionally substituted or unsubstituted, and when it is substituted, the substituent, in some embodiments, is selected from the group consisting of one or more of the following groups, independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, wherein the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl are optionally substituted with one or more groups selected from the group consisting of halogen, deuterium, hydroxy, oxo, nitro, and cyano. Likewise, the definitions of "alkynyloxy", "alkenyloxy", "cycloalkoxy", "heterocycloalkoxy", and "cycloalkenyloxy" are similar to the above definition of "alkoxy".

The term "alkenyl" refers to a straight-chain or branched-chain non-aromatic hydrocarbon group containing at least one carbon-carbon double bond and having 2-10 carbon atoms. Up to 5 carbon-carbon double bonds may be present in such groups. For example, "$C_2$-$C_6$" alkenyl is defined as an alkenyl group having 2-6 carbon atoms. Examples of alkenyl groups include, but are not limited to: ethenyl, propenyl, butenyl, and cyclohexenyl. The straight, branched, or cyclic portion of an alkenyl group may contain a double bond and is optionally mono-, di-, tri-, tetra-, or penta-substituted at any position as permitted by normal valency.

The term "cycloalkenyl" refers to a monocyclic hydrocarbon group having the specified number of carbon atoms and at least one carbon-carbon double bond.

The term "alkynyl" refers to a straight-chain or branched-chain hydrocarbon group containing 2-10 carbon atoms and containing at least one carbon-carbon triple bond. Up to 5 carbon-carbon triple bonds may be present. Thus, "$C_2$-$C_6$ alkynyl" refers to an alkenyl group having 2-6 carbon atoms. Examples of alkynyl groups include, but are not

limited to: ethynyl, 2-propynyl, and 2-butynyl. The straight or branched portion of an alkynyl group may contain a triple bond as permitted by normal valency and is optionally mono-, di-, tri-, tetra-, or penta-substituted at any position as permitted by normal valency.

The term "keto" refers to any alkyl, alkenyl, alkynyl, cycloalkyl, cycloalkenyl, heterocyclyl, heteroaryl, or aryl group as described herein attached through a carbonyl bridge. Examples of keto groups include, but are not limited to: alkanoyl (e.g., acetyl, propionyl, butyryl, pentanoyl, hexanoyl), alkenoyl (e.g., acryloyl), alkynoyl (e.g., ethynoyl, propynoyl, butynoyl, pentynoyl, hexynoyl), aroyl (e.g., benzoyl), heteroaroyl (e.g., pyrroloyl, imidazoloyl, quinolinoyl, pyridinoyl).

The term "alkoxycarbonyl" refers to any alkoxy group as defined above attached through a carbonyl bridge (i.e., -C(O)O-alkyl). Examples of alkoxycarbonyl groups include, but are not limited to: methoxycarbonyl, ethoxycarbonyl, isopropoxycarbonyl, n-propoxycarbonyl, tert-butoxycarbonyl, benzyloxycarbonyl, or n-pentoxycarbonyl. The term "aryloxycarbonyl" refers to any aryl group as defined above attached through an oxycarbonyl bridge (i.e., -C(O)O-aryl). Examples of aryloxycarbonyl groups include, but are not limited to: phenoxycarbonyl and naphthyloxycarbonyl.

The term "heteroaryloxycarbonyl" refers to any heteroaryl group as defined above attached through an oxycarbonyl bridge (i.e., -C(O)O-heteroaryl). Examples of heteroaryloxycarbonyl groups include, but are not limited to: 2-pyridinyloxycarbonyl, 2-oxazolyloxycarbonyl, 4-thiazolyloxycarbonyl, or pyrimidinyloxycarbonyl.

The term "cycloalkyl" or "carbocycle" refers to a saturated or partially unsaturated, monocyclic or polycyclic hydrocarbon substituent; the cycloalkyl ring contains 3 to 20 carbon atoms. In some embodiments, it is selected from the group consisting of those containing 3 to 7 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, and the like. Polycyclic cycloalkyl groups include spiro, fused, and bridged cycloalkyl groups. Cycloalkyl may be substituted or unsubstituted. When it is substituted, the substituent may be substituted at any accessible point of attachment. In some embodiments, it is selected from the group consisting of one or more of the following groups, independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, wherein the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl are optionally substituted with one or more groups selected from the group consisting of halogen, deuterium, hydroxy, oxo, nitro, and cyano.

The cycloalkyl ring may be fused to an aryl or heteroaryl ring, wherein the ring attached to the parent structure is a cycloalkyl group; non-limiting examples include indanyl, tetrahydronaphthyl, benzocycloheptyl, and the like. Cycloalkyl may be optionally substituted or unsubstituted, and when it is substituted, the substituent, in some embodiments, is selected from the group consisting of one or more of the following groups, independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, wherein the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl are optionally substituted with one or more groups selected from the group consisting of halogen, deuterium, hydroxy, oxo, nitro, and cyano.

The term "heterocycloalkyl" or "heterocycle" or "heterocyclyl" refers to a saturated or partially unsaturated, monocyclic or polycyclic hydrocarbon substituent containing 3 to 20 ring atoms, wherein one or more of the ring atoms are heteroatoms selected from the group consisting of nitrogen, oxygen, or $S(O)_m$ (where m is an integer of 0 to 2), excluding a ring moiety of -O-O-, -O-S-, or -S-S-, and the other ring atoms are carbon atoms. In some embodiments, it is selected from the group consisting of those containing 3 to 12 ring atoms, of which 1 to 4 are heteroatoms. In some embodiments, it is selected from the group consisting of those containing 3 to 7 ring atoms. Non-limiting examples of monocyclic heterocycloalkyl groups include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, and the like. Polycyclic heterocycloalkyl groups include spiro, fused, and bridged heterocycloalkyl groups. Non-limiting examples of "heterocycloalkyl" groups include:

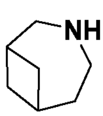, 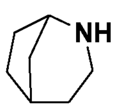, 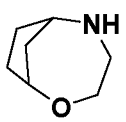, 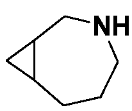, 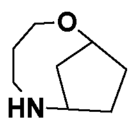, 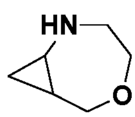,

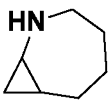, 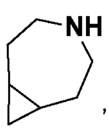, 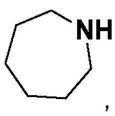, 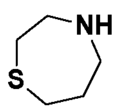, 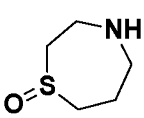, 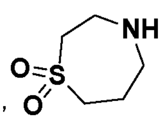,

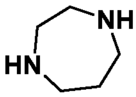 , 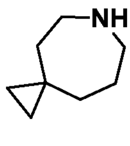 , 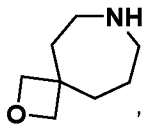 , 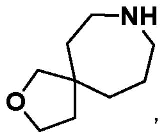 , 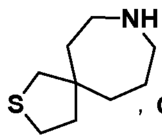 , 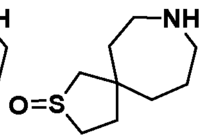 ,

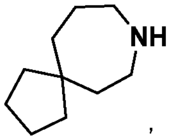 , 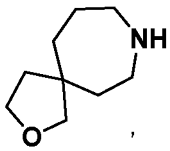 , 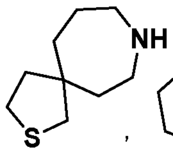 , 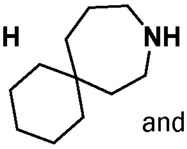 and 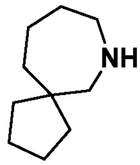 ,

and the like.

The term "hydroxy" refers to the -OH group.

The term "halogen" refers to fluorine, chlorine, bromine, or iodine.

The term "haloalkyl" refers to an alkyl group substituted with a halogen, wherein the alkyl group is as defined above.

The term "cyano" refers to -CN.

The term "nitro" refers to $-NO_2$.

The term "oxo" refers to the =O group; for example, a carbon atom is attached to an oxygen atom through a double bond, wherein a keto or aldehyde group is formed.

The term "amino" refers to $-NH_2$.

The term "carboxyl" refers to -C(O)OH.

The term "aldehyde" refers to -CHO.

In the chemical structural formulas of the present disclosure,

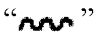

or

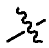

may be linked to any group or groups in accordance with the scope of the invention described herein; the asterisk "*" indicates a chiral center.

In the present disclosure, a "phosphoester group" is a phosphodiester group unless otherwise specified.

In the present disclosure, the phosphorothioate group refers to a phosphodiester group modified by replacing one non-bridging oxygen atom with a sulfur atom, and is used interchangeably with

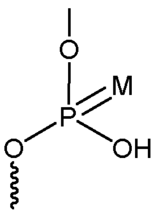 and 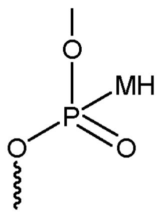

(M is a S atom).

In the context of the present disclosure, the

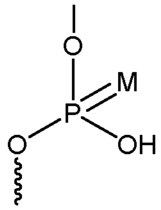

in the group

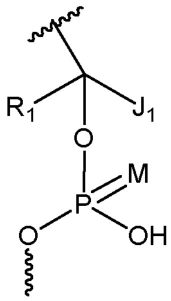

can be replaced with any group capable of linking to an adjacent nucleotide.

The term "link", "connect", or "attach", when referring to a relationship between two molecules, means that the two molecules are linked by a covalent bond or that the two molecules are associated via a non-covalent bond (e.g., a hydrogen bond or an ionic bond), and includes direct linkage and indirect linkage.

The term "directly linked" means that a first compound or group is linked to a second compound or group without any atom or group of atoms interposed between.

The term "indirectly linked" means that a first compound or group is linked to a second compound or group by an intermediate group, a compound, or a molecule (e.g., a linking group).

The term "substituted" means that any one or more hydrogen atoms on the specified atom (usually a carbon, oxygen, or nitrogen atom) are replaced with any group as defined herein, provided that the normal valency of the specified atom is not exceeded and that the substitution results in a stable compound. Non-limiting examples of substituents include C1-C6 alkyl, C2-C6 alkenyl, C2-C6 alkynyl, cyano, hydroxy, oxo, carboxyl, cycloalkyl, cycloalkenyl, heterocyclyl, heteroaryl, aryl, keto, alkoxycarbonyl, aryloxycarbonyl, heteroaryloxycarbonyl, or halogens (e.g., F, Cl, Br, I). When the substituent is ketone or oxo (i.e., =O), then two (2) hydrogens on the atom are replaced. "Substituted with one or more" means that it may be substituted with a single substituent or multiple substituents. In the case of substitution with multiple substituents, there may be a plurality of identical substituents, or one combination of or a plurality of combinations of different substituents.

Some abbreviations in the present disclosure are defined as follows:

DCE: dichloroethane;
$Sc(OTf)_3$: scandium trifluoromethanesulfonate;
TFH: tetrahydrofuran;
Pd/C: palladium-carbon;
TFA: trifluoroacetic acid;
DMF: dimethylformamide;
DIPEA: N-ethyl diisopropylamine;
HoBt: 1-hydroxybenzotriazole;
EDCI: 1-ethyl-(3-dimethylaminopropyl)carbodiimide hydrochloride;
DMTrCl: 4,4 -dimethoxytrityl chloride;
DIEA: N,N-diisopropylethylamine;
HATU: 2-(7-azabenzotriazole)-N,N,N',N'-tetramethyluronium hexafluorophosphate;
LiOH: lithium hydroxide;
DMAP: 4-dimethylaminopyridine;
HBTU: benzotriazole-N,N,N',N'-tetramethyluronium hexafluorophosphate;
DMTrCl: 1-[chloro(4-methoxyphenyl)benzyl]-4-methoxybenzene
$CF_3SO_3H$: trifluoromethanesulfonic acid;
BnBr: benzyl bromide;

DEPBT: 3-(diethoxyphosphoryloxy)-1,2,3-benzotriazin-4-one;
Bz: a benzoyl protecting group;
MMTr: methoxyphenyl diphenylmethyl;
DMTr: a dimethoxytrityl protecting group.

## BRIEF DESCRIPTION OF THE DRAWINGS

FIG. 1 shows the TTR mRNA expression levels at day 7 after administration of TRD002218 and TRD007205.

FIG. 2 shows the TTR mRNA expression levels at day 28 after administration of TRD002218 and TRD007205.

## DETAILED DESCRIPTION

The present disclosure is further described below with reference to examples; however, these examples are not intended to limit the scope of the present disclosure. Experimental procedures without conditions specified in the examples of the present disclosure were generally conducted under conventional conditions or conditions recommended by the manufacturers of the starting materials or commercial products. Where the source of a reagent is not shown, the reagent may be obtained from any supplier of reagents for molecular biology at a quality/purity level for application in molecular biology.

### Part One. Anti-Off-Target Modifications

### Example 1. Preparation of Chemical Modifications

### 1.1. Synthesis of compound 1-1a and compound 1-1b

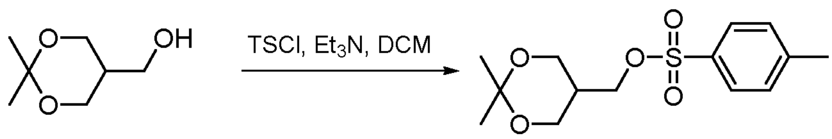


**1** **2**

Compound 1 (500 mg, 3.42 mmol) and triethylamine ($Et_3N$, 692 mg, 6.84 mmol, 0.95 mL) were dissolved in dichloromethane (DCM, 10 mL). A solution of 4-toluenesulfonyl chloride (tsCl, 717 mg, 3.76 mmol) in dichloromethane (10 mL) was added dropwise under an ice bath. After the dropwise addition, the mixture was stirred overnight at room temperature. After the reaction was complete, the reaction mixture was quenched with water. The aqueous phase was extracted three times with dichloromethane (15 mL). The combined organic phases were washed first with saturated aqueous sodium bicarbonate solution (10 mL) and then with saturated brine (20 mL) and were then concentrated under reduced pressure to remove the solvent to give a crude product **2** (820 mg, 80%). The crude product was directly used in the next step. MS m/z: $C_{14}H_{21}O_5S$, $[M+H]^+$ calculated: 301.10, found: 301.2.

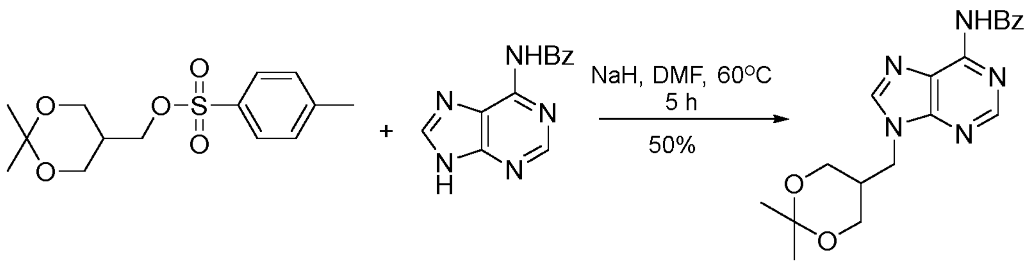


**2** **3** **4**

Compound **3** (239 mg, 1.22 mmol) was dissolved in dimethylformamide (DMF, 10 mL). A solution of NaH (60%

in mineral oil, 93 mg, 2.33 mmol) was added under an ice bath. The mixture was stirred for 30 min, and then compound **2** (350 mg, 1.16 mmol) was added dropwise. After the dropwise addition, the mixture was stirred at 60 °C for 5 h. After the reaction was complete, the reaction mixture was quenched with water. The aqueous phase was extracted three times with ethyl acetate (15 mL). The combined organic phases were washed first with water (10 mL) three times and then with saturated brine (10 mL) and were then concentrated under reduced pressure to remove the solvent. The residue was purified by preparative reversed-phase HPLC ($C^{18}$, conditions: 5-50% (A: $H_2O$, B: $CH_3CN$), flow rate: 70 mL/min) and lyophilized to give compound **4** (220 mg). MS m/z: $C_{19}H_{21}N_5O_3Na$, $[M+Na]^+$ calculated: 390.16, found: 390.3.

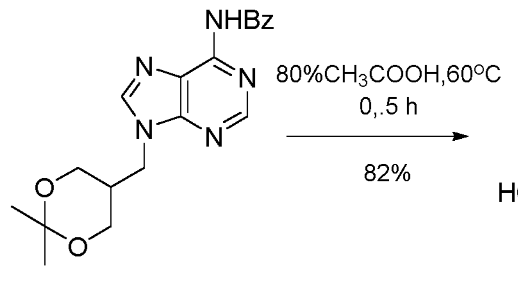


**4** **5**

Compound **4** (1.50 g, 4.08 mmol) was dissolved in 20 mL of a mixed solution of acetic acid and water (4:1) at room temperature, and the mixture was stirred at 60 °C for 30 min. After the reaction was complete, the reaction mixture was concentrated under reduced pressure to remove the solvent. The residue was purified by preparative reversed-phase HPLC ($C^{18}$, conditions: 5-25% (A: $H_2O$, B: $CH_3CN$), flow rate: 70 mL/min) and lyophilized to give compound **5** (1.10 g). MS m/z: $C_{16}H_{18}N_5O_3$, $[M+H]^+$ calculated: 328.13, found: 328.4.

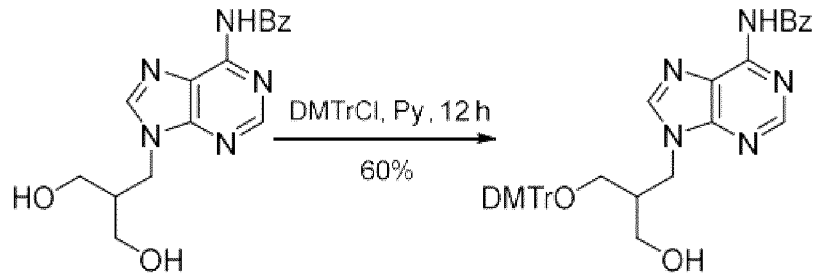


**5** **6**

Compound **5** (1.00 g, 3.05 mmol) was dissolved in pyridine (Py, 10 mL). A solution of 4,4'-dimethoxytrityl chloride (DMTrCl, 1.50 g, 4.58 mmol) in pyridine (5 mL) was added dropwise under an ice bath. After the dropwise addition, the mixture was stirred overnight at room temperature. After the reaction was complete, the reaction mixture was quenched with water and concentrated under reduced pressure to remove the solvent. The residue was purified by preparative reversed-phase HPLC ($C^{18}$, conditions: 5-80% (A: $H_2O$, B: $CH_3CN$), flow rate: 70 mL/min) and lyophilized to give compound **6** (1.00 g). MS m/z: $C_{37}H_{36}N_5O_5$, $[M-H]^+$ calculated: 630.26, found: 630.5. The racemic compound **6** was resolved using a chiral column (Daicel CHIRALPAK® IE 250 × 4.6 mm, 5 μm, A: n-hexane, B: ethanol) to give 6A(-) (410 mg) and 6B(+) (435 mg).

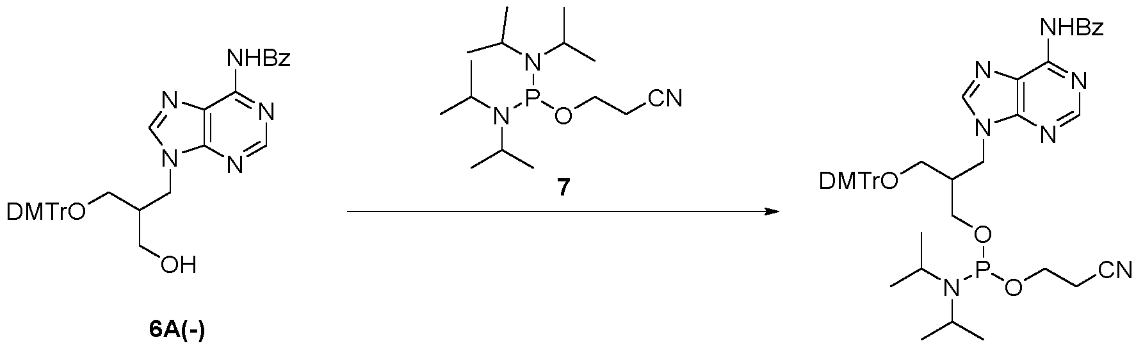

6A(-)

7

1-1a

Compound **6A(-)** (200 mg, 0.32 mmol), tetrazole (11 mg, 0.16 mmol), N-methylimidazole (5 mg, 0.06 mmol), and 3A molecular sieve (500 mg) were dissolved in 10 mL of acetonitrile. Compound **7** (144 mg, 0.48 mmol) was added at room temperature. The mixture was stirred overnight at room temperature. After the reaction was complete, the molecular sieve was filtered out, and dichloromethane (30 mL) was added. The mixture was washed with saturated aqueous sodium bicarbonate solution (10 mL) three times and then with saturated brine (20 mL). The filtrate was concentrated to dryness by rotary evaporation, and the residue was purified by preparative reversed-phase HPLC ($C^{18}$, conditions: 5-100% (A: water, B: $CH_3CN$), flow rate: 70 mL/min) and lyophilized to give compound **1-1a** (200 mg). MS m/z: $C_{40}H_{39}N_6O_7P$, $[M\text{-diisopropyl+OH}]^+$ calculated: 747.26, found: 747.6. 1H NMR (400 MHz, acetonitrile-*d*$_3$) $\delta$ 7.56, 7.54 (2s, 1H), 7.36-7.27 (m, 2H), 7.24-7.21 (m, 7H), 6.83-6.80 (m, 4H), 4.12-4.10 (m, 2H), 3.75-3.68 (m, 10H), 3.20-2.80 (m, 2H), 2.68-2.54 (m, 4H), 1.22-1.04 (m, 18H).

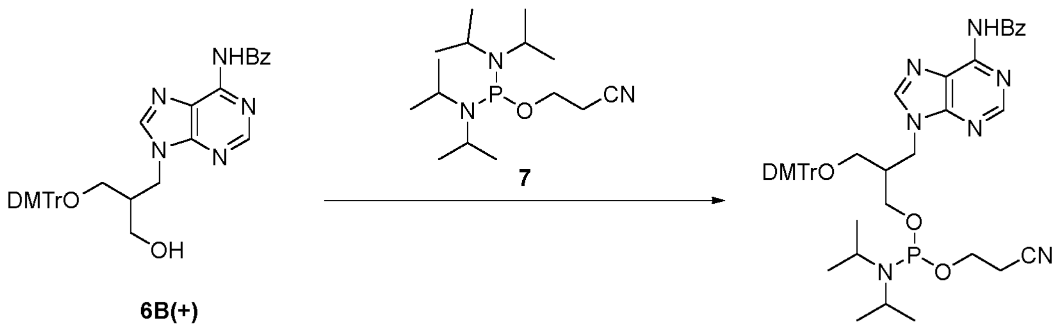

6B(+)

7

1-1b

Compound **6B**(+) (200 mg, 0.32 mmol), tetrazole (11 mg, 0.16 mmol), N-methylimidazole (5 mg, 0.06 mmol), and 3A molecular sieve (500 mg) were dissolved in 10 mL of acetonitrile. Compound 7 (144 mg, 0.48 mmol) was added at room temperature. The mixture was stirred overnight at room temperature. After the reaction was complete, the molecular sieve was filtered out, and dichloromethane (30 mL) was added. The mixture was washed with saturated aqueous sodium bicarbonate solution (10 mL) three times and then with saturated brine (20 mL). The filtrate was concentrated to dryness by rotary evaporation, and the residue was purified by preparative reversed-phase HPLC ($C^{18}$, conditions: 5-100% (A: water, B: $CH_3CN$), flow rate: 70 mL/min) and lyophilized to give compound **1-1b** (200 mg). MS m/z: $C_{40}H_{39}N6O_7P$, $[M\text{-diisopropyl+OH}]^+$ calculated: 747.26, found: 747.5.

**1.2. Synthesis of compound 1-6a**

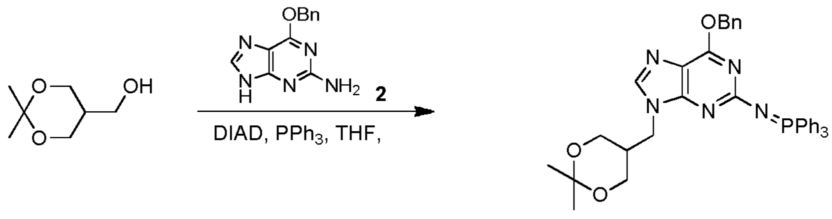


1 3

Compound 1 (10 g, 68.404 mmol), compound 2 (15 g, 62.186 mmol), and triphenylphosphine (32.62 g, 124.371 mmol) were dissolved in anhydrous THF (30 mL). DIAD (24.656 mL, 124.371 mmol) was slowly added dropwise at 0 °C. The reaction mixture was left to react at 25 °C for 12 h, and LCMS analysis showed the reaction had been complete. The reaction mixture was extracted with ethyl acetate (200 mL) and water (200 mL). The organic phase was dried. The filtrate was concentrated, and the resulting residue was purified using a normal-phase column (DCM/MeOH = 10/1) to give the target product 3 (20 g).

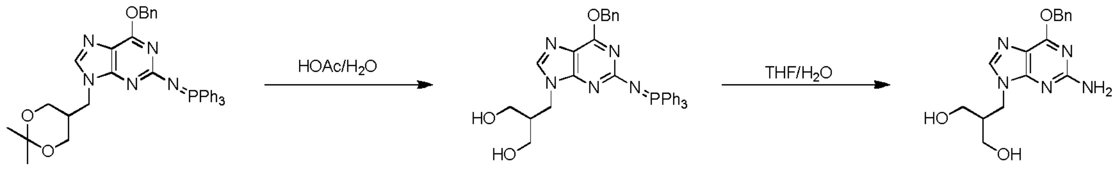


3 4 5

Compound **3** (20 g, 28.585 mmol) was dissolved in acetic acid (24 mL, 426.016 mmol) and H2O (12 mL). The mixture was stirred at 60 °C for 1 h. The reaction mixture was then concentrated to dryness by rotary evaporation. THF (12 mL) and H2O (12 mL) were added. The mixture was stirred at 80 °C for 7 h. LCMS analysis showed the reaction had been complete. The reaction mixture was extracted with ethyl acetate (200 mL) and water (100 mL). Solid sodium carbonate was added to the aqueous phase until a large amount of solid precipitated from the aqueous phase. The solid was collected by filtration and washed with water. The filter cake was dried using an oil pump to give the target compound **5** (9 g).

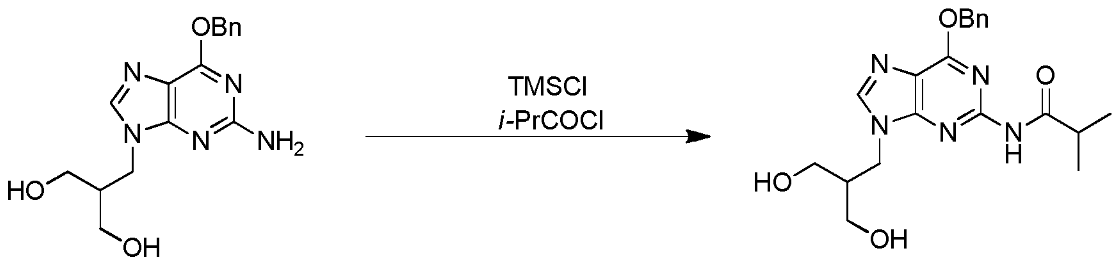


**5** **6**

Compound 5 (6.8 g, 18.581 mmol) was dissolved in pyridine (80 mL) in a nitrogen atmosphere. TMSCl (14.250 mL, 111.489 mmol) was slowly added at 0 °C. The mixture was stirred for 2 h. Isobutyryl chloride (2.044 mL, 19.511 mmol) was then added at 0 °C. The mixture was stirred at 25 °C for 1 h, and LCMS analysis showed the reaction had been complete. The reaction mixture was extracted with dichloromethane (200 mL) and water (200 mL). After the organic phase was dried and concentrated to dryness by rotary evaporation, a sample to be purified was prepared. The sample was purified using a normal-phase column (elution with DCM:MeOH = 10:1, peak at 4.8%) to give compound 6 as a yellow oil (12 g).

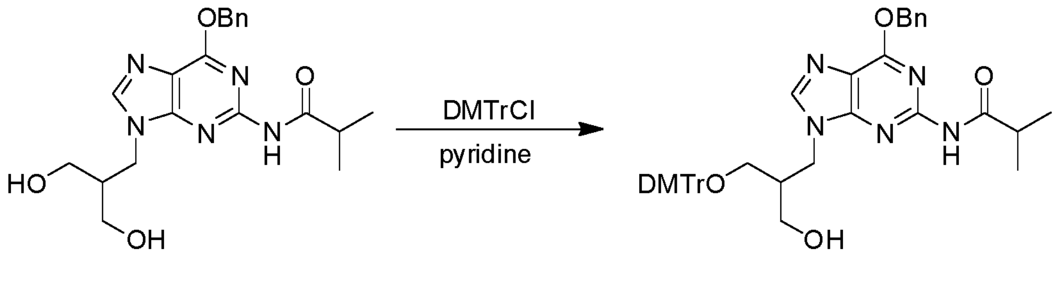


6 7

Compound 6 (5.5 g, 12.392 mmol) was dissolved in pyridine (30 mL) in a nitrogen atmosphere. Molecular sieve 4A 1/16 (7 g, 12.392 mmol) was added, and then solid DMTrCl (5.04 g, 14.870 mmol) was added in batches at 0 °C. The mixture was left to react at 25 °C for 2 h, and TLC analysis (PE:EtOAc = 1:1, Rf = 0.69) showed the reaction had been complete. The reaction mixture and TJN200879-040-P1 were combined and treated together. The reaction mixture was extracted with ethyl acetate (200 mL) and water (200 mL). After the organic phase was dried and concentrated to dryness by rotary evaporation, a sample to be purified was prepared. The sample was purified using a normal-phase column (elution with PE:EtOAc, peak at 84%) to give compound 7 as a yellow oil (12 g).

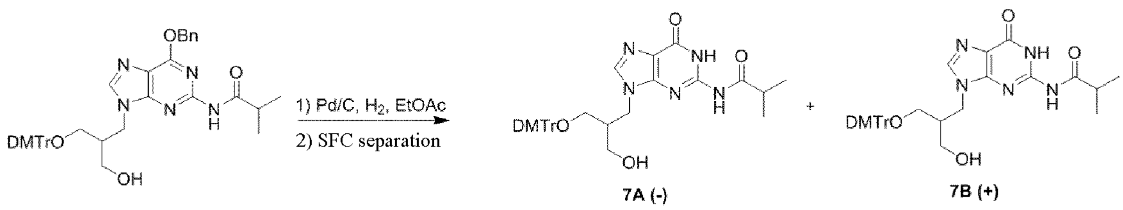


7 7A (-) 7B (+)

Compound 7 (12 g, 15.389 mmol) was dissolved in EtOAc (140 mL). Wet palladium on carbon Pd/C (7 g, 15.389 mmol) was added. The reaction mixture was left to react at 25 °C in a hydrogen atmosphere (15 Psi) for 2 h. TLC analysis (PE:EtOAc = 0: 1, Rf = 0.09) showed the reaction had been complete. The reaction mixture was filtered. After the filter cake was rinsed three times with ethyl acetate (30 mL), the filtrate was collected. After the filtrate was concentrated to dryness by rotary evaporation, 50 mL of dichloromethane and 2 mL of triethylamine were added to prepare a sample to be purified. The sample was purified using a normal-phase column (elution with DCM:MeOH = 10:1, peak at 0.5%) to give 9 g (a yellow foamy solid). The resulting racemic compound was resolved by SFC to give products, the target compound 7A(-) (3.9 g) and the target compound 7B(+) (3.8 g).

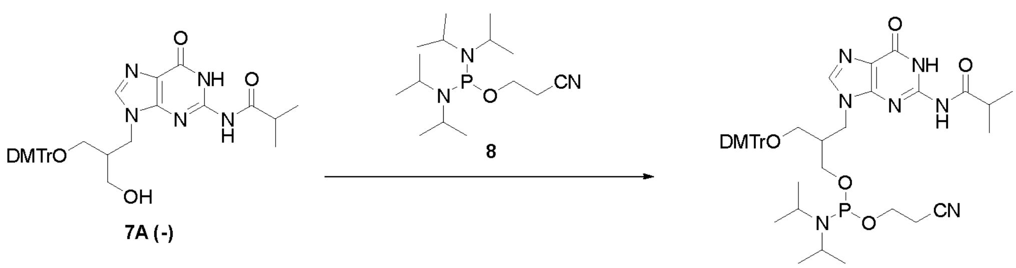

7A (-) 8 1-6a

Compound 7A(-) (3.30 g, 5.40 mmol), tetrazole (190 mg, 2.70 mmol), 1-methylimidazole (90 mg, 1.10 mmol), and 3A molecular sieve (500 mg) were dissolved in 30 mL of acetonitrile. Compound 8 (2.50 g, 8.10 mmol) was added at room temperature. The mixture was stirred at room temperature for 2 h. After the reaction was complete, the molecular sieve was filtered out, and DCM (150 mL) was added. The mixture was washed with saturated aqueous sodium bicarbonate solution (30 mL × 3) and then with saturated brine (30 mL). The filtrate was concentrated to dryness by rotary evaporation, and the residue was purified by preparative reversed-phase HPLC (C18, conditions: 5-100% (A: water, B: CH3CN), flow rate: 70 mL/min) and lyophilized to give compound 1-6a (2.9 g, 66%). MS m/z: C43H55N7O7P [M+H]+, calculated: 812.38, found: 812.5. 1H NMR (400 MHz, acetonitrile-d3) δ 7.56, 7.54 (2s, 1H), 7.36-7.27 (m, 2H), 7.24-7.21 (m, 7H), 6.83-6.80 (m, 4H), 4.12-4.10 (m, 2H), 3.75-3.68 (m, 10H), 3.20-2.80 (m, 2H), 2.68-2.54 (m, 4H), 1.22-1.04 (m,

18H).

**1.3. Synthesis of compound 1-7a**

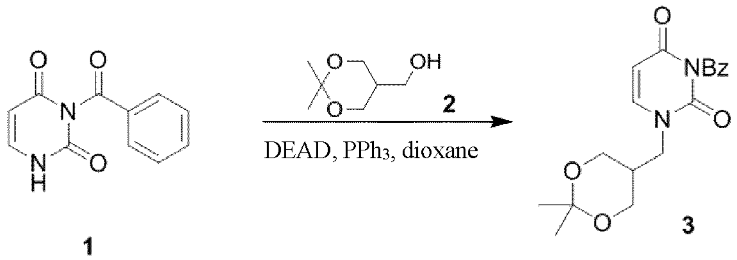


1 3

Compound 1 (5 g, 23.1272 mmol), compound 2 (6.76 g, 46.254 mmol), and triphenylphosphine (7.28 g, 27.753 mmol) were dissolved in 30 mL of dioxane in a nitrogen atmosphere. DEAD (5.502 mL, 27.753 mmol) was slowly added dropwise at 0 °C. After the dropwise addition, the reaction mixture was slowly warmed to 25 °C and left to react for another hour. 100 mL of H2O and 100 mL of EtOAc were added to the reaction mixture for extraction. After the organic phases were combined, dried, filtered, and concentrated, a sample to be purified was prepared. The sample was purified using a normal-phase column (elution with PE:EtOAc = 1: 1) to give the target product (4 g).

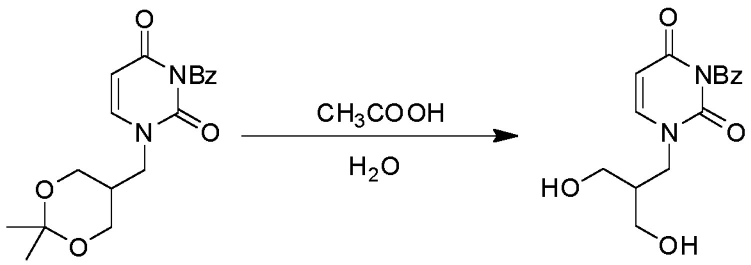


3 4

Compound 3 (3.3 g) was dissolved in HOAc (16 mL) and H2O (4 mL), and the solution was heated in an oil bath at 60 °C for 0.5 h. The reaction mixture was concentrated to dryness by rotary evaporation, and the resulting residue was purified using a normal-phase column (elution with PE:EtOAc = 0:1) to give the target product 4 (3 g).

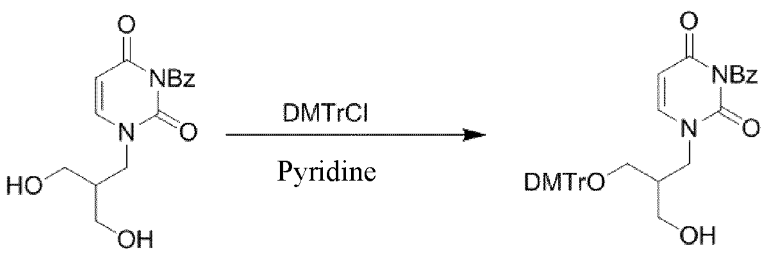


4 5

Compound 4 (3 g, 8.873 mmol) was dissolved in 5 mL of pyridine. A solution of DMTrCl (3.91 g, 11.535 mmol) in 10 mL of pyridine was slowly added dropwise at 0 °C in a nitrogen atmosphere. After the dropwise addition, the reaction mixture was warmed to 25 °C and left to react for another hour. 50 mL of water and 100 mL of ethyl acetate were added to the reaction mixture for extraction. The aqueous phase was extracted three times with 100 mL of ethyl acetate. The organic phases were combined, dried, filtered, and concentrated, and the residue was purified using a normal-phase column (with PE:EtOAc = 2:1) to give the target product 5 (4 g).

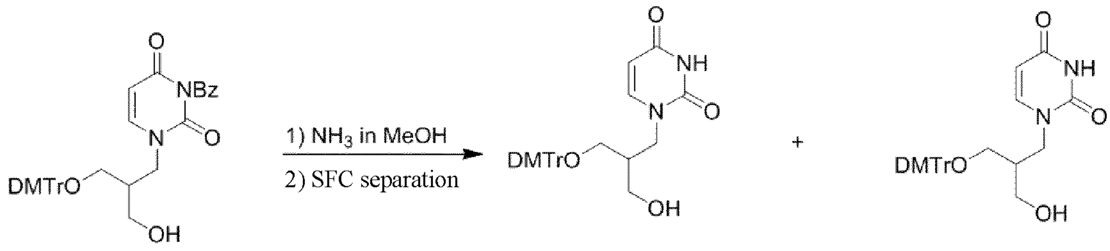


5 6A (-) 6B (+)

Compound 5 (4 g, 5.769 mmol) was dissolved in methanol (10 mL). A saturated solution of NH3 in methanol (40 mL) was added. The mixture was left to react at 0 °C for 6 h. The reaction mixture was concentrated to dryness by rotary evaporation, and the residue was purified using a normal-phase column (PE:EtOAc = 0:1) to give a racemic compound (2.4 g). The racemic compound was resolved by SFC to give the target product 6A (750 mg, 100% purity) and the target product 6B (400 mg, 99.16% purity).

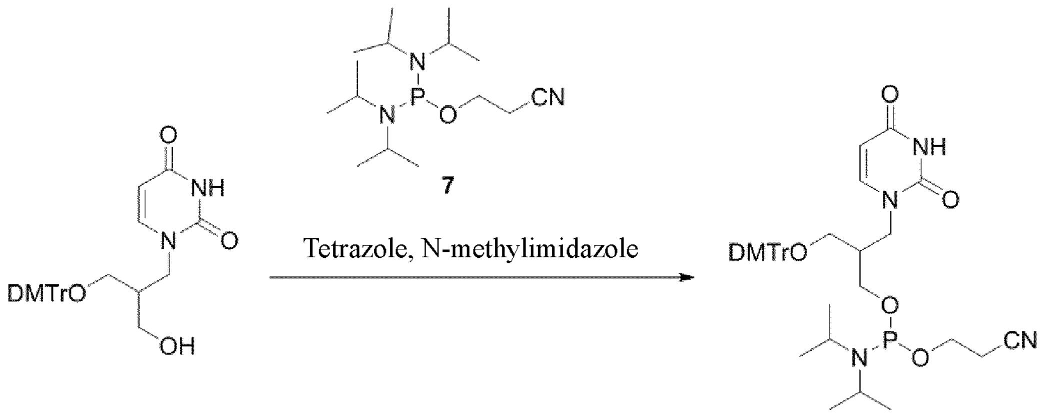


7

6A (-) 1-7a

Compound 6A(-) (700 mg, 1.40 mmol), tetrazole (50 mg, 0.70 mmol), 1-methylimidazole (23 mg, 0.28 mmol), and 3A molecular sieve (500 mg) were dissolved in 10 mL of acetonitrile. Compound 7 (630 mg, 2.10 mmol) was added at room temperature. The mixture was stirred at room temperature for 2 h. After the reaction was complete, the molecular sieve was filtered out, and DCM (50 mL) was added. The mixture was washed with saturated aqueous sodium bicarbonate solution (10 mL × 3) and then with saturated brine (20 mL). The filtrate was concentrated to dryness by rotary evaporation, and the residue was purified by preparative reversed-phase HPLC (C18, conditions: 5-100% (A: water, B: CH3CN), flow rate: 70 mL/min) and lyophilized to give compound **1-7a** (700 mg, 72%). MS m/z: C38H47N4O7PNa [M+Na]+, calculated: 725.32, found: 725.5.

**1.4. Synthesis of compound 1-8a**

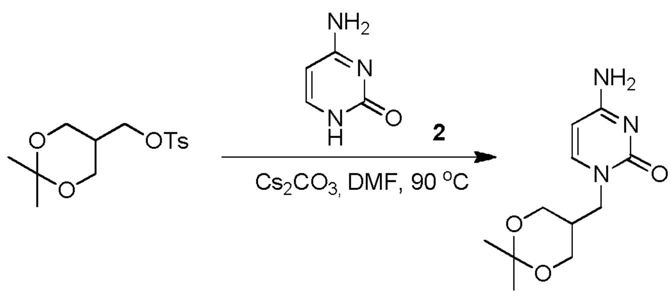


2

1 3

Compound 1 (8.5 g, 76.508 mmol) and compound 2 (30.64 g, 91.809 mmol) were dissolved in DMF (150 mL). CS2CO3 (29.91 g, 91.809 mmol) was added. The mixture was left to react in a nitrogen atmosphere at 90 °C for 12 h. LCMS analysis showed the reaction had been complete. The reaction mixture was filtered and concentrated to dryness by rotary evaporation using an oil pump, and the residue was separated and purified using a normal-phase column (80 g, DCM/MeOH = 10/1 to 5/1) to give the target product 3 (13.5 g, 80% purity).

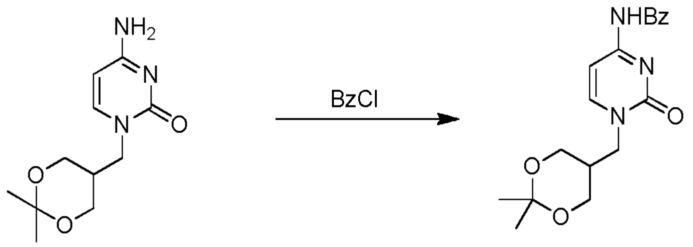


**3** **4**

Compound 3 (10.5 g, 35.105 mmol) was dissolved in pyridine (65 mL) and CH3CN (65 mL). BzCl (4.894 mL, 42.126 mmol) was added dropwise to the solution. The mixture was left to react at 25 °C for 2 h. LCMS analysis showed most of the starting material had been consumed. The reaction mixture was quenched with H2O (100 mL) and extracted with EtOAc (100 mL × 3). The organic phase was dried and concentrated to dryness by rotary evaporation, and the residue was separated (combined with TJN200872-101) and purified by column chromatography (80 g, PE/EtOAc = 10/1 to 0/1, DCM/MeOH = 10/1) to give the target product **4** (14 g, 90% purity).

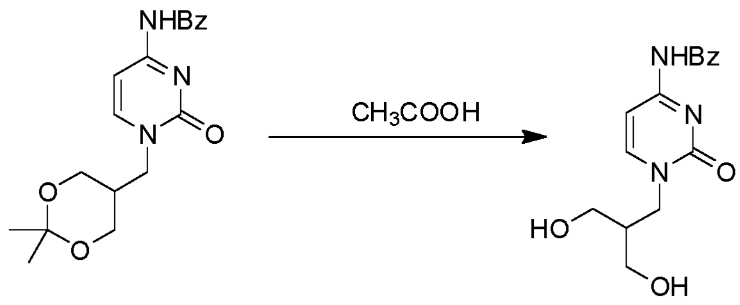


**4** **5**

Compound 4 (14 g, 36.694 mmol) was dissolved in HOAc (56 mL, 314.796 mmol) and H2O (14 mL). The mixture was left to react at 60 °C for 2 h, and LCMS analysis showed the reaction had been complete. The reaction mixture was concentrated using an oil pump, and the residue was separated using a normal-phase column (40 g, DCM/MeOH = 1/0 to 5/1) to give the target product 5 (8.4 g, 90% purity & 2.4 g, 80% purity).

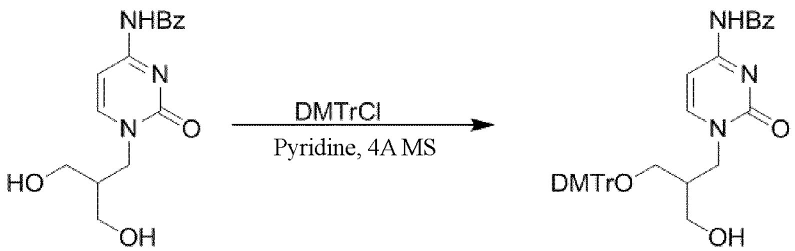


**5** **6**

Compound 5 (7.4 g, 21.957 mmol), DMAP (0.54 g, 4.391 mmol), and molecular sieve 4A (11.1 g, 2.967 mmol) were dissolved in pyridine (60 mL). The mixture was stirred under an ice bath for 10 min, and then DMTrCl (8.93 g, 26.348 mmol) was added. The mixture was stirred for 1.8 h, and LCMS analysis showed about 19% of the starting material remained and about 60% was target MS. The reaction mixture and TJN200872-105&106 were combined and

purified together. $H_2O$ (50 mL) was added to the reaction mixture, and extraction was performed with DCM (50 mL × 3). The organic phase was dried and concentrated to dryness by rotary evaporation, and the residue was separated by column chromatography (120 g, PE/(EA:DCM:TEA = 1:1:0.05) = 1/0 to 0/1 to DCM/MeOH = 10/1) to give compound 6 as a yellow solid (11 g, 89% purity, TJN200872-105&106&107). The starting material was recovered (3.0 g, 70% purity).

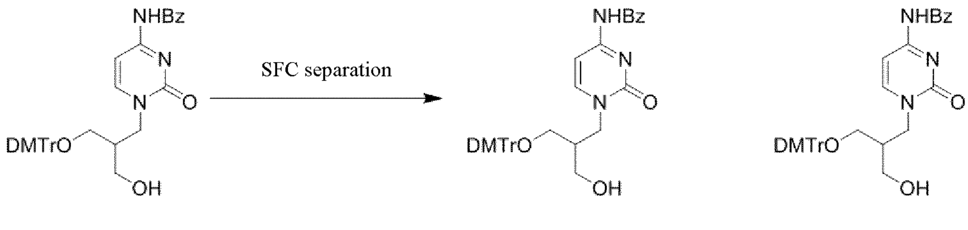


6 6A (+) 6B (-)

Compound 6 (15 g, 22.041 mmol) was resolved by SFC (DAICEL CHIRALPAK AD (250 mm × 50 mm,10 μm); 0.1% $NH_3H_2O$ EtOH, B: 45%-45%; 200 mL/min) to give the target product 6A (5.33 g, 94.29% purity) and the target product 6B (6.14 g, 97.91% purity). 1.0 g of compound 6 was recovered.

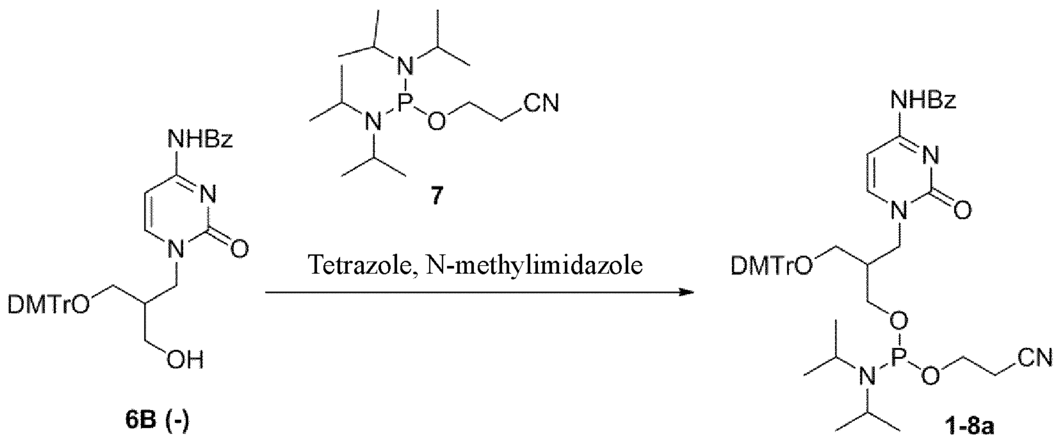


7

6B (-) 1-8a

Compound 6B(-) (5.4 g, 8.92 mmol), tetrazole (312 mg, 4.46 mmol), 1-methylimidazole (146 mg, 1.78 mmol), and 3A molecular sieve (500 mg) were dissolved in 40 mL of acetonitrile. Compound 7 (4 g, 13.4 mmol) was added at room temperature. The mixture was stirred at room temperature for 2 h. After the reaction was complete, the molecular sieve was filtered out, and DCM (200 mL) was added. The mixture was washed with saturated aqueous sodium bicarbonate solution (30 mL × 3) and then with saturated brine (50 mL). The filtrate was concentrated to dryness by rotary evaporation, and the residue was purified by preparative reversed-phase HPLC (C18, conditions: 5-100% (A: water, B: $CH_3CN$), flow rate: 70 mL/min) and lyophilized to give compound **1-8a** (5.8 g, 80%). MS m/z: $C_{45}H_{51}N_5O_7P$, $[M+H]^+$, calculated: 804.36, found: 804.4.

**Example 2. Synthesis of siRNA**

The siRNA synthesis was the same as the conventional phosphoramidite solid-phase synthesis. In synthesizing the modified nucleotide at position 7 of the 5' end of the AS strand, the original nucleotide of the parent sequence was replaced with the phosphoramidite monomer synthesized above. The synthesis process is briefly described below: Nucleoside phosphoramidite monomers were linked one by one according to the synthesis program on a Dr. Oligo48 synthesizer (Biolytic), starting at a universal CPG support. Other than the phosphoramidite monomer at position 7 of the 5' end of the AS strand described above, the other nucleoside monomer materials 2'-F RNA, 2'-O-methyl RNA, and other nucleoside phosphoramidite monomers were purchased from Hongene, Shanghai or Genepharma, Suzhou. 5-Ethylthio-1H-tetrazole (ETT) was used as an activator (a 0.6 M solution in acetonitrile), a 0.22 M solution of PADS in acetonitrile and collidine (1:1 by volume) (Kroma, Suzhou) as a sulfurizing agent, and iodopyridine/water solution (Kroma) as an oxidant.

After completion of solid phase synthesis, oligoribonucleotides were cleaved from the solid support and soaked in a solution of 28% ammonia water and ethanol (3:1) at 50 °C for 16 h. The mixture was centrifuged, and the supernatant was transferred to another centrifuge tube. After the supernatant was concentrated to dryness by evaporation, the residue

was purified by C18 reversed-phase chromatography using 0.1 M TEAA and acetonitrile as the mobile phase, and DMTr was removed using 3% trifluoroacetic acid solution. The target oligonucleotides were collected, then lyophilized, identified as the target products by LC-MS, and quantified by UV (260 nm).

The resulting single-stranded oligonucleotides were paired in an equimolar ratio in a complementary manner and annealed. The final double-stranded siRNA was dissolved in 1× PBS, and the solution was adjusted to the concentration required for the experiment so it was ready to be used.

**Example 3. psiCHECK Activity Screening**

The synthesis of siRNA samples is as described before. The plasmids were obtained from Sangon Biotech (Shanghai) Co., Ltd. The experimental consumables for psiCHECK are shown in Table 1.

Table 1. Experimental consumables and reagents for psiCHECK

| **Reagent consumables** | | | | |
|---|---|---|---|---|
| **Name** | **Company** | **Catalog number/model** | **Batch No.** | **Shelf life** |
| Huh 7 cells | Cobioer, Nanjing | ATCC-Cobioer/CBP60202 | / | / |
| Dual-Glo® Luciferase Assay System | Promega | E2940 | 0000363099 | 2020/5/13 |
| Lipofectamine® 2000 | Invitrogen | 11668-019 | / | 2021/6/14 |

Experimental procedure: Cell plating and cell transfection were carried out. The specific amounts for preparing the transfection complex are shown in Table 2.

Table 2. Amounts required for transfection complex in each well of a 96-well plate

| | Amount/well | Opti-MEM |
|---|---|---|
| Plasmid Mix | 0.05 μL | 10 μL |
| Lipofectamine 2000 | 0.2 μL | 10 μL |
| Notes: Lipo: 0.2 μL/well; plasmid: 0.05 μL/well; Opti-MEM: 10 μL/well. | | |

According to Table 3, dilutions with different concentrations were prepared as working solutions for immediate use based on the different experimental requirements. 24 h after transfection, assays were carried out according to the instructions of the Dual-Glo® Luciferase Assay System kit.

Calculation of a relative value:

$$\text{Ratio} = \text{Ren/Fir (a renilla/firefly ratio)};$$

Calculation of the inhibition rate:

1- (ratio + siRNA/reporter gene only) × 100% = inhibition rate (%);

In the present disclosure, residual activity% (also referred to as the remaining mRNA expression level% or remaining mRNA expression ratio) = 100% - inhibition rate (%).

Table 3. Multi-concentration dilution protocol

| Final concentration (nM) | Addition of water and sample |
|---|---|
| / | / |
| 40 | 4 μL siRNA(20 NM) + 96 μL $H_2O$ |

(continued)

| Final concentration (nM) | Addition of water and sample |
|---|---|
| 13.33333333 | 30 μL siRNA + 60 μL $H_2O$ |
| 4.444444444 | 30 μL siRNA + 60 μL $H_2O$ |
| 1.481481481 | 30 μL siRNA + 60 μL $H_2O$ |
| 0.49382716 | 30 μL siRNA + 60 μL $H_2O$ |
| 0.164609053 | 30 μL siRNA + 60 μL $H_2O$ |
| 0.054869684 | 30 μL siRNA + 60 μL $H_2O$ |
| 0.018289895 | 30 μL siRNA + 60 μL $H_2O$ |
| 0.006096632 | 30 μL siRNA + 60 μL $H_2O$ |
| 0.002032211 | 30 μL siRNA + 60 μL $H_2O$ |
| 0.000677404 | 30 μL siRNA + 60 μL $H_2O$ |

**Example 4. On-Target and Off-Target Activity Experiments of siRNAs Comprising Different Chemical Modifications**

The siRNAs were synthesized using the compounds of Example 1 and the method of Example 2, and the on-target activity and off-target activity of each siRNA were verified using the method of Example 3. The siRNAs had identical sense strands and contained the following modified nucleotides/chemical modifications, respectively, at position 7 of the 5' end of the antisense strand:

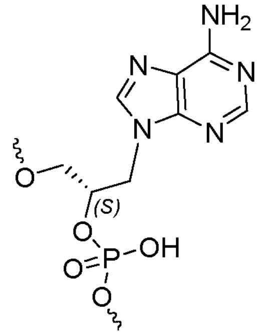
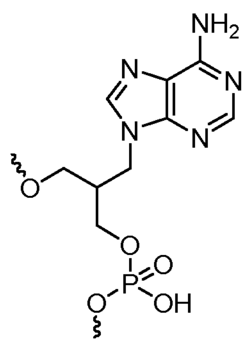
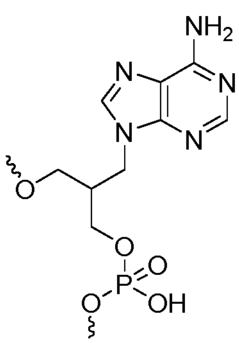

GNA(A) (-)hmpNA(A) (+)hmpNA(A)

wherein the nucleotide synthesized using 2-hydroxymethyl-1,3-propanediol as the starting material was defined as hmpNA;
(+)hmpNA(A) was obtained by solid-phase synthesis using the nucleoside phosphoramidite monomer 1-1b of example section 1.1, and its absolute configuration was (S)-hmpNA(A);
(-)hmpNA(A) was obtained by solid-phase synthesis using the nucleoside phosphoramidite monomer 1-1a of example section 1.1, and its absolute configuration was (*R*)-hmpNA(A).

Similarly, the following structures were obtained by solid-phase synthesis and by changing the base species of hmpNA, and their absolute configurations were determined:

(+)hmpNA(G), with the absolute configuration (*S*)-hmpNA(G);
(-)hmpNA(G), with the absolute configuration (*R*)-hmpNA(G);
(+)hmpNA(C), with the absolute configuration (*S*)-hmpNA(C);
(-)hmpNA(C), with the absolute configuration (*R*)-hmpNA(C);
(+)hmpNA(U), with the absolute configuration (*R*)-hmpNA(U); and

(-)hmpNA(U), with the absolute configuration (*S*)-hmpNA(U).

The absolute configurations (*S*)-hmpNA(G), (*R*)-hmpNA(G), (*S*)-hmpNA(C), (*R*)-hmpNA(C), (*S*)-hmpNA(U), and (*R*)-hmpNA(U) were determined from their intermediates or derivatives by X-Ray diffraction.

The structures of the intermediates or derivatives are:

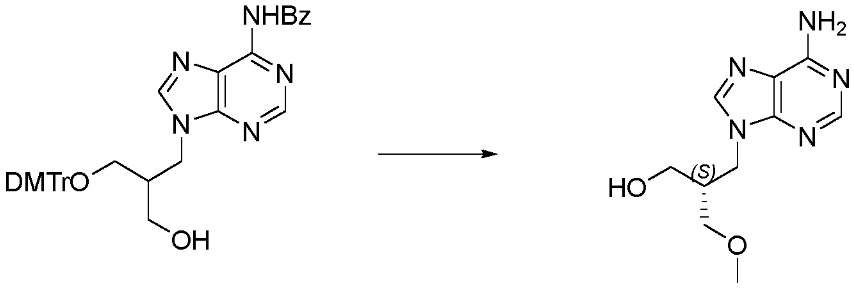

**15.1 6B(+)** **TJ-NA067**

**TJ-NA067:** determined as a colorless massive crystal (0.30 × 0.10 × 0.04 mm3), belonging to the monoclinic crystal system with a P21 space group. Lattice parameter a = 16.0496(5) Å, b = 4.86260(10) Å, c = 16.4686(5) Å, $\alpha$ = 90°, $\beta$ = 118.015(4)°, $y$ = 90°, V = 1134.65(7) Å3, Z = 4. Calculated density Dc = 1.389 g/cm3; the number of electrons in a unit cell F(000) = 504.0; linear absorption coefficient of a unit cell $\mu$ (Cu K$\alpha$) = 0.840 mm-1; diffraction experiment temperature T = 150.00(11) K.

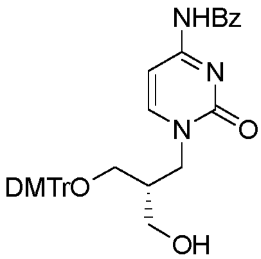

**15.8 6A (+)**

6A(+): determined as a colorless massive crystal (0.30 × 0.20 × 0.10 mm3), belonging to the monoclinic crystal system with a P21 space group. Lattice parameter a = 22.6688(7) Å, b = 8.5595(2) Å, c = 23.3578(5) Å, $\alpha$ = 90°, $\beta$ = 113.876(3)°, $\gamma$ = 90°, V = 4144.3(2) Å3, Z = 2. Calculated density Dc = 0.999 g/cm3; the number of electrons in a unit cell F(000) = 1318.0; linear absorption coefficient of a unit cell $\mu$ (Cu K$\alpha$) = 0.570 mm-1; diffraction experiment temperature T = 100.01(18) K.

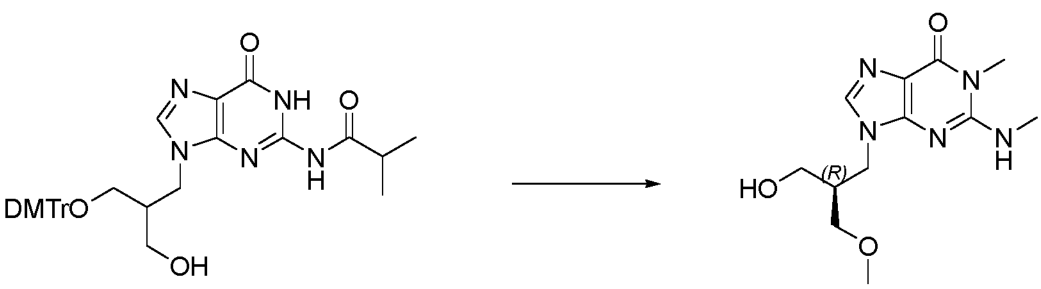

**15.6 7A (-)** **TJ-NA068**

**TJ-NA048:** determined as a colorless acicular crystal (0.30 × 0.04 × 0.04 mm3), belonging to the monoclinic crystal system with a P1 space group. Lattice parameter a = 7.6165(4) Å, b = 11.3423(5) Å, c = 17.3991(8) Å, $\alpha$ = 85.007(4)°, $\beta$ = 88.052(4)°, $\gamma$ = 70.532(4)°, V = 1411.75(12) Å3, Z = 2. Calculated density Dc = 1.366 g/cm3; the number of electrons in a unit cell F(000) = 620.0; linear absorption coefficient of a unit cell $\mu$ (Cu K$\alpha$) = 0.856 mm-1; diffraction

experiment temperature T = 150.00(13) K.

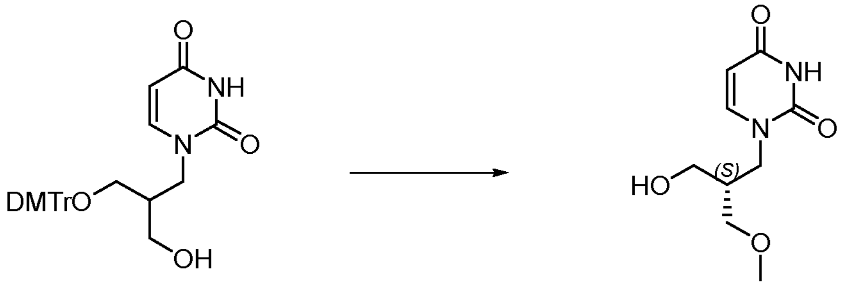

**15.7 6A (-)** **TJ-NA092**

**TJ-NA092:** determined as a colorless prismatic crystal (0.30 × 0.10 × 0.10 mm3), belonging to the triclinic crystal system with a P1 space group. Lattice parameter a = 5.17960(10) Å, b = 8.0667(2) Å, c = 12.4077(2) Å, $\alpha$ = 93.146(2)°, $\beta$ = 101.266(2)°, $\gamma$ = 96.134(2)°, V = 503.993(18) Å3, Z = 2. Calculated density Dc = 1.412 g/cm3; the number of electrons in a unit cell F(000) = 228.0; linear absorption coefficient of a unit cell $\mu$ (Cu K$\alpha$) = 0.945 mm-1; diffraction experiment temperature T = 100.00(10) K.

**Example 5. Sequence-Dependence Experiment of siRNAs Comprising Different Chemical Modifications**

The experimental compounds of the present disclosure were tested on multiple different sequences. siRNAs targeting the mRNAs of different genes were used, and position 7 of the 5' end of the AS strands was modified using **(+)hmpNA(A), (-)hmpNA(A),** and the compound GNA**(A)**, which was used as a control (the sequences are shown in Table 4-1 and Table 4-2).

Table 4-1. siRNA sense strands targeting different genes

| siRNA target gene | SS strand 5'-3' |
|---|---|
| ANGPTL3 | GmsAmsAmCmUfAmCfUfCfCmCmUmUmUmCmU mUmCmAm (SEQ ID NO:431) |
| HBV-S | CmsCmsAmUmUfUmGfUfUfCmAmGmUmGmGmU mUmCmsGm(SEQ ID NO:432) |
| HBV-X | CmsAmsCmCmUfCmUfGfCfAmCmGmUmCmGmCm AmUmsGm(SEQ ID NO:433) |

Table 4-2. siRNA antisense strands targeting different genes and comprising chemical modifications

| Target mRNA | siRNA double strand No. | AS strand modification (SEQ ID NO:) |
|---|---|---|
| ANGPTL3 | TRD5841 | UmsGfsAmAfGmAfGNA(A)AmGfGmGmAfGmUfAmGf UmUfCmsUmsUm (SEQ ID NO:369) |
| | TRD5845 | UmsGfsAmAfGmAf**(+)hmpNA(A)**AmGfGmGmAfGmUfAmGf UmUfCmsUmsUm (SEQ ID NO:370) |
| | TRD5846 | UmsGfsAmAfGmAf**(-)hmpNA(A)**AmGfGmGmAfGmUfAmGf UmUfCmsUmsUm(SEQ ID NO:371) |
| HBV-S | TRD5848 | UmsGfsAmAmCmCfGNA(A)CmUmGmAmAmCmAfAm AfUmGmGmsCmsAm(SEQ ID NO:372) |

(continued)

| Target mRNA | siRNA double strand No. | AS strand modification (SEQ ID NO:) |
|---|---|---|
| | TRD5852 | UmsGfsAmAmCmCf**(+)hmpNA(A)**CmUmGmAmAmCmAfAmAf UmGmGmsCmsAm (SEQ ID NO:373) |
| | TRD5853 | UmsGfsAmAmCmCf**(-)hmpNA(A)**CmUmGmAmAmCmAfAmAf UmGmGmsCmsAm(SEQ ID NO:374) |
| HBV-X | TRD5855 | UmsAfsUmGfCmGfGNA(A)CmGfUmGmCfAmGfAmGfGm UfGmsAmsAm(SEQ ID NO:375) |
| | TRD5859 | UmsAfsUmGfCmGf**(+)hmpNA(A)**CmGfUmGmCfAmGfAmGfGmUf GmsAmsAm(SEQ ID NO:376) |
| | TRD5860 | UmsAfsUmGfCmGf**(-)hmpNA(A)**CmGfUmGmCfAmGfAmGfGmUf GmsAmsAm (SEQ ID NO:377) |

The results of the on-target activity experiment are shown in Table 5. GNA**(A)** showed significant sequence dependence, and different sequences had significantly different on-target activity. The experimental compounds of the present disclosure did not show significant sequence dependence, which indicates that they are more universally applicable.

Table 5. On-target activity results of siRNAs for different target sequences

| Double strand code | Remaining percentage of target gene mRNA (on-target activity) expression (mean) | | | | | | | | | | |
|---|---|---|---|---|---|---|---|---|---|---|---|
| | 40 nM | 13.3 nM | 4.44 nM | 1.48 nM | 0.493 nM | 0.164 nM | 0.054 nM | 0.0182 nM | 0.00609 nM | 0.00203 nM | 0.00067 nM | IC50 value (nM) |
| TRD 5841 | 57.5% | 51.1% | 55.5% | 68.3% | 76.5% | 85.9% | 82.9% | 87.8% | 81.5% | 64.0% | 97.8% | >40 |
| TRD 5845 | 28.2% | 30.5% | 41.7% | 55.0% | 63.9% | 78.0% | 77.1% | 84.1% | 95.8% | 83.2% | 91.9% | 1.9953 |
| TRD 5846 | 31.6% | 26.8% | 34.1% | 59.1% | 84.8% | 102.1% | 97.2% | 108.9% | 95.6% | 107.2% | 102.1% | 1.9055 |
| TRD 5848 | 46.5% | 35.1% | 26.6% | 36.0% | 67.3% | 76.3% | 88.4% | 104.1% | 91.6% | 95.1% | 98.1% | 0.7943 |
| TRD 5852 | 24.7% | 17.5% | 13.1% | 21.1% | 40.5% | 64.1% | 84.3% | 94.5% | 88.4% | 100.2% | 95.1% | 0.2951 |
| TRD 5853 | 17.5% | 11.5% | 9.9% | 13.5% | 30.3% | 54.5% | 74.6% | 86.3% | 90.3% | 91.0% | 84.1% | 0.1905 |
| TRD 5855 | 41.3% | 40.7% | 36.9% | 73.6% | 71.7% | 87.0% | 89.0% | 85.8% | 94.9% | 104.4% | 101.6% | 4.2658 |
| TRD 5860 | 43.5% | 37.1% | 34.1% | 50.8% | 77.6% | 88.5% | 86.6% | 100.0% | 95.1% | 97.8% | 110.8% | 1.5488 |

The experimental results of the off-target activity are shown in Table 6. It can be seen that the experimental compounds of the present disclosure significantly reduced the off-target activity of siRNA relative to the parent sequences.

Table 6. Off-target activity results of siRNAs for different target sequences

| Double strand code | Remaining percentage of target gene mRNA (off-target activity) expression (mean) | | | | | | | | | | |
|---|---|---|---|---|---|---|---|---|---|---|---|
| | 40 nM | 13.3 nM | 4.44 nM | 1.48 nM | 0.493 nM | 0.164 nM | 0.054 nM | 0.0182 nM | 0.00609 nM | 0.00203 nM | 0.00067 nM |
| TRD 5855 | 71.1% | 78.2% | 81.6% | 92.0% | 91.0% | 94.1% | 87.3% | 93.6% | 99.4% | 119.9% | 96.6% |
| TRD 5859 | 79.8% | 81.0% | 86.0% | 96.4% | 101.9% | 98.8% | 99.8% | 118.4% | 101.3% | 93.3% | 103.2% |
| TRD 5860 | 78.4% | 75.6% | 80.6% | 86.1% | 83.2% | 95.9% | 91.6% | 91.5% | 95.6% | 97.3% | 98.6% |

**Example 6. Evaluation of Different Modifications at Positions 9 and Position 10 of AS strands**

In this experiment, the efficiency of the siRNA conjugates or dsRNAs, with 2'-fluoro modifications at different sites, of the present disclosure in inhibiting the target gene mRNA expression level *in vivo* was investigated.

6- to 8-week-old male C57BL/6 mice were randomized into groups of 6, 3 mice per time point, and each group of mice was given test conjugates (2 conjugates, TRD007047 and TRD006870), a control conjugate (TRD002218), and PBS. All the animals were dosed once by subcutaneous injection based on their body weight. The siRNA conjugates or dsRNAs were administered at a dose of 1 mg/kg (calculated based on siRNA), with a volume of 5 mL/kg. The mice were sacrificed 7 days after administration, and their livers were collected and stored with RNA later (Sigma Aldrich). Then the liver tissue was homogenized using a tissue homogenizer, and the total RNA was extracted from the liver tissue using a tissue RNA extraction kit (FireGen Biomedicals, FG0412) by following the procedure described in the instructions. The total RNA was reverse-transcribed into cDNA, and the TTR mRNA expression level in liver tissue was measured by real-time fluorescence quantitative PCR. In the fluorescence quantitative PCR method, the glyceraldehyde 3-phosphate dehydrogenase (GAPDH) gene was used as an internal reference gene, and the TTR and GAPDH mRNA expression levels were measured using Taqman probe primers for TTR and GAPDH, respectively. The compounds are shown in Table 7, the compound groups for the *in vivo* experiment on mice are shown in Table 8, and the primers are shown in Table 9.

Table 7. Modification of position 9 and position 10 of AS strands

| | SS strand (SEQ ID NO:) | AS strand (SEQ ID NO:) |
|---|---|---|
| TRD002218 | CmsAmsGmUmGfUmUfCfUfUmGmCmUmCmUmAmUmAmAm-L96 (SEQ ID NO:378) | UmsUfsAmUmAmGfAmGmCmAmAmGmAmAfCmAfCmUmGmsUmsUm (SEQ ID NO:381) |
| TRD007047 | CmsAmsGmUmGfUmUfCfUfUmGmCmUmCmUmAmUmAmsAms-NAG1(SEQ ID NO:379) | UmsUfsAmUfAmGf**(-)hmpNA(A)**GmCfAmAmGfAmAfCmAfCmUfGmsUmsUm SEQ ID NO:382) |
| TRD006870 | CmsAmsGmUmGfUmUfCfUfUmGmCmUmCmUmAmUmAmsAms-NAG1(SEQ ID NO:380) | UmsUfsAmUfAmGf**(-)hmpNA(A)**GmCmAfAmGfAmAfCmAfCmUfGmsUmsUm (SEQ ID NO:383) |

The structure of NAG1 is:

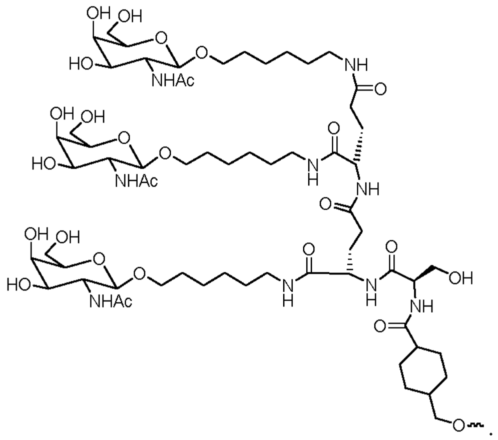

The compound NAG1 was prepared by following the method described in the patent WO2021254360A1; the structure of L96 is:

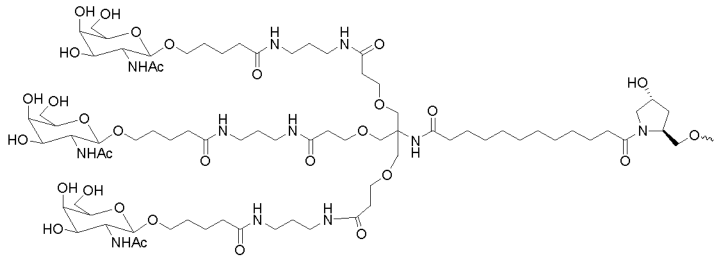

The control compound L96 was prepared by following the method described in the patent WO2014025805A1.

Table 8. The groups for the *in vivo* experiment on mice

| No. | Dose | mRNA quantification | Number of animals | Note |
|---|---|---|---|---|
| PBS | - | D7, 28 | 6 | 3 mice per time point |
| TRD002218 | 1mpk s.c. | D7, 28 | 6 | 3 mice per time point |
| TRD007047 | 1mpk s.c. | D7, 28 | 6 | 3 mice per time point |
| TRD006870 | 1mpk s.c. | D7, 28 | 6 | 3 mice per time point |

Table 9. The sequences of detection primers

| Primer name | Forward primer |
|---|---|
| mTTR-F | GGGAAGACCGCGGAGTCT (SEQ ID NO:384) |
| mTTR-R | CAGTTCTACTCTGTACACTCCTTCTACAAA (SEQ ID NO:385) |
| mTTR-P | 5'6-FAM-CTGCACGGGCTCACCACAGATGA-3'BHQ1 (SEQ ID NO:386) |
| mGAPDH-F | CGGCAAATTCAACGGCACAG (SEQ ID NO:387) |
| mGAPDH-R | CCACGACATACTCAGCACCG(SEQ ID NO:388) |
| mGAPDH-P | 5'TET-ACCATCTTCCAGGAGCGAGACCCCACT-3'BHQ2 (SEQ ID NO:389) |

The efficiency of the siRNA conjugates or dsRNAs, with fluoro modifications at different sites, of the present disclosure in inhibiting the target gene mRNA expression level *in vivo* 28 days after administration was shown in Table 10. The siRNA conjugates with fluoro modifications at different sites inhibited more TTR mRNA expression than the reference positive control TRD002218 28 days after administration. Both the modification methods showed high inhibition

efficiency and the inhibitory effects were not significantly different, which indicates that both the AS strand position 9 modification and position 10 modification methods can mediate higher inhibition efficiency.

Table 10. 7-day and 28-day test results

| | | 7 days | | 28 days | |
|---|---|---|---|---|---|
| 9/10F | No. | Remaining mRNA | SD | Remaining mRNA | SD |
| | PBS | 100% | 11% | 100% | 9% |
| PC | TRD002218 | 31% | 7% | 49% | 5% |
| mTTR 9F | TRD007047 | 15% | 5% | 39% | 10% |
| mTTR 10F | TRD006870 | 13% | 4% | 36% | 3% |

The TTR mRNA expression level was calculated according to the equation below:

TTR mRNA expression level = [(TTR mRNA expression level of test group/GAPDH mRNA expression level of test group)/(TTR mRNA expression level of control group/GAPDH mRNA expression level of control group)] × 100%

**Part Two**. **Targeting Ligands**

**Example 7. Preparation of NAG0052 and L96**

The compounds NAG0024 and NAG0026 were purchased from WuXi AppTec (Tianjin) Co. Ltd. Unless otherwise specified, all reagents used in the following examples were commercially available.

**(1) Synthesis of compound NAG0052**

The starting material compound 1 was purchased from Jiangsu Beida Pharmatech Ltd.

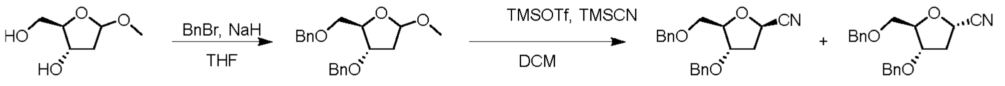


1 2 3 4

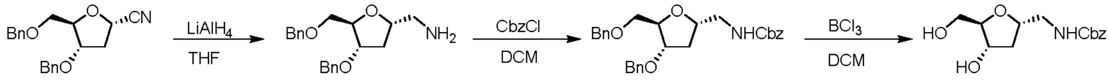


4 5 6 7

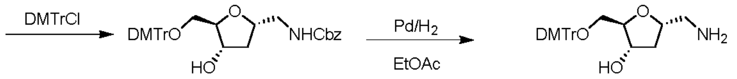


8 9

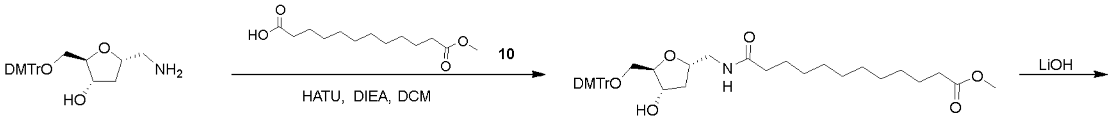


9 11

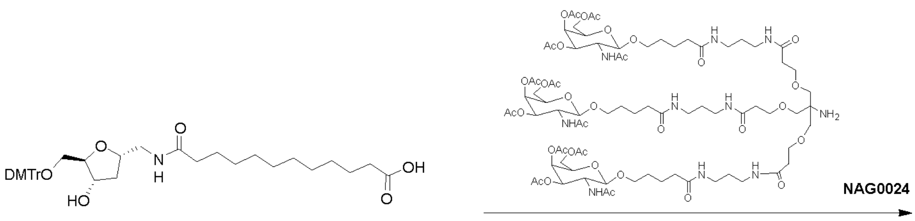


12

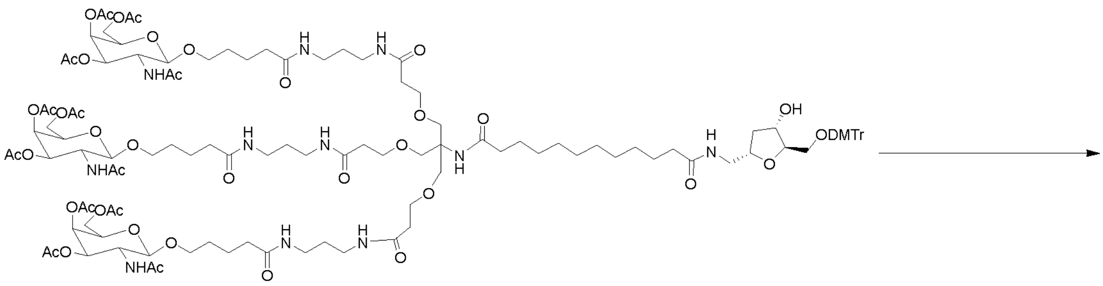

13

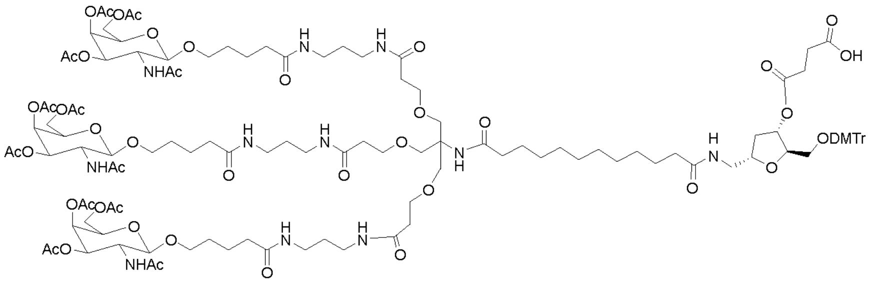

NAG0052

**Compound 2**

NaH (12.2 g, 304 mmol, purity 60%) was added portionwise to a solution of compound 1 (12.3 mL, 101 mmol) in THF (300 mL) at 0 °C in a nitrogen atmosphere. The mixture was stirred at 20 °C for 1 h and then cooled to 0 °C again. Then benzyl bromide (36.3 mL, 304 mmol) was added dropwise to the system, and the mixture was stirred at 20 °C for 12 h. The reaction mixture was quenched with $H_2O$ (100 mL) and then extracted with EtOAc (200 mL × 2). The combined organic phases were washed with saturated brine (100 mL), dried over $Na_2SO_4$, filtered, and concentrated, and the resulting residue was separated by silica gel column chromatography to give the target compound **2** (20.0 g, 51.8 mmol, yield 51%).

**LCMS:** $t_R$ = 2.615 and 2.820 min in 30-90AB_7 min_220&254_Shimadzu.lcm (Xtimate C18, 3 μm, 2.1 × 30 mm), MS (ESI) m/z = 351.2 $[M+Na]^+$.

**$^1$H NMR:** (400 MHz, $CDCl_3$) δ ppm 7.35-7.12 (m, 10H), 5.06-4.95 (m, 1H), 4.51-4.39 (m, 4H), 4.24-3.87 (m, 2H), 3.50-3.40 (m, 2H), 3.38-3.20 (m, 3H), 2.20-1.91 (m, 2H).

**Compound 3 and compound 4**

TMSCN (13.5 mL, 101 mmol) was added in one go to a solution of compound **2** (13.0 g, 33.6 mmol) in DCM (300 mL) at 20 °C in a nitrogen atmosphere, and a solution of TMSOTf (9.14 mL, 50.5 mmol) in DCM (30 mL) was then added dropwise. The reaction mixture was stirred at 20 °C for 15 h. After the reaction was complete, the system was quenched with saturated aqueous $NaHCO_3$ solution (80 mL) and extracted with DCM (150 mL × 2). The combined organic phases were washed with saturated brine (80 mL), dried over $Na_2SO_4$, filtered, and concentrated. The residue was separated by silica gel column chromatography to give the target compound **3** (3.30 g, 9.18 mmol, yield 27%) and compound **4** (8.50 g, 9.18 mmol, yield 70%) as a pale yellow oily liquid.

**Compound 3**

**$^1$H NMR:** (400 MHz, $CDCl_3$) *δ* ppm 7.42-7.29 (m, 10H), 4.81 (t, *J* = 7.8 Hz, 1H), 4.65-4.49 (m, 4H), 4.30-4.21 (m, 2H), 3.65-3.57 (m, 1H), 3.57-3.49 (m, 1H), 2.49-2.40 (m, 2H).

**Compound 4**

**$^1$H NMR:** (400 MHz, $CDCl_3$) *δ* ppm 7.42-7.26 (m, 10H), 4.93-4.87 (m, 1H), 4.65-4.48 (m, 4H), 4.43-4.38 (m, 1H), 4.21-4.17 (m, 1H), 3.79-3.70 (m, 1H), 3.54 (d, *J* = 4.0 Hz, 1H), 2.45-2.37 (m, 2H).

**Compound 5**

A solution of compound **4** (3.00 g, 9.28 mmol) in THF (15 mL) was added dropwise to a solution of $LiAlH_4$ (0.79 g, 20.9 mmol) in THF (15 mL) at 0 °C in a nitrogen atmosphere. After the dropwise addition, the system was left to react at 0 °C for 1 h. TLC monitoring (PE:EtOAc = 3:1) showed the starting material was completely consumed. Sodium sulfate decahydrate was slowly added to the reaction mixture until no more bubbles were produced. The reaction mixture was then filtered, and the filter cake was washed three times with dichloromethane (60 mL). The filtrate was collected and concentrated to dryness by rotary evaporation to give compound **5** (3.00 g, yield 90%).

**$^1$H NMR:** (400 MHz, DMSO-$d_6$) δ ppm 7.40-7.14 (m, 10H), 4.54-4.38 (m, 4H), 4.06-3.99 (m, 2H), 3.91 (q, *J* = 6.4 Hz, 1H), 3.48-3.37 (m, 2H), 2.67-2.52 (m, 2H), 2.21-2.18 (m, 1H), 1.77-1.73 (m, 1H).

**Compound 6**

Compound **5** (3.00 g, 8.25 mmol) was dissolved in DCM (30 mL) in a nitrogen atmosphere, and TEA (3.44 mL, 24.7 mmol) and CbzCl (1.76 mL, 12.4 mmol) were added. The mixture was left to react at 20 °C for 2 h. LCMS analysis showed the reaction had been complete. The reaction mixture was added to dichloromethane (30 mL) and water (60 mL) for extraction. The organic phase was washed three times with water (60 mL × 3), dried over anhydrous sodium sulfate, and concentrated, and the residue was purified using a normal-phase column (PE:EtOAc = 1:1) to give the target compound 6 (2.5 g, yield 90%).

**LCMS:** $t_R$ = 0.810 min in 5-95AB_1min, MS (ESI) m/z = 462.2 $[M+H]^+$.

**$^1$H NMR:** (400 MHz, $CDCl_3$) δ ppm 7.39-7.29 (m, 15H), 5.35 (s, 1H), 5.15-5.01 (m, 2H), 4.72 (d, *J* = 6.0 Hz, 1H), 4.54-4.40 (m, 3H), 4.26 (s, 1H), 4.23-4.18 (m, 1H), 4.11-4.04 (m, 1H), 3.54-3.41 (m, 3H), 3.37-3.25 (m, 1H), 2.34-2.23 (m, 1H), 1.85-1.79 (m, 1H).

**Compound 7**

Compound **6** (2.00 g, 3.90 mmol) was dissolved in DCM (5 mL) in a nitrogen atmosphere, and a solution of $BCl_3$ in THF (1 M, 27.3 mL) was added at -78 °C. The mixture was left to react for 1 h. TLC monitoring (DCM:MeOH = 10: 1) showed the starting material was completely consumed. The reaction mixture was quenched with methanol (20 mL) at - 78 °C and concentrated, and the residue was purified using a normal-phase column (DCM:MeOH = 10: 1) to give the target compound 7 (2.00 g, yield 60%).

**$^1$H NMR:** (400 MHz, $CD_3OD$) $\delta$ ppm 7.41-7.23 (m, 5H), 5.08 (s, 2H), 4.25-4.07 (m, 2H), 3.85-3.75 (m, 1H), 3.63-3.56 (m, 1H), 3.54-3.48 (m, 1H), 3.30-3.27 (m, 2H), 2.34-2.21 (m, 1H), 1.71-1.64 (m, 1H).

**Compound 8**

Compound **7** (0.50 g, 1.78 mmol) was dissolved in pyridine (5 mL) in a nitrogen atmosphere, and 4A molecular sieve (500 mg) and DMTrCl (0.66 mL, 2.13 mmol) were added at 0 °C. Then the mixture was warmed to 20 °C and left to react for 1.5 h. TLC monitoring (PE:EtOAc = 2:1) showed the starting material was completely consumed. The reaction mixture was added to ethyl acetate (60 mL) and water (60 mL) for extraction. The organic phase was washed three times with water (60 mL $\times$ 3), then dried over anhydrous sodium sulfate, and concentrated, and the residue was purified using a normal-phase column (PE:EtOAc = 1:1) to give the target compound **8** (800 mg, yield 90%).

**$^1$H NMR:** (400 MHz, $CDCl_3$) $\delta$ ppm 7.44 (d, J = 7.6 Hz, 2H), 7.37-7.23 (m, 11H), 7.22-7.15 (m, 1H), 6.84 (d, J = 8.8 Hz, 4H), 5.09 (s, 2H), 4.31-4.17 (m, 2H), 4.02-3.91 (m, 1H), 3.84-3.73 (m, 6H), 3.33 (s, 1H), 3.28 (s, 1H), 3.19-3.01 (m, 2H), 2.34-2.25 (m, 1H), 1.70-1.62 (m, 1H).

**Compound 9**

Compound **8** (800 mg, 1.234 mmol) was dissolved in EtOAc (5 mL), and Pd/C 10% (800 mg, 7.517 mmol) was added. The mixture was left to react in a $H_2$ atmosphere (15 Psi) at 20 °C for 1 h. LCMS analysis showed the reaction had been complete. The reaction mixture was filtered, and the filter cake was washed three times with dichloromethane (100 mL) and methanol (100 mL). The filtrate was concentrated, and the residue was separated using a reversed-phase column to give compound **9** (300 mg, 54%).

**LCMS:** $t_R$ = 2.586 min in 10-80CD_3min MS (ESI) m/z = 450.2 $[M+H]^+$.

**Compound 11**

Compound **10** (435 mg, 1.780 mmol) was dissolved in DCM (10 mL), and DIEA(0.441 mL, 2.67 mmol) and HATU (677 mg, 1.78 mmol) were added. Then compound **9** (400 mg, 0.890 mmol) was added. The mixture was left to react at 20 °C for 1 h. TLC monitoring (DCM:MeOH = 10:1) showed the reaction had been complete. The reaction mixture was added to dichloromethane (60 mL) and water (60 mL) for extraction. The organic phase was washed three times with water (60 mL $\times$ 3), dried over anhydrous sodium sulfate, and concentrated, and the residue was purified using a normal-phase column (elution with PE:EtOAc = 0:1, product peak at 100%) to give the target compound **11** (600 mg, yield 90%).

**LCMS:** $t_R$ = 2.745 min in 30-90CD_3min, MS (ESI) m/z = 698.4 $[M+Na]^+$.

**$^1$H NMR:** (400 MHz, $CD_3OD$) $\delta$ ppm 7.46-7.38 (m, 2H), 7.35-7.24 (m, 6H), 7.22-7.16 (m, 1H), 6.90-6.78 (m, 4H), 4.29-4.21 (m, 2H), 4.02-3.95 (m, 1H), 3.77 (s, 6H), 3.66-3.62 (m, 3H), 3.41 (s, 1H), 3.18-3.04 (m, 2H), 2.36-2.17 (m, 5H), 1.71-1.50 (m, 5H), 1.39-1.25 (m, 14H).

**Compound 12**

Compound **11** (600 mg, 0.799 mmol) was dissolved in THF (3 mL) and $H_2O$ (1 mL), and $LiOH.H_2O$ (134 mg, 3.20 mmol) was added. The mixture was left to react at 20 °C for 12 h. TLC analysis (DCM:MeOH = 10:1) showed the reaction had been complete. The reaction mixture was concentrated to dryness by rotary evaporation. The residue was dissolved in water (5 mL) and methanol (5 mL) and purified using a reversed-phase column ($H_2O$:$CH_3CN$ = 1:1, peak at around 35%) to give the target compound **12** (460 mg, yield 100%, lithium salt).

**LCMS:** $t_R$ = 1.346 min in 10-80CD_3min, MS (ESI) m/z = 684.3 $[M+Na]^+$.

**HPLC:** $t_R$ = 1.879 min in 10-80CD_6min.

**$^1$H NMR:** (400 MHz, $CD_3OD$) $\delta$ ppm 7.47-7.39 (m, 2H), 7.35-7.24 (m, 6H), 7.22-7.15 (m, 1H), 6.91-6.79 (m, 4H), 4.31-4.18 (m, 2H), 4.02-3.95 (m, 1H), 3.78 (s, 6H), 3.44-3.33 (m, 2H), 3.18-3.04 (m, 2H), 2.35-2.27 (m, 1H), 2.24-2.10 (m, 4H), 1.70-1.51 (m, 5H), 1.31-1.23 (m, 12H).

## Compound 13

The compound **NAG0024** (271 mg, 0.151 mmol) was dissolved in anhydrous THF (2 mL) and anhydrous DMF (4 mL) at room temperature in a nitrogen atmosphere, and 3A molecular sieve was added. Then compound **12** (100 mg, 0.151 mmol), HOBt (25 mg, 0.181 mmol), DCC (38 mg, 0.181 mmol), and DIEA (39 mg, 0.302 mmol) were sequentially added. The reaction mixture was left to react at 45 °C for 16 h. After LC-MS analysis showed the reaction had been complete, the reaction mixture was quenched with water and filtered. The filtrate was concentrated, and the residue was then purified using a C18 reversed-phase column ($H_2O$/MeCN) to give compound **13** (210 mg, yield 57%).

## Compound NAG0052

Compound **13** (230 mg, 0.094 mmol) was dissolved in pyridine (5 mL) at room temperature, and a molecular sieve was added. DMAP (12 mg, 0.283 mmol) and succinic anhydride (28 mg, 0.283 mmol) were added. The mixture was stirred at 50 °C for 16 h in a nitrogen atmosphere. LCMS analysis showed the reaction had been complete. The reaction mixture was filtered and concentrated to dryness by rotary evaporation. After purification on a C18 reversed-phase column, secondary purification was performed by preparative HPLC to give the target compound NAG0052 (123 mg, 0.048 mmol, yield 51%).

MS (ESI) m/z = 2535.3 $[M-1]^-$. Calculated: 2536.2.

$^1H$ NMR (400 MHz, acetonitrile-$d_3$) $\delta$ 7.48-7.43 (m, 2H), 7.37-7.12 (m, 11H), 7.00-6.85 (m, 10H), 6.66 (s, 1H), 5.31 (dd, *J* = 3.4, 1.1 Hz, 3H), 5.20-5.13 (m, 1H), 5.05 (dd, *J* = 11.3, 3.4 Hz, 3H), 4.56 (d, *J* = 8.5 Hz, 3H), 4.30 (dd, *J* = 7.7, 5.3 Hz, 1H), 4.18-3.93 (m, 14H), 3.79 (s, 10H), 3.65 (q, *J* = 4.7, 3.6 Hz, 13H), 3.56-3.07 (m, 24H), 2.56 (s, 6H), 2.37 (t, *J* = 5.8 Hz, 10H), 2.17 (t, *J* = 7.5 Hz, 9H), 2.02-1.96 (m, 20H), 1.88 (s, 8H), 1.82-1.73 (m, 2H), 1.60 (dt, *J* = 15.0, 7.3 Hz, 16H), 1.27 (s, 13H).

## Example 8. Synthesis of siRNA Conjugates or dsRNAs

1. In-house preparation of resin with support

The carboxylic acid group-containing compound **NAG0052** (157 mg, 0.062 mmol) was dissolved in anhydrous DMF (3 mL). After the substrate was completely dissolved, anhydrous acetonitrile (4 mL), DIEA (0.03 mL, 0.154 mmol, 2.5 eq), and HBTU (35 mg, 0.093 mmol, 1.5 eq) were sequentially added. After the reaction mixture was mixed well, macroporous aminomethyl resin (476 mg, blank loading 0.41 mmol/g, target loading 0.1 mmol/g) was added. The reaction mixture was shaken overnight on a shaker (temperature: 25 °C; rotational speed: 200 rpm). The reaction mixture was filtered, and the filter cake was washed with DCM and then with anhydrous acetonitrile. The solid was collected and dried overnight under vacuum.

The solid from the previous step was dispersed in anhydrous acetonitrile (5 mL), and pyridine (0.18 mL), DMAP (3 mg), NMI (0.12 mL), and CapB1 (2.68 mL) were sequentially added. The reaction mixture was shaken on a shaker (temperature: 25 °C; rotational speed: 200 rpm) for 2 h. The reaction mixture was filtered, and the filter cake was washed with anhydrous acetonitrile. The solid was collected and dried overnight under vacuum to give a resin with a support. The loading was measured at 0.1 mmol/g. 2. For NAG0052 that had been attached to the resin, the resin was used as a start, and nucleoside monomers were attached one by one in the 3'-5' direction in the order in which nucleotides were arranged. Each time a nucleoside monomer was attached, four reactions-deprotection, coupling, capping, and oxidation or sulfurization-were involved. The procedure is conventional in the art.

The prepared siRNA conjugates or dsRNAs had sense and antisense strands as shown in Table 11 and Table 12.

Table 11. siRNA conjugates or dsRNAs

| siRNA conjugate or dsRNA No. | Sense strand No. | Antisense strand No. |
|---|---|---|
| TRD002218 | TJR4373-SS | TJR0414-AS |
| TRD007205 | TJR013485S | TJR0414-AS |

Table 12. The nucleic acid sequences of the sense and antisense strands

| | No. | **Sequence direction 5'-3'** (SEQ ID NO:) |
|---|---|---|
| **Sense strand** | TJR4373-SS | CmsAmsGm UmGfUm UfCfUf UmGmCm UmCmUm AmUmAm Am-**L96** (SEQ ID NO:390) |
| | TJR013485S | CmsAmsGm UmGfUm UfCfUf UmGmCm UmCmUm AmUmAms Ams-**NAG0052'**(SEQ ID NO:391) |
| **antisense strand** | TJR0414-AS | UmsUfsAm UmAmGf AmGmCm AmAmGm AmAfCm AfCmUm GmsUmsUm(SEQ ID NO:392) |

Table 13. The structures of conjugates

| siRNA conjugate or dsRNA sense strand No. | Structure |
|---|---|
| TJR4373-SS | 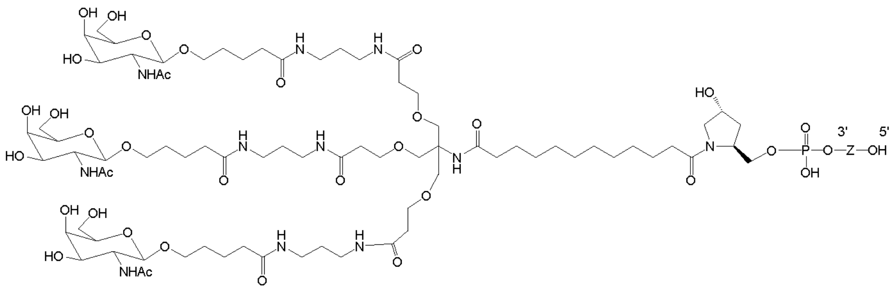 |
| TJR013485S | 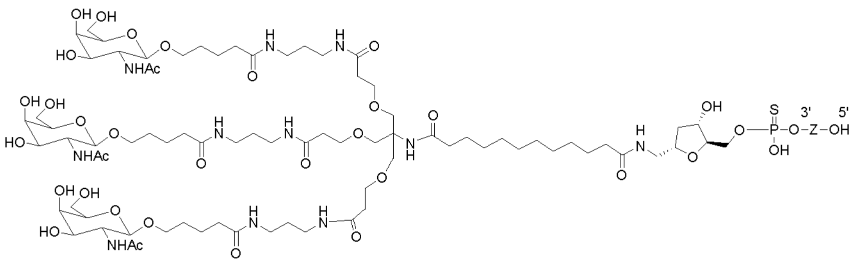 |

The conjugate TRD002218 was used as a reference positive compound.

**Example 9. *In Vivo* Inhibition of Target Gene mRNA Expression Level by siRNA Conjugates or dsRNAs**

In this experiment, the efficiency of the siRNA conjugates or dsRNAs of the present disclosure, which are conjugated with different structures, in inhibiting the target gene mRNA expression level *in vivo* was investigated.

Male C57BL/6 mice aged 6-8 weeks were randomly divided into groups of 6, with 3 mice per time point. These groups of mice were administered the conjugate of the present disclosure TRD007205, the reference positive nucleic acid ligand conjugate TRD002218, and PBS.

All the animals were dosed once by subcutaneous injection based on their body weight. The siRNA conjugates or dsRNAs were administered at a dose of 1 mg/kg (calculated based on siRNA), with a volume of 5 mL/kg. The mice were sacrificed 7 days or 28 days after administration, and their livers were collected and stored with RNA later (Sigma Aldrich). Then the liver tissue was homogenized using a tissue homogenizer, and the total RNA was extracted from the liver tissue using a tissue RNA extraction kit (FireGen Biomedicals, FG0412) by following the procedure described in the instructions. The total RNA was reverse-transcribed into cDNA, and the TTR mRNA expression level in liver tissue was measured by real-time fluorescence quantitative PCR. In the fluorescence quantitative PCR method, the glyceraldehyde 3-phosphate dehydrogenase (GAPDH) gene was used as an internal reference gene, and the TTR and GAPDH mRNA expression levels were measured using Taqman probe primers for TTR and GAPDH, respectively. The sequences of the detection primers were the same as shown in Table 9.

Table 14. The compound groups for the *in vivo* experiment on mice

| Compound No. | Dose | mRNA quantification | Number of animals | Note |
|---|---|---|---|---|
| PBS | - | D7, 28 | 6 | 3 mice per time point |
| TRD002218 | 1mpk s.c. | D7, 28 | 6 | 3 mice per time point |
| TRD007205 | 1mpk s.c. | D7, 28 | 6 | 3 mice per time point |

The TTR mRNA expression level was calculated according to the equation below:

TTR mRNA expression level = [(TTR mRNA expression level of test group/GAPDH mRNA expression level of test group)/(TTR mRNA expression level of control group/GAPDH mRNA expression level of control group)] × 100%.

The efficiency of the siRNA conjugates or dsRNAs of the present disclosure, which are conjugated with different structures, in inhibiting the target gene mRNA expression level *in vivo* 7 days and 28 days after administration are shown in FIG. 1 and FIG. 2, respectively.

As can be seen from the results in FIG. 1, the conjugate TRD007205 well inhibited TTR mRNA expression 7 days after administration, indicating that it can mediate more efficient delivery. As can be seen from FIG. 2, 28 days after administration, TRD007205 inhibited the target gene mRNA expression level better than TRD002218.

**Example 10. Synthesis of siRNA-NAG0052 Conjugates**

1. In-house preparation of resin with support

The procedure was the same as in Example 8.

2. A resin with NAG0052 was used as a start, and nucleoside monomers were attached one by one in the 3'-5' direction in the order in which nucleotides were arranged. Each time a nucleoside monomer was attached, four reactions-deprotection, coupling, capping, and oxidation or sulfurization-were involved.

Specifically, reference was made to the synthesis method of Example 2. The prepared siRNA conjugates or dsRNAs had sense and antisense strands as shown in Table 15, Table 16-1, and Table 16-1.

Table 15. siRNA conjugates or dsRNAs

| Target | siRNA conjugate or dsRNA No. | Sense strand No. | Antisense strand No. |
|---|---|---|---|
| AGT | TRD008028 | TJR014967S | TJR014818A |
| | TRD008029 | TJR014968S | TJR014819A |
| | TRD008030 | TJR014969S | TJR014831A |
| | TRD008031 | TJR014970S | TJR014832A |
| | TRD008028-1 | TJR014967S-1 | TJR014818A |
| | TRD008029-1 | TJR014968S-1 | TJR014819A |
| | TRD008030-1 | TJR014969S-1 | TJR014831A |
| | TRD008031-1 | TJR014970S-1 | TJR014832A |

Table 16-1. The sense and antisense strands of siRNA conjugates or dsRNAs

| Single strand No. | SEQ ID NO | **Sequence direction 5'-3'** | Single strand No. | SEQ ID NO | **Sequence direction 5'-3'** |
|---|---|---|---|---|---|
| TJR0149 67S | 393 | UmsCmsAmAmCfUmGfGfAfUmGmAmAmGmAmAmAmCmUm-**NAG0052'** | TJR01496 7S-1 | 397 | UmsCmsAmAmCmUmGfGfAfUmGmAmAmGmAmAmAmCmUm-**NAG0052'** |
| TJR0149 68S | 394 | CmsGmsUmUmUfCmUfCfCfUmUmGmGmUmCmU | TJR01496 8S-1 | 398 | CmsGmsUmUmUmCmUfCfCfUmUmGmGmU |
| | | mAmAmAm-**NAG0052'** | | | mCmUmAmAmAm-**NAG0052'** |
| TJR0149 69S | 395 | CmsCmsAmCmAfAmUfGfAfGmAmGmUmAmCmCmUmGmUm-**NAG0052'** | TJR01496 9S-1 | 399 | CmsCmsAmCmAmAmUfGfAfGmAmGmUmAmCmCmUmGmUm-**NAG0052'** |
| TJR0149 70S | 396 | CmsGmsAmCmCfAmGfCfUfUmGmUmUmUmGmUmGmAmAm-**NAG0052'** | TJR01497 0S-1 | 400 | CmsGmsAmCmCmAmGfCfUfUmGmUmUmUmGmUmGmAmAm-**NAG0052'** |
| Single strand No. | | **Sequence direction 5'-3** | | | |
| TJR0148 18A | 401 | AmsGfsUmUfUmCf(-)hmpNA(U)UmCmAfUmCfCmAfGmUfUmGfAmsGmsGm | | | |
| TJR0148 19A | 402 | UmsUfsUmAfGmAf(-)hmpNA(C)CmAmAfGmGfAmGfAmAfAmCfGmsGmsCm | | | |
| TJR0148 31A | 403 | AmsCfsAmGfGmUf(-)hmpNA(A)CmUmCfUmCfAmUfUmGfUmGfGmsAmsUm | | | |
| TJR0148 32A | 404 | UmsUfsCmAfCmAf(-)hmpNA(A)AmCmAfAmGfCmUfGmGfUmCfGmsGmsUm | | | |

Table 16-2. The naked sequences of the corresponding nucleic acid sequences of the sense and antisense strands of siRNA conjugates or dsRNAs

| Single strand No. | SEQ ID NO | Naked sequence corresponding to single strand No. **Sequence direction 5'-3'** | Single strand No. | SEQ ID NO | Naked sequence corresponding to single strand No. **Sequence direction 5'-3'** |
|---|---|---|---|---|---|
| TJR014967S | 405 | UCAACUGGAUG AAGAAACU | TJR014 967S-1 | 409 | UCAACUGGAUG AAGAAACU |
| TJR014968S | 406 | CGUUUCUCCUU GGUCUAAA | TJR014 968S-1 | 410 | CGUUUCUCCUU GGUCUAAA |
| TJR014969S | 407 | CCACAAUGAGA GUACCUGU | TJR014 969S-1 | 411 | CCACAAUGAGA GUACCUGU |
| TJR014970S | 408 | CGACCAGCUUG UUUGUGAA | TJR014 970S-1 | 412 | CGACCAGCUUG UUUGUGAA |
| Single strand No. | SEQ ID NO | Naked sequence corresponding to single strand No. **Sequence direction 5'-3'** | | | |
| TJR014818A | 413 | AGUUUCUUCAUCCAGUUGAGG | | | |
| TJR014819A | 414 | UUUAGACCAAGGAGAAACGGC | | | |
| TJR014831A | 415 | ACAGGUACUCUCAUUGUGGAU | | | |
| TJR014832A | 416 | UUCACAAACAAGCUGGUCGGU | | | |

For the structures of (-)hmpNA(A), (-)hmpNA(G), (-)hmpNA(C), and (-)hmpNA(U), see Example 4.

The structure of **NAG0052'** is:

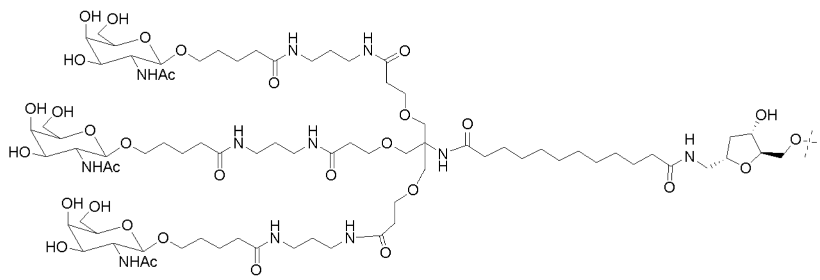

.

**Example 11. On-target Activity of siRNA-NAG0052 Conjugates on Different Targets**

Table 17 shows a positive control.

Table 17. The sequences of the positive control compound

| Target | Positive control No. | SEQ ID NO | Sense strand (5'-3') | SEQ ID NO | Antisense strand (5'-3') |
|---|---|---|---|---|---|
| AGT | TRD007779 | 417 | GmsUmsCmAmUmCmCfAmCfAfAfUmGmAmGmAmGmUmAmCmAmL96 | 418 | UmsGfsUmAmCmGNA(t)CmUmCmAmUmUmGmUfGmGfAmUmGmAmCmsGmsAm |

TRD007779 was prepared according to US 11015201B;

For the structure of GNA(A), see Example 4; the structure of GNA(t) is

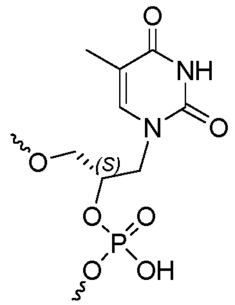

GNA(T) ;

the structure of L96 is

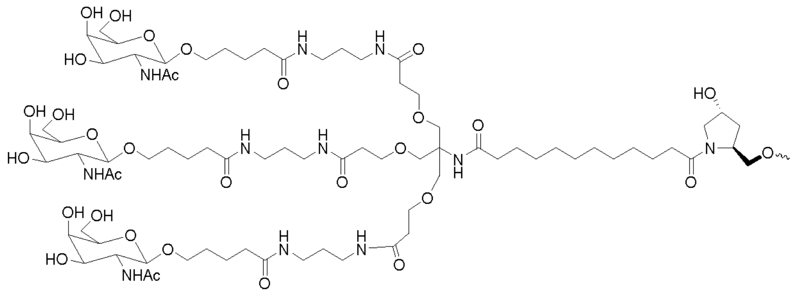

.

The siRNA conjugates or dsRNAs in Table 15 and Table 17 were subjected to an *in vitro* molecular-level simulation of on-target activity screening in HEK293A cells using 11 concentration gradients.

Corresponding on-target sequences of the siRNAs were constructed from the AGT gene and inserted into psiCHECK-2 plasmids. The plasmids contained the renilla luciferase gene and the firefly luciferase gene. The plasmids were dual reporter gene systems. The target sequence of siRNA was inserted into the 3' UTR region of the renilla luciferase gene. The activity of siRNA for the target sequence was reflected by the measurements of renilla luciferase expression corrected with firefly luciferase. The measurements used Dual-Luciferase Reporter Assay System (Promega, E2940).

HEK293A cells were cultured at 37 °C with 5% $CO_2$ in a DMEM high glucose medium containing 10% fetal bovine serum. 24 h prior to transfection, the HEK293A cells were inoculated onto a 96-well plate at a density of 8 $\times$ $10^3$ cells per well, with each well containing 100 $\mu$L of medium.

The cells were co-transfected with siRNA and the corresponding plasmid using Lipofectamine2000 (ThermoFisher, 11668019) according to the instructions. 0.2 $\mu$L of Lipofectamine2000 was used for each well. The amount of plasmid for transfection was 20 ng per well. For the on-target sequence plasmids, a total of 11 concentration points were set up for the siRNAs, with the highest concentration point final concentration being 20 nM. A 3-fold serial dilution was performed, resulting in 20 nM, 6.6667 nM, 2.2222 nM, 0.7407 nM, 0.2469 nM, 0.0823 nM, 0.0274 nM, 0.0091 nM, 0.0030 nM, 0.0010 nM, and 0.0003 nM. 24 h after transfection, the on-target levels were determined using Dual-Luciferase Reporter Assay System (Promega, E2940).

The experimental materials and instruments are shown in Table 18 and Table 19. The dilution process is shown in Table 20. The results are shown in Table 21.

The Psi-CHECK plasmids were purchased from Sangon Biotech (Shanghai) Co., Ltd.

Table 18. Experimental consumables and reagents for psi-CHECK

| Reagent consumables | | | | |
|---|---|---|---|---|
| **Name** | **Company** | **Catalog number/model** | **Batch No.** | **Shelf life** |
| HEK293A cells | Cobioer, Nanjing | ATCC-Cobioer/CBP60202 | / | / |
| Dual-Glo® Luciferase Assay System | Promega | E2940 | 0000363099 | 2022/5/13 |

(continued)

| Reagent consumables | | | | |
|---|---|---|---|---|
| Name | Company | Catalog number/model | Batch No. | Shelf life |
| Lipofectamine® 2000 | Invitrogen | 11668-019 | / | 2022/6/14 |

Table 19. Experimental instruments for psi-CHECK

| Instruments | | |
|---|---|---|
| Name | Company | Catalog number/model |
| Nanodrop | Thermo | Nanodrop One |
| Microplate reader | PerkinElmer | EnVision2105 |
| Graphing software | / | Graph Prism 5 |

Table 20. Multi-concentration dilution protocol for samples

| Final concentration of siRNA (nM) | Addition of water and siRNA |
|---|---|
| 20 | 4 μ of L siRNA (stock solution) + 36 μL of $H_2O$ |
| 6.6667 | 10 μL siRNA + 20 μL $H_2O$ |
| 2.2222 | 10 μL siRNA + 20 μL $H_2O$ |
| 0.7407 | 10 μL siRNA + 20 μL $H_2O$ |
| 0.2469 | 10 μL siRNA + 20 μL $H_2O$ |
| 0.0823 | 10 μL siRNA + 20 μL $H_2O$ |
| 0.0274 | 10 μL siRNA + 20 μL $H_2O$ |
| 0.0091 | 10 μL siRNA + 20 μL $H_2O$ |
| 0.0030 | 10 μL siRNA + 20 μL $H_2O$ |
| 0.0010 | 10 μL siRNA + 20 μL $H_2O$ |
| 0.0003 | 10 μL siRNA + 20 μL $H_2O$ |

Table 21. psi-CHECK on-target activity screening results

| Remaining percentage of target gene mRNA) expression (mean) | | | | | | |
|---|---|---|---|---|---|---|
| Double strand code | 20 nM | 6.6667 nM | 2.2222 nM | 0.7407 nM | 0.2469 nM | 0.0823 nM |
| TRD007779 | 20.94% | 25.94% | 19.71% | 22.83% | 30.25% | 52.84% |
| TRD008028 | 15.98% | 15.62% | 14.82% | 15.21% | 18.89% | 28.25% |
| TRD008029 | 19.62% | 18.32% | 17.67% | 16.43% | 19.09% | 29.18% |
| TRD008030 | 22.06% | 20.44% | 21.77% | 23.54% | 30.98% | 51.09% |
| TRD008031 | 24.20% | 24.39% | 22.08% | 22.82% | 28.27% | 41.84% |
| Double strand code | 0.0274 nM | 0.0091 nM | 0.0030 nM | 0.0010 nM | 0.0003 nM | GSCM $IC_{50}$ (nM) |
| TRD007779 | 80.38% | 0.8691 | 100.43% | 98.43% | 90.27% | 0.0885 |
| TRD008028 | 46.07% | 57.83% | 78.32% | 87.88% | 92.34% | 0.0171 |

(continued)

| Double strand code | 0.0274 nM | 0.0091 nM | 0.0030 nM | 0.0010 nM | 0.0003 nM | GSCM $IC_{50}$ (nM) |
|---|---|---|---|---|---|---|
| TRD008029 | 43.94% | 59.89% | 83.88% | 84.74% | 82.90% | 0.0185 |
| TRD008030 | 79.10% | 0.9093 | 110.38% | 106.16% | 94.68% | 0.0845 |
| TRD008031 | 65.76% | 0.8225 | 93.05% | 97.54% | 84.17% | 0.0521 |

The above results indicate that the reference controls TRD007779, TRD008028, TRD008029, TRD008030, and TRD008031 have high levels of on-target inhibitory activity against the AGT gene in the psiCHECK system.

**Example 12. Inhibition of Human AGT in Huh7 Cells - 7-Concentration-Point Inhibitory Activity**

The siRNA conjugates or dsRNAs in Table 15 and Table 17 were subjected to Huh7 cell activity screening in Huh7 cells using 11 concentration gradients. The initial final concentration of each sample for transfection was 10 nM, and a 5-fold serial dilution was performed to obtain 7 concentration points.

Huh7 cells were cultured at 37 °C with 5% $CO_2$ in a DMEM high glucose medium containing 10% fetal bovine serum. 24 h prior to transfection, the Huh7 cells were inoculated onto a 96-well plate at a density of 10,000 cells per well, with each well containing 100 μL of medium.

The cells were transfected with the samples using Lipofectamine RNAi MAX (ThermoFisher, 13778150) by following the instruction manual of the product, with the final concentrations of the samples for transfection being 10 nM, 2 nM, 0.4 nM, 0.08 nM, 0.016 nM, 0.0032 nM, and 0.00064 nM. 24 h after treatment, the total cellular RNA was extracted using a high-throughput cellular RNA extraction kit, and RNA reverse transcription and quantitative real-time PCR detection were carried out. The mRNA level of human AGT was measured and corrected based on the GAPDH internal reference gene level.

The results are expressed relative to the remaining percentage of human AGT mRNA expression in cells treated with the control sample. The inhibition rate $IC_{50}$ results are shown in Table 22.

Table 22. Multi-dose inhibitory activity against human AGT in Huh7

| siRNA conjugate or dsRNA No. | Remaining percentage of target gene mRNA (Huh7) expression (mean) | | | | | | | $IC_{50}$ value (nM) |
|---|---|---|---|---|---|---|---|---|
| | 10 nM | 2nM | 0.4 nM | 0.08 nM | 0.016 nM | 0.0032 nM | 0.00064 nM | |
| TRD008028 | 15.78% | 28.84% | 30.04% | 56.13% | 83.80% | 98.80% | 99.84% | 0.1072 |
| TRD008029 | 19.93% | 29.35% | 28.36% | 47.77% | 77.51% | 95.34% | 97.08% | 0.0692 |
| TRD008030 | 18.25% | 26.25% | 33.09% | 54.32% | 76.46% | 91.36% | 83.78% | 0.1096 |
| TRD008031 | 20.14% | 26.06% | 38.04% | 61.23% | 93.19% | 103.16% | 100.07% | 0.1585 |

The results indicate that TRD008028, TRD008029, TRD008030, and TRD008031 have excellent inhibitory activity against AGT in Huh7 cells.

**Example 13. Inhibition of Human AGT in Hep3B Cells - 7-Concentration-Point Inhibitory Activity**

Samples were subjected to activity screening in Hep3B cells using 7 concentration gradients. The initial final concentration of each sample for transfection was 10 nM, and a 5-fold serial dilution was performed to obtain 7 concentration points.

Hep3B cells were cultured at 37 °C with 5% CO2 in a MEM medium containing 10% fetal bovine serum. 24 h prior to transfection, the Hep3B cells were inoculated onto a 96-well plate at a density of 10,000 cells per well, with each well containing 100 μL of medium.

The cells were transfected with the samples using Lipofectamine RNAi MAX (ThermoFisher, 13778150) by following the instruction manual of the product, with the final concentrations of the samples for transfection being 10 nM, 2 nM, 0.4 nM, 0.08 nM, 0.016 nM, 0.0032 nM, and 0.00064 nM. 24 h after treatment, the total cellular RNA was extracted using a high-throughput cellular RNA extraction kit, and RNA reverse transcription and quantitative real-time PCR detection were carried out. The mRNA level of human AGT was measured and corrected based on the GAPDH internal reference gene level.

The results are expressed relative to the remaining percentage of human AGT mRNA expression in cells treated with the control sample. The inhibition rate $IC_{50}$ results are shown in Table 23.

Table 23. Multi-dose inhibitory activity against human AGT in Hep3B

| No. | Remaining percentage of target gene mRNA (Hep3B) expression (mean) | | | | | | | $IC_{50}$ value (nM) |
|---|---|---|---|---|---|---|---|---|
| | 10 nM | 2nM | 0.4 nM | 0.08 nM | 0.016 nM | 0.0032 nM | 0.00064 nM | |
| TRD008028 | 17.04% | 24.90% | 38.71% | 53.23% | 81.87% | 91.96% | 100.22% | 0.1318 |
| TRD008029 | 17.97% | 22.05% | 42.58% | 44.35% | 71.44% | 88.81% | 100.34% | 0.0851 |
| TRD008030 | 15.18% | 23.69% | 33.91% | 57.68% | 91.42% | 97.66% | 100.21% | 0.1318 |
| TRD008031 | 14.69% | 16.16% | 26.27% | 56.92% | 81.95% | 94.32% | 100.11% | 0.1000 |

The results indicate that TRD008028, TRD008029, TRD008030, and TRD008031 have excellent inhibitory activity against AGT in Hep3B cells.

**Part Three. Biological Evaluation of Targeting AGT**

**Example 14. Design of Human AGT siRNAs**

With the human AGT gene (NM_001384479.1) as a target gene, siRNAs of 19/21 nt were designed in accordance with the general rules for active siRNAs. The sequences of unmodified sense and antisense strands are detailed in Table 24 and Table 25; antisense strands with a chemical modification at position 7 are detailed in Table 24; modified sense and antisense strands are shown in Table 26, Table 27, and Table 28; the present disclosure also provides the sequences of the sense and antisense strands in Table 26, Table 27, and Table 28 free of phosphorothioate groups.

Table 24. Unmodified sense and antisense strands of human siRNAs; antisense strands with a chemical modification at position 7

| Double strand No. of unmodified sequence | SEQ ID NO: | Unmodified SS strand (5'-3') | SEQ ID NO: | Unmodified AS strand (5'-3') | SEQ ID NO: | AS strand with modification at position 7 (5'-3') |
|---|---|---|---|---|---|---|
| TJR100283 | 1 | CGUUUCUCCUUGGUCUAAA | 7 | UUUAGACCAAGGAGAAACGGC | 13 | UUUAGAW'CAAGGAGAAACGGC |
| TJR100299 | 2 | CAUGCACAGUGAGCUAUGA | 8 | UCAUAGCUCACUGUGCAUGCC | 14 | UCAUAGW'UCACUGUGCAUGCC |
| TJR100320 | 3 | GGUGCUGCAAGGAUCUUAU | 9 | AUAAGAUCCUUGCAGCACCAG | 15 | AUAAGAW'CCUUGCAGCACCAG |
| | 4 | UCAACUGGAUGAAGAAACU | 10 | AGUUUCUUCAUCCAGUUGAGG | 16 | AGUUUCW'UCAUCCAGUUGAGG |
| | 5 | CCACAAUGAGAGUACCUGU | 11 | ACAGGUACUCUCAUUGUGGAU | 17 | ACAGGUW'CUCUCAUUGUGGAU |
| | 6 | CGACCAGCUUGUUUGUGAA | 12 | UUCACAAACAAGCUGGUCGGU | 18 | UUCACAW'ACAAGCUGGUCGGU |

In synthesizing the modified nucleotide at position 7 of the 5' end of the AS strand, the original nucleotide of the parent sequence was replaced with a phosphoramidite monomer synthesized in Example 1 or a 2'-methoxy-modified phosphoramidite monomer. W' is selected from the group consisting of:
a nucleotide modified by a chemical modification represented by

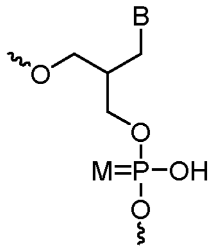

,

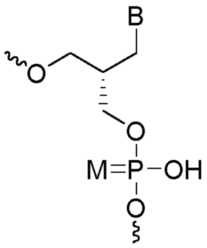

, or

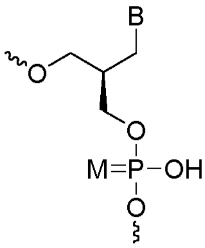

or a tautomer thereof, or a 2'-methoxy-modified nucleotide;
wherein M is O or S;
B is selected from the group consisting of the bases corresponding to position 7 of the 5' end of SEQ ID NO: 7 to SEQ ID NO: 12 in Table 24. For example, SEQ ID NO: 13 corresponds to SEQ ID NO: 7, and SEQ ID NO: 17 corresponds to SEQ ID NO: 11.

Table 25. Unmodified sense and antisense strands of human siRNAs

| Double strand No. | SEQ ID NO: | Unmodified SS strand (5'-3') | SEQ ID NO: | Unmodified AS strand (5'-3') |
|---|---|---|---|---|
| | 19 | GAUCUUAUGACCUGCAGGA | 77 | UCCUGCAGGUCAUAAGAUCCU |
| | 20 | GUUUCUCCUUGGUCUAAGU | 78 | ACUUAGACCAAGGAGAAACGG |
| | 21 | CGUAUAUAUGGCAUGCACA | 79 | UGUGCAUGCCAUAUAUACGGA |
| | 22 | GUAUAUAUGGCAUGCACAA | 80 | UUGUGCAUGCCAUAUAUACGG |
| | 23 | UAUAUAUGGCAUGCACAGU | 81 | ACUGUGCAUGCCAUAUAUACG |
| | 24 | AUAUAUGGCAUGCACAGUA | 82 | UACUGUGCAUGCCAUAUAUAC |
| | 25 | UAUAUGGCAUGCACAGUGA | 83 | UCACUGUGCAUGCCAUAUAUA |
| | 26 | AUAUGGCAUGCACAGUGAA | 84 | UUCACUGUGCAUGCCAUAUAU |
| | 27 | UAUGGCAUGCACAGUGAGA | 85 | UCUCACUGUGCAUGCCAUAUA |
| | 28 | AUGGCAUGCACAGUGAGCU | 86 | AGCUCACUGUGCAUGCCAUAU |
| | 29 | GCAUGCACAGUGAGCUAUA | 87 | UAUAGCUCACUGUGCAUGCCA |
| | 30 | AUGCACAGUGAGCUAUGGA | 88 | UCCAUAGCUCACUGUGCAUGC |
| | 31 | UGCACAGUGAGCUAUGGGA | 89 | UCCCAUAGCUCACUGUGCAUG |
| | 32 | CAGUGAGCUAUGGGGCGUA | 90 | UACGCCCCAUAGCUCACUGUG |
| | 33 | AAGGAUCUUAUGACCUGCA | 91 | UGCAGGUCAUAAGAUCCUUGC |
| | 34 | AGGAUCUUAUGACCUGCAA | 92 | UUGCAGGUCAUAAGAUCCUUG |
| | 35 | GGAUCUUAUGACCUGCAGA | 93 | UCUGCAGGUCAUAAGAUCCUU |
| | 36 | UUUCUCCUUGGUCUAAGUA | 94 | UACUUAGACCAAGGAGAAACG |
| | 37 | UUCUCCUUGGUCUAAGUGU | 95 | ACACUUAGACCAAGGAGAAAC |

(continued)

| Double strand No. | SEQ ID NO: | Unmodified SS strand (5'-3') | SEQ ID NO: | Unmodified AS strand (5'-3') |
|---|---|---|---|---|
| | 38 | UCUCCUUGGUCUAAGUGUA | 96 | UACACUUAGACCAAGGAGAAA |
| | 39 | UGGCAUGCACAGUGAGCUA | 97 | UAGCUCACUGUGCAUGCCAUA |
| | 40 | GGCAUGCACAGUGAGCUAU | 98 | AUAGCUCACUGUGCAUGCCAU |
| TJR100286 | 41 | CGUUUCUCCUUGGUCUAAG | 99 | CUUAGACCAAGGAGAAACG |
| TJR100290 | 42 | GUUUCUCCUUGGUCUAAGU | 100 | ACUUAGACCAAGGAGAAACGG |
| TJR100291 | 43 | UUUCUCCUUGGUCUAAGUG | 101 | CACUUAGACCAAGGAGAAACG |
| TJR100292 | 44 | AGCCGUUUCUCCUUGGUCUAA | 102 | UUAGACCAAGGAGAAACGGCU |
| TJR100293 | 45 | GCCGUUUCUCCUUGGUCUAAG | 103 | CUUAGACCAAGGAGAAACGGC |
| TJR100295 | 46 | GUUUCUCCUUGGUCUAAGU | 104 | ACUUAGACCAAGGAGAAAC |
| TJR100302 | 47 | CAUGCACAGUGAGCUAUGC | 105 | GCAUAGCUCACUGUGCAUGCC |
| TJR100303 | 48 | GGCAUGCACAGUGAGCUAU | 106 | AUAGCUCACUGUGCAUGCC |
| TJR100304 | 49 | UAUGGCAUGCACAGUGAGA | 107 | UCUCACUGUGCAUGCCAUA |
| TJR100305 | 50 | ACAGUGAGCUAUGGGGCGU | 108 | ACGCCCCAUAGCUCACUGU |
| TJR100306 | 51 | AUGGCAUGCACAGUGAGCUAU | 109 | AUAGCUCACUGUGCAUGCCAUAU |
| TJR100307 | 52 | UAUGGCAUGCACAGUGAGC | 110 | GCUCACUGUGCAUGCCAUAUU |
| TJR100308 | 53 | AUGGCAUGCACAGUGAGCU | 111 | AGCUCACUGUGCAUGCCAUAU |
| TJR100309 | 54 | UGGCAUGCACAGUGAGCUA | 112 | UAGCUCACUGUGCAUGCCAUA |
| TJR100310 | 55 | GGCAUGCACAGUGAGCUAU | 113 | AUAGCUCACUGUGCAUGCCAU |
| TJR100311 | 56 | GCAUGCACAGUGAGCUAUG | 114 | CAUAGCUCACUGUGCAUGCCA |
| TJR100312 | 57 | AUGCACAGUGAGCUAUGGG | 115 | CCCAUAGCUCACUGUGCAUGC |
| TJR100313 | 58 | UGCACAGUGAGCUAUGGGG | 116 | CCCCAUAGCUCACUGUGCAUG |
| TJR100321-1 | 59 | UGCACAGUGAGCUAUGGGG | 117 | CCCCAUAGCUCACUGUGCAUG |
| TJR100322 | 60 | GGUGCUGCAAGGAUCUUAG | 118 | CUAAGAUCCUUGCAGCACCAG |
| TJR100323 | 61 | GGUGCUGCAAGGAUCUUAG | 119 | GUAAGAUCCUUGCAGCACCAG |
| TJR100324 | 62 | UGCUGCAAGGAUCUUAUGA | 120 | UCAUAAGAUCCUUGCAGCA |
| TJR100325 | 63 | UGGUGCUGCAAGGAUCUUA | 121 | UAAGAUCCUUGCAGCACCAGU |
| TJR100326 | 64 | CUGGUGCUGCAAGGAUCUU | 122 | AAGAUCCUUGCAGCACCAGUU |
| TJR100327 | 65 | GUGCUGCAAGGAUCUUAUG | 123 | CAUAAGAUCCUUGCAGCACCA |
| TJR100328 | 66 | UGCUGCAAGGAUCUUAUGA | 124 | UCAUAAGAUCCUUGCAGCACC |
| TRD0321-1 | 67 | GCAUUUUUUUUGAGCUUGA | 125 | UCAAGCUCAAAAAAAAUGCUG |
| TJR100193-1 | 68 | GCCGUUUCUCCUUGGUCUAAU | 126 | AUUAGACCAAGGAGAAACGGC |
| TJR100192-1 | 69 | GCCGUUUCUCCUUGGUCUAAC | 127 | GUUAGACCAAGGAGAAACGGC |
| TJR100189-1 | 70 | AGCCGUUUCUCCUUGGUCUAA | 128 | UUAGACCAAGGAGAAACGGCUUU |
| TJR100167-1 | 71 | GUUUCUCCUUGGUCUAAGU | 129 | ACUUAGACCAAGGAGAAAC |
| TJR100162-1 | 72 | GCAUUUUUUUUGAGCUUGA | 130 | UCAAGCUCAAAAAAAAUGCUG |
| TJR100161-1 | 73 | GCAUUUUUUUUGAGCUUGA | 131 | UCAAGCUCAAAAAAAAUGCUG |
| TJR100160-1 | 74 | GCAUUUUUUUUGAGCUUGAAG | 132 | UCAAGCUCAAAAAAAAUGCUG |

(continued)

| Double strand No. | SEQ ID NO: | Unmodified SS strand (5'-3') | SEQ ID NO: | Unmodified AS strand (5'-3') |
|---|---|---|---|---|
| TJR100191-1 | 75 | GCCGUUUCUCCUUGGUCUAAG | 133 | CUUAGACCAAGGAGAAACGGC |
| TJR100190-1 | 76 | AGCCGUUUCUCCUUGGUCUAA | 134 | UUAGACCAAGGAGAAACGGCU |

Table 26. Modified sense and antisense strands

| Double strand No. | SEQ ID NO: | SS strand (5'-3') | SEQ ID NO: | AS strand (5'-3') |
|---|---|---|---|---|
| TRD007716 | 135 | CmsAmsUmGmCfAmCfAfGfUmG mAmGmCmUmAmUmGmAm | 189 | UmsCfsAmUmAmGfCmUmCmAmCmU mGmUfGmCfAmUmGmsCmsCm |
| TRD007770 | 136 | CmsGmsUmUmUfCmUfCfCfUmU mGmGmUmCmUmAmAmAm | 190 | UmsUfsUmAmGmAfCmCmAmAmGmG mAmGfAmAfAmCmGmsGmsCm |
| TRD007920 | 137 | CmsGmsUmUmUfCmUfCfCfUmU mGmGmUmCmUmAmAmsAm | 191 | UmsUfsUmAfGmAf(-)hmpNA(C)CmAm AfGmGfAmGfAmAfAmCfGmsGmsCm |
| TRD007925 | 138 | CmsAmsUmGmCfAmCfAfGfUmG mAmGmCmUmAmUmGmsAm | 192 | UmsCfsAmUfAmGf(-)hmpNA(C)UmCm AfCmUfGmUfGmCfAmUfGmsCmsCm |
| TJR100072 | 139 | CmsAmsUmGmCmAmCfAfGfUm GmAmGmCmUmAmUmGmAm | 193 | UmsCfsAmUfAmGfCmUmCmAfCmUfG mUfGmCfAmUfGmsCmsCm |
| TJR100073 | 140 | CmsAmsUmGmCmAmCfAfGfUm GmAmGmCmUmAmUmGmAm | 194 | UmsCfsAmUfAmGf(-)hmpNA(C)UmCm AfCmUfGmUfGmCfAmUfGmsCmsCm |
| TJR100075 | 141 | GmsGmsUmGmCmUmGfCfAfAm GmGm AmUmCmUmUmAm Um | 195 | AmsUfsAmAfGmAf(-)hmpNA(U)CmCm UfUmGfCmAfGmCfAmCfCmsAmsGm |
| TJR100076 | 142 | GmsCmsAmGmCmCmGfUfUfUm CmUm CmCmUmUmGmGm Um | 196 | AmsCfsCmAfAmGfGmAmGmAfAmAfC mGfGmCfUmGfCmsUmsUm |
| TRD008096-1 | 143 | CmsGmsUmUmUmCmUfCfCfUm UmGmGmUmCmUmAmAmAm | 197 | UmsUfsUmAfGmAfCmCmAmAfGmGfA mGfAmAfAmCfGmsGmsCm |
| | 144 | CmsGmsUmUmUmCmUfCfCfUm UmGmGmUmCmUmAmAmAm | 198 | UmsUfsUmAfGmAf(-)hmpNA(C)CmAm AfGmGfAmGfAmAfAmCfGmsGmsCm |
| | 145 | CmsGmsUmUmUfCmUfCfCfUmU mGmGmUmCmUmAmAmAm | 199 | UmsUfsUmAfGmAf(-)hmpNA(C)CmAm AfGmGfAmGfAmAfAmCfGmsGmsCm |
| | 146 | CmsAmsUmGmCmAmCfAfGfUm GmAmGmCmUmAmUmGmAm | 200 | UmsCfsAmUfAmGfCmUmCmAfCmUfG mUfGmCfAmUfGmsCmsCm |
| | 147 | CmsAmsUmGmCmAmCfAfGfUm GmAmGmCmUmAmUmGmAm | 201 | UmsCfsAmUfAmGf(-)hmpNA(C)UmCm AfCmUfGmUfGmCfAmUfGmsCmsCm |
| | 148 | CmsGmsUmUmUmCmUfCfCfUm UmGmGmUmCmUmAmAmAm | 202 | UmsUfsUmAfGmAfCmCmAmAfGmGfA mGfAmAfAmCfGmsGmsCm |

(continued)

| Double strand No. | SEQ ID NO: | SS strand (5'-3') | SEQ ID NO: | AS strand (5'-3') |
|---|---|---|---|---|
| | 149 | CmsGmsUmUmUmCmUfCfCfUm UmGmGmUmCmUmAmAmAm | 203 | UmsUfsUmAfGmAf(-)hmpNA(C)CmAm AfGmGfAmGfAmAfAmCfGmsGmsCm |
| | 150 | GmsGmsUmGmCmUmGfCfAfAm GmGmAmUmCmUmUmAmUm | 204 | AmsUfsAmAfGmAf(-)hmpNA(U)CmCm UfUmGfCmAfGmCfAmCfCmsAmsGm |
| | 151 | GmsGmsUmGmCmUmGfCfAfAm GmGmAmUmCmUmUmAmUm | 205 | AmsUfsAmAfGmAfUmCmCmUfUmGfC mAfGmCfAmCfCmsAmsGm |
| | 152 | CmsAmsUmGmCmAmCfAfGfUm GmAmGmCmUmAmUmGmAm | 206 | UmsCfsAmUfAmGfCmUmCmAfCmUfG mUfGmCfAmUfGmsCmsCm |
| | 153 | CmsGmsUmUmUmCmUfCfCfUm UmGmGmUmCmUmAmAmAm | 207 | UmsUfsUmAfGmAfCmCmAmAfGmGfA mGfAmAfAmCfGmsGmsCm |
| | 154 | CmsAmsUmGmCmAmCfAfGfUm GmAmGmCmUmAmUmGmAm | 208 | UmsCfsAmUfAmGfCmUmCmAfCmUfG mUfGmCfAmUfGmsCmsCm |
| | 155 | CmsAmsUmGmCmAmCfAfGfUm GmAmGmCmUmAmUmGmAm | 209 | UmsCfsAmUfAmGf(-)hmpNA(C)UmCm AfCmUfGmUfGmCfAmUfGmsCmsCm |
| | 156 | GmsGmsUmGmCmUmGfCfAfAm GmGmAmUmCmUmUmAmUm | 210 | AmsUfsAmAfGmAf(-)hmpNA(U)CmCm UfUmGfCmAfGmCfAmCfCmsAmsGm |
| | 157 | UmsCmsAmAmCfUmGfGfAfUmG mAmAmGmAmAmAmCmUm | 211 | AmsGfsUmUfUmCf(-)hmpNA(U)UmCm AfUmCfCmAfGmUfUmGfAmsGmsGm |
| | 158 | CmsCmsAmCmAfAmUfGfAfGmA mGmUmAmCmCmUmGmUm | 212 | AmsCfsAmGfGmUf(-)hmpNA(A)CmUm CfUmCfAmUfUmGfUmGfGmsAmsUm |
| | 159 | CmsGmsAmCmCfAmGfCfUfUmG mUmUmUmGmUmGmAmAm | 213 | UmsUfsCmAfCmAf(-)hmpNA(A)AmCm AfAmGfCmUfGmGfUmCfGmsGmsUm |
| | 160 | UmsCmsAmAmCmUmGfGfAfUm GmAmAmGmAmAmAmCmUm | 214 | AmsGfsUmUfUmCf(-)hmpNA(U)UmCm AfUmCfCmAfGmUfUmGfAmsGmsGm |
| | 161 | CmsCmsAmCmAmAmUfGfAfGm AmGmUmAmCmCmUmGmUm | 215 | AmsCfsAmGfGmUf(-)hmpNA(A)CmUm CfUmCfAmUfUmGfUmGfGmsAmsUm |
| | 162 | CmsGmsAmCmCmAmGfCfUfUm GmUmUmUmGmUmGmAmAm | 216 | UmsUfsCmAfCmAf(-)hmpNA(A)AmCm AfAmGfCmUfGmGfUmCfGmsGmsUm |

(continued)

| Double strand No. | SEQ ID NO: | SS strand (5'-3') | SEQ ID NO: | AS strand (5'-3') |
|---|---|---|---|---|
| TRD008096 | 163 | CmsGmsUmUmUmCmUfCfCfUmUmGmGmUmCmUmAmAmAm-NAG0052 | 217 | UmsUfsUmAfGmAf(-)hmpNA(C)CmAmAfGmGfAmGfAmAfAmCfGmsGmsCm |
| TRD008029 | 164 | CmsGmsUmUmUfCmUfCfCfUmUmGmGmUmCmUmAmAmAm-NAG0052 | 218 | UmsUfsUmAfGmAf(-)hmpNA(C)CmAmAfGmGfAmGfAmAfAmCfGmsGmsCm |
| TJR100104 | 165 | CmsAmsUmGmCmAmCfAfGfUmGmAmGmCmUmAmUmGmAm-L96 | 219 | UmsCfsAmUfAmGfCmUmCmAfCmUfGmUfGmCfAmUfGmsCmsCm |
| TJR100103 | 166 | CmsAmsUmGmCmAmCfAfGfUmGmAmGmCmUmAmUmGmAm-L96 | 220 | UmsCfsAmUfAmGf(-)hmpNA(C)UmCmAfCmUfGmUfGmCfAmUfGmsCmsCm |
| TJR100102 | 167 | CmsGmsUmUmUmCmUfCfCfUmUmGmGmUmCmUmAmAmAm-L96 | 221 | UmsUfsUmAfGmAfCmCmAmAfGmGfAmGfAmAfAmCfGmsGmsCm |
| TJR100101 | 168 | CmsGmsUmUmUmCmUfCfCfUmUmGmGmUmCmUmAmAmAm-L96 | 222 | UmsUfsUmAfGmAf(-)hmpNA(C)CmAmAfGmGfAmGfAmAfAmCfGmsGmsCm |
| TJR100100 | 169 | GmsGmsUmGmCmUmGfCfAfAmGmGmAmUmCmUmUmAmUm-L96 | 223 | AmsUfsAmAfGmAf(-)hmpNA(U)CmCmUfUmGfCmAfGmCfAmCfCmsAmsGm |
| TJR100099 | 170 | GmsGmsUmGmCmUmGfCfAfAmGmGmAmUmCmUmUmAmUm-L96 | 224 | AmsUfsAmAfGmAfUmCmCmUfUmGfCmAfGmCfAmCfCmsAmsGm |
| TJR100072 | 171 | CmsAmsUmGmCmAmCfAfGfUmGmAmGmCmUmAmUmGmAm | 225 | UmsCfsAmUfAmGfCmUmCmAfCmUfGmUfGmCfAmUfGmsCmsCm |
| TJR100053 | 172 | CmsGmsUmUmUmCmUfCfCfUmUmGmGmUmCmUmAmAmAm-NAG0052 | 226 | UmsUfsUmAfGmAfCmCmAmAfGmGfAmGfAmAfAmCfGmsGmsCm |

(continued)

| Double strand No. | SEQ ID NO: | SS strand (5'-3') | SEQ ID NO: | AS strand (5'-3') |
|---|---|---|---|---|
| TJR100024 | 173 | CmsAmsUmGmCmAmCfAfGfUmGmAmGmCmUmAmUmGmAm-NAG0052 | 227 | UmsCfsAmUfAmGfCmUmCmAfCmUfGmUfGmCfAmUfGmsCmsCm |
| TJR100023 | 174 | CmsAmsUmGmCmAmCfAfGfUmGmAmGmCmUmAmUmGmAm-NAG0052 | 228 | UmsCfsAmUfAmGf(-)hmpNA(C)UmCmAfCmUfGmUfGmCfAmUfGmsCmsCm |
| TJR100019 | 175 | GmsGmsUmGmCmUmGfCfAfAmGmGmAmUmCmUmUmAmUm-NAG0052 | 229 | AmsUfsAmAfGmAf(-)hmpNA(U)CmCmUfUmGfCmAfGmCfAmCfCmsAmsGm |
| TJR100296 | 176 | GmsGmsUmGmCmUmGfCfAfAmGmGmAmUmCmUmUmAmUm-NAG0052 | 230 | AmsUfsAmAfGmAfUmCmCmUfUmGfCmAfGmCfAmCfCmsAmsGm |
| TRD008028 | 177 | UmsCmsAmAmCfUmGfGfAfUmGmAmAmGmAmAmAmCmUm-**NAG0052** | 231 | AmsGfsUmUfUmCf(-)hmpNA(U)UmCmAfUmCfCmAfGmUfUmGfAmsGmsGm |
| TRD008030 | 178 | CmsCmsAmCmAfAmUfGfAfGmAmGmUmAmCmCmUmGmUm-**NAG0052** | 232 | AmsCfsAmGfGmUf(-)hmpNA(A)CmUmCfUmCfAmUfUmGfUmGfGmsAmsUm |
| TRD008031 | 179 | CmsGmsAmCmCfAmGfCfUfUmGmUmUmUmGmUmGmAmAm-**NAG0052** | 233 | UmsUfsCmAfCmAf(-)hmpNA(A)AmCmAfAmGfCmUfGmGfUmCfGmsGmsUm |
| TRD008028-1 | 180 | UmsCmsAmAmCmUmGfGfAfUmGmAmAmGmAmAmAmCmUm-**NAG0052** | 234 | AmsGfsUmUfUmCf(-)hmpNA(U)UmCmAfUmCfCmAfGmUfUmGfAmsGmsGm |
| TRD008030-1 | 181 | CmsCmsAmCmAmAmUfGfAfGmAmGmUmAmCmCmUmGmUm-**NAG0052** | 235 | AmsCfsAmGfGmUf(-)hmpNA(A)CmUmCfUmCfAmUfUmGfUmGfGmsAmsUm |
| TRD008031-1 | 182 | CmsGmsAmCmCmAmGfCfUfUmGmUmUmUmGmUmGmAmAm-**NAG0052** | 236 | UmsUfsCmAfCmAf(-)hmpNA(A)AmCmAfAmGfCmUfGmGfUmCfGmsGmsUm |

(continued)

| Double strand No. | SEQ ID NO: | SS strand (5'-3') | SEQ ID NO: | AS strand (5'-3') |
|---|---|---|---|---|
| | 183 | UmsCmsAmAmCfUmGfGfAfUmGmAmAmGmAmAmAmCmUm-**NAG0052** | 237 | AmsGfsUmUfUmCfUmUmCmAfUmCfCmAfGmUfUmGfAmsGmsGm |
| | 184 | CmsCmsAmCmAfAmUfGfAfGmAmGmUmAmCmCmUmGmUm-**NAG0052** | 238 | AmsCfsAmGfGmUfAmCmUmCfUmCfAmUfUmGfUmGfGmsAmsUm |
| | 185 | CmsGmsAmCmCfAmGfCfUfUmGmUmUmUmGmUmGmAmAm-**NAG0052** | 239 | UmsUfsCmAfCmAfAmAmCmAfAmGfCmUfGmGfUmCfGmsGmsUm |
| | 186 | UmsCmsAmAmCmUmGfGfAfUmGmAmAmGmAmAmAmCmUm-**NAG0052** | 240 | AmsGfsUmUfUmCfUmUmCmAfUmCfCmAfGmUfUmGfAmsGmsGm |
| | 187 | CmsCmsAmCmAmAmUfGfAfGmAmGmUmAmCmCmUmGmUm-**NAG0052** | 241 | AmsCfsAmGfGmUfAmCmUmCfUmCfAmUfUmGfUmGfGmsAmsUm |
| | 188 | CmsGmsAmCmCmAmGfCfUfUmGmUmUmUmGmUmGmAmAm-**NAG0052** | 242 | UmsUfsCmAfCmAfAmAmCmAfAmGfCmUfGmGfUmCfGmsGmsUm |

The structure of NAG0052 is:

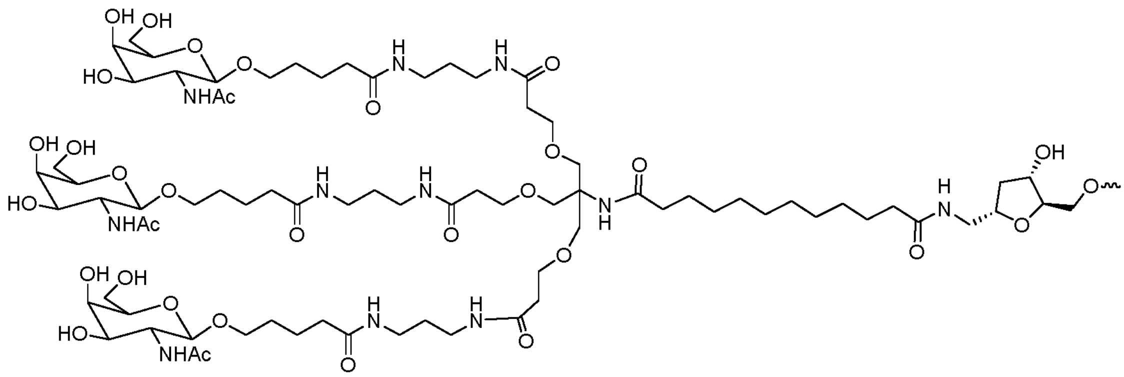

The structure of L96 is:

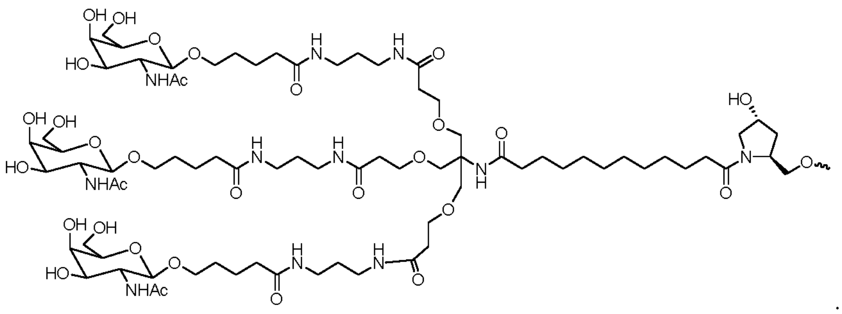

Table 27. Differential optical chemical modifications at position 7 of AS strands

| SEQ ID NO: | AS strand (5'-3') | SEQ ID NO: | AS strand (5'-3') | SEQ ID NO: | AS strand (5'-3') |
|---|---|---|---|---|---|
| 191 | UmsUfsUmAfGmAf(-)hmpNA(C)CmAmAfGmGfAmGfAmAfAmCfGmsGmsCm | 243 | UmsUfsUmAfGmAf(+)hmpNA(C)CmAmAfGmGfAmGfAmAfAmCfGmsGmsCm | 273 | UmsUfsUmAfGmAfhmpNA(C)CmAmAfGmGfAmGfAmAfAmCfGmsGmsCm |
| 192 | UmsCfsAmUfAmGf(-)hmpNA(C)UmCmAfCmUfGmUfGmCfAmUfGmsCmsCm | 244 | UmsCfsAmUfAmGf(+)hmpNA(C)UmCmAfCmUfGmUfGmCfAmUfGmsCmsCm | 274 | UmsCfsAmUfAmGfhmpNA(C)UmCmAfCmUfGmUfGmCfAmUfGmsCmsCm |
| 194 | UmsCfsAmUfAmGf(-)hmpNA(C)UmCmAfCmUfGmUfGmCfAmUfGmsCmsCm | 245 | UmsCfsAmUfAmGf(+)hmpNA(C)UmCmAfCmUfGmUfGmCfAmUfGmsCmsCm | 275 | UmsCfsAmUfAmGfhmpNA(C)UmCmAfCmUfGmUfGmCfAmUfGmsCmsCm |
| 195 | AmsUfsAmAfGmAf(-)hmpNA(U)CmCmUfUmGfCmAfGmCfAmCfCmsAmsGm | 246 | AmsUfsAmAfGmAf(+)hmpNA(U)CmCmUfUmGfCmAfGmCfAmCfCmsAmsGm | 276 | AmsUfsAmAfGmAfhmpNA(U)CmCmUfUmGfCmAfGmCfAmCfCmsAmsGm |
| 198 | UmsUfsUmAfGmAf(-)hmpNA(C)CmAmAfGmGfAmGfAmAfAmCfGmsGmsCm | 247 | UmsUfsUmAfGmAf(+)hmpNA(C)CmAmAfGmGfAmGfAmAfAmCfGmsGmsCm | 277 | UmsUfsUmAfGmAfhmpNA(C)CmAmAfGmGfAmGfAmAfAmCfGmsGmsCm |

(continued)

| SEQ ID NO: | AS strand (5'-3') | SEQ ID NO: | AS strand (5'-3') | SEQ ID NO: | AS strand (5'-3') |
|---|---|---|---|---|---|
| 199 | UmsUfsUmAfGmAf(-)hmpNA(C)CmAmAfGmGfAmGfAmAfAmCfGmsGmsCm | 248 | UmsUfsUmAfGmAf(+)hmpNA(C)CmAmAfGmGfAmGfAmAfAmCfGmsGmsCm | 278 | UmsUfsUmAfGmAfhmpNA(C)CmAmAfGmGfAmGfAmAfAmCfGmsGmsCm |
| 201 | UmsCfsAmUfAmGf(-)hmpNA(C)UmCmAfCmUfGmUfGmCfAmUfGmsCmsCm | 249 | UmsCfsAmUfAmGf(+)hmpNA(C)UmCmAfCmUfGmUfGmCfAmUfGmsCmsCm | 279 | UmsCfsAmUfAmGfhmpNA(C)UmCmAfCmUfGmUfGmCfAmUfGmsCmsCm |
| 203 | UmsUfsUmAfGmAf(-)hmpNA(C)CmAmAfGmGfAmGfAmAfAmCfGmsGmsCm | 250 | UmsUfsUmAfGmAf(+)hmpNA(C)CmAmAfGmGfAmGfAmAfAmCfGmsGmsCm | 280 | UmsUfsUmAfGmAfhmpNA(C)CmAmAfGmGfAmGfAmAfAmCfGmsGmsCm |
| 204 | AmsUfsAmAfGmAf(-)hmpNA(U)CmCmUfUmGfCmAfGmCfAmCfCmsAmsGm | 251 | AmsUfsAmAfGmAf(+)hmpNA(U)CmCmUfUmGfCmAfGmCfAmCfCmsAmsGm | 281 | AmsUfsAmAfGmAfhmpNA(U)CmCmUfUmGfCmAfGmCfAmCfCmsAmsGm |
| 209 | UmsCfsAmUfAmGf(-)hmpNA(C)UmCmAfCmUfGmUfGmCfAmUfGmsCmsCm | 252 | UmsCfsAmUfAmGf(+)hmpNA(C)UmCmAfCmUfGmUfGmCfAmUfGmsCmsCm | 282 | UmsCfsAmUfAmGfhmpNA(C)UmCmAfCmUfGmUfGmCfAmUfGmsCmsCm |
| 210 | AmsUfsAmAfGmAf(-)hmpNA(U)CmCmUfUmGfCmAfGmCfAmCfCmsAmsGm | 253 | AmsUfsAmAfGmAf(+)hmpNA(U)CmCmUfUmGfCmAfGmCfAmCfCmsAmsGm | 283 | AmsUfsAmAfGmAfhmpNA(U)CmCmUfUmGfCmAfGmCfAmCfCmsAmsGm |
| 211 | AmsGfsUmUfUmCf(-)hmpNA(U)UmCmAfUmCfCmAfGmUfUmGfAmsGmsGm | 254 | AmsGfsUmUfUmCf(+)hmpNA(U)UmCmAfUmCfCmAfGmUfUmGfAmsGmsGm | 284 | AmsGfsUmUfUmCfhmpNA(U)UmCmAfUmCfCmAfGmUfUmGfAmsGmsGm |
| 212 | AmsCfsAmGfGmUf(-)hmpNA(A)CmUmCfUmCfAmUfUmGfUmGfGmsAmsUm | 255 | AmsCfsAmGfGmUf(+)hmpNA(A)CmUmCfUmCfAmUfUmGfUmGfGmsAmsUm | 285 | AmsCfsAmGfGmUfhmpNA(A)CmUmCfUmCfAmUfUmGfUmGfGmsAmsUm |
| 213 | UmsUfsCmAfCmAf(-)hmpNA(A)AmCmAfAmGfCmUfGmGfUmCfGmsGmsUm | 256 | UmsUfsCmAfCmAf(+)hmpNA(A)AmCmAfAmGfCmUfGmGfUmCfGmsGmsUm | 286 | UmsUfsCmAfCmAfhmpNA(A)AmCmAfAmGfCmUfGmGfUmCfGmsGmsUm |
| 214 | AmsGfsUmUfUmCf(-)hmpNA(U)UmCmAfUmCfCmAfGmUfUmGfAmsGmsGm | 257 | AmsGfsUmUfUmCf(+)hmpNA(U)UmCmAfUmCfCmAfGmUfUmGfAmsGmsGm | 287 | AmsGfsUmUfUmCfhmpNA(U)UmCmAfUmCfCmAfGmUfUmGfAmsGmsGm |
| 215 | AmsCfsAmGfGmUf(-)hmpNA(A)CmUmCfUmCfAmUfUmGfUmGfGmsAmsUm | 258 | AmsCfsAmGfGmUf(+)hmpNA(A)CmUmCfUmCfAmUfUmGfUmGfGmsAmsUm | 288 | AmsCfsAmGfGmUfhmpNA(A)CmUmCfUmCfAmUfUmGfUmGfGmsAmsUm |

(continued)

| SEQ ID NO: | AS strand (5'-3') | SEQ ID NO: | AS strand (5'-3') | SEQ ID NO: | AS strand (5'-3') |
|---|---|---|---|---|---|
| 216 | UmsUfsCmAfCmAf(-)hmpNA(A)AmCmAfAmGfCmUfGmGfUmCfGmsGmsUm | 259 | UmsUfsCmAfCmAf(+)hmpNA(A)AmCmAfAmGfCmUfGmGfUmCfGmsGmsUm | 289 | UmsUfsCmAfCmAfhmpNA(A)AmCmAfAmGfCmUfGmGfUmCfGmsGmsUm |
| 217 | UmsUfsUmAfGmAf(-)hmpNA(C)CmAmAfGmGfAmGfAmAfAmCfGmsGmsCm | 260 | UmsUfsUmAfGmAf(+)hmpNA(C)CmAmAfGmGfAmGfAmAfAmCfGmsGmsCm | 290 | UmsUfsUmAfGmAfhmpNA(C)CmAmAfGmGfAmGfAmAfAmCfGmsGmsCm |
| 218 | UmsUfsUmAfGmAf(-)hmpNA(C)CmAmAfGmGfAmGfAmAfAmCfGmsGmsCm | 261 | UmsUfsUmAfGmAf(+)hmpNA(C)CmAmAfGmGfAmGfAmAfAmCfGmsGmsCm | 291 | UmsUfsUmAfGmAfhmpNA(C)CmAmAfGmGfAmGfAmAfAmCfGmsGmsCm |
| 220 | UmsCfsAmUfAmGf(-)hmpNA(C)UmCmAfCmUfGmUfGmCfAmUfGmsCmsCm | 262 | UmsCfsAmUfAmGf(+)hmpNA(C)UmCmAfCmUfGmUfGmCfAmUfGmsCmsCm | 292 | UmsCfsAmUfAmGfhmpNA(C)UmCmAfCmUfGmUfGmCfAmUfGmsCmsCm |
| 222 | UmsUfsUmAfGmAf(-)hmpNA(C)CmAmAfGmGfAmGfAmAfAmCfGmsGmsCm | 263 | UmsUfsUmAfGmAf(+)hmpNA(C)CmAmAfGmGfAmGfAmAfAmCfGmsGmsCm | 293 | UmsUfsUmAfGmAfhmpNA(C)CmAmAfGmGfAmGfAmAfAmCfGmsGmsCm |
| 223 | AmsUfsAmAfGmAf(-)hmpNA(U)CmCmUfUmGfCmAfGmCfAmCfCmsAmsGm | 264 | AmsUfsAmAfGmAf(+)hmpNA(U)CmCmUfUmGfCmAfGmCfAmCfCmsAmsGm | 294 | AmsUfsAmAfGmAfhmpNA(U)CmCmUfUmGfCmAfGmCfAmCfCmsAmsGm |
| 228 | UmsCfsAmUfAmGf(-)hmpNA(C)UmCmAfCmUfGmUfGmCfAmUfGmsCmsCm | 265 | UmsCfsAmUfAmGf(+)hmpNA(C)UmCmAfCmUfGmUfGmCfAmUfGmsCmsCm | 295 | UmsCfsAmUfAmGfhmpNA(C)UmCmAfCmUfGmUfGmCfAmUfGmsCmsCm |
| 229 | AmsUfsAmAfGmAf(-)hmpNA(U)CmCmUfUmGfCmAfGmCfAmCfCmsAmsGm | 266 | AmsUfsAmAfGmAf(+)hmpNA(U)CmCmUfUmGfCmAfGmCfAmCfCmsAmsGm | 296 | AmsUfsAmAfGmAfhmpNA(U)CmCmUfUmGfCmAfGmCfAmCfCmsAmsGm |
| 231 | AmsGfsUmUfUmCf(-)hmpNA(U)UmCmAfUmCfCmAfGmUfUmGfAmsGmsGm | 267 | AmsGfsUmUfUmCf(+)hmpNA(U)UmCmAfUmCfCmAfGmUfUmGfAmsGmsGm | 297 | AmsGfsUmUfUmCfhmpNA(U)UmCmAfUmCfCmAfGmUfUmGfAmsGmsGm |
| 232 | AmsCfsAmGfGmUf(-)hmpNA(A)CmUmCfUmCfAmUfUmGfUmGfGmsAmsUm | 268 | AmsCfsAmGfGmUf(+)hmpNA(A)CmUmCfUmCfAmUfUmGfUmGfGmsAmsUm | 298 | AmsCfsAmGfGmUfhmpNA(A)CmUmCfUmCfAmUfUmGfUmGfGmsAmsUm |
| 233 | UmsUfsCmAfCmAf(-)hmpNA(A)AmCmAfAmGfCmUfGmGfUmCfGmsGmsUm | 269 | UmsUfsCmAfCmAf(+)hmpNA(A)AmCmAfAmGfCmUfGmGfUmCfGmsGmsUm | 299 | UmsUfsCmAfCmAfhmpNA(A)AmCmAfAmGfCmUfGmGfUmCfGmsGmsUm |

(continued)

| SEQ ID NO: | AS strand (5'-3') | SEQ ID NO: | AS strand (5'-3') | SEQ ID NO: | AS strand (5'-3') |
|---|---|---|---|---|---|
| 234 | AmsGfsUmUfUmCf(-)hmpNA(U)UmCmAfUmCfCmAfGmUfUmGfAmsGmsGm | 270 | AmsGfsUmUfUmCf(+)hmpNA(U)UmCmAfUmCfCmAfGmUfUmGfAmsGmsGm | 300 | AmsGfsUmUfUmCfhmpNA(U)UmCmAfUmCfCmAfGmUfUmGfAmsGmsGm |
| 235 | AmsCfsAmGfGmUf(-)hmpNA(A)CmUmCfUmCfAmUfUmGfUmGfGmsAmsUm | 271 | AmsCfsAmGfGmUf(+)hmpNA(A)CmUmCfUmCfAmUfUmGfUmGfGmsAmsUm | 301 | AmsCfsAmGfGmUfhmpNA(A)CmUmCfUmCfAmUfUmGfUmGfGmsAmsUm |
| 236 | UmsUfsCmAfCmAf(-)hmpNA(A)AmCmAfAmGfCmUfGmGfUmCfGmsGmsUm | 272 | UmsUfsCmAfCmAf(+)hmpNA(A)AmCmAfAmGfCmUfGmGfUmCfGmsGmsUm | 302 | UmsUfsCmAfCmAfhmpNA(A)AmCmAfAmGfCmUfGmGfUmCfGmsGmsUm |

Table 28. Modified sense and antisense strands

| Double strand No. | SEQ ID NO: | SS strand (5'-3') | SEQ ID NO: | AS strand (5'-3') |
|---|---|---|---|---|
| TRD007751 | 303 | GmsAmsUmCmUfUmAfUfGfAmCmCmUmGmCmAmGmGmAm | 336 | UmsCfsCmUmGmCfAmGmGmUmCmAmUmAfAmGfAmUmCmsCmsUm |
| TRD007771 | 304 | GmsUmsUmUmCfUmCfCfUfUmGmGmUmCmUmAmAmGmUm | 337 | AmsCfsUmUmAmGfAmCmCmAmAmGmGmAfGmAfAmAmCmsGmsGm |
| TRD007343 | 305 | CmsGmsUmAmUfAmUfAfUfGmGmCmAmUmGmCmAmCmAm | 338 | UmsGfsUmGmCmAfUmGmCmCmAmUmAmUfAmUfAmCmGmsGmsAm |
| TRD007344 | 306 | GmsUmsAmUmAfUmAfUfGfGmCmAmUmGmCmAmCmAmAm | 339 | UmsUfsGmUmGmCfAmUmGmCmCmAmUmAfUmAfUmAmCmsGmsGm |
| TRD007345 | 307 | UmsAmsUmAmUfAmUfGfGfCmAmUmGmCmAmCmAmGmUm | 340 | AmsCfsUmGmUmGfCmAmUmGmCmCmAmUfAmUfAmUmAmsCmsGm |
| TRD007346 | 308 | AmsUmsAmUmAfUmGfGfCfAmUmGmCmAmCmAmGmUmAm | 341 | UmsAfsCmUmGmUfGmCmAmUmGmCmCmAfUmAfUmAmUmsAmsCm |
| TRD007347 | 309 | UmsAmsUmAmUfGmGfCfAfUmGmCmAmCmAmGmUmGmAm | 342 | UmsCfsAmCmUmGfUmGmCmAmUmGmCmCfAmUfAmUmAmsUmsAm |
| TRD007348 | 310 | AmsUmsAmUmGfGmCfAfUfGmCmAmCmAmGmUmGmAmAm | 343 | UmsUfsCmAmCmUfGmUmGmCmAmUmGmCfCmAfUmAmUmsAmsUm |
| TRD007349 | 311 | UmsAmsUmGmGfCmAfUfGfCmAmCmAmGmUmGmAmGmAm | 344 | UmsCfsUmCmAmCfUmGmUmGmCmAmUmGfCmCfAmUmAmsUmsAm |

(continued)

| Double strand No. | SEQ ID NO: | SS strand (5'-3') | SEQ ID NO: | AS strand (5'-3') |
|---|---|---|---|---|
| TRD007350 | 312 | AmsUmsGmGmCfAmUfGfCfAmCmAmGmUmGmAmGmCmUm | 345 | AmsGfsCmUmCmAfCmUmGmUmGmCmAmUfGmCfCmAmUmsAmsUm |
| TRD007351 | 313 | GmsCmsAmUmGfCmAfCfAfGmUmGmAmGmCmUmAmUmAm | 346 | UmsAfsUmAmGmCfUmCmAmCmUmGmUmGfCmAfUmGmCmsCmsAm |
| TRD007352 | 314 | AmsUmsGmCmAfCmAfGfUfGmAmGmCmUmAmUmGmGmAm | 347 | UmsCfsCmAmUmAfGmCmUmCmAmCmUmGfUmGfCmAmUmsGmsCm |
| TRD007353 | 315 | UmsGmsCmAmCfAmGfUfGfAmGmCmUmAmUmGmGmGmAm | 348 | UmsCfsCmCmAmUfAmGmCmUmCmAmCmUfGmUfGmCmAmsUmsGm |
| TRD007354 | 316 | CmsAmsGmUmGfAmGfCfUfAmUmGmGmGmGmCmGmUmAm | 349 | UmsAfsCmGmCmCfCmCmAmUmAmGmCmUfCmAfCmUmGmsUmsGm |
| TRD0269 | 317 | UmsGmsGmCmAfUmGfCfAfCmAmGmUmGmAmGmCmUmAm | 350 | UmsAfsGmCmUmCfAmCmUmGmUmGmCmAfUmGfCmCmAmsUmsAm |
| TRD0270 | 318 | GmsGmsCmAmUfGmCfAfCfAmGmUmGmAmGmCmUmAmUm | 351 | AmsUfsAmGmCmUfCmAmCmUmGmUmGmCfAmUfGmCmCmsAmsUm |
| TRD007566 | 319 | AmsAmsGmGmAfUmCfUfUfAmUmGmAmCmCmUmGmCmAm | 352 | UmsGfsCmAmGmGfUmCmAmUmAmAmGmAfUmCfCmUmUmsGmsCm |
| TRD007567 | 320 | AmsGmsGmAmUfCmUfUfAfUmGmAmCmCmUmGmCmAmAm | 353 | UmsUfsGmCmAmGfGmUmCmAmUmAmAmGfAmUfCmCmUmsUmsGm |
| TRD007568 | 321 | GmsGmsAmUmCfUmUfAfUfGmAmCmCmUmGmCmAmGmAm | 354 | UmsCfsUmGmCmAfGmGmUmCmAmUmAmAfGmAfUmCmCmsUmsUm |
| TRD007657 | 322 | UmsUmsUmCmUfCmCfUfUfGmGmUmCmUmAmAmGmUmAm | 355 | UmsAfsCmUmUmAfGmAmCmCmAmAmGmGfAmGfAmAmAmsCmsGm |
| TRD007658 | 323 | UmsUmsCmUmCfCmUfUfGfGmUmCmUmAmAmGmUmGmUm | 356 | AmsCfsAmCmUmUfAmGmAmCmCmAmAmGfGmAfGmAmAmsAmsCm |
| TRD007659 | 324 | UmsCmsUmCmCfUmUfGfGfUmCmUmAmAmGmUmGmUmAm | 357 | UmsAfsCmAmCmUfUmAmGmAmCmCmAmAfGmGfAmGmAmsAmsAm |
| TRD0321 | 325 | GmsCmsAmUmUfUmUfUfUfUmUmGmAmGmCmUmUmGmAm | 358 | UmsCfsAmAmGmCfUmCmAmAmAmAmAmAfAmAfUmGmCmsUmsGm |

(continued)

| Double strand No. | SEQ ID NO: | SS strand (5'-3') | SEQ ID NO: | AS strand (5'-3') |
|---|---|---|---|---|
| TJR100162 | 326 | GmsCmsAmUmUfUmUfUfUfUmUmGmAmGmCmUmUmGmAm | 359 | UmsCmsAmAmGmCmUfCmAmAmAmAfAmAfAmAmUmGmCmsUmsGm |
| TJR100161 | 327 | GmsCmsAmUmUfUmUfUfUfUmUmGmAmGmCmUmUmsGmsAm | 360 | UmsCmsAmAmGmCmUfCmAmAmAmAfAmAfAmAmUmGmCmsUmsGm |
| TJR100160 | 328 | GmsCmsAmUmUfUmUfUfUfUmUmGmAmGmCmUmUmGmAmsAmsGm | 361 | UmsCmsAmAmGmCmUfCmAmAmAmAfAmAfAmAmUmGmCmsUmsGm |
| TJR100025 | 329 | AmsUmsGmGmCmAmUfGmCfAfCfAmGmUmGmAmGmCmUmAmUm -L96 | 362 | AmsUfsAmGmCmUfCmAfCfUmGmUmGmCfAmUfGmCmCmAmUms AmsUm |
| TJR100189 | 330 | AmsGmsCmCmGmUmUfUfCfUmCmCmUmUmGmGmUmCmUmAmAm-L96 | 363 | UmsUfsAmGfAmCfCmAmAmGfGmAfGmAfAmAfCmGfGmCmUmsUmsUm |
| TJR100190 | 331 | AmsGmsCmCmGmUmUfUfCfUmCmCmUmUmGmGmUmCmUmAmAm-L96 | 364 | UmsUfsAmGfAmCfCmAmAmGfGmAfGmAfAmAfCmGfGmsCmsUm |
| TJR100191 | 332 | GmsCmsCmGmUmUmUfCfUfCmCmUmUmGmGmUmCmUmAmAmGm-L96 | 365 | CmsUfsUmAfGmAfCmCmAmAfGmGfAmGfAmAfAmCfGmsGmsCm |
| TJR100192 | 333 | GmsCmsCmGmUmUmUfCfUfCmCmUmUmGmGmUmCmUmAmAmCm-L96 | 366 | GmsUfsUmAfGmAfCmCmAmAfGmGfAmGfAmAfAmCfGmsGmsCm |
| TJR100193 | 334 | GmsCmsCmGmUmUmUfCfUfCmCmUmUmGmGmUmCmUmAmAmUm-L96 | 367 | AmsUfsUmAfGmAfCmCmAmAfGmGfAmGfAmAfAmCfGmsGmsCm |
| TJR100167 | 335 | GmsUmsUmUmCmUmCfCfUfUmGmGmUmCmUmAmAmGmUm-L96 | 368 | AmsCfsUmUfAmGfAmCmCmAfAmGfGmAfGmAfAmAfCm |

The structure of NAG0052 is:

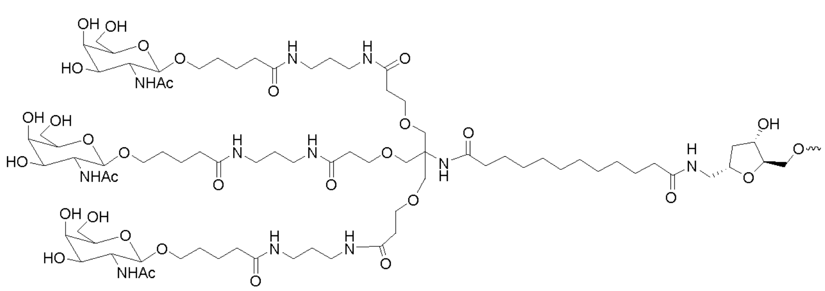

.

**Example 15. Inhibitory Activity Against AGT in Human Hepatoma Cell Line (Hep3B)**

In the human hepatoma cell line (Hep3B), a double-dose experiment was carried out with final duplex concentrations of 10 nM and 0.1 nM, and a single-dose experiment was carried out with a final duplex concentration of 0.1 nM.

The experimental consumables are shown in Table 29.

24 h prior to transfection, Hep3B cells were applied onto a 96-well plate at about 20,000 cells/well, with each well containing 100 μL of medium. The cells were transfected with the samples using Lipofectamine RNAi MAX (ThermoFisher, 13778150) by following the instruction manual of the product, with the final concentrations of the samples for transfection being 10 nM and/or 0.1 nM. 24 h after treatment, the total cellular RNA was extracted using a high-throughput cellular RNA extraction kit (FG0417-L/ FG0418-XL, magnetic bead method), and RNA reverse transcription (Takara PrimeScript™ II 1st Strand cDNA Synthesis Kit (6210A)) and quantitative real-time PCR detection (Takara PrimeScript™ II 1st Strand cDNA Synthesis Kit (6210A)) were carried out. The Taqman probe primers are shown in Table 30. The mRNA level of human AGT was measured and corrected based on the GAPDH internal reference gene level.

After the Taqman probe Q-PCR detection was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene, and is also referred to as $2^{-\triangle\triangle Ct}$, $\triangle\triangle Ct$ = [(target gene of Ct experimental group - internal reference of Ct experimental group) - (target gene of Ct control group - internal reference of Ct control group)]. Inhibition rate (%) = (1 - remaining level of target gene expression) × 100%.

Table 29. Experimental consumables for cell activity screening (nucleic acid extractor)

| Name | Company | Catalog number/model |
|---|---|---|
| Lipofectamine RNAi Max | Invitrogen | 13778-150 |
| Cells-to-CT Bulk Lysis Reagents | Thermo | 4391851C |
| High-throughput cellular RNA extraction kit | FireGen | FG0417 |
| PrimeScript™ II 1st Strand cDNA Synthesis Kit | Takara | 6210B (A × 4) |
| TaqMan™ Fast Advanced Master Mix | Thermo | 4444557 |

Table 30. Taqman probe primers

| Primer name | Sequence |
|---|---|
| hAGT-PF1-MGB | TGTGAGCAGCTGGCAAAGG (SEQ ID NO:425) |
| hAGT-PR1-MGB | GATGTCTTGGCCTGAATTGGA (SEQ ID NO:426) |
| hAGT-P1-MGB | CAATGCCGGGAAGC (SEQ ID NO:427) |
| hGAPDH-PF1-MGB | GACCCCTTCATTGACCTCAACTAC (SEQ ID NO:428) |
| hGAPDH-PR1-MGB | TTGACGGTGCCATGGAATTT (SEQ ID NO:429) |
| hGAPDH-P1-MGB | TTACATGTTCCAATATGATTCC (SEQ ID NO:430) |

The results are expressed relative to the remaining percentage of human AGT mRNA expression in cells treated with the control sample. The inhibition rate $IC_{50}$ results are shown in Table 31.

Table 31. Inhibitory activity against human AGT in the human hepatoma cell line (Hep3B)

| | Remaining target gene mRNA expression level | | | |
|---|---|---|---|---|
| | 10 nM | SD | 0.1 nM | SD |
| TRD007716 | 14.2% | 0.7% | 24.0% | 4.8% |
| TRD007343 | | | 105.4% | 5.7% |
| TRD007344 | | | 102.2% | 6.5% |
| TRD007345 | | | 85.4% | 4.2% |
| TRD007346 | | | 78.1% | 15.9% |
| TRD007347 | | | 101.5% | 14.2% |

(continued)

| | Remaining target gene mRNA expression level | | | |
|---|---|---|---|---|
| | 10 nM | SD | 0.1 nM | SD |
| TRD007348 | | | 106.4% | 1.3% |
| TRD007349 | | | 100.3% | 15.0% |
| TRD007350 | | | 91.5% | 1.3% |
| TRD007351 | | | 99.3% | 2.8% |
| TRD007352 | | | 96.0% | 0.9% |
| TRD0269 | | | 104.5% | 1.2% |
| TRD007353 | | | 90.0% | 15.0% |
| TRD007354 | | | 81.4% | 1.3% |
| TRD0270 | | | 106.7% | 8.0% |
| TRD007770 | 19.0% | 0.9% | 29.9% | 4.5% |
| TRD007657 | | | 100.3% | 8.4% |
| TRD007658 | | | 166.4% | 18.7% |
| TRD007659 | | | 111.7% | 10.4% |
| TRD007751 | 118.0% | 3.4% | 126.8% | 15.4% |
| TRD007566 | | | 103.3% | 1.9% |
| TRD007567 | | | 83.4% | 12.9% |
| TRD007568 | | | 80.7% | 1.9% |

From the data in Table 31, it can be seen that:
At the low concentrations of 0.1 nM, TRD007716 exhibited significantly better activity in inhibiting the proliferation of AGT than TRD007343, TRD007344, TRD007345, TRD007346, TRD007347, TRD007348, TRD007349, TRD007350, TRD007351, TRD007352, TRD007353, TRD007354, TRD0269, and TRD0270. The parent sequence of the 14 sequences was obtained by shifting the parent sequence of TRD007716, i.e., TJR100299, by n bases to the left or right, and the modifications were the same.

At the low concentrations of 0.1 nM, TRD007770 exhibited significantly better activity in inhibiting the proliferation of AGT than TRD007657, TRD007658, and TRD007659. The parent sequence of the 3 sequences was obtained by shifting the parent sequence of TRD007770, i.e., TJR100283, by n bases to the left or right, and the modifications were the same.

The above results show that the sequences derived from TJR100299 and TJR100283 per se have superior expression-inhibiting activity against AGT.

**Example 16. Inhibition of AGT in Human Hepatoma Cell Line (Hep3B) - Multi-Concentration-Point Inhibitory Activity**

A dose response experiment was carried out in the human hepatoma cell line (Hep3B) using 7 concentration gradients.

24 h prior to transfection, Hep3B cells were applied onto a 96-well plate at about 20,000 cells/well, with each well containing 100 μL of medium. The cells were transfected with the samples using Lipofectamine RNAi MAX (ThermoFisher, 13778150) by following the instruction manual of the product, with the final concentrations of the samples for transfection being 10, 2, 0.4, 0.08, 0.016, 0.0032, and 0.00064 nM. 24 h after treatment, the total cellular RNA was extracted using a high-throughput cellular RNA extraction kit, and RNA reverse transcription and quantitative real-time PCR detection were carried out. The human mRNA level was measured, and the mRNA level of human AGT was corrected based on the GAPDH internal reference gene level.

The results are expressed relative to the remaining percentage of human AGT mRNA expression in cells treated with the control sample. The inhibition rate $IC_{50}$ results are shown in Table 32. Reference was made to the experimental

procedure in Example 15.

Table 32. Inhibitory activity against human AGT in Hep3B cells

| Sample No. | Remaining percentage of target gene mRNA (Hep3B) expression (mean) | | | | | | | Hep3B $IC_{50}$ (nM) |
|---|---|---|---|---|---|---|---|---|
| | 10 | 2.0 | 0.4 | 0.08 | 0.016 | 0.0032 | 0.00064 | |
| TRD007716 | 19.12% | 14.22% | 12.95% | 35.90% | 77.55% | 103.03% | 113.12% | 0.0406 |
| TRD007770 | 16.5% | 8.9% | 14.0% | 31.1% | 67.6% | 96.4% | 113.7% | 0.0302 |
| TRD007920 | 16.1% | 16.0% | 22.2% | 45.8% | 90.2% | 113.8% | 118.5% | 0.0668 |
| TRD007925 | 23.4% | 32.2% | 40.1% | 73.2% | 97.9% | 111.1% | 116.4% | 0.2455 |
| TRD008029 | 8.80% | 17.19% | 15.74% | 28.34% | 79.22% | 105.53% | 103.22% | 0.0363 |
| TJR100023 | 12.18% | 22.03% | 47.90% | 70.13% | 106.02% | 115.36% | 107.98% | 0.2911 |
| TJR100024 | 12.25% | 18.66% | 31.11% | 46.67% | 97.55% | 96.31% | 103.30% | 0.0784 |
| TJR100025 | 17.38% | 36.16% | 68.71% | 95.80% | 110.63% | 104.47% | 104.63% | 0.9097 |
| TJR100189 | 20.85% | 27.64% | 46.21% | 75.05% | 83.00% | 95.33% | 100.00% | 0.3311 |
| TJR100190 | 19.74% | 28.81% | 50.88% | 77.82% | 99.64% | 94.97% | 100.00% | 0.3890 |
| TJR100191 | 24.71% | 54.04% | 81.89% | 88.12% | 79.19% | 100.03% | 100.00% | 3.1046 |
| TJR100192 | 19.67% | 67.60% | 100.26% | 112.63% | 113.00% | 112.45% | 100.00% | 3.2359 |
| TJR100193 | 13.04% | 36.98% | 79.89% | 107.30% | 116.21% | 123.19% | 100.00% | 1.1588 |
| TJR100101 | 11.75% | 15.93% | 22.13% | 36.53% | 62.79% | 88.16% | 100.00% | 0.0344 |
| TJR100102 | 12.73% | 16.91% | 23.44% | 35.16% | 67.80% | 94.77% | 100.00% | 0.0372 |
| TJR100104 | 10.96% | 17.53% | 22.87% | 40.82% | 72.71% | 96.48% | 100.00% | 0.0505 |
| TJR100167 | 15.21% | 26.24% | 35.54% | 66.47% | 105.99% | 110.78% | 100.00% | 0.1603 |
| TJR100283 | 7.0% | 12.1% | 14.8% | 24.5% | 48.4% | 105.3% | 104.9% | 0.0163 |
| TJR100286 | 8.7% | 12.5% | 15.5% | 25.5% | 58.4% | 110.6% | 109.0% | 0.0214 |
| TJR100290 | 10.8% | 18.2% | 23.1% | 35.0% | 61.7% | 92.3% | 115.0% | 0.0325 |
| TJR100291 | 41.9% | 57.1% | 70.4% | 102.3% | 119.6% | 125.9% | 122.5% | 2.7102 |
| TJR100292 | 11.5% | 15.8% | 19.0% | 31.3% | 62.8% | 96.9% | 105.1% | 0.0286 |
| TJR100293 | 10.1% | 16.3% | 24.2% | 40.2% | 68.9% | 92.9% | 109.9% | 0.0447 |
| TJR100299 | 13.4% | 13.7% | 18.6% | 31.6% | 57.8% | 85.7% | 94.3% | 0.0263 |
| TJR100302 | 14.2% | 16.2% | 23.6% | 39.4% | 71.5% | 102.3% | 97.8% | 0.0447 |
| TJR100303 | 16.5% | 20.0% | 33.7% | 56.3% | 87.6% | 118.2% | 104.2% | 0.1081 |
| TJR100304 | 44.9% | 55.3% | 69.3% | 94.7% | 106.0% | 119.0% | 105.5% | 3.0200 |
| TJR100305 | 79.2% | 88.1% | 93.8% | 106.8% | 104.5% | 110.0% | 105.9% | >10 |
| TJR100306 | 21.4% | 30.4% | 42.8% | 65.8% | 87.2% | 102.7% | 103.0% | 0.2344 |
| TJR100307 | 108.6% | 123.8% | 115.2% | 111.5% | 111.4% | 107.8% | 104.9% | >10 |
| TJR100308 | 21.7% | 25.1% | 34.0% | 61.7% | 88.4% | 106.7% | 105.9% | 0.1413 |
| TJR100309 | 21.9% | 24.5% | 33.2% | 57.0% | 89.1% | 103.7% | 104.5% | 0.1380 |
| TJR100310 | 22.9% | 23.8% | 30.3% | 59.6% | 80.6% | 102.1% | 101.8% | 0.1122 |
| TJR100311 | 14.6% | 19.2% | 27.5% | 47.7% | 73.1% | 90.0% | 93.4% | 0.0692 |
| TJR100312 | 64.5% | 85.3% | 92.8% | 95.8% | 93.2% | 97.2% | 98.9% | >10 |

(continued)

| Sample No. | Remaining percentage of target gene mRNA (Hep3B) expression (mean) | | | | | | | Hep3B $IC_{50}$ (nM) |
|---|---|---|---|---|---|---|---|---|
| | 10 | 2.0 | 0.4 | 0.08 | 0.016 | 0.0032 | 0.00064 | |
| TJR100313 | 32.9% | 38.8% | 57.1% | 76.9% | 90.0% | 93.5% | 94.3% | 0.6607 |
| TJR100320 | 30.8% | 36.5% | 49.8% | 75.4% | 93.3% | 88.3% | 85.8% | 0.3784 |
| TJR100322 | 32.0% | 42.3% | 61.4% | 87.6% | 108.1% | 119.1% | 104.1% | 0.7943 |
| TJR100323 | 97.2% | 108.2% | 116.5% | 129.4% | 127.2% | 131.0% | 118.4% | >10 |
| TJR100324 | 48.0% | 62.2% | 76.3% | 100.6% | 115.2% | 117.2% | 109.7% | >10 |
| TJR100325 | 69.3% | 78.5% | 93.1% | 108.9% | 110.0% | 124.1% | 105.8% | >10 |
| TJR100326 | 50.2% | 62.8% | 85.5% | 99.3% | 112.9% | 107.9% | 100.7% | >10 |
| TJR100327 | 78.8% | 86.6% | 93.9% | 106.2% | 108.2% | 104.8% | 88.9% | >10 |
| TJR100328 | 54.4% | 74.0% | 89.7% | 97.0% | 110.4% | 108.7% | 88.3% | >10 |

From the data in Table 32, it can be seen that:
The inhibitory activity of TRD007716, TRD007925, TJR100023, TJR100024, and TJR100104 against AGT is better than that of TJR100025; the inhibitory activity of TRD008029, TRD007920, TRD007770, TJR100101, and TJR100102 against AGT is better than that of TJR100189, TJR100190, TJR100191, TJR100192, TJR100193, and TJR100167. The results indicate that the sequences with various modifications derived from the TJR100283 and TJR100299 parent sequences exhibited better inhibitory activity than the control sequence.

The inhibitory activity of TJR100283 against AGT is better than that of TJR100286, TJR100290, TJR100291, TJR100292, and TJR100293; the inhibitory activity of TJR100299 against AGT is significantly better than that of TJR100302, TJR100303, TJR100304, TJR100305, TJR100306, TJR100307, TJR100308, TJR100309, TJR100310, TJR100311, TJR100312, and TJR100313; the inhibitory activity of TJR100320 against AGT is significantly better than that of TJR100322, TJR100323, TJR100324, TJR100325, TJR100326, TJR100327, and TJR100328. The sequences of these samples were derived from the three sequences TJR100283, TJR100299, and TJR100320 by mutating the first base at the 5' end, shifting bases to the left or right, and the like. The results indicate that the 3 sequences per se, namely TJR100283, TJR100299, and TJR100320, exhibited better inhibitory activity against AGT, and that all the base changes weakened the inhibitory activity against AGT.

**Example 17. psiCHECK Validation of On-Target Levels**

The samples were subjected to an *in vitro* molecular-level simulation of off-target level screening in HEK 293A cells using 11 concentration gradients. The experimental results are shown in Table 34.

Corresponding on-target sequences were constructed for the sample sequences. That is, on-target plasmids (GSCM) of the sample target sequences were constructed and inserted into psiCHECK plasmids. The plasmids contained the renilla luciferase gene and the firefly luciferase gene. The plasmids were dual reporter gene systems. The activity of the samples for the target sequence was reflected by the measurements of renilla luciferase expression corrected with firefly luciferase. The measurements used Dual-Luciferase Reporter Assay System (Promega, E2940).

HEK 293A cells were cultured at 37 °C with 5% $CO_2$ in a DMEM high glucose medium containing 10% fetal bovine serum. 24 h prior to transfection, the HEK 293A cells were inoculated onto a 96-well plate at a density of 8000 cells per well, with each well containing 100 μL of medium.

The cells were co-transfected with the samples and the corresponding plasmids using Lipofectamine2000 (ThermoFisher, 11668019) according to the instructions. 0.2 μL of Lipofectamine2000 was used for each well. The transfection amount of plasmid was 10 ng per well. For the off-target sequence plasmids, a total of 11 concentration points were set up for the samples, with the highest concentration point final concentration being 20 nM. A 3-fold serial dilution was performed, resulting in 20 nM, 6.667 nM, 2.222 nM, 0.741 nM, 0.247 nM, 0.082 nM, 0.0274 nM, 0.0091 nM, 0.0030 nM, 0.001 nM, and 0.0003 nM. A multi-concentration-point dilution protocol for the samples is shown in Table 33. 24 h after transfection, the off-target levels were determined using Dual-Luciferase Reporter Assay System (Promega, E2940). Calculation of a relative value:

Ratio = Ren/Fir; calculation of the inhibition rate: 1- (Ratio + siRNA/reporter gene only) × 100% = inhibition rate (%).

Table 33. Multi-concentration dilution protocol

| siRNA concentration (nM) | Final concentration (nM) | Addition of water and siRNA |
|---|---|---|
| 20000 | 20 | |
| 20 | 6.667 | 4μL siRNA + 36μL $H_2O$ |
| 6.667 | 2.222 | 10μL siRNA + 20μL $H_2O$ |
| 2.222 | 0.741 | 10μL siRNA + 20μL $H_2O$ |
| 0.741 | 0.247 | 10μL siRNA + 20μL $H_2O$ |
| 0.247 | 0.082 | 10μL siRNA + 20μL $H_2O$ |
| 0.082 | 0.0274 | 10μL siRNA + 20μL $H_2O$ |
| 0.0274 | 0.0091 | 10μL siRNA + 20μL $H_2O$ |
| 0.0091 | 0.0030 | 10μL siRNA + 20μL $H_2O$ |
| 0.0030 | 0.0010 | 10μL siRNA + 20μL $H_2O$ |
| 0.0010 | 0.0003 | 10μL siRNA + 20μL $H_2O$ |

Table 34. The screening results for the psiCHECK on-target activity - 11-concentration-point inhibitory activity

| No. | Remaining percentage of target gene mRNA (GSCM) expression (mean) | | | | | |
|---|---|---|---|---|---|---|
| | 20 nM | 6.667 nM | 2.222 nM | 0.741 nM | 0.247 nM | 0.082 nM |
| TJR100019 | 81.6% | 52.7% | 36.3% | 34.1% | 46.6% | 69.3% |
| TJR100024 | 16.6% | 14.9% | 15.0% | 15.5% | 22.3% | 32.7% |
| TJR100025 | 26.6% | 24.1% | 25.4% | 26.8% | 32.7% | 62.7% |
| TJR100072 | 13.9% | 12.2% | 12.9% | 12.9% | 17.6% | 25.1% |
| TRD008029 | 29.5% | 20.0% | 17.6% | 17.5% | 17.3% | 25.3% |
| TJR100053 | 14.5% | 13.2% | 15.9% | 15.5% | 14.8% | 20.6% |
| TRD008096-1 | 21.7% | 18.4% | 14.0% | 14.7% | 14.1% | 19.2% |
| TJR100103 | 14.7% | 16.6% | 13.0% | 14.1% | 23.8% | 31.6% |
| TJR100104 | 14.8% | 13.4% | 13.1% | 14.6% | 18.4% | 29.0% |
| TJR100101 | 19.3% | 18.8% | 18.4% | 18.1% | 18.3% | 24.2% |
| TJR100102 | 16.0% | 12.9% | 16.6% | 15.9% | 15.5% | 19.5% |
| TJR100160 | 21.5% | 14.1% | 14.4% | 20.3% | 41.1% | 68.4% |
| TJR100161 | 18.1% | 13.2% | 11.7% | 15.3% | 26.8% | 48.1% |
| TJR100162 | 22.5% | 13.9% | 11.6% | 15.4% | 27.6% | 50.9% |
| TRD0321 | 19.8% | 9.1% | 8.1% | 12.7% | 25.4% | 48.0% |
| No. | Remaining percentage of target gene mRNA expression (mean) | | | | | GSCM IC50 (nM) |
| | 0.027 nM | 0.009 nM | 0.003 nM | 0.001 nM | 0.0003 nM | |
| TJR100019 | 100.3% | 117.2% | 127.7% | 135.3% | 120.6% | 0.1932 |
| TJR100024 | 52.8% | 84.1% | 111.0% | 104.5% | 87.9% | 0.0344 |

(continued)

| No. | Remaining percentage of target gene mRNA expression (mean) | | | | | GSCM IC50 (nM) |
|---|---|---|---|---|---|---|
| | 0.027 nM | 0.009 nM | 0.003 nM | 0.001 nM | 0.0003 nM | |
| TJR100025 | 91.6% | 111.4% | 124.8% | 126.8% | 104.0% | 0.1132 |
| TJR100072 | 42.8% | 63.6% | 89.1% | 101.8% | 75.8% | 0.0195 |
| TRD008029 | 39.5% | 80.7% | 105.0% | 102.9% | 90.6% | 0.0201 |
| TJR100053 | 39.1% | 66.7% | 89.7% | 100.2% | 96.5% | 0.0171 |
| TRD008096-1 | 29.6% | 50.6% | 77.0% | 99.0% | 84.1% | 0.0096 |
| TJR100103 | 51.5% | 84.3% | 107.3% | 100.0% | | 0.0314 |
| TJR100104 | 45.1% | 76.8% | 93.3% | 99.0% | 102.3% | 0.0244 |
| TJR100101 | 42.2% | 71.6% | 87.6% | 93.3% | 94.0% | 0.0195 |
| TJR100102 | 34.9% | 62.5% | 86.5% | 94.2% | 94.6% | 0.0141 |
| TJR100160 | 83.1% | 93.3% | 96.7% | 98.0% | 92.6% | 0.1660 |
| TJR100161 | 72.2% | 87.9% | 89.0% | 84.0% | 87.4% | 0.0748 |
| TJR100162 | 71.1% | 91.6% | 86.6% | 94.3% | 90.5% | 0.0794 |
| TRD0321 | 72.5% | 94.8% | 97.6% | 106.9% | 100.4% | 0.0703 |

It can be seen from the data that:
The inhibiting activity of TRD008029, TJR100053, TJR100101, TJR100102, and TRD008096-1 against AGT is better than that of TJR100160, TJR100161, TJR100162, and TRD0321. This indicates that the various dsRNAs or siRNA conjugates derived from the TJR100283 parent sequence exhibited better inhibitory activity than the control sequence.
The inhibitory activity of TJR100024, TJR100072, TJR100103, and TJR100104 against AGT is better than that of TJR100025. This indicates that the various dsRNAs or siRNA conjugates derived from the TJR100299 parent sequence exhibited better inhibitory activity.

**Example 18. psiCHECK Validation of Off-Target Levels of AS and SS Strands**

An *in vitro* molecular-level simulation of off-target level screening was performed in HEK 293A cells using 11 concentration gradients. The experimental results are shown in Table 35.
The rules for constructing corresponding target plasmids are as follows:
The design of off-target sequences for the AS strand of each sample. That is, off-target plasmids (GSSM) that are completely complementary to the 5' end positions 1-8 of the antisense strands and the bases of which at the other positions do not match at all were constructed. The corresponding rules for base mismatches were that A pairs with C and G pairs with T. To improve detection sensitivity, GSSM-5hits off-target plasmids were constructed, where 5 identical GSSM sequences were connected by TTCC and inserted into psiCHECK plasmids; the plasmids contained the renilla luciferase gene and the firefly luciferase gene. The plasmids were dual reporter gene systems. The target sequence was inserted into the 3' UTR region of the renilla luciferase gene.
For the sense strands, off-target plasmids (PSCM) that are completely complementary to the SS strands were constructed. Off target plasmids (PSSM) that are completely complementary to positions 1-8 of the 5' end of the sense strands and the bases of which at the other positions do not match at all were constructed. The corresponding rules for base mismatches were that A pairs with C and G pairs with T. To improve detection sensitivity, PSSM-5hits off-target plasmids were constructed, where 5 identical PSSM sequences were connected by TTCC.
The activity for the target sequence was reflected by the measurements of renilla luciferase expression corrected with firefly luciferase. The measurements used Dual-Luciferase Reporter Assay System (Promega, E2940). For the rest of the experimental procedure, see Example 17.

Table 35. The screening results for the psiCHECK off-target activity of the sense and antisense strands

| No. | IC50 (nM) | | |
|---|---|---|---|
| | AS strand seed region off-target activity | SS strand full-length off-target activity | SS strand seed region off-target activity |
| TJR100100 | >20 | >20 | >20 |
| TJR100101 | >20 | >20 | >20 |
| TJR100103 | >20 | >20 | >20 |
| TRD007920 | >20 | >20 | >20 |

The results show that the samples listed involve no or minimal off-target risk.

**Example 19. Inhibition of AGT in Human Hepatoma Cell Line (Huh7) - Multi-Concentration-Point Inhibitory Activity**

The siRNAs were subjected to a dose response experiment in the human hepatoma cell line (Huh7) using 7 concentration gradients.

24 h prior to transfection, Huh7 cells were applied onto a 96-well plate at about 20,000 cells/well, with each well containing 100 μL of medium. The cells were transfected with the samples using Lipofectamine RNAiMAX (ThermoFisher, 13778150) by following the instruction manual of the product, with the final concentrations of the samples for transfection being 10, 2, 0.4, 0.08, 0.016, 0.0032, and 0.00064 nM. 24 h after treatment, the total cellular RNA was extracted using a high-throughput cellular RNA extraction kit, and RNA reverse transcription and quantitative real-time PCR detection were carried out. The human mRNA level was measured, and the mRNA level of human AGT was corrected based on the GAPDH internal reference gene level.

The results are expressed relative to the remaining percentage of human AGT mRNA expression in cells treated with the control sample. The inhibition rate $IC_{50}$ results are shown in Table 36. Reference was made to the experimental procedure in Example 12.

Table 36. 7-concentration inhibitory activity against human AGT in Huh7 cells

| Sample No. | Remaining percentage of target gene mRNA (Huh7) expression (mean) | | | | | | | Huh7 $IC_{50}$ (nM) |
|---|---|---|---|---|---|---|---|---|
| | 10 | 2.0 | 0.4 | 0.08 | 0.016 | 0.0032 | 0.00064 | |
| TRD007920 | 9.34% | 11.58% | 23.53% | 53.89% | 84.07% | 98.77% | 98.43% | 0.1122 |
| TRD008096 | 15.4% | 14.1% | 28.6% | 60.2% | 88.2% | 103.6% | 100.0% | 0.1204 |
| TJR100053 | 14.8% | 11.9% | 20.5% | 44.9% | 79.8% | 93.8% | 100.0% | 0.0615 |
| TJR100189 | 18.9% | 32.3% | 57.3% | 81.0% | 100.6% | 97.5% | 100.0% | 0.5960 |
| TJR100190 | 18.3% | 37.2% | 59.5% | 101.4% | 106.4% | 101.2% | 100.0% | 0.6430 |
| TJR100191 | 24.6% | 57.0% | 83.5% | 96.1% | 88.1% | 94.4% | 100.0% | 2.7100 |
| TJR100192 | 15.3% | 63.8% | 86.7% | 104.5% | 106.5% | 99.0% | 100.0% | 3.1770 |
| TJR100193 | 13.1% | 41.7% | 78.9% | 102.8% | 111.3% | 109.6% | 100.0% | 1.3590 |

In Huh7, the inhibitory activity of the three sequences TRD007920, TRD008096, and TJR100053 against AGT mRNA is better than that of TJR100189, TJR100190, TJR100191, TJR100192, and TJR100193.

**Example 20. Inhibitory Activity Against AGT in Primary Human Hepatocytes (PHHs)**

Dose response experiments were carried out in primary human hepatocytes (PHHs) using 8 concentration gradients and 7 concentration gradients.

The cells were transfected with the samples using Lipofectamine RNAiMAX (ThermoFisher, 13778150) by following the instruction manual of the product, with the final concentrations of the samples for transfection being 20, 6.67, 2.22 0.74, 0.25, 0.082, 0.027, and 0.009 nM. For another group, the gradient concentrations used were: 10, 2,

0.4, 0.08, 0.016, 0.0032, and 0.00064 nM. Equal volumes of RNAiMAX and sample diluted with Opti-MEM I were mixed. The mixture was incubated at room temperature for 15 min and then added to a 96-well plate at 10 μL/well, and a suspension of thawed frozen PHHs (6.7E5 cells/mL) was then added at 90 μL/well. Cells were harvested 24 h after transfection and the total cellular RNA was extracted using the RNeasy 96 Kit (Qiagen-74182). A random primer was added by following the instructions in the reverse transcription kit (Tiangen, KR106) to reverse-transcribe the RNA into a cDNA. The mRNA levels of AGT in the samples were measured by qPCR, and the mRNA levels of human AGT were corrected based on the GAPDH internal reference gene level.

The expression level of the target AGT mRNA for each sample was calculated by the ΔΔCt relative quantitation method. The relative expression level of the target gene is represented by $2^{-\Delta\Delta CT}$, and the calculation formula is as follows:

$$\Delta CT = \text{mean Ct value of target gene - mean Ct value of internal reference gene;}$$

$$\Delta\Delta CT = \Delta CT \text{ (drug adding group) group - } \Delta CT \text{ (control group);}$$

Relative expression level of target gene mRNA = $2^{-\Delta\Delta CT}$

$IC_{50}$ was calculated using GraphPad Prism software (four parameter logistic equations). The results are shown in Table 37.

Table 37. Inhibitory activity against AGT in PHHs

| Sample No. | Inhibition rate of target gene mRNA (PHH) expression (mean) | | | | | | | | PHH $IC_{50}$ (nM) |
|---|---|---|---|---|---|---|---|---|---|
| | 20 nM | 6.667 nM | 2.222 nM | 0.741 nM | 0.247 nM | 0.082 nM | 0.027 nM | 0.009 nM | |
| TRD007716 | 69.9% | 69.7% | 62.9% | 62.7% | 51.3% | 38.1% | 25.9% | 14.4% | 0.0652 |
| Sample No. | Inhibition rate of target gene mRNA (PHH) expression (mean) | | | | | | | | PHH $IC_{50}$ (nM) |
| | 10 nM | 2.0 nM | 0.4 nM | 0.08 nM | 0.016 nM | 0.0032 nM | 0.00064 nM | | |
| TJR100099 | 19.1% | 24.2% | 32.0% | 57.8% | 83.0% | 101.1% | 111.1% | | 0.1137 |
| TJR100100 | 19.4% | 19.8% | 37.3% | 71.6% | 91.0% | 98.7% | 91.5% | | 0.2100 |
| TJR100101 | 20.3% | 21.5% | 32.9% | 56.8% | 81.2% | 98.9% | 103.8% | | 0.1108 |
| TJR100102 | 17.4% | 20.5% | 25.4% | 43.9% | 68.1% | 92.0% | 91.4% | | 0.0525 |
| TJR100103 | 13.5% | 14.9% | 29.6% | 54.5% | 86.7% | 93.1% | 95.0% | | 0.1080 |
| TJR100104 | 12.8% | 10.7% | 12.8% | 20.1% | 51.7% | 83.4% | 100.0% | | 0.0154 |

All the sequences exhibited good inhibitory activity against AGT mRNA in PHHs.

**Example 21. Inhibition of AGT in Primary Monkey Hepatocytes (PCHs) - Multi-Concentration-Point Inhibitory Activity**

The samples were subjected to a dose response experiment in primary monkey hepatocytes (PCHs) using 7 concentration gradients.

The cells were transfected with the samples using Lipofectamine RNAiMAX (ThermoFisher, 13778150) by following the instruction manual of the product, with the final concentrations of the samples for transfection being 10, 2, 0.4, 0.08, 0.016, 0.0032, and 0.00064 nM. Equal volumes of RNAiMAX and sample diluted with Opti-MEM I were mixed. The mixture was incubated at room temperature for 15 min and then added to a 96-well plate at 10 μL/well, and a suspension of thawed frozen PCHs (6.7E5 cells/mL) was then added at 90 μL/well. Cells were harvested 24 h after transfection and the total cellular RNA was extracted using the RNeasy 96 Kit (Qiagen-74182). A random primer was added by following the instructions in the reverse transcription kit (Tiangen, KR106) to reverse-transcribe the RNA into a cDNA. The mRNA levels of AGT in the samples were measured by qPCR, and the mRNA levels of monkey AGT were corrected based on the GAPDH internal reference gene level. The probe primer sequences are shown in Table 38.

The expression level of the target AGT mRNA for each sample was calculated by the ΔΔCt relative quantitation

method. The relative expression level of the target gene is represented by $2^{-\Delta\Delta CT}$, and the calculation formula is as follows:

$$\Delta CT = \text{mean Ct value of target gene - mean Ct value of internal reference gene;}$$

$$\Delta\Delta CT = \Delta CT \text{ (drug adding group) group - } \Delta CT \text{ (control group);}$$

Relative expression level of target gene mRNA = $2^{-\Delta\Delta CT}$

$IC_{50}$ was calculated using GraphPad Prism software (four parameter logistic equations). The results are shown in Table 39.

Table 38. Taqman probe primers

| Primer name | Sequence |
|---|---|
| mkAGT-PF1-MGB | AAAGCAGTCGTTTCTCCTTGGT(SEQ ID NO:419) |
| mkAGT-PR1-MGB | CCTGGGAGGCGCATTTG(SEQ ID NO:420) |
| mkAGT-P1-MGB | CTGCGTGAGCAGTAAG(SEQ ID NO:421) |
| mkGAPDH-PF1-MGB | AGTCAGCCGCATTTTCTCTTG(SEQ ID NO:422) |
| mkGAPDH-PR1-MGB | AAATCCGTTGACTCCGACCTT(SEQ ID NO:423) |
| mkGAPDH-P1-MGB | ATCGCCAGCGCATC(SEQ ID NO:424) |

Table 39. 7-concentration inhibitory activity against monkey AGT in PCHs

| Sample No. | Remaining percentage of target gene mRNA (PCH) expression (mean) | | | | | | | PCH $IC_{50}$ (nM) |
|---|---|---|---|---|---|---|---|---|
| | 10 | 2.0 | 0.4 | 0.08 | 0.016 | 0.0032 | 0.00064 | |
| TJR100099 | 8.8% | 20.3% | 31.3% | 55.6% | 102.0% | 106.5% | 109.4% | 0.1166 |
| TJR100104 | 7.6% | 18.7% | 27.8% | 37.2% | 63.5% | 86.9% | 100.0% | 0.0404 |
| TJR100167 | 19.6% | 22.4% | 31.5% | 46.0% | 75.0% | 96.8% | 102.4% | 0.0668 |
| TJR100101 | 4.7% | 16.2% | 21.4% | 32.6% | 51.2% | 91.6% | 93.1% | 0.0243 |
| TJR100102 | 4.2% | 18.7% | 25.8% | 34.0% | 49.7% | 86.1% | 113.1% | 0.0229 |
| TRD008096 | 6.4% | 22.1% | 29.2% | 39.7% | 64.3% | 109.8% | 112.7% | 0.0437 |
| TJR100053 | 3.8% | 17.5% | 24.3% | 32.8% | 46.4% | 84.6% | 99.1% | 0.0214 |
| TJR100283 | 18.2% | 20.4% | 22.0% | 25.6% | 33.8% | 61.5% | 90.6% | 0.0060 |
| TJR100292 | 17.9% | 22.4% | 26.6% | 34.7% | 51.5% | 82.9% | 105.8% | 0.0209 |
| TJR100293 | 19.7% | 27.9% | 43.8% | 67.7% | 104.0% | 117.3% | 111.5% | 0.2213 |
| TJR100295 | 17.9% | 24.5% | 27.0% | 31.1% | 45.9% | 75.1% | 99.2% | 0.0135 |

The first 7 sequences exhibited excellent inhibitory activity against AGT in PCHs, and the activity of TJR100283 is significantly better than that of TJR100292, TJR100293, and TJR100295. This indicates that the sequence TJR100283 per se or its conjugates or dsRNAs exhibited very strong inhibitory activity against AGT.

## Claims

**1.** An siRNA , comprising a sense strand and an antisense strand forming a double-stranded region, wherein

the sense strand comprises at least 15 contiguous nucleotides and differs by no more than 3 nucleotides from

any one of the nucleotide sequences of SEQ ID NOs: 1, 2, 3, 4, 5 and 6;
the antisense strand comprises at least 15 contiguous nucleotides and differs by no more than 3 nucleotides from any one of the nucleotide sequences of SEQ ID NOs: 7, 8, 9, 10, 11 and 12;
the siRNA targets angiotensinogen.

**2.** The siRNA according to claim 1, wherein

the sense strand comprises at least 17 contiguous nucleotides of a nucleotide sequence selected from any one of SEQ ID NO: 1 to SEQ ID NO: 6;
the antisense strand comprises at least 19 contiguous nucleotides of a nucleotide sequence selected from any one of SEQ ID NO: 7 to SEQ ID NO: 12;
preferably, the sense strand comprises a nucleotide sequence selected from any one of SEQ ID NO: 1 to SEQ ID NO: 6;
preferably, the antisense strand comprises a nucleotide sequence selected from any one of SEQ ID NO: 7 to SEQ ID NO: 12.

**3.** The siRNA according to claim 1 or 2, comprising or selected from any one of the following groups:

group 1), a sense strand set forth in SEQ ID NO: 1 and an antisense strand set forth in SEQ ID NO: 7;
group 2), a sense strand set forth in SEQ ID NO: 2 and an antisense strand set forth in SEQ ID NO: 8;
group 3), a sense strand set forth in SEQ ID NO: 3 and an antisense strand set forth in SEQ ID NO: 9;
group 4), a sense strand set forth in SEQ ID NO: 4 and an antisense strand set forth in SEQ ID NO: 10;
group 5), a sense strand set forth in SEQ ID NO: 5 and an antisense strand set forth in SEQ ID NO: 11;
group 6), a sense strand set forth in SEQ ID NO: 6 and an antisense strand set forth in SEQ ID NO: 12.

**4.** The siRNA according to any one of the preceding claims, wherein at least one nucleotide in the sense strand and/or the antisense strand is a modified nucleotide.

**5.** The siRNA according to any one of the preceding claims, wherein at least one nucleotide at positions 2 to 8 of the 5' end of the antisense strand is a modified nucleotide, wherein:

- the modified nucleotide is a 2'-methoxy-modified nucleotide, or
- the modified nucleotide comprises a chemical modification of formula (I) or a tautomeric modification thereof, the chemical modification of formula (I) being selected from the group consisting of:

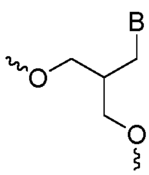 , 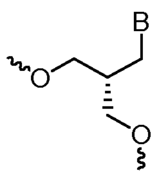 or 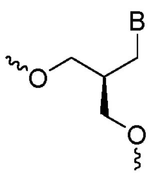 ;

each B is independently selected from the group consisting of bases corresponding to positions 2 to 8 of the 5' end of the antisense strand.

**6.** The siRNA according to any one of the preceding claims, wherein the nucleotide at position 5, 6 or 7 of the 5' end of the antisense strand

- is a 2'-methoxy-modified nucleotide, or
- comprises the chemical modification of formula (I) or the tautomeric modification thereof as defined in claim 5;
when the chemical modification of formula (I) or the tautomeric modification thereof is at position 5 of the 5' end, B is a base at position 5 of the 5' end of the antisense strand;
when the chemical modification of formula (I) or the tautomeric modification thereof is at position 6 of the 5' end, B is a base at position 6 of the 5' end of the antisense strand;
when the chemical modification of formula (I) or the tautomeric modification thereof is at position 7 of the 5' end, B is a base at position 7 of the 5' end of the antisense strand.

**7.** The siRNA according to any one of the preceding claims, wherein three contiguous nucleotides in the sense strand

are 2’-fluoro-modified nucleotides; and/or

in a 5’-end to 3’-end direction, nucleotides at positions 2, 4, 6, 9, 12, 14, 16 and 18 of the antisense strand are each independently a 2’-fluoro-modified nucleotide; or
in a 5’-end to 3’-end direction, nucleotides at positions 2, 4, 6, 10, 12, 14, 16 and 18 of the antisense strand are each independently a 2’-fluoro-modified nucleotide.

**8.** The siRNA according to any one of the preceding claims, wherein the sense strand comprises or is selected from a nucleotide sequence of the formula shown below:

5’-$N_aN_aN_aN_aN_aN_aN_bN_bN_bN_aN_aN_aN_aN_aN_aN_aN_aN_aN_a$-3’; or,
5’-$N_aN_aN_aN_aN_bN_aN_bN_bN_bN_aN_aN_aN_aN_aN_aN_aN_aN_aN_a$-3’;
wherein $N_a$ is a 2’-methoxy-modified nucleotide, and $N_b$ is a 2’-fluoro-modified nucleotide.

**9.** The siRNA according to any one of the preceding claims, wherein the antisense strand comprises or is selected from a nucleotide sequence of the formula shown below:

5’-$N_a'N_b'N_a'N_b'N_a'N_b'W'N_a'N_a'N_b'N_a'N_b'N_a'N_b'N_a'N_b'N_a'N_b'N_a'N_a'N_a'$-3’; or,
5’-$N_aN_b'N_aN_b'N_aN_b'W'N_aN_b'N_aN_aN_b'N_aN_b'N_aN_b'N_aN_b'N_aN_aN_a$-3’;
wherein,
$N_a'$ is a 2’-methoxy-modified nucleotide;
$N_b'$ is a 2’-fluoro-modified nucleotide;
W’ is a 2’-methoxy-modified nucleotide, or W’ is a nucleotide comprising the chemical modification of formula (I) or the tautomeric modification thereof as defined in claim 5.

**10.** The siRNA according to claim 9, wherein W’ is selected from a nucleotide comprising a

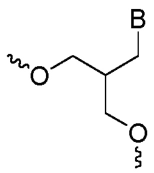 , 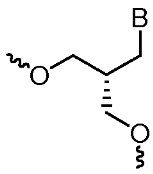 or 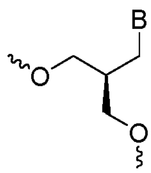

modification; wherein: B is a base; preferably, B is selected from a base corresponding to position 7 of 5’ end of the antisense strand.

**11.** The siRNA according to claim 10, wherein W’ is selected from a 2’-methoxy-modified nucleotide.

**12.** The siRNA according to any one of the preceding claims, wherein

the sense strand comprises or is selected from any one of SEQ ID NO: 135 to SEQ ID NO: 162;
the antisense strand comprises or is selected from any one of SEQ ID NO: 189 to SEQ ID NO: 216.

**13.** A double-stranded ribonucleic acid, comprising:

the siRNA according to any one of claims 1-12, and a targeting ligand linked to the end of the siRNA; wherein preferably, the targeting ligand is linked to the 3’ end of the sense strand of the siRNA.

**14.** The double-stranded ribonucleic acid according to claim 13, wherein:

the targeting ligand comprises at least one targeting moiety, and
the targeting moieties are each independently selected from the group consisting of:

galactose, galactosamine, N-formyl-galactosamine, N-acetyl-galactosamine, N-propionyl-galactosamine, N-n-butyryl-galactosamine and N-isobutyryl-galactosamine;
preferably, the targeting moiety is N-acetyl-galactosamine;
more preferably, the targeting ligand comprises three identical or different targeting moieties.

**15.** The double-stranded ribonucleic acid according to claim 14, wherein the targeting ligand is a compound of formula (II) or a pharmaceutically acceptable salt thereof, wherein formula (II) is:

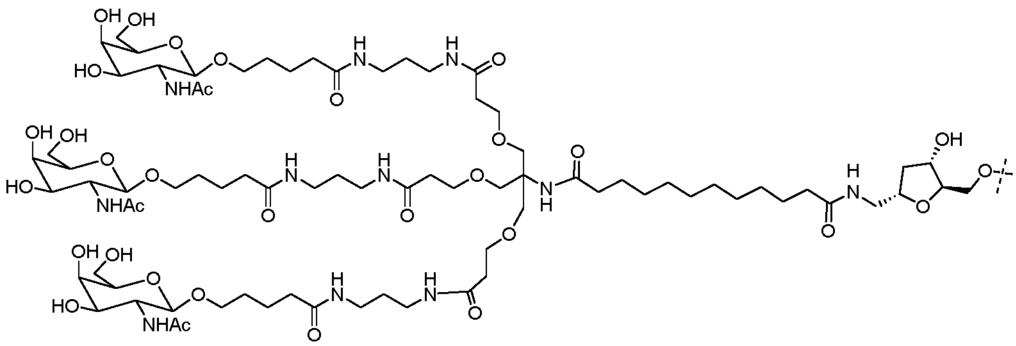

.

**16.** A double-stranded ribonucleic acid, comprising a sense strand and an antisense strand forming a double-stranded region, wherein

the sense strand is selected from any one of SEQ ID NO: 163 to SEQ ID NO: 188;
the antisense strand is selected from any one of SEQ ID NO: 217 to SEQ ID NO: 242;
preferably, the double-stranded ribonucleic acid is selected from the group consisting of TRD008096, TJR100053 or TJR100296.

**17.** A pharmaceutical composition, comprising:

the siRNA according to any one of claims 1-12 or the double-stranded ribonucleic acid according to any one of claims 13-16, and
a pharmaceutically acceptable carrier.

**18.** A method for inhibiting the expression of an angiotensinogen gene, comprising administering to a subject an effective amount or effective dose of:

the siRNA according to any one of claims 1-12, or
the double-stranded ribonucleic acid according to any one of claims 13-16, or
the pharmaceutical composition according to claim 17.

**19.** A method for treating and/or preventing a disease, comprising administering to a subject an effective amount or effective dose of the siRNA according to any one of claims 1-12, or the double-stranded ribonucleic acid according to any one of claims 13-16, or the pharmaceutical composition according to claim 17;
wherein preferably, the disease is hypertension.

**20.** A method for delivering an siRNA or a double-stranded ribonucleic acid to the liver *in vivo,* wherein:

the siRNA or the double-stranded ribonucleic acid inhibits the expression and/or replication of an angiotensinogen gene, and
the method comprises administering to a subject the double-stranded ribonucleic acid according to any one of claims 13-16, or the pharmaceutical composition according to claim 17.

**21.** A method for preparing an siRNA or a double-stranded ribonucleic acid, comprising: synthesizing the siRNA according to any one of claims 1-12 or the double-stranded ribonucleic acid according to any one of claims 13-16.

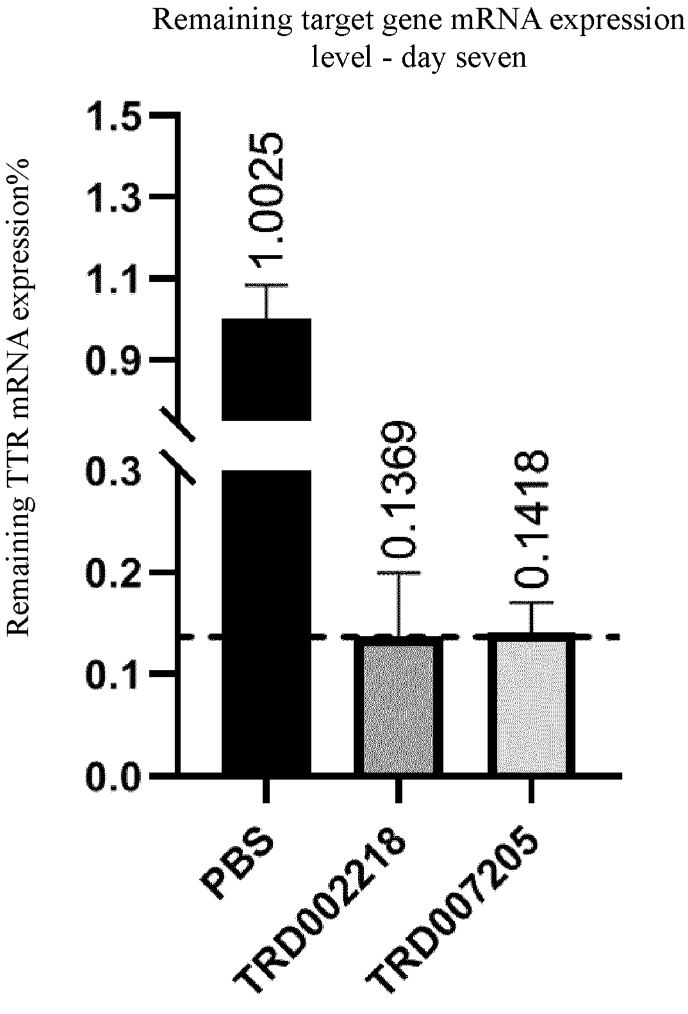
Remaining target gene mRNA expression
level - day seven
Remaining TTR mRNA expression%
1.5
1.3
1.1
0.9
0.3
0.2
0.1
0.0
1.0025
0.1369
0.1418
PBS
TRD002218
TRD007205

FIG. 1

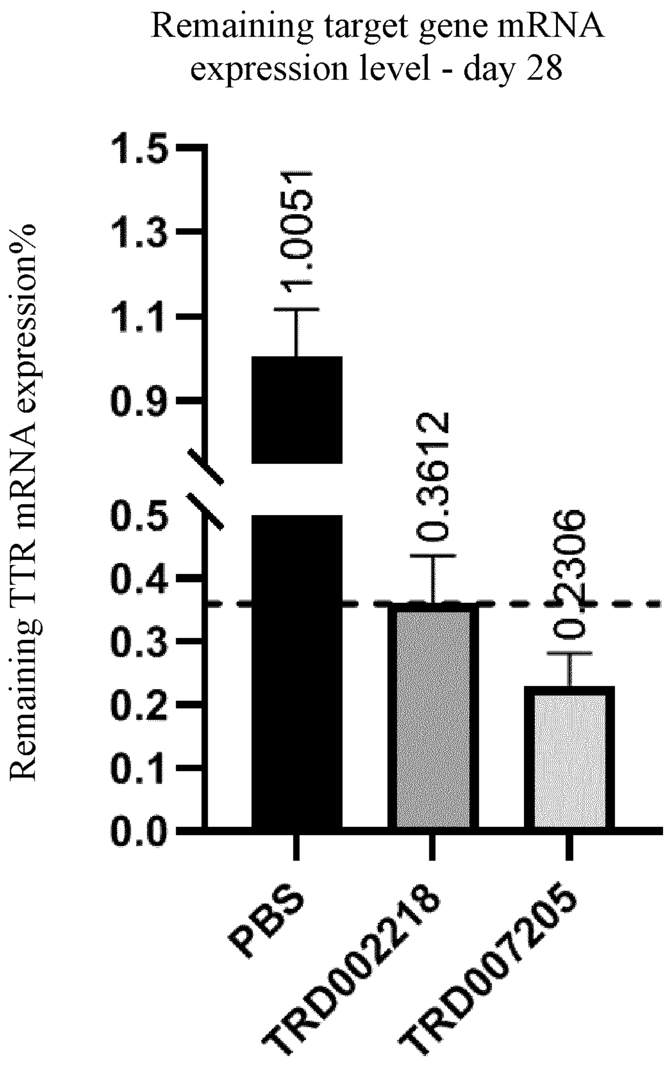
Remaining target gene mRNA
expression level - day 28
Remaining TTR mRNA expression%
1.5
1.3
1.1
0.9
0.5
0.4
0.3
0.2
0.1
0.0
1.0051
0.3612
0.2306
PBS
TRD002218
TRD007205

FIG. 2

## INTERNATIONAL SEARCH REPORT

International application No.
**PCT/CN2022/132883**

**A. CLASSIFICATION OF SUBJECT MATTER**

C12N15/113(2010.01)i;A61K31/713(2006.01)i;A61P9/12(2006.01)i;A61P9/10(2006.01)i;A61P13/12(2006.01)i

According to International Patent Classification (IPC) or to both national classification and IPC

**B. FIELDS SEARCHED**

Minimum documentation searched (classification system followed by classification symbols)

C12N,A61K,A61P

Documentation searched other than minimum documentation to the extent that such documents are included in the fields searched

Electronic data base consulted during the international search (name of data base and, where practicable, search terms used)

CJFD, CNTXT, ENTXT, ENTXTC, STN, PUBMED, SpringerLink, ISI Web of Knowledge, Elsevier Science Direct, Wiley InterScience, Nature, Science, CNKI, 万方, WANFANG, NCBI: SEQ ID NO: 1-242, 血管紧张素原, AGT, ANHU, SERPINA8, NM_001384479, 乙酰基-半乳糖胺, 甲氧基, 氟代, siRNA, dsRNA, 糖基.

**C. DOCUMENTS CONSIDERED TO BE RELEVANT**

| Category* | Citation of document, with indication, where appropriate, of the relevant passages | Relevant to claim No. |
|---|---|---|
| PX | WO 2022159158 A1 (ALNYLAM PHARMACEUTICALS, INC. et al.) 28 July 2022 (2022-07-28)<br>claims 1-57, and description, pp. 94-103, and table 3-table 4 | 1-21 |
| PX | CN 114763547 A (SNCON BIOTECHNOLOGY CO., LTD.) 19 July 2022 (2022-07-19)<br>claims 1-9, and description, paragraphs 21-55, table 1, SEQ ID NO: 409 | 1-21 |
| X | CN 106574268 A (ALNYLAM PHARMACEUTICALS INC.) 19 April 2017 (2017-04-19)<br>claims 1-85, description, paragraphs 55-63, 447, and 812-843, and sequence listing | 1-21 |
| X | CN 112313335 A (ALNYLAM PHARMACEUTICALS INC.) 02 February 2021 (2021-02-02)<br>claims 1-78, abstract, sequence listing | 1-21 |
| X | CN 108271351 A (IONIS PHARMACEUTICALS, INC.) 10 July 2018 (2018-07-10)<br>claims 1-35, and description, pp. 60-114 | 1-21 |
| X | WO 2021096763 A1 (ALNYLAM PHARMACEUTICALS, INC.) 20 May 2021 (2021-05-20)<br>claims 1-94, and description, pp. 75-76 | 1-21 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 February 2023** | **13 February 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

| International application No. |
|---|
| **PCT/CN2022/132883** |

**C. DOCUMENTS CONSIDERED TO BE RELEVANT**

| Category* | Citation of document, with indication, where appropriate, of the relevant passages | Relevant to claim No. |
|---|---|---|
| A | OLEARCZYK, J. et al. "Targeting of hepatic angiotensinogen using chemically modified siRNAs results in significant and sustained blood pressure lowering in a rat model of hypertension" *Hypertension Research,* Vol. 37, 12 December 2013 (2013-12-12), pp. 405-412 | 1-21 |

Form PCT/ISA/210 (second sheet) (July 2022)

**INTERNATIONAL SEARCH REPORT**

International application No.
**PCT/CN2022/132883**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☐ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☑ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2022/132883**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **18-20**
because they relate to subject matter not required to be searched by this Authority, namely:

   Claims 18-20 relate to a method for treating and/or preventing a disease, which belongs to a method for treating a disease as defined in PCT Rule 39.1(iv); however, a search was still conducted on the basis of a pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2022/132883**

| Patent document cited in search report | Publication date (day/month/year) | Patent family member(s) | Publication date (day/month/year) |
|---|---|---|---|
| WO 2022159158 A1 | 28 July 2022 | None | |
| CN 114763547 A | 19 July 2022 | None | |
| CN 106574268 A | 19 April 2017 | SG 11201609376 SA | 29 December 2016 |
| | | WO 2015179724 A1 | 26 November 2015 |
| | | WO 2015179724 A8 | 18 February 2016 |
| | | WO 2015179724 A9 | 10 March 2016 |
| | | JP 2017517511 A | 29 June 2017 |
| | | JP 6811094 B2 | 13 January 2021 |
| | | IL 248816 A0 | 31 January 2017 |
| | | IL 248816 B | 29 July 2021 |
| | | IL 281638 A | 31 May 2021 |
| | | AU 2015264038 A1 | 01 December 2016 |
| | | AU 2015264038 A2 | 22 December 2016 |
| | | AU 2015264038 B2 | 11 February 2021 |
| | | EP 3739048 A1 | 18 November 2020 |
| | | US 2019298842 A1 | 03 October 2019 |
| | | US 10814007 B2 | 27 October 2020 |
| | | AU 2021202552 A1 | 27 May 2021 |
| | | CA 2948381 A1 | 26 November 2015 |
| | | BR 112016026950 A2 | 31 October 2017 |
| | | KR 20170005130 A | 11 January 2017 |
| | | KR 102410687 B1 | 22 June 2022 |
| | | US 2017189541 A1 | 06 July 2017 |
| | | US 10238749 B2 | 26 March 2019 |
| | | JP 2022104985 A | 12 July 2022 |
| | | KR 20220087576 A | 24 June 2022 |
| | | JP 2021063085 A | 22 April 2021 |
| | | JP 7101748 B2 | 15 July 2022 |
| | | SG 10202104570 TA | 29 June 2021 |
| | | EA 201692370 A1 | 31 March 2017 |
| | | US 2021046187 A1 | 18 February 2021 |
| | | US 11419942 B2 | 23 August 2022 |
| | | EP 3146049 A1 | 29 March 2017 |
| | | EP 3146049 B1 | 26 February 2020 |
| | | MX 2016015126 A | 23 February 2017 |
| | | ZA 201607666 B | 30 January 2019 |
| CN 112313335 A | 02 February 2021 | PE 20210114 A1 | 19 January 2021 |
| | | US 2021310006 A1 | 07 October 2021 |
| | | EP 3794122 A1 | 24 March 2021 |
| | | JP 2021522841 A | 02 September 2021 |
| | | IL 278539 B | 01 June 2022 |
| | | IL 292877 A | 01 July 2022 |
| | | CO 2020015314 A2 | 19 March 2021 |
| | | WO 2019222166 A1 | 21 November 2019 |
| | | KR 20210010529 A | 27 January 2021 |
| | | SG 11202011144 QA | 30 December 2020 |
| | | CL 2021002326 A1 | 13 May 2022 |
| | | US 2021095290 A1 | 01 April 2021 |
| | | US 11015201 B2 | 25 May 2021 |
| | | BR 112020022943 A2 | 17 February 2021 |

Form PCT/ISA/210 (patent family annex) (July 2022)

**INTERNATIONAL SEARCH REPORT**
**Information on patent family members**

International application No.
**PCT/CN2022/132883**

| Patent document cited in search report | Publication date (day/month/year) | Patent family member(s) | Publication date (day/month/year) |
|---|---|---|---|
| | | TW 202016304 A | 01 May 2020 |
| | | CA 3100003 A1 | 21 November 2019 |
| | | AU 2019269418 A1 | 07 January 2021 |
| | | CL 2020002865 A1 | 30 April 2021 |
| | | PH 12020551904 A1 | 21 June 2021 |
| CN 108271351 A | 10 July 2018 | HK 1258275 A1 | 08 November 2019 |
| | | CA 2998898 A1 | 13 April 2017 |
| | | JP 2018530325 A | 18 October 2018 |
| | | IL 295971 A | 01 October 2022 |
| | | US 2019160090 A1 | 30 May 2019 |
| | | US 10912792 B2 | 09 February 2021 |
| | | CO 2018002598 A2 | 12 June 2018 |
| | | IL 257989 A | 31 May 2018 |
| | | IL 257989 B | 01 October 2022 |
| | | CL 2018000899 A1 | 07 September 2018 |
| | | MX 2018004307 A | 01 May 2018 |
| | | AU 2022202671 A1 | 19 May 2022 |
| | | BR 112018005223 A2 | 16 October 2018 |
| | | BR 112018005223 B1 | 09 August 2022 |
| | | KR 20180055898 A | 25 May 2018 |
| | | WO 2017062816 A2 | 13 April 2017 |
| | | WO 2017062816 A3 | 08 June 2017 |
| | | WO 2017062816 A8 | 11 January 2018 |
| | | CL 2020001453 A1 | 13 November 2020 |
| | | EP 3960183 A1 | 02 March 2022 |
| | | BR 122020023854 B1 | 29 November 2022 |
| | | EP 3359164 A2 | 15 August 2018 |
| | | EP 3359164 A4 | 12 June 2019 |
| | | SG 10202003148 VA | 28 May 2020 |
| | | PE 20181085 A1 | 05 July 2018 |
| | | US 2022000901 A1 | 06 January 2022 |
| | | ZA 201801600 B | 30 January 2019 |
| | | AU 2016334114 A1 | 12 April 2018 |
| | | AU 2016334114 B2 | 27 January 2022 |
| | | JP 2022008677 A | 13 January 2022 |
| WO 2021096763 A1 | 20 May 2021 | KR 20220115946 A | 19 August 2022 |
| | | TW 202132568 A | 01 September 2021 |
| | | CO 2022006092 A2 | 20 May 2022 |
| | | BR 112022009216 A2 | 02 August 2022 |
| | | AU 2020382478 A1 | 02 June 2022 |
| | | IL 292865 A | 01 July 2022 |
| | | CA 3161703 A1 | 20 May 2021 |
| | | WO 2021096763 A8 | 24 June 2021 |
| | | EP 4058577 A1 | 21 September 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202111398206
- CN 202111542323X
- CN 202210554195
- WO 2021254360 A1
- WO 2014025805 A1
- US 11015201 B